# EUROPEAN PATENT APPLICATION

(11) **EP 4 813 987 A1**
(43) Date of publication of application: **30.09.2026**
(21) Application number: 25166483.5
(22) Date of filing: 26.03.2025
(51) Int. Cl.: C07D 471/04, A61P 25/28, A61K 31/437

(54) **MUTS-BETA INHIBITORS**

(71) Applicant: LoQus23 Therapeutics Limited, Great Abington, Cambridgeshire CB21 6GP (GB)
(72) Inventor: The designation of the inventor has not yet been filed
(74) Representative: HGF

(57) **Abstract**

The present invention relates at least in part to compounds of formula (I) that function as inhibitors of MutSβ. The present invention also relates to processes for the preparation of these compounds, to pharmaceutical compositions comprising them, and to their use in the treatment of diseases or conditions in which MutSβ activity is implicated, such as, for example, repeat expansion diseases or disorders, as well as to increase the efficiency of genome editing.

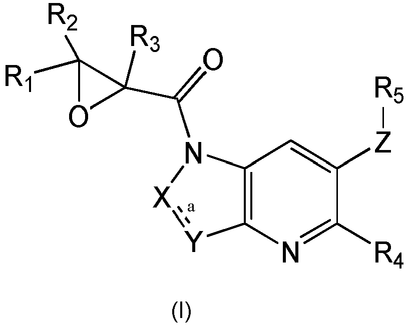

## Description

### FIELD OF THE INVENTION

The present invention relates to certain compounds that function as inhibitors of MutSβ. The present invention also relates to processes for the preparation of these compounds, to pharmaceutical compositions comprising them, and to their use in the treatment of diseases or conditions in which MutSβ activity is implicated, such as, for example, diseases related to somatic expansion of expanded simple nucleotide repeats.

### BACKGROUND OF THE INVENTION

Mismatch repair (MMR) is a highly conserved DNA repair pathway that promotes genome stability in all organisms which recognises and repairs erroneous insertion, deletion or mis-incorporation of nucleotides that can arise during DNA replication, repair or recombination. MMR is thus one of the key pathways that participates in the cellular response to certain types of DNA damage. In addition to DNA damage response roles, MMR is employed during meiosis in eukaryotes and immunoglobulin maturation/diversification in mammals.

MMR predominantly corrects DNA base mismatches and insertion/deletion (indel) loops that can occasionally arise during normal DNA replication processes. Base pair mismatches occur when incorrect nucleotides (for example, in response to oxidative damage) are inserted into the newly synthesised DNA strand and escape the proofreading function of DNA polymerases. Indel loops commonly arise in the context of microsatellites which are highly polymorphic, short tandem repeat DNA sequences distributed throughout genomes. Typically, at microsatellites, especially in the context of longer repeats, the template and primer strands are prone to slippage (dissociation and reannealing) during replication; this can lead to the formation of loop structures and ultimately result in a discordant number of repeat units between the template and newly synthesised strand.

Mammalian MMR can be separated into four steps: (1) mismatch recognition mediated by MutS heterodimers; (2) recruitment of MutL heterodimers which connect the mismatch recognition signal to where the DNA strand scission begins in a strand-specific fashion; (3) excision of the errant, error-containing DNA strand and (4) re-synthesis of the excision gap using the remaining DNA strand as a template.

In humans, mismatch recognition by MutSα (comprised of *MSH2* and *MSH6* gene products) or MutSβ (comprised of *MSH2* and *MSH3* gene products) heterodimers initiate the MMR pathway. These obligate heterodimers differ in their substrate preferences but there are considerable overlaps between the substrates recognised and processed. MutSα recognizes base-base mismatches and some insertion-deletion mismatches with a preference for shorter indels (1-3 nucleotides), whereas MutSβ predominantly processes larger indels of 3+ nucleotides. MutSα also contributes to the recognition of certain types of DNA damage such as oxidised nucleotides and can participate in the checkpoint response to such lesions. Similarly, MutSβ may cooperate with the nucleotide excision repair machinery in the repair of interstrand cross-links. Furthermore, both heterodimers have been implicated in the production of certain genetic variants. MutSα participates in the somatic hypermutation phase of immunoglobulin gene affinity maturation, and MutSβ is required for the triplet repeat expansions that are responsible for a number of neurodegenerative diseases. Additionally, when MMR pathway signalling is abolished (termed deficient MMR or dMMR), this typically results in increased instability at these repetitive regions at multiple genome loci, a process termed microsatellite instability (MSI).

Binding of MutSα or MutSβ heterodimers to the mismatch results in the ATP-dependent recruitment of a MutL heterodimer (either MutLα comprising *MLH1* and *PMS2* gene products, MutLβ comprising *MLH1* and *PMS1* or MutLγ comprising *MLH1* or *MLH3* gene products) to form a ternary complex. This ternary complex forms interactions with additional proteins including proliferating cell nuclear antigen (*PCNA*) and exonuclease 1 (*EXO1*) which enable successful completion of MMR sequelae.

Short tandem repeats (STR) represent one of the most abundant class of variations in human genomes, are polymorphic by nature and become highly unstable in a length dependent fashion. The expansion of repeat length above a threshold is a well-established feature of more than 50 hereditary human disorders mainly affecting the nervous system, with many likely remaining to be identified due to improvements in detection by sequencing-based methods. Coding repeat expansion diseases include but are not limited to brachydactyly and cleidocranial dysplasia (BCCD); blepharophimosis, ptosis and epicanthus inversus (BPES); cleidocranial dysplasia (CCD); congenital central hypoventilation syndrome (CCHS); dentatorubropallidoluysian atrophy (DRPLA); early infantile epileptic encephalopathy type 1 (EIEE1); Huntington's disease (HD); Huntington disease-like 2 (HDL2); hand-foot-genital syndrome (HFGS); holoprosencephaly type 5 (HPE5); mental retardation with isolated growth hormone deficiency (MRGH); oculopharyngeal muscular dystrophy (OPMD); spinobulbar muscular atrophy (SBMA); spinocerebellar ataxias type 1, 2, 3, 6, 7, 17 (SCA1, SCA2, SCA3, SCA6, SCA7, SCA17); synpolydactyly (SPD); X-linked mental retardation and abnormal genitalia (XLAG); X-linked mental retardation with or without growth hormone deficiency (XLMR, XLMRGHD). Non-coding repeat expansion diseases include but are not limited to benign adult familial myoclonic epilepsy (BAFME); Baratela-Scott syndrome (BSS); cerebellar ataxia, neuropathy and vestibular areflexia syndrome (CANVAS); myotonic dystrophy type 1 (DM1); myotonic dystrophy type 2 (DM2); progressive myoclonus epilepsy type 1 or Unverricht-Lundborg diseae (EPM1); familial adult myoclonic epilepsy (FAME); Fuch's endothelial corneal dystrophy type 3 (FECD3); fragile XE syndrome (FRAXE); Friedreich ataxia (FRDA); frontotemporal dementia/amyotrophic lateral sclerosis (FTD/ALS); fragile X-associated premature ovarian infertility (FXPOI); fragile X syndrome (FXS); fragile X-associated tremor ataxia syndrome (FXTAS); global development delay, progressive ataxia and elevated glutamine (GDPAG); neuronal intranuclear inclusion disease (NIID); oculopharyngodistal myopathy type 1 (OPDM1); oculopharyngodistal myopathy type 2 (OPDM2); oculopharyngeal myopathy with leukoencephalopathy type 1 (OPML1), spinocerebellar ataxias types 8, 10, 12, 27B, 31, 36, 37 (SCA8, SCA10, SCA12, SCA27B, SCA31, SCA36, SCA37); X-linked dystonia parkinsonism (XDP).

Repeat expansion diseases can be dominant (e.g. Huntington's disease) or recessive (e.g. Friedreich's ataxia) and have the common feature of inheritance of an expansion of a normally polymorphic repeat in the disease-associated gene above a defined threshold. The number of repeats required to pass this threshold varies for each disease, although exonic repeats generally have lower thresholds than intronic repeats. Coding or exonic repeat diseases generally lead to a toxic gain of function of the resulting protein gene product, whereas non-coding intronic repeats generally lead to reduced expression of the protein gene product. In both cases this leads to dysfunction and death in vulnerable cell types, eventually resulting in clinical signs and symptoms. In addition to inheritance of an expanded allele there is now strong evidence that these repeat expansions are unstable and expand further during the lifespan of the individual. This expansion happens at the individual cell level (i.e. somatic expansion) and the extent of expansion in a given cell type/tissue typically correlates tightly with how vulnerable it is to degeneration during the course of the disease. The faster the expansion in mutation carriers, the greater the impact on disease measures such as age-of-onset, progression and severity.

The term "somatic instability" describes the fact that a repetitive sequence (e.g. a trinucleotide repeat tract such as CAG for huntingtin) is unstable at an individual cell level (i.e. somatic) which leads to a shift in the CAG repeat profiles at the population level. Somatic instability can lead to expansion or contraction of the repeat sequence but is usually expansion-biased in the context of a disease-causing repeat expansion. Huntington's disease is triggered by the inheritance of an expanded CAG repeat sequence in the HTT gene longer than 40 consecutive CAG repeats (though it should be noted that 36-39 consecutive CAG repeats align with incomplete penetrance): these repeats have increased propensity to expand further. The rate of somatic CAG repeat expansion is influenced by the number of consecutive repeats, time and cell-type. The pathogenic repeat threshold in expansion-prone cell types such as striatal projection neurons (also known as medium spiny neurons) is longer than 40 CAG and is currently unknown. However, post-mortem brain tissue data indicate that greater than 150 CAG repeats (via somatic expansion over time) is associated with measurable pathophysiology (profoundly altered gene expression profiles) in the cells bearing these highly expanded repeats. It is now widely accepted that the somatic expansion of a CAG repeat sequence in HTT in brain cells is the first step in the molecular pathogenesis of Huntington's disease, followed by a second process that leads to cell dysfunction once a CAG repeat threshold has been achieved. The rate of somatic CAG repeat expansion is thus the key driver for the age of onset and rate of progression of the disease. Slowing or stopping somatic instability could significantly mitigate disease progression and provide a much sought after disease-modifying therapy.

For at least a subset of these repeat expansion diseases, MMR proteins are modifiers of repeat expansions and clinical profile. Strong genetic and mechanistic data indicate that MutSβ (gene products of *MSH2* and *MSH3*) drives somatic expansion of these repeats. Reduction in MutSβ expression or function, for example by point mutations to ablate its ATPase function, block somatic expansion. Thus, methods of arresting, or reversing, somatic repeat expansions is hypothesised to be an effective therapeutic approach to slow or stop repeat disease progression.

While MMR is essential for maintaining the integrity of the genome, it can also act as a barrier to efficient genome editing.

More recently, multiple labs have shown that reduction or inhibition of MMR significantly enhanced the efficiency of prime editing (PE) while abating the frequency of unintended indels. PE was established in the Liu lab and utilises three components: (i) Cas9 nickase to nick the non-target strand; (ii) prime editing single guide RNA (pegRNA) to bind the target strand and (iii) a reverse transcriptase to reverse transcribe from the 3' end of the non-target strand according to the information provided by the pegRNA. PE is a precise and versatile genome editing technique that enables all types of base conversions and small fragment deletion or insertion in the genome. One challenge with PE is it has a much lower efficiency than other types of Cas9-derived editing approaches because endogenous MMR processes often revert the sequence back to the original sequence. Accordingly, increasing the efficiency of PE, together with reducing indels, through the use of MutSα and/or MutSβ inhibitor treatment *ex vivo* and/or *in vivo* may enhance the potential for clinical translation and therapeutic use of prime editors.

Other genome editing techniques aiming to introduce specific changes in the DNA sequence by providing a template that serves as a repair template could benefit from inhibiting MMR. As MMR can recognize and repair mismatches between the template and the target DNA, leading to the removal or correction of the desired edits, MutSα and/or MutSβ inhibitors could prevent the rejection of the edit and increase editing efficiency. The use of a template is a crucial aspect of certain gene editing techniques, such as homology-directed repair (HDR). HDR relies on the introduction of an exogenous DNA template that serves as a repair template to guide the desired genetic modifications.

Some HDR-based gene editing methods that commonly utilize a template include CRISPR-Cas9, ZFN (Zinc Finger Nucleases) and TALENs (Transcription Activator-Like Effector Nucleases). In these approaches, nucleases introduce double-strand breaks (DSBs) at specific target sites in the genome. These DSBs can then be repaired using an exogenous DNA template as a guide for HDR, enabling precise insertion, deletion, or replacement of genetic material. Nuclease-free editing methods utilising ssODN (single-stranded oligonucledotides) as a template for desired edit were also shown to benefit from lowering activity of MMR factors and has been used to create mouse strains.

Therefore, there is an on-going need for compounds that function as inhibitors of MutSβ.

The present invention was devised with the foregoing in mind.

### SUMMARY OF THE INVENTION

According to a first aspect of the present invention, there is provided a compound, or a pharmaceutically acceptable salt, hydrate or solvate thereof, as defined herein.

According to a further aspect of the present invention, there is provided a pharmaceutical composition comprising a compound as defined herein, or a pharmaceutically acceptable salt, hydrate or solvate thereof, in admixture with a pharmaceutically acceptable diluent or carrier.

According to a further aspect of the present invention, there is provided a method of inhibiting MutSβ activity *in vitro, ex vivo* or *in vivo,* said method comprising contacting a cell with an effective amount of a compound or a pharmaceutically acceptable salt, hydrate or solvate thereof as defined herein. Suitably, the method comprises the selective inhibition of MutSβ activity *in vitro, ex vivo* or *in vivo.*

According to a further aspect of the present invention, there is provided a method of inhibiting somatic expansion of expanded simple nucleotide repeats *in vitro, ex vivo* or *in vivo* (e.g. in treating a repeat expansion disease or disorder), said method comprising contacting a cell with an effective amount of a compound or a pharmaceutically acceptable salt, hydrate or solvate thereof as defined herein. Suitably, the somatic expansion is associated with MutSβ activity.

According to a further aspect of the present invention, there is provided a method of treating a disease or disorder in which MutSβ activity is implicated (e.g. a repeat expansion disease or disorder) in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a method of treating a repeat expansion disease or disorder in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a method of treating a triplet repeat disease or disorder in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a compound, or a pharmaceutically acceptable salt, hydrate or solvate thereof, or a pharmaceutical composition as defined herein, for use in therapy.

According to a further aspect of the present invention, there is provided a compound, or a pharmaceutically acceptable salt, hydrate or solvate thereof, as defined herein for use in the inhibition of MutSβ activity, e.g. in cellular systems. The inhibition may be the selective inhibition of MutSβ activity.

According to a further aspect of the present invention, there is provided a compound, or a pharmaceutically acceptable salt, hydrate or solvate thereof, as defined herein for use in inhibiting somatic expansion of expanded simple nucleotide repeats, e.g. in the treatment of a repeat expansion disease or disorder.

According to a further aspect of the present invention, there is provided a compound, or a pharmaceutically acceptable salt, hydrate or solvate thereof, as defined herein for use in the treatment of a disease or condition in which MutSβ activity is implicated (e.g. a repeat expansion disease or disorder).

According to a further aspect of the present invention, there is provided a compound, or a pharmaceutically acceptable salt, hydrate or solvate thereof, or a pharmaceutical composition as defined herein for use in the treatment of a repeat expansion disease or disorder.

According to a further aspect of the present invention, there is provided a compound, or a pharmaceutically acceptable salt, hydrate or solvate thereof, or a pharmaceutical composition as defined herein for use in the treatment of a triplet repeat disease or disorder.

According to a further aspect of the present invention, there is provided a use of a compound, or a pharmaceutically acceptable salt, hydrate or solvate thereof, as defined herein in the manufacture of a medicament for the inhibition of MutSβ activity, e.g. in cellular systems.

According to a further aspect of the present invention, there is provided a use of a compound, or a pharmaceutically acceptable salt, hydrate or solvate thereof, as defined herein in the manufacture of a medicament for inhibiting somatic expansion of expanded simple nucleotide repeats, e.g. in treating a repeat expansion disease or disorder.

According to a further aspect of the present invention, there is provided a use of a compound, or a pharmaceutically acceptable salt, hydrate or solvate thereof, as defined herein in the manufacture of a medicament for the treatment of a disease or condition in which MutSβ activity is implicated (e.g. a repeat expansion disease or disorder).

According to a further aspect of the present invention, there is provide the use of a compound, or a pharmaceutically acceptable salt, hydrate or solvate thereof, as defined herein in the manufacture of a medicament for the treatment of a repeat expansion disease or disorder.

According to a further aspect of the present invention, there is provide the use of a compound, or a pharmaceutically acceptable salt, hydrate or solvate thereof, as defined herein in the manufacture of a medicament for the treatment of a triplet repeat disease or disorder.

Suitably, the repeat expansion disease or disorder may be selected from benign adult familial myoclonic epilepsy (BAFME); brachydactyly and cleidocranial dysplasia (BCCD); blepharophimosis, ptosis and epicanthus inversus (BPES); Baratela-Scott syndrome (BSS); cerebellar ataxia, neuropathy and vestibular areflexia syndrome (CANVAS); congenital central hypoventilation syndrome (CCHS); dentatorubropallidoluysian atrophy (DRPLA); early infantile epileptic encephalopathy type 1 (EIEE1); familial adult myoclonic epilepsy (FAME); Fuch's endothelial corneal dystrophy type 3 (FECD3); fragile XE syndrome (FRAXE); Friedreich ataxia (FRDA) frontotemporal dementia/amyotrophic lateral sclerosis (FTD/ALS); fragile X-associated premature ovarian infertility (FXPOI); fragile X syndrome (FXS); fragile X-associated tremor ataxia syndrome (FXTAS); global development delay, progressive ataxia and elevated glutamine (GDPAG); Huntington's disease (HD); Huntington disease-like 2 (HDL2); hand-foot-genital syndrome (HFGS); holoprosencephaly type 5 (HPE5); mental retardation with isolated growth hormone deficiency (MRGH); myotonic dystrophy type 1 (DM1); myotonic dystrophy type 2 (DM2); progressive myoclonus epilepsy type 1 or Unverricht-Lundborg diseae (EPM1); neuronal intranuclear inclusion disease (NIID); oculopharyngodistal myopathy type 1 (OPDM1); oculopharyngodistal myopathy type 2 (OPDM2); oculopharyngeal myopathy with leukoencephalopathy type 1 (OPML1); spinobulbar muscular atrophy (SBMA); spinocerebellar ataxias type 1, 2, 3, 6, 7, 8, 10, 12, 17, 27B, 31, 36, 37 (SCA1, SCA2, SCA3, SCA6, SCA7, SCA8, SCA10, SCA12, SCA17, SCA27B, SCA31, SCA36, SCA37); synpolydactyly (SPD); X-linked dystonia parkinsonism (XDP); X-linked mental retardation and abnormal genitalia (XLAG); or X-linked mental retardation with or without growth hormone deficiency (XLMR, XLMRGHD).

Suitably, the repeat expansion disease or disorder may be a triplet repeat disease or disorder. The triplet repeat disease or disorder may be selected from dentatorubropallidoluysian atrophy (DRPLA); Fuch's endothelial corneal dystrophy type 3 (FECD3); fragile XE syndrome (FRAXE); Friedreich ataxia (FRDA); fragile X-associated premature ovarian infertility (FXPOI); fragile X syndrome (FXS); fragile X-associated tremor ataxia syndrome (FXTAS); Huntington's disease (HD); Huntington disease-like 2 (HDL2); myotonic dystrophy type 1 (DM1); neuronal intranuclear inclusion disease (NIID; )spinobulbar muscular atrophy (SBMA); spinocerebellar ataxias type 1, 2, 3, 6, 7, 8, 17, (SCA1, SCA2, SCA3, SCA6, SCA7, SCA8, SCA17). More suitably, the triplet repeat disease or disorder is Huntington's disease (HD).

According to a further aspect of the present invention, there is provided a method of increasing the efficiency of a gene editing technique, the method comprising conducting the gene editing technique in the presence of a compound as defined herein, or a pharmaceutically acceptable salt, hydrate or solvate thereof.

According to a further aspect of the present invention, there is provided a method of prime editing, the method comprising contacting a cell with: (1) a Cas9 nickase to nick a non-target strand; (2) a prime editing single guide RNA (pegRNA) to bind the target strand; (3) a reverse transcriptase to reverse transcribe from the 3' end of the non-target strand according to the information provided by the pegRNA; and (4) a compound as defined herein, or a pharmaceutically acceptable salt, hydrate or solvate thereof.

In the method of prime editing described herein, the step of "contacting a cell" suitably comprises contacting a target gene or DNA in a cell.

According to a further aspect of the present invention, there is provided the use of a compound, or a pharmaceutically acceptable salt, hydrate or solvate thereof, as defined herein for gene editing *in vitro, ex vivo or in vivo.*

According to a further aspect of the present invention, there is provided a compound, or a pharmaceutically acceptable salt, hydrate or solvate thereof, as defined herein for use in gene editing *in vitro, ex vivo or in vivo.*

Suitably, the gene editing technique referred to herein is any suitable gene editing technique in which host cell mis-match repair (MMR) factors are known to decrease the efficiency of the gene editing. Several available tools for gene editing use enzymes acting on DNA called nucleases and exploit intrinsic DNA repair pathways such as homology-directed repair (HDR), non-homologous end joining (NHEJ) which are both associated with double strand breaks (DSB)s. Specific examples of such DSB nucleases include ZFN (zinc finger nucleases), TALENs (Transcription activator like effector nucleases), CRISPR-Cas9 or CRISPR-Cas12 nucleases and Fanzor (an eukaryotic CRISPR-like RNA guided nucleases related to prokaryotic OMEGA system nucleases). Other forms of gene editing technology do not induce DSBs and use engineered or inactivated nucleases to either cut only a single strand of DNA. Specific examples of gene editing technologies that do not leverage DSBs include base editing (fusion of catalytically dead dCas9 enzymes to DNA deaminases which enable single nucleotide substitutions) and prime editing (fusion of a Cas9 nickase to a reverse transcriptase) and its related technology PASTE (programmable addition via site-specific targeting elements which leverages prime editing to insert AttB sites and then facilitate serine integrase proteins to insert larger sequences). Indeed, all editing methods using a single-stranded oligonucleotide (ssODN) as a template should benefit from MMR inhibition.

The gene editing technique may be a HDR-based gene editing method, e.g. CRISPR-Cas9, ZFN and TALENs.

According to a further aspect of the present invention, there is provided a process for preparing a compound, or a pharmaceutically acceptable salt, hydrate or solvate thereof, as defined herein.

According to a further aspect of the present invention, there is provided a compound, or a pharmaceutically acceptable salt, hydrate or solvate thereof, obtainable by, or obtained by, or directly obtained by a process of preparing a compound as defined herein.

According to a further aspect of the present invention, there are provided novel intermediates as defined herein which are suitable for use in any one of the synthetic methods set out herein.

Features, including optional, suitable, and preferred features in relation to one aspect of the invention may also be features, including optional, suitable and preferred features in relation to any other aspect of the invention.

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

In this specification, "simple nucleotide repeats" (also known as microsatellites or short tandem repeat sequences) are known in the art and are short tandemly repeated DNA sequences comprising a repetitive unit of from 1-6 base pairs, where the motifs are repeated >5 times.

The term "prime editing" refers to programmable editing of a target DNA using a prime editor complexed with a pegRNA to incorporate an intended nucleotide edit into the target DNA through target-primed DNA synthesis. A target polynucleotide, e.g., a target gene of prime editing may comprise a double stranded DNA molecule having two complementary strands: a first strand that may be referred to as a "target strand" or a "non-edit strand," and a second strand that may be referred to as a "non-target strand," or an "edit strand".

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For Example, "(1-6C)alkyl" includes (1-4C)alkyl, (1-3C)alkyl, methyl, ethyl, n-propyl, isopropyl, n-butyl, sec-butyl, and t-butyl, as well as pentyl and hexyl isomers. A similar convention applies to other radicals, for example "phenyl(1-6C)alkyl" includes phenyl(1-4C)alkyl, benzyl, 1-phenylethyl and 2-phenylethyl etc.

The term "(m-nC)" or "(m-nC) group" used alone or as a prefix, refers to any group having m to n carbon atoms.

An "alkylene," "alkenylene," or "alkynylene" group is an alkyl, alkenyl, or alkynyl group that is positioned between and serves to connect two other chemical groups. Thus, "(1-6C)alkylene" means a linear saturated divalent hydrocarbon radical of one to six carbon atoms or a branched saturated divalent hydrocarbon radical of three to six carbon atoms, for example, methylene, ethylene, propylene, 2-methylpropylene, pentylene, and the like.

"(2-6C)alkenylene" means a linear divalent hydrocarbon radical of two to six carbon atoms or a branched divalent hydrocarbon radical of three to six carbon atoms, containing at least one double bond, for example, as in ethenylene, 2,4-pentadienylene, and the like.

"(2-6C)alkynylene" means a linear divalent hydrocarbon radical of two to six carbon atoms or a branched divalent hydrocarbon radical of three to six carbon atoms, containing at least one triple bond, for example, as in ethynylene, propynylene, and butynylene and the like.

"(3-8C)cycloalkyl" means a hydrocarbon ring containing from 3 to 8 carbon atoms, for example, cyclopropyl, cyclobutyl, cyclopentyl, cyclohexyl, cycloheptyl or bicyclo[2.2.1]heptyl.

"(3-8C)cycloalkenyl" means a hydrocarbon ring containing at least one double bond, for example, cyclobutenyl, cyclopentenyl, cyclohexenyl or cycloheptenyl, such as 3-cyclohexen-1-yl, or cyclooctenyl.

"(3-8C)cycloalkyl-(1-6C)alkyl" means a (3-8C)cycloalkyl group covalently attached to a (1-6C)alkylene group, both of which are defined herein.

The term "halo" or "halogeno" refers to fluoro, chloro, bromo and iodo.

The term "heterocyclyl", "heterocyclic" or "heterocycle" means a non-aromatic saturated or partially saturated monocyclic, fused, bridged, or spiro bicyclic heterocyclic ring system(s). Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring, with from 1 to 8 (suitably 1, 2, 3, 4 or 5, more suitably 1, 2 or 3) heteroatoms selected from nitrogen, oxygen or sulfur in the ring. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2H-thiopyran, and hexahydrothiepine. Other heterocycles include dihydrooxathiolyl, tetrahydrooxazolyl, tetrahydro-oxadiazolyl, tetrahydrodioxazolyl, tetrahydrooxathiazolyl, hexahydrotriazinyl, tetrahydrooxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO₂ groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group via any suitable atom, such as via a carbon or nitrogen atom. However, reference herein to piperidino or morpholino refers to a piperidin-1-yl or morpholin-4-yl ring that is linked via the ring nitrogen.

By "bridged ring systems" it is meant ring systems in which two rings share more than two atoms, see for example Advanced Organic Chemistry, by Jerry March, 4th Edition, Wiley Interscience, pages 131-133, 1992. Examples of bridged heterocyclyl ring systems include, aza-bicyclo[2.2.1]heptane, 2-oxa-5-azabicyclo[2.2.1]heptane, aza-bicyclo[2.2.2]octane, aza-bicyclo[3.2.1]octane and quinuclidine.

By "carbocycle" or a "carbocyclic ring", we mean a saturated or partially saturated ring formed exclusively from carbon atoms. For example, the term "carbocyclic ring" encompasses cycloalkyl or cycloalkenyl ring systems.

By "spiro bicyclic ring systems" we mean that the two ring systems share one common spiro carbon atom, i.e. the heterocyclic ring is linked to a further carbocyclic or heterocyclic ring through a single common spiro carbon atom. Examples of spiro ring systems include 6-azaspiro[3.4]octane, 2-oxa-6-azaspiro[3.4]octane, 2-azaspiro[3.3]heptanes, 2-oxa-6-azaspiro[3.3]heptanes, 7-oxa-2-azaspiro[3.5]nonane, 6-oxa-2-azaspiro[3.4]octane, 2-oxa-7-azaspiro[3.5]nonane and 2-oxa-6-azaspiro[3.5]nonane.

"Heterocyclyl(1-6C)alkyl" means a heterocyclyl group covalently attached to a (1-6C)alkylene group, both of which are defined herein.

The term "heteroaryl" or "heteroaromatic" means an aromatic mono-, bi-, or polycyclic ring incorporating one or more (for example 1-4, particularly 1, 2 or 3) heteroatoms selected from nitrogen, oxygen or sulfur. The term heteroaryl includes both monovalent species and divalent species. Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically, the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general, the number of basic nitrogen atoms present in the heteroaryl group, including any amino group substituents of the ring, will be less than five.

Examples of heteroaryl include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, isoindolyl, benzothienyl, benzoxazolyl, benzimidazolyl, benzothiazolyl, benzothiazolyl, indazolyl, purinyl, benzofurazanyl, quinolyl, isoquinolyl, quinazolinyl, quinoxalinyl, cinnolinyl, pteridinyl, naphthyridinyl, carbazolyl, phenazinyl, benzisoquinolinyl, pyridopyrazinyl, thieno[2,3b]-furanyl-, 2H-furo[3,2b]-pyranyl-, 5H-pyrido[2,3-d]-ooxazinyl-, 1H-pyrazolo[4,3-d]-oxazolyl, 4H-imidazo[4,5d]thiazolyl, pyrazino[2,3d]pyridazinyl, -imidazo[2,1b]thiazolyl, -imidazo[1,2b][1,2,4]-triazinyl. "Heteroaryl" also covers partially aromatic bi- or polycyclic ring systems wherein at least one ring is an aromatic ring and one or more of the other ring(s) is a nonaromatic, saturated or partially saturated ring, provided at least one ring contains one or more heteroatoms selected from nitrogen, oxygen or -sulfur-. Examples of partially aromatic heteroaryl groups include for example, tetrahydroisoquinolinyl, tetrahydroquinolinyl, 2-oxo-1,2,3,4-tetrahydroquinolinyl, dihydrobenzthienyl, dihydrobenzfuranyl, 2,3-dihydro-benzo[1,4]dioxinyl, benzo[1,3]dioxolyl, 2,2-dioxo-1,3-dihydro-2-benzothienyl, 4,5,6,7-tetrahydrobenzofuranyl, indolinyl, 1,2,3,4-tetrahydro-1,8-naphthyridinyl, 1,2,3,4-tetrahydropyrido[2,3-*b*]pyrazinyl, 3,4-dihydro-2*H*-pyrido[3,2-*b*][1,4]oxazinyl and 6,8-dihydro-5H-[1,2,4]triazolo[4,3-a]pyrazinyl.

Examples of five membered heteroaryl groups include but are not limited to pyrrolyl, furanyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered heteroaryl groups include but are not limited to pyridyl, pyrazinyl, pyridazinyl, pyrimidinyl and triazinyl.

A bicyclic heteroaryl group may be, for example, a group selected from:
a benzene ring fused to a 5- or 6-membered ring containing 1, 2 or 3 ring heteroatoms;
a pyridine ring fused to a 5- or 6-membered ring containing 1, 2 or 3 ring heteroatoms;
a pyrimidine ring fused to a 5- or 6-membered ring containing 1 or 2 ring heteroatoms;
a pyrrole ring fused to a 5- or 6-membered ring containing 1, 2 or 3 ring heteroatoms;
a pyrazole ring fused to a 5- or 6-membered ring containing 1 or 2 ring heteroatoms;
a pyrazine ring fused to a 5- or 6-membered ring containing 1 or 2 ring heteroatoms;
an imidazole ring fused to a 5- or 6-membered ring containing 1 or 2 ring heteroatoms;
an oxazole ring fused to a 5- or 6-membered ring containing 1 or 2 ring heteroatoms;
an isoxazole ring fused to a 5- or 6-membered ring containing 1 or 2 ring heteroatoms;
a thiazole ring fused to a 5- or 6-membered ring containing 1 or 2 ring heteroatoms;
an isothiazole ring fused to a 5- or 6-membered ring containing 1 or 2 ring heteroatoms;
a thiophene ring fused to a 5- or 6-membered ring containing 1, 2 or 3 ring heteroatoms;
a furan ring fused to a 5- or 6-membered ring containing 1, 2 or 3 ring heteroatoms;
a cyclohexyl ring fused to a 5- or 6-membered heteroaromatic ring containing 1, 2 or 3 ring heteroatoms; and
a cyclopentyl ring fused to a 5- or 6-membered heteroaromatic ring containing 1, 2 or 3 ring heteroatoms.

Particular examples of bicyclic heteroaryl groups containing a six membered ring fused to a five membered ring include but are not limited to benzfuranyl, benzthiophenyl, benzimidazolyl, benzoxazolyl, benzisoxazolyl, benzthiazolyl, benzisothiazolyl, isobenzofuranyl, indolyl, isoindolyl, indolizinyl, indolinyl, isoindolinyl, purinyl (e.g., adeninyl, guaninyl), indazolyl, benzodioxolyl and pyrazolopyridinyl groups.

Particular examples of bicyclic heteroaryl groups containing two fused six membered rings include but are not limited to quinolinyl, isoquinolinyl, chromanyl, thiochromanyl, chromenyl, isochromenyl, chromanyl, isochromanyl, benzodioxanyl, quinolizinyl, benzoxazinyl, benzodiazinyl, pyridopyridinyl, quinoxalinyl, quinazolinyl, cinnolinyl, phthalazinyl, naphthyridinyl and pteridinyl groups.

"Heteroaryl(1-6C)alkyl" means a heteroaryl group covalently attached to a (1-6C)alkylene group, both of which are defined herein. Examples of heteroaralkyl groups include pyridin-3-ylmethyl, 3-(benzofuran-2-yl)propyl, and the like.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. In particular embodiment, an aryl is phenyl.

The term "aryl(1-6C)alkyl" means an aryl group covalently attached to a (1-6C)alkylene group, both of which are defined herein. Examples of aryl-(1-6C)alkyl groups include benzyl, phenylethyl, and the like.

This specification also makes use of several composite terms to describe groups comprising more than one functionality. Such terms will be understood by a person skilled in the art. For example heterocyclyl(m-nC)alkyl comprises (m-nC)alkyl substituted by heterocyclyl.

The term "optionally substituted" refers to either groups, structures, or molecules that are substituted and those that are not substituted. The term "wherein a/any CH, CH₂, CH₃ group or heteroatom (i.e. NH) within a R¹ group is optionally substituted" suitably means that (any) one of the hydrogen radicals of the R¹ group is substituted by a relevant stipulated group.

Where optional substituents are chosen from "one or more" groups it is to be understood that this definition includes all substituents being chosen from one of the specified groups or the substituents being chosen from two or more of the specified groups.

The phrase "compound of the invention" means those compounds which are disclosed herein, both generically and specifically.

### Compounds of the invention

In a first aspect, the present invention relates to compounds, or pharmaceutically acceptable salts, hydrates or solvates thereof, having the structural formula (I) shown below: 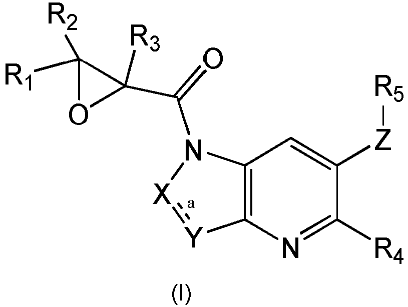 wherein:
R₁ is selected from hydrogen or a group:

   -L^{B}-X^{B}-Q^{B}

   wherein:
   L^{B} is absent or -[CR_{L1}R_{L2}]ₘ-, wherein:
      m is an integer selected from 1, 2, 3 or 4;
      each occurrence of R_{L1} and R_{L2} is independently selected from hydrogen or (1-2C)alkyl), or R_{L1} and R_{L2} taken together may form an oxo group;
   X^{B} is absent or is selected from the group consisting of -O-, -S-, -S(O)-, - S(O)₂-, -C(O)-, -C(O)O-, -OC(O)-, -C(O)N(R_{XB1})-, -N(R_{XB1})C(O)-, -N(R_{XB1})-, -N(R_{XB1})C(O)O-, -OC(O)N(R_{XB1})-, -N(R_{XB1})C(O)N(R_{XB2})-, -S(O)₂N(R_{XB1})-, or - N(R_{XB1})SO₂-, wherein R_{XB1} and R_{XB2} are each independently selected from hydrogen and (1-2C)alkyl;
   Q^{B} is selected from a group consisting of hydrogen, (1-6C)alkyl, -[CR_{Q1}R_{Q2}]ₙ-, -[CR_{Q1}R_{Q2}]ₙ-(3-10C)cycloalkyl, -[CR_{Q1}R_{Q2}]ₙ-(3 to 10-membered heterocyclyl), - [CR_{Q1}R_{Q2}]ₙ-aryl and -[CR_{Q1}R_{Q2}]ₙ₋(5 to 10-membered heteroaryl);
   wherein:
      integer n is 0, 1 or 2; and
      R_{Q1} and R_{Q2} are each independently selected from hydrogen and (1-2C)alkyl; and
      any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy, NR_{Q3}R_{Q4}, C(O)R_{Q3}, C(O)OR_{Q3}, OC(O)R_{Q3}, C(O)N(R_{Q3})R_{Q4}, N(R_{Q3})C(O)R_{Q4}, S(O)_{y}Rₘ (where y is 0, 1 or 2), SO₂N(R_{Q3})R_{Q4} or N(R_{Q3})SO₂Rₙ; wherein R_{Q3} and R_{Q4} are each independently selected from H or (1-2C)alkyl;
R₂ is selected from hydrogen and (1-3C)alkyl;
R₃ is selected from hydrogen and (1-3C)alkyl;
bond a is either a single bond or a double bond, wherein:
   when bond a is a single bond:
      X is selected from -C(R_{X1})(R_{X2})-, -C(=O)-, -C(=NH)-, -C(=S)-; and
      Y is selected from -C(R_{Y1})(R_{Y2})-, -C(=O)-, -C(=NH)-, -C(=S)-; or
      when bond a is a double bond:
   X is -C(R_{X1})- and
   Y is -C(R_{Y1})-;
      wherein:
      R_{X1} and R_{X2} are each independently selected from hydrogen, halo, hydroxy, (1-3C)alkyl or (1-3C)alkoxy; and any alkyl is optionally further substituted with one or more substituents selected from: halo, hydroxy or (1-3C)alkoxy; or
      R_{Y1} and R_{Y2} are each independently selected from hydrogen, halo, hydroxy, (1-3C)alkyl or (1-3C)alkoxy; and any alkyl is optionally further substituted with one or more substituents selected from: halo, hydroxy or (1-3C)alkoxy; or
      R_{X1} and R_{X2} are linked such that, together with the carbon atom to which they are attached, form a (3-6C)cycloalkyl ring; or
      or R_{Y1} and R_{Y2} are linked such that, together with the carbon atom to which they are attached, form a (3-6C)cycloalkyl ring, optionally substituted with halo, hydroxy, (1-3C)alkyl or (1-3C)alkoxy; and any alkyl is optionally further substituted with one or more substituents selected from: halo, hydroxy or (1-3C)alkoxy;
R₄ is selected from hydrogen, halo, cyano, hydroxy, NR₄ₐR_{4b}, (1-6C)alkyl, (1-6C)alkoxy, (1-6C)haloalkyl, (1-6C)haloalkoxy, -[CH₂]ᵣ-(3-12C)cycloalkyl, -[CH₂]ᵣ-(3- to 12-membered)heterocyclyl, -[CH₂]ᵣ-aryl or -[CH₂]ᵣ-heteroaryl wherein each of R₄ₐ and
R_{4b} is independently selected from hydrogen or (1-3C)alkyl and integer r is 0, 1 or 2;
   wherein:
   any alkyl, cycloalkyl, heterocyclyl, aryl or heteroaryl in an R₄ group is optionally substituted by one or more R₁₆ substituents independently selected from halo, hydroxy, cyano, (1-3C)alkyl, (1-3C)alkoxy, (1-3C)haloalkyl, (1-3C)haloalkoxy or NR₄ₐR_{4b}, wherein R₄ₐ and R_{4b} are each independently selected from H or (1-2C)alkyl;
Z is selected from -C(R_{Z2})(R_{Z3})-, -O-, or -NR_{Z1}, wherein:
   R_{Z1} is selected from hydrogen or (1-2C)alkyl;
   R_{Z2} and R_{Z3} are each independently selected from hydrogen or (1-3C)alkyl; or
   R_{Z2} and R_{Z3} are linked such that, together with the carbon atom to which they are attached, form a (3-5C)cycloalkyl or heterocyclyl ring;
R₅ is selected from phenyl, naphthyl or heteroaryl, wherein R₅ is optionally substituted by one or more substituents R_{A}, wherein each R_{A} is independently selected from: halo, hydroxy, cyano, (1-6C)alkyl, (1-6C)alkoxy, (1-6C)haloalkyl, (1-6C)haloalkoxy, cycloalkyl, (3-6-membered)heterocyclyl and -[CH₂]ₛ-NR₅ₐR_{5b}; wherein R₅ₐ and R_{5b} are each independently selected from hydrogen or (1-2C)alkyl and integer s is 0, 1 or 2.

Particular compounds of the invention include, for example, compounds of the formula I, or pharmaceutically acceptable salts, hydrates and/or solvates thereof, wherein, unless otherwise stated, each of R₁, R₂, R₃, R₄, R₅, X, Y, Z, bond a and any associated sub groups, have any of the meanings defined hereinbefore or in any of paragraphs (1) to (26) hereinafter:
(1) R₁ is selected from hydrogen or a group:

   -L^{B}-X^{B}-Q^{B}

   wherein:
   L^{B} is absent or -[CR_{L1}R_{L2}]ₘ-, wherein:
      m is an integer selected from 1, 2 or 3;
      each occurrence of R_{L1} and R_{L2} is independently selected from hydrogen or (1-2C)alkyl);
   X^{B} is absent or is selected from the group consisting of -O-, -S-, -S(O)-, - S(O)₂-, -C(O)-, -C(O)O-, -OC(O)-, -C(O)N(R_{XB1})-, -N(R_{XB1})C(O)- or -N(R_{XB1})-, wherein R_{XB1} is selected from hydrogen and (1-2C)alkyl;
   Q^{B} is selected from a group consisting of hydrogen, (1-6C)alkyl, -[CR_{Q1}R_{Q2}]ₙ-, -[CR_{Q1}R_{Q2}]ₙ-(3-10C)cycloalkyl, -[CR_{Q1}R_{Q2}]ₙ-(3 to 10-membered heterocyclyl), - [CR_{Q1}R_{Q2}]ₙ-aryl or -[CR_{Q1}R_{Q2}]ₙ₋(5 to 10-membered heteroaryl);
   wherein:
      integer n is 0, 1 or 2; and
      R_{Q1} and R_{Q2} are each independently selected from hydrogen and (1-2C)alkyl; and
      any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy, N(R_{Q3})R_{Q4}, C(O)R_{Q3}, C(O)OR_{Q3}, OC(O)R_{Q3}, C(O)N(R_{Q3})R_{Q4} or N(R_{Q3})C(O)R_{Q4}; wherein R_{Q3} and R_{Q4} are each independently selected from H or (1-2C)alkyl.
(2) R₁ is selected from hydrogen or a group:

   -L^{B}-X^{B}-Q^{B}

   wherein:
   L^{B} is absent or -[CR_{L1}R_{L2}]ₘ-, wherein:
      m is an integer selected from 1 or 2;
      each occurrence of R_{L1} and R_{L2} is independently selected from hydrogen or (1-2C)alkyl);
   X^{B} is absent or is selected from the group consisting of -O-, -S-, -C(O)-, - C(O)N(R_{XB1})-, -N(R_{XB1})C(O)- or -N(R_{XB1})-, wherein R_{XB1} is selected from hydrogen and (1-2C)alkyl;
   Q^{B} is selected from a group consisting of hydrogen, (1-6C)alkyl, -[CR_{Q1}R_{Q2}]ₙ-, -[CR_{Q1}R_{Q2}]ₙ-(3-6C)cycloalkyl, -[CR_{Q1}R_{Q2}]ₙ-(3 to 6-membered heterocyclyl), - [CR_{Q1}R_{Q2}]ₙ-phenyl or -[CR_{Q1}R_{Q2}]ₙ₋(5 or 6-membered heteroaryl);
   wherein:
      integer n is selected from 0, 1 or 2; and
      R_{Q1} and R_{Q2} are each independently selected from hydrogen and (1-2C)alkyl; and
      any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy or NR_{Q3}R_{Q4}; wherein R_{Q3} and R_{Q4} are each independently selected from H or (1-2C)alkyl.
(3) R₁ is selected from hydrogen or a group:

   -L^{B}-X^{B}-Q^{B}

   wherein:
   L^{B} is absent or -[CR_{L1}R_{L2}]ₘ-, wherein:
      m is an integer selected from 1 or 2;
      each occurrence of R_{L1} and R_{L2} is independently selected from hydrogen or (1-2C)alkyl);
   X^{B} is absent or is selected from the group consisting of -O-, -S-, -C(O)-, - C(O)N(R_{XB1})-, -N(R_{XB1})C(O)-, -N(R_{XB1})-, wherein R_{XB1} is selected from hydrogen and (1-2C)alkyl;
   Q^{B} is selected from a group consisting of hydrogen, (1-4C)alkyl, -[CR_{Q1}R_{Q2}]ₙ-, -[CR_{Q1}R_{Q2}]ₙ-(3-6C)cycloalkyl, -[CR_{Q1}R_{Q2}]ₙ-(3 to 6-membered heterocyclyl), - [CR_{Q1}R_{Q2}]ₙ-phenyl or -[CR_{Q1}R_{Q2}]ₙ₋(5 or 6-membered heteroaryl);
   wherein:
      integer n is selected from 0, 1 or 2; an R_{Q1} and R_{Q2} are each independently selected from hydrogen or (1-2C)alkyl; and
      any Q^{B} is optionally further substituted with one or more substituents independently selected from: halo, oxo, hydroxy, cyano, (1-2C)alkyl, (1-2C)alkoxy, (1-2C)haloalkyl, (1-2C)haloalkoxy or NR_{Q3}R_{Q4}; wherein R_{Q3} and R_{Q4} are each independently selected from hydrogen or methyl.
(4) R₁ is selected from hydrogen or a group:

   -L^{B}-X^{B}-Q^{B}

   wherein:
   L^{B} is absent or -[CR_{L1}R_{L2}]-, wherein:
      each occurrence of R_{L1} and R_{L2} is independently selected from hydrogen or methyl;
   X^{B} is absent or is selected from the group consisting of -O-, -C(O)N(R_{XB1})-, wherein R_{XB1} is selected from hydrogen and methyl;
   Q^{B} is selected from a group consisting of hydrogen, (1-4C)alkyl, -[CR_{Q1}R_{Q2}]ₙ-(3-6C)cycloalkyl, -[CR_{Q1}R_{Q2}]ₙ-(4 to 6-membered heterocyclyl), -[CR_{Q1}R_{Q2}]ₙ-phenyl or -[CR_{Q1}R_{Q2}]ₙ₋(5 or 6-membered heteroaryl); wherein:
      integer n is selected from 0 or 1; and
      R_{Q1} and R_{Q2} are each independently selected from hydrogen and
      methyl; and
      any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy or NR_{Q3}R_{Q4}; wherein R_{Q3} and R_{Q4} are each independently selected from H or (1-2C)alkyl.
(5) R₁ is a group of the formula:

   -L^{B}-X^{B}-Q^{B}

   wherein:
   L^{B} is absent or -[CR_{L1}R_{L2}]-, wherein each occurrence of R_{L1} and R_{L2} is independently selected from hydrogen or (1-2C)alkyl);
   X^{B} is absent or is selected from the group consisting of -O-, -S-, -C(O)-, - C(O)N(R_{XB1})-, -N(R_{XB1})C(O)-, -N(R_{XB1})-, wherein R_{XB1} is selected from hydrogen and (1-2C)alkyl;
   Q^{B} is selected from a group consisting of hydrogen, (1-4C)alkyl, (3-6C)cycloalkyl, 3 to 6-membered heterocyclyl (e.g. oxetane, azetidine, 1,3-dioxolane or morpholine), phenyl or 5 or 6-membered heteroaryl;
   wherein:
      any Q^{B} is optionally further substituted with one or more substituents independently selected from: halo, oxo, hydroxy, cyano, (1-2C)alkyl, (1-2C)alkoxy.
(6) R₁ is selected from (1-6C)alkyl, (1-6C)hydroxyalkyl, (1-6C)haloalkyl, -(CH₂)₀₋₁C(O)N(R_{XB1})R_{QB}, 5- or 6-membered heteroaryl (e.g. pyridyl), phenyl, (4-8-membered)heterocyclyl or -[CH₂]-(4- to 8-membered)heterocyclyl, wherein (1-6C)alkyl is optionally substituted with one or more substituents selected from (1-4C)alkoxy, (1-4C)alkoxy, hydroxy, (1-4C)alkoxy, cyano or N(R_{XB1})R_{QB};
   and any 5- or 6-membered heteroaryl (e.g. pyridyl), phenyl, (4-8-membered)heterocyclyl or -[CH₂]-(4- to 8-membered)heterocyclyl is optionally substituted with one or more substituents selected from (1-2Calkyl), halo, (1-4C)alkoxy, (1-4C)haloalkoxy, hydroxy, cyano or N(R_{XB1})R_{QB}; wherein R_{XB1} and R_{QB} are each independently selected from hydrogen or (1-2C)alkyl.
(7) R₁ is selected from (1-6C)alkyl, (1-6C)hydroxyalkyl, (1-6C)haloalkyl or C(O)N(R_{XB1})R_{QB}, wherein (1-6C)alkyl is optionally substituted with one or more substituents selected from (1-2C)alkoxy, hydroxy, cyano or N(R_{XB1})R_{QB};
   wherein R_{XB1} and R_{QB} are each independently selected from hydrogen or methyl.
(8) R₁ is selected from (1-4C)alkyl, (1-4C)hydroxyalkyl, (1-4C)haloalkyl or C(O)N(R_{XB1})R_{QB}, wherein (1-4C)alkyl is optionally substituted with one or more substituents selected from (1-2C)alkoxy or cyano;
   wherein any (1-4C)alkyl, (1-4C)hydroxyalkyl or (1-4C)haloalkyl comprises a branched alkyl group (e.g. an isopropyl group).
(9) R₁ is a group of the formula: wherein L^{B} is absent or -[CH₂]-; 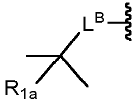 R₁ₐ is selected from hydrogen, methyl, halo, hydroxy, cyano, methoxy.
(10) R₁ is a group of the formula: wherein L^{B} is absent or -[CH₂]-; 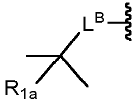 R₁ₐ is selected from hydrogen or hydroxy.
(11) R₁ is a group with a formula selected from: 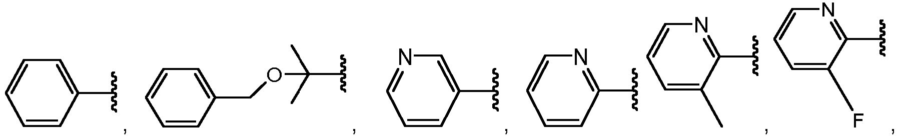 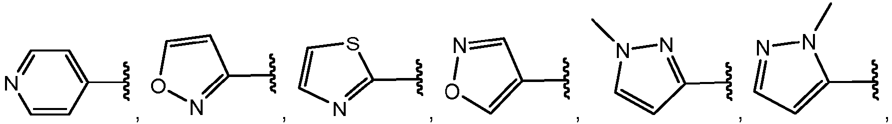 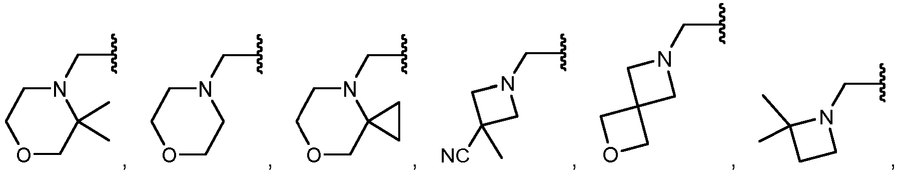 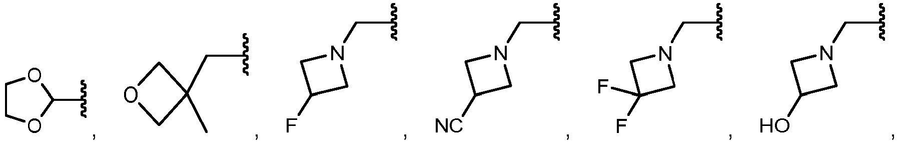 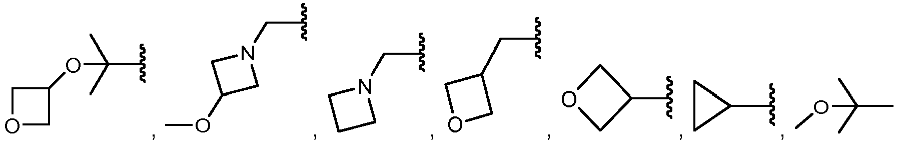 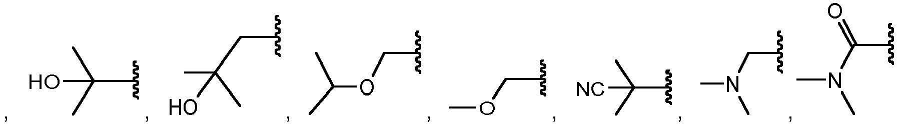 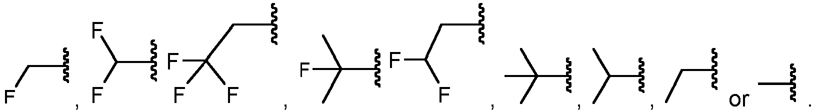
(12) R₂ is selected from hydrogen or methyl.
(13) R₂ is hydrogen.
(14) R₃ is selected from hydrogen or methyl.
(15) R₃ is hydrogen.
(16) bond a is either a single or a double bond, and wherein:
   when bond a is a single bond:
      X is -CH₂-, and
      Y is selected from -C(R_{Y1})(R_{Y2})- or -C(=O)-; or
   when bond a is a double bond:
      X is -CH- and
      Y is -C(R_{Y1})-;
         wherein:
         R_{Y1} and R_{Y2} are each independently selected from hydrogen or
         (1-2C)alkyl; or
         or R_{Y1} and R_{Y2} are linked such that, together with the carbon atom to which they are attached, form a (3-4C)cycloalkyl ring.
(17) bond a is a single bond and:
   X is -CH₂-, and
   Y is selected from -C(R_{Y1})(R_{Y2})-;
      wherein:
      R_{Y1} and R_{Y2} are each independently selected from hydrogen or methyl; or
      R_{Y1} and R_{Y2} are linked such that, together with the carbon atom to which they are attached, form a cyclopropyl ring.
(18) R₄ is selected from hydrogen, halo (e.g. F or Cl), NR₄ₐR_{4b}, (1-4C)alkyl (e.g. CH₃ or CD₃), (1-4C)alkoxy, (1-4C)haloalkyl, (1-4C)haloalkoxy, -[CH₂]ᵣ-(3-6C)cycloalkyl, - [CH₂]ᵣ-(3- to 7-membered)heterocyclyl, -[CH₂]ᵣ-phenyl or -[CH₂]ᵣ-(5- or 6-membered) heteroaryl;
   wherein:
   each of R₄ₐ and R_{4b} is independently selected from hydrogen or (1-2C)alkyl and integer r is 0 or 1; and
   any alkyl, cycloalkyl, heterocyclyl, phenyl or heteroaryl in an R₄ group is optionally substituted by one or more substituents independently selected from halo, hydroxy, cyano or amino.
(19) R₄ is selected from hydrogen, halo (e.g. F or Cl), NR₄ₐR_{4b}, (1-3C)alkyl (e.g. CH₃ or CD₃), (1-2C)alkoxy, (1-3C)haloalkyl, (3-6C)cycloalkyl, (4- to 6-membered)heterocyclyl, phenyl or (5- or 6-membered)heteroaryl;
   wherein:
   each of R₄ₐ and R_{4b} is independently selected from hydrogen or (1-2C)alkyl and integer r is 0 or 1; and
   any alkyl, cycloalkyl, heterocyclyl, phenyl or heteroaryl in an R₄ group is optionally substituted by one or more substituents independently selected from halo, hydroxy, cyano or amino.
(20) R₄ is selected from hydrogen, halo (e.g. F or Cl), NR₄ₐR_{4b}, (1-3C)alkyl (e.g. CH₃ or CD₃), (1-2C)alkoxy, (1-3C)haloalkyl, (3-6C)cycloalkyl, (4- to 6-membered)heterocyclyl, phenyl or (5- or 6-membered)heteroaryl;
   wherein:
   each of R₄ₐ and R_{4b} is independently selected from hydrogen or methyl;
   any alkyl, cycloalkyl, heterocyclyl, phenyl or heteroaryl in an R₄ group is optionally substituted by one or more substituents independently selected from halo, hydroxy, cyano or amino.
(21) R₄ is selected from hydrogen, F or Cl, NR₄ₐR_{4b}, (1-2C)alkyl (e.g. CH₃ or CD₃), methoxy, (1-2C)haloalkyl, (3-4C)cycloalkyl, (4- to 5-membered)heterocyclyl or (5- or 6-membered)heteroaryl;
   wherein:
   each of R₄ₐ and R_{4b} is independently selected from hydrogen or methyl;
   any alkyl, cycloalkyl, heterocyclyl or heteroaryl in an R₄ group is optionally substituted by one or more substituents independently selected from halo, hydroxy, cyano or amino;
(22) R₄ is methyl.
(23) Z is selected from -C(R_{Z2})(R_{Z3})-, -O-, - or -NR_{Z1}; wherein:
   R_{Z1} is selected from hydrogen or methyl; and
   R_{Z2} and R_{Z3} are independently selected from hydrogen or methyl.
(24) Z is selected from -CH₂-, -O- or -NR_{Z1}; wherein:
   R_{Z1} is selected from hydrogen or methyl.
(25) Z is -O-.
(26) R₅ is selected from phenyl, 5- or 6-membered heteroaryl, or a bicyclic heteroaryl (e.g.
   a bicyclic heteroaryl comprising a phenyl ring fused to a 4- to 6 membered heterocyclic ring);
   wherein R₅ is optionally substituted by one or more substituents R_{A}, wherein each R_{A} is independently selected from: halo, hydroxy, cyano, (1-4C)alkyl, (1-4C)alkoxy, (1-4C)haloalkyl, (1-4C)haloalkoxy, (3-6C)cycloalkyl, (3-6-membered)heterocyclyl and NR₅ₐR_{5b}; wherein R₅ₐ and R_{5b} are each independently selected from hydrogen or methyl.
(27) R₅ is selected from phenyl, pyridyl, or a phenyl ring fused to a 4- to 6 membered heterocyclic ring;
   wherein R₅ is optionally substituted by one or more substituents R_{A}, wherein each R_{A} is independently selected from: halo, hydroxy, cyano, (1-2C)alkyl, (1-2C)alkoxy, (1-2C)haloalkyl, (1-2C)haloalkoxy, (3-6C)cycloalkyl or (3-6-membered) heterocyclyl;
(28) R₅ is a group of the formula: 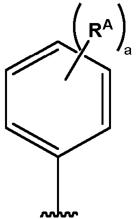 wherein a is an integer selected from 0, 1, 2, 3 or 4; each occurrence of R_{A} is independently as defined anywhere herein.
(29) R₅ is a group of the formula: 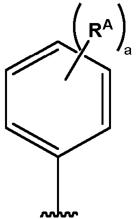 wherein a is an integer selected from 0, 1, 2, 3 or 4; and
   each occurrence of R_{A} is independently selected from: halo, hydroxy, cyano, (1-2C)alkyl, (1-2C)alkoxy, (1-2C)haloalkyl, (1-2C)haloalkoxy, (3-6C)cycloalkyl or (3-6-membered)heterocyclyl;
   optionally wherein each occurrence of R_{A} is independently selected from fluoro, chloro, hydroxy, cyano, or methoxy;
   further optionally wherein each occurrence of R_{A} is independently selected from fluoro or chloro.
(30) R₅ is a group of the formula: 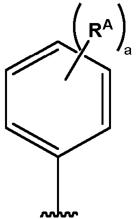
   wherein a is an integer selected from 0, 1, 2, 3 or 4; and
   each occurrence of R_{A} is independently selected from fluoro, chloro, hydroxy, cyano, or methoxy.
(31) R₅ is a group of the formula: 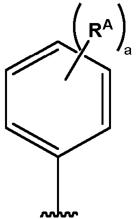
   wherein a is an integer selected from 0, 1, 2 or 3; and
   each occurrence of R_{A} is independently selected from each occurrence of R_{A} is independently selected from fluoro, chloro, hydroxy, cyano, or methoxy.
(32) R₅ is a group of the formula: 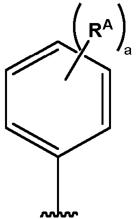 wherein:
   a is an integer selected from 0, 1 or 2;
   each occurrence of R_{A} is independently as defined anywhere herein;
   at least one occurrence of R_{A} is in the meta position.
(33) R₅ is a group of the formula: 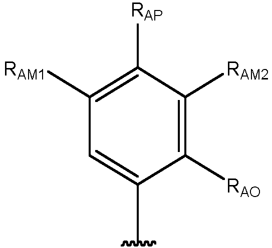
   each of R_{AM1}, R_{AM2}, R_{AO} and R_{AP} are independently selected from hydrogen, fluoro and chloro;
   wherein at least one of R_{AM1}, R_{AM2}, R_{AO} and R_{AP} is not hydrogen.
(34) R₅ is a group of the formula: 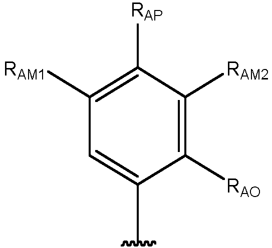
   each of R_{AM1}, R_{AO} and R_{AP} are independently selected from hydrogen, fluoro and chloro; and
   R_{AM2} is selected from fluoro and chloro.
(35) R₅ is a group with a formula selected from: 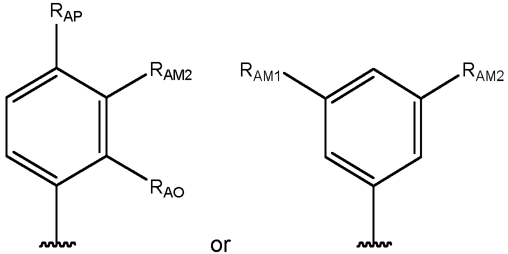
   each of R_{AO} and R_{AP} are independently selected from hydrogen, fluoro and chloro;
   R_{AM1} is selected from fluoro and chloro; and
   R_{AM2} is selected from fluoro and chloro.
(36) R₅ is a group with a formula selected from: 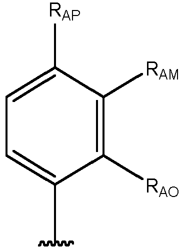
   each of R_{AO} and R_{AP} are independently selected from hydrogen, fluoro and chloro; R_{AM} is selected from fluoro and chloro;
   optionally wherein at least one of R_{AO} and R_{AP} is hydrogen, and the other is selected from fluoro and chloro.

Suitably, a heteroaryl or heterocyclyl group as defined herein is a monocyclic heteroaryl or mono, bicyclic or bridged heterocyclyl group comprising one, two or three heteroatoms selected from N, O or S.

Suitably, a heteroaryl is a 5- or 6-membered heteroaryl ring comprising one, two or three heteroatoms selected from N, O or S.

Suitably, a heterocyclyl group is a 4-, 5-, 6-, 7-, 8- or 9-membered heterocyclyl ring comprising one, two or three heteroatoms selected from N, O or S. Most suitably, a heterocyclyl group is a 5-, 6- or 7-membered monocyclic or bicyclic ring comprising one, two or three heteroatoms selected from N, O or S [e.g. morpholinyl (e.g. 4-morpholinyl), pyridinyl, piperazinyl, homopiperazinyl or pyrrolidinonyl].

Suitably, an aryl group is phenyl.

Suitably, R₁ is as defined in any one of paragraphs (1) to (11) above. More suitably, R₁ is as defined in any one of paragraphs (6) to (11) above. Most suitably, R₁ is as defined in paragraph (11) above.

Suitably, R₂ is as defined in paragraph (12) or (13) above. More suitably, Rₙ is as defined in paragraph (13) above.

Suitably, R₃ is as defined in paragraph (14) or (15) above. More suitably, R₃ is as defined in paragraph (15) above.

Suitably, bond a is as defined in paragraph (16) or (17) above. More suitably, bond a is as defined in paragraph (17).

Suitably, X is as defined in paragraph (16) or (17) above. More suitably, X is as defined in paragraph (17).

Suitably, Y is as defined in paragraph (16) or (17) above. More suitably, Y is as defined in paragraph (17).

Suitably, R₄ is as defined in any one of paragraphs (18) to (22) above. More suitably, R₄ is as defined in any one of paragraphs (20) to (22) above. Most suitably, R₄ is as defined in paragraph (22) above.

Suitably, Z is as defined in any one of paragraphs (23) to (25) above. More suitably, Z is as defined in paragraph (24) or (25) above. Most suitably, Z is as defined in paragraph (25) above.

Suitably, R₅ is as defined in any one of paragraphs (26) to (36) above. More suitably, R₅ is as defined in any one of paragraphs (32) to (36) above. Most suitably, R₅ is as defined in paragraph (35) or (36) above.

In an embodiment of the compounds of formula (I):
R₁ is as defined in any one of paragraphs (1) to (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (18) to (22) above;
Z is as defined in any one of paragraphs (23) to (25) above; and
R₅ is as defined in any one of paragraphs (26) to (36) above.

In an embodiment of the compounds of formula (I):
R₁ is as defined in any one of paragraphs (6), (7), (8), (9), (10) or (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (18) to (22) above;
Z is as defined in any one of paragraphs (23) to (25) above; and
R₅ is as defined in any one of paragraphs (26) to (36) above.

In an embodiment of the compounds of formula (I):
R₁ is as defined in any one of paragraphs (6) to (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (20) to (22) above;
Z is as defined in paragraph (24) or (25) above; and
R₅ is as defined in any one of paragraphs (33) to (36) above.

In an embodiment of the compounds of formula (I):
R₁ is as defined in any one of paragraphs (9), (10) or (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in any one of paragraphs (34), (35) or (36) above.

In an embodiment of the compounds of formula (I):
R₁ is as defined in any one of paragraphs (9), (10) or (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in paragraph (36) above.

In an embodiment of the compounds of formula (I):
R₁ is as defined in paragraph (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in any one of paragraphs (34), (35) or (36) above.

In an embodiment of the compounds of formula (I):
R₁ is as defined in paragraph (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in paragraph (36) above.

In a particular embodiment, the compounds have the structural formula (II) (a sub-definition of formula (I)) shown below: 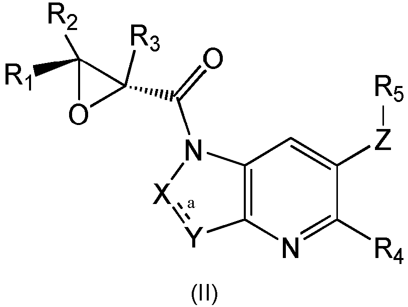 wherein R₁, R₂, R₃, R₄, R₅, X, Y, Z and bond a are as defined herein, or a pharmaceutically acceptable salt thereof.

In an embodiment of the compounds of formula (II):
R₁ is as defined in any one of paragraphs (1) to (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (18) to (22) above;
Z is as defined in any one of paragraphs (23) to (25) above; and
R₅ is as defined in any one of paragraphs (26) to (36) above.

In an embodiment of the compounds of formula (II):
R₁ is as defined in any one of paragraphs (6), (7), (8), (9), (10) or (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (18) to (22) above;
Z is as defined in any one of paragraphs (23) to (25) above; and
R₅ is as defined in any one of paragraphs (26) to (36) above.

In an embodiment of the compounds of formula (II):
R₁ is as defined in any one of paragraphs (6) to (11) above;
R₂ is as defined in paragraph (12) or (13) above;
R₃ is as defined in paragraph (14) or (15) above;
bond a is as defined in paragraph (16) or (17) above;
X is as defined in paragraph (16) or (17) above;
Y is as defined in paragraph (16) or (17) above;
R₄ is as defined in any one of paragraphs (20) to (22) above;
Z is as defined in paragraph (24) or (25) above; and
R₅ is as defined in any one of paragraphs (33) to (36) above.

In an embodiment of the compounds of formula (II):
R₁ is as defined in any one of paragraphs (9), (10) or (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in any one of paragraphs (34), (35) or (36) above.

In an embodiment of the compounds of formula (II):
R₁ is as defined in any one of paragraphs (9), (10) or (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in paragraph (36) above.

In an embodiment of the compounds of formula (II):
R₁ is as defined in paragraph (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in any one of paragraphs (34), (35) or (36) above.

In an embodiment of the compounds of formula (II):
R₁ is as defined in paragraph (11) above;
R₂ is as defined in paragraph (13) above;
R₃ is as defined in paragraph (15) above;
bond a is as defined in paragraph (17) above;
X is as defined in paragraph (17) above;
Y is as defined in paragraph (17) above;
R₄ is as defined in paragraph (22) above;
Z is as defined in paragraph (25) above; and
R₅ is as defined in paragraph (36) above.

Particular compounds of the present invention include any of the compounds described in the example section of the present application, or a pharmaceutically acceptable pharmaceutically acceptable salt, hydrate or solvate thereof, and, in particular, any of the following:
trans-(6-(3-chloro-4-fluorobenzyl)-5-methyl-2,3-dihydro-1H-pyrrolo[3,2-b]pyridin-1-yl)(3-((dimethylamino)methyl)oxiran-2-yl)methanone;
trans-(6-(3-chloro-4-fluorobenzyl)-5-methyl-2,3-dihydro-1H-pyrrolo[3,2-b]pyridin-1-yl)(3-(methoxymethyl)oxiran-2-yl)methanone;
trans-(6-(3-chloro-4-fluorobenzyl)-5-methyl-2,3-dihydro-1H-pyrrolo[3,2-b]pyridin-1-yl)(3-(fluoromethyl)oxiran-2-yl)methanone;
trans-(6-(3-chloro-4-fluorobenzyl)-5-methyl-2,3-dihydro-1H-pyrrolo[3,2-b]pyridin-1-yl)(3-ethyloxiran-2-yl)methanone;
trans-(6-(3-chloro-4-fluorobenzyl)-5-methyl-2,3-dihydro-1H-pyrrolo[3,2-b]pyridin-1-yl)(3-methyloxiran-2-yl)methanone;
(6-(3-chloro-4-fluorobenzyl)-5-methyl-2,3-dihydro-1H-pyrrolo[3,2-b]pyridin-1-yl)((2R,3S)-3-methyloxiran-2-yl)methanone;
(6-(3-chloro-4-fluorobenzyl)-5-methyl-2,3-dihydro-1H-pyrrolo[3,2-b]pyridin-1-yl)(oxiran-2-yl)methanone;
(R)-(6-(3-chlorobenzyl)-5-methyl-2,3-dihydro-1H-pyrrolo[3,2-b]pyridin-1-yl)(oxiran-2-yl)methanone;
trans-(6-(3-chloro-4-fluorobenzyl)-5-(oxetan-3-yl)-2,3-dihydro-1H-pyrrolo[3,2-b]pyridin-1-yl)(3-methyloxiran-2-yl)methanone;
trans-(6-(3-chloro-4-fluorobenzyl)-2,3-dihydro-1H-pyrrolo[3,2-b]pyridin-1-yl)(3-methyloxiran-2-yl)methanone;
trans-(6-(3-chloro-4-fluorobenzyl)-5-(dimethylamino)-2,3-dihydro-1H-pyrrolo[3,2-b]pyridin-1-yl)(3-methyloxiran-2-yl)methanone;
trans-(6-(3-chloro-4-fluorobenzyl)-5-(isoxazol-5-yl)-2,3-dihydro-1H-pyrrolo[3,2-b]pyridin-1-yl)(3-methyloxiran-2-yl)methanone;
trans-(6-(3-chloro-4-fluorobenzyl)-5-cyclopropyl-2,3-dihydro-1H-pyrrolo[3,2-b]pyridin-1-yl)(3-methyloxiran-2-yl)methanone;
trans-(6-(3-chloro-4-fluorobenzyl)-5-(isoxazol-4-yl)-2,3-dihydro-1H-pyrrolo[3,2-b]pyridin-1-yl)(3-methyloxiran-2-yl)methanone;
trans-(6-(3-chloro-4-fluorobenzyl)-5-(hydroxymethyl)-2,3-dihydro-1H-pyrrolo[3,2-b]pyridin-1-yl)(3-methyloxiran-2-yl)methanone;
trans-(6-(3-chloro-4-fluorobenzyl)-5-ethyl-2,3-dihydro-1H-pyrrolo[3,2-b]pyridin-1-yl)(3-methyloxiran-2-yl)methanone;
(6-(3-chloro-4-fluorobenzyl)-5-methoxy-2,3-dihydro-1H-pyrrolo[3,2-b]pyridin-1-yl)((2R,3S)-3-methyloxiran-2-yl)methanone;
trans-(6-(3-chloro-4-fluorobenzyl)-5-(methyl-d3)-2,3-dihydro-1H-pyrrolo[3,2-b]pyridin-1-yl)(3-methyloxiran-2-yl)methanone;
trans-(5-chloro-6-(3-chloro-4-fluorobenzyl)-2,3-dihydro-1H-pyrrolo[3,2-b]pyridin-1-yl)(3-methyloxiran-2-yl)methanone;
(6-(3-chloro-4-fluorobenzyl)-5-fluoro-2,3-dihydro-1H-pyrrolo[3,2-b]pyridin-1-yl)((2R,3S)-3-methyloxiran-2-yl)methanone;
(5-chloro-6-(3-chloro-4-fluorobenzyl)-2,3-dihydro-1H-pyrrolo[3,2-b]pyridin-1-yl)((2R,3S)-3-methyloxiran-2-yl)methanone;
trans-(6-(4-fluorophenoxy)-5-methyl-2,3-dihydro-1H-pyrrolo[3,2-b]pyridin-1-yl)(3-(2-methoxypropan-2-yl)oxiran-2-yl)methanone;
trans-(6-(3,4-difluorophenoxy)-5-methyl-2,3-dihydro-1H-pyrrolo[3,2-b]pyridin-1-yl)(3-(2-methoxypropan-2-yl)oxiran-2-yl)methanone;
trans-(6-(2,3-difluorophenoxy)-5-methyl-2,3-dihydro-1H-pyrrolo[3,2-b]pyridin-1-yl)(3-(2-methoxypropan-2-yl)oxiran-2-yl)methanone;
trans- (6-(3-fluorophenoxy)-5-methyl-2,3-dihydro-1H-pyrrolo[3,2-b]pyridin-1-yl)(3-(2-(oxetan-3-yloxy)propan-2-yl)oxiran-2-yl)methanone;
trans-(6-(4-fluorophenoxy)-5-methyl-2,3-dihydro-1H-pyrrolo[3,2-b]pyridin-1-yl)(3-(2-fluoropropan-2-yl)oxiran-2-yl)methanone;
trans-(6-(3,4-difluorophenoxy)-5-methyl-2,3-dihydro-1H-pyrrolo[3,2-b]pyridin-1-yl)(3-(oxetan-3-ylmethyl)oxiran-2-yl)methanone;
trans-(6-(2,3-difluorophenoxy)-5-methyl-2,3-dihydro-1H-pyrrolo[3,2-b]pyridin-1-yl)(3-((3-methyloxetan-3-yl)methyl)oxiran-2-yl)methanone;
trans-(6-(3,4-difluorophenoxy)-5-methyl-2,3-dihydro-1H-pyrrolo[3,2-b]pyridin-1-yl)(3-(2-hydroxy-2-methylpropyl)oxiran-2-yl)methanone;
trans-(6-(2,3-difluorophenoxy)-5-methyl-2,3-dihydro-1H-pyrrolo[3,2-b]pyridin-1-yl)(3-(2-hydroxy-2-methylpropyl)oxiran-2-yl)methanone;
trans-(3-(2,2-difluoroethyl)oxiran-2-yl)(6-(4-fluorophenoxy)-5-methyl-2,3-dihydro-1H-pyrrolo[3,2-b]pyridin-1-yl)methanone;
(6-(2,3-difluorophenoxy)-5-methyl-2,3-dihydro-1H-pyrrolo[3,2-b]pyridin-1-yl)((2R,3S)-3-((3-methyloxetan-3-yl)methyl)oxiran-2-yl)methanone;
trans-(6-(3-fluorophenoxy)-5-methyl-2,3-dihydro-1H-pyrrolo[3,2-b]pyridin-1-yl)(3-(2-fluoropropan-2-yl)oxiran-2-yl)methanone;
trans-(6-(3-fluorophenoxy)-5-methyl-2,3-dihydro-1H-pyrrolo[3,2-b]pyridin-1-yl)(3-(2,2,2-trifluoroethyl)oxiran-2-yl)methanone;
trans-(3-(2,2-difluoroethyl)oxiran-2-yl)(6-(3-fluorophenoxy)-5-methyl-2,3-dihydro-1H-pyrrolo[3,2-b]pyridin-1-yl)methanone;
(6-(2,3-difluorophenoxy)-5-methyl-2,3-dihydro-1H-pyrrolo[3,2-b]pyridin-1-yl)((2R,3S)-3-(2-hydroxy-2-methylpropyl)oxiran-2-yl)methanone;
trans-2-(3-(6-(3-fluorophenoxy)-5-methyl-2,3-dihydro-1H-pyrrolo[3,2-b]pyridine-1-carbonyl)oxiran-2-yl)-2-methylpropanenitrile;
trans-2-(3-(6-(2,3-difluorophenoxy)-5-methyl-2,3-dihydro-1H-pyrrolo[3,2-b]pyridine-1-carbonyl)oxiran-2-yl)-2-methylpropanenitrile;
(6-(3,4-difluorophenoxy)-5-methyl-2,3-dihydro-1H-pyrrolo[3,2-b]pyridin-1-yl)((2S,3R)-3-(2-hydroxy-2-methylpropyl)oxiran-2-yl)methanone;
(6-(3-fluorophenoxy)-5-methyl-2,3-dihydro-1H-pyrrolo[3,2-b]pyridin-1-yl)((2R,3S)-3-(oxetan-3-ylmethyl)oxiran-2-yl)methanone;
(6-(3,4-difluorophenoxy)-5-methyl-2,3-dihydro-1H-pyrrolo[3,2-b]pyridin-1-yl)((2R,3S)-3-(oxetan-3-ylmethyl)oxiran-2-yl)methanone;
2-((2S,3R)-3-(6-(3-fluorophenoxy)-5-methyl-2,3-dihydro-1H-pyrrolo[3,2-b]pyridine-1-carbonyl)oxiran-2-yl)-2-methylpropanenitrile;
trans-(3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone;
2-((2S,3R)-3-(6-(2,3-difluorophenoxy)-5-methyl-2,3-dihydro-1H-pyrrolo[3,2-b]pyridine-1-carbonyl)oxiran-2-yl)-2-methylpropanenitrile;
((2R,3R)-3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone;
trans-2-(3-(6-(4-fluorophenoxy)-5-methyl-2,3-dihydro-1H-pyrrolo[3,2-b]pyridine-1-carbonyl)oxiran-2-yl)-2-methylpropanenitrile;
trans-(6-(3-fluorophenoxy)-5-methyl-2,3-dihydro-1H-pyrrolo[3,2-b]pyridin-1-yl)(3-(oxetan-3-ylmethyl)oxiran-2-yl)methanone;
trans-(6-(2,3-difluorophenoxy)-5-methyl-2,3-dihydro-1H-pyrrolo[3,2-b]pyridin-1-yl)(3-(isoxazol-4-yl)oxiran-2-yl)methanone;
trans-(3-(tert-butyl)oxiran-2-yl)(6-(4-fluorophenoxy)-5-methyl-2,3-dihydro-1H-pyrrolo[3,2-b]pyridin-1-yl)methanone;
trans-(6-(3-fluoro-4-morpholinophenoxy)-5-methyl-2,3-dihydro-1H-pyrrolo[3,2-b]pyridin-1-yl)(3-(oxetan-3-yl)oxiran-2-yl)methanone;
trans-(3-(2-(benzyloxy)propan-2-yl)oxiran-2-yl)(6-(3-fluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-(2-hydroxypropan-2-yl)oxiran-2-yl)methanone;
trans-(6-(3-fluorophenoxy)-5-methyl-2,3-dihydro-1H-pyrrolo[3,2-b]pyridin-1-yl)(3-(2-hydroxypropan-2-yl)oxiran-2-yl)methanone;
trans-(6-(3,4-difluorophenoxy)-5-methyl-2,3-dihydro-1H-pyrrolo[3,2-b]pyridin-1-yl)(3-phenyloxiran-2-yl)methanone;
trans-(6-(3-chloro-4-fluorophenoxy)-5-methyl-2,3-dihydro-1H-pyrrolo[3,2-b]pyridin-1-yl)(3-phenyloxiran-2-yl)methanone;
(6-(2,3-difluorophenoxy)-5-methyl-2,3-dihydro-1H-pyrrolo[3,2-b]pyridin-1-yl)((2R,3R)-3-(2-hydroxypropan-2-yl)oxiran-2-yl)methanone;
trans-(6-(3-chloro-4-fluorophenoxy)-5-methyl-2,3-dihydro-1H-pyrrolo[3,2-b]pyridin-1-yl)(3-cyclopropyloxiran-2-yl)methanone;
trans-(6-(4-fluorophenoxy)-5-methyl-2,3-dihydro-1H-pyrrolo[3,2-b]pyridin-1-yl)(3-(1-methyl-1H-pyrazol-3-yl)oxiran-2-yl)methanone;
trans-(6-(2,3-difluorophenoxy)-5-methyl-2,3-dihydro-1H-pyrrolo[3,2-b]pyridin-1-yl)(3-(pyridin-3-yl)oxiran-2-yl)methanone;
trans-(6-(2,3-difluorophenoxy)-5-methyl-2,3-dihydro-1H-pyrrolo[3,2-b]pyridin-1-yl)(3-(pyridin-2-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-3-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-4-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-2-yl)oxiran-2-yl)methanone;
(6-(3-chloro-4-fluorophenoxy)-5-methyl-2,3-dihydro-1H-pyrrolo[3,2-b]pyridin-1-yl)((2S,3R)-3-phenyloxiran-2-yl)methanone;
trans-(6-(3,4-difluorophenoxy)-5-methyl-2,3-dihydro-1H-pyrrolo[3,2-b]pyridin-1-yl)(3-(oxetan-3-yl)oxiran-2-yl)methanone;
trans-(6-(4-fluorophenoxy)-5-methyl-2,3-dihydro-1H-pyrrolo[3,2-b]pyridin-1-yl)(3-(1-methyl-1H-pyrazol-5-yl)oxiran-2-yl)methanone;
trans-(6-(2,3-difluorophenoxy)-5-methyl-2,3-dihydro-1H-pyrrolo[3,2-b]pyridin-1-yl)(3-(oxetan-3-yl)oxiran-2-yl)methanone;
(6-(3,4-difluorophenoxy)-5-methyl-2,3-dihydro-1H-pyrrolo[3,2-b]pyridin-1-yl)((2R,3S)-3-(pyridin-2-yl)oxiran-2-yl)methanone;
trans-(6-(3,4-difluorophenoxy)-5-methyl-2,3-dihydro-1H-pyrrolo[3,2-b]pyridin-1-yl)(3-(isoxazol-5-yl)oxiran-2-yl)methanone;
(6-(2,3-difluorophenoxy)-5-methyl-2,3-dihydro-1H-pyrrolo[3,2-b]pyridin-1-yl)((2R,3S)-3-(oxetan-3-yl)oxiran-2-yl)methanone;
(6-(2,3-difluorophenoxy)-5-methyl-2,3-dihydro-1H-pyrrolo[3,2-b]pyridin-1-yl)((2R,3S)-3-(pyridin-3-yl)oxiran-2-yl)methanone;
(6-(3-chloro-4-fluorophenoxy)-5-methyl-2,3-dihydro-1H-pyrrolo[3,2-b]pyridin-1-yl)((2R,3S)-3-cyclopropyloxiran-2-yl)methanone;
(6-(3,4-difluorophenoxy)-5-methyl-2,3-dihydro-1H-pyrrolo[3,2-b]pyridin-1-yl)(3-(isoxazol-3-yl)oxiran-2-yl)methanone;
trans-(6-(3,4-difluorophenoxy)-5-methyl-2,3-dihydro-1H-pyrrolo[3,2-b]pyridin-1-yl)(3-(isoxazol-5-yl)oxiran-2-yl)methanone;
trans-(6-(2,3-difluorophenoxy)-5-methyl-2,3-dihydro-1H-pyrrolo[3,2-b]pyridin-1-yl)(3-(3-fluoropyridin-2-yl)oxiran-2-yl)methanone;
trans-(6-(2,3-difluorophenoxy)-5-methyl-2,3-dihydro-1H-pyrrolo[3,2-b]pyridin-1-yl)(3-(3-methylpyridin-2-yl)oxiran-2-yl)methanone;
trans-(6-(3-chloro-4-fluorophenoxy)-5-methyl-2,3-dihydro-1H-pyrrolo[3,2-b]pyridin-1-yl)(3-isopropyloxiran-2-yl)methanone;
trans-(3-cyclopropyloxiran-2-yl)(6-(3,4-difluorophenoxy)-5-methyl-2,3-dihydro-1H-pyrrolo[3,2-b]pyridin-1-yl)methanone;
trans-(6-(3-fluoro-2-hydroxyphenoxy)-5-methyl-2,3-dihydro-1H-pyrrolo[3,2-b]pyridin-1-yl)(3-(morpholinomethyl)oxiran-2-yl)methanone;
trans-(6-(3-fluoro-2-methoxyphenoxy)-5-methyl-2,3-dihydro-1H-pyrrolo[3,2-b]pyridin-1-yl)(3-(morpholinomethyl)oxiran-2-yl)methanone;
trans-(3-(azetidin-1-ylmethyl)oxiran-2-yl)(6-(3-fluoro-2-methoxyphenoxy)-5-methyl-2,3-dihydro-1H-pyrrolo[3,2-b]pyridin-1-yl)methanone;
trans-(3-(azetidin-1-ylmethyl)oxiran-2-yl)(6-(3-fluorophenoxy)-5-methyl-2,3-dihydro-1H-pyrrolo[3,2-b]pyridin-1-yl)methanone;
trans-(6-(2,5-difluorophenoxy)-5-methyl-2,3-dihydro-1H-pyrrolo[3,2-b]pyridin-1-yl)(3-(morpholinomethyl)oxiran-2-yl)methanone;
trans-(6-(2-fluorophenoxy)-5-methyl-2,3-dihydro-1H-pyrrolo[3,2-b]pyridin-1-yl)(3-(morpholinomethyl)oxiran-2-yl)methanone;
trans-(6-(2,4-difluorophenoxy)-5-methyl-2,3-dihydro-1H-pyrrolo[3,2-b]pyridin-1-yl)(3-((dimethylamino)methyl)oxiran-2-yl)methanone;
(6-(2-fluorophenoxy)-5-methyl-2,3-dihydro-1H-pyrrolo[3,2-b]pyridin-1-yl)((2R,3S)-3-(morpholinomethyl)oxiran-2-yl)methanone;
trans-(6-(3-fluorophenoxy)-5-methyl-2,3-dihydro-1H-pyrrolo[3,2-b]pyridin-1-yl)(3-((3-methoxyazetidin-1-yl)methyl)oxiran-2-yl)methanone;
trans-(3-((3-fluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(3-((3-fluoroazetidin-1-yl)methyl)oxiran-2-yl)(6-(4-fluorophenoxy)-5-methyl-2,3-dihydro-1H-pyrrolo[3,2-b]pyridin-1-yl)methanone;
trans-(6-(3,4-difluorophenoxy)-5-methyl-2,3-dihydro-1H-pyrrolo[3,2-b]pyridin-1-yl)(3-(morpholinomethyl)oxiran-2-yl)methanone;
trans-(6-(3-fluorophenoxy)-5-methyl-2,3-dihydro-1H-pyrrolo[3,2-b]pyridin-1-yl)(3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-(morpholinomethyl)oxiran-2-yl)methanone;
trans-(3-((dimethylamino)methyl)oxiran-2-yl)(6-(3-fluorophenoxy)-5-methyl-2,3-dihydro-1H-pyrrolo[3,2-b]pyridin-1-yl)methanone;
trans-(3-(azetidin-1-ylmethyl)oxiran-2-yl)(6-(4-fluorophenoxy)-5-methyl-2,3-dihydro-1H-pyrrolo[3,2-b]pyridin-1-yl)methanone;
trans-(3-((2-oxa-6-azaspiro[3.3]heptan-6-yl)methyl)oxiran-2-yl)(6-(3-fluorophenoxy)-5-methyl-2,3-dihydro-1H-pyrrolo[3,2-b]pyridin-1-yl)methanone;
trans-(6-(4-fluorophenoxy)-5-methyl-2,3-dihydro-1H-pyrrolo[3,2-b]pyridin-1-yl)(3-(isopropoxymethyl)oxiran-2-yl)methanone;
(6-(2,3-difluorophenoxy)-5-methyl-2,3-dihydro-1H-pyrrolo[3,2-b]pyridin-1-yl)((2R,3S)-3-(morpholinomethyl)oxiran-2-yl)methanone;
trans-1-((3-(6-(2,3-difluorophenoxy)-5-methyl-2,3-dihydro-1H-pyrrolo[3,2-b]pyridine-1-carbonyl)oxiran-2-yl)methyl)azetidine-3-carbonitrile;
(6-(2,3-difluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone;
trans-(6-(3,4-difluorophenoxy)-5-methyl-2,3-dihydro-1H-pyrrolo[3,2-b]pyridin-1-yl)(3-((dimethylamino)methyl)oxiran-2-yl)methanone;
trans-(3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((2,2-dimethylazetidin-1-yl)methyl)oxiran-2-yl)methanone;
trans-(6-(2,3-difluorophenoxy)-5-methyl-2,3-dihydro-1H-pyrrolo[3,2-b]pyridin-1-yl)(3-((3-fluoroazetidin-1-yl)methyl)oxiran-2-yl)methanone;
trans-(3-((2-oxa-6-azaspiro[3.3]heptan-6-yl)methyl)oxiran-2-yl)(6-(3,4-difluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((3-hydroxyazetidin-1-yl)methyl)oxiran-2-yl)methanone;
trans-(3-((2-oxa-6-azaspiro[3.3]heptan-6-yl)methyl)oxiran-2-yl)(6-(2,3-difluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((dimethylamino)methyl)oxiran-2-yl)methanone;
(6-(3,4-difluorophenoxy)-5-methyl-2,3-dihydro-1H-pyrrolo[3,2-b]pyridin-1-yl)((2R,3S)-3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-((3-methoxyazetidin-1-yl)methyl)oxiran-2-yl)methanone;
(6-(3-fluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone;
((2R,3S)-3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(6-(3-chlorophenoxy)-5-methyl-2,3-dihydro-1H-pyrrolo[3,2-b]pyridin-1-yl)(3-(morpholinomethyl)oxiran-2-yl)methanone;
(6-(2,3-difluorophenoxy)-5-methyl-2,3-dihydro-1H-pyrrolo[3,2-b]pyridin-1-yl)((2R,3S)-3-((3-fluoroazetidin-1-yl)methyl)oxiran-2-yl)methanone;
trans-(6-(3-chloro-4-fluorophenoxy)-5-methyl-2,3-dihydro-1H-pyrrolo[3,2-b]pyridin-1-yl)(3-(morpholinomethyl)oxiran-2-yl)methanone;
(6-(2,3-difluorophenoxy)-5-methyl-2,3-dihydro-1H-pyrrolo[3,2-b]pyridin-1-yl)((2R,3S)-3-((3-hydroxyazetidin-1-yl)methyl)oxiran-2-yl)methanone;
trans-(3-(azetidin-1-ylmethyl)oxiran-2-yl)(6-(2,3-difluorophenoxy)-5-methyl-2,3-dihydro-1H-pyrrolo[3,2-b]pyridin-1-yl)methanone;
trans-(3-(azetidin-1-ylmethyl)oxiran-2-yl)(6-(3,4-difluorophenoxy)-5-methyl-2,3-dihydro-1H-pyrrolo[3,2-b]pyridin-1-yl)methanone;
trans-(6-(3,4-difluorophenoxy)-5-methyl-2,3-dihydro-1H-pyrrolo[3,2-b]pyridin-1-yl)(3-(methoxymethyl)oxiran-2-yl)methanone;
trans-(6-(3-fluorophenoxy)-5-methyl-2,3-dihydro-1H-pyrrolo[3,2-b]pyridin-1-yl)(3-(isopropoxymethyl)oxiran-2-yl)methanone;
((2R,3S)-3-(azetidin-1-ylmethyl)oxiran-2-yl)(6-(2,3-difluorophenoxy)-5-methyl-2,3-dihydro-1H-pyrrolo[3,2-b]pyridin-1-yl)methanone;
((2R,3S)-3-((2-oxa-6-azaspiro[3.3]heptan-6-yl)methyl)oxiran-2-yl)(6-(2,3-difluorophenoxy)-5-methyl-2,3-dihydro-1H-pyrrolo[3,2-b]pyridin-1-yl)methanone;
trans-(6-(3-chlorophenoxy)-5-methyl-2,3-dihydro-1H-pyrrolo[3,2-b]pyridin-1-yl)(3-((dimethylamino)methyl)oxiran-2-yl)methanone;
(6-(2,3-difluorophenoxy)-5-methyl-2,3-dihydro-1H-pyrrolo[3,2-b]pyridin-1-yl)((2R,3S)-3-((2,2-dimethylazetidin-1-yl)methyl)oxiran-2-yl)methanone;
(6-(2,3-difluorophenoxy)-5-methyl-2,3-dihydro-1H-pyrrolo[3,2-b]pyridin-1-yl)((2R,3S)-3-((dimethylamino)methyl)oxiran-2-yl)methanone;
trans-(6-(3-chloro-4-fluorophenoxy)-3,3,5-trimethyl-2,3-dihydro-1H-pyrrolo[3,2-b]pyridin-1-yl)(3-((dimethylamino)methyl)oxiran-2-yl)methanone;
((2R,3S)-3-((dimethylamino)methyl)oxiran-2-yl)(6-(3-fluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-((dimethylamino)methyl)oxiran-2-yl)methanone;
(6-(3,4-difluorophenoxy)-5-methyl-2,3-dihydro-1H-pyrrolo[3,2-b]pyridin-1-yl)((2R,3S)-3-((dimethylamino)methyl)oxiran-2-yl)methanone;
((2R,3S)-3-(azetidin-1-ylmethyl)oxiran-2-yl)(6-(3,4-difluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-(methoxymethyl)oxiran-2-yl)methanone;
(6-(3-chloro-4-fluorophenoxy)-3,3,5-trimethyl-2,3-dihydro-1H-pyrrolo[3,2-b]pyridin-1-yl)((2R,3S)-3-((dimethylamino)methyl)oxiran-2-yl)methanone;
(6-(3-chlorophenoxy)-5-methyl-2,3-dihydro-1H-pyrrolo[3,2-b]pyridin-1-yl)((2R,3S)-3-(morpholinomethyl)oxiran-2-yl)methanone;
(6-(3-chloro-4-fluorophenoxy)-5-methyl-2,3-dihydro-1H-pyrrolo[3,2-b]pyridin-1-yl)((2R,3S)-3-(morpholinomethyl)oxiran-2-yl)methanone;
trans-(3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone;
((2R,3S)-3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-(fluoromethyl)oxiran-2-yl)methanone;
(6-(3-chloro-4-fluorophenoxy)-5-methyl-2,3-dihydro-1H-pyrrolo[3,2-b]pyridin-1-yl)((2S,3R)-3-((dimethylamino)methyl)oxiran-2-yl)methanone;
(6-(3-chloro-4-fluorophenoxy)-5-methyl-2,3-dihydro-1H-pyrrolo[3,2-b]pyridin-1-yl)((2R,3R)-3-(fluoromethyl)oxiran-2-yl)methanone;
trans-1-((3-(6-(2,3-difluorophenoxy)-5-methyl-2,3-dihydro-1H-pyrrolo[3,2-b]pyridine-1-carbonyl)oxiran-2-yl)methyl)-3-methylazetidine-3-carbonitrile;
trans-(6-(2,6-difluorophenoxy)-5-methyl-2,3-dihydro-1H-pyrrolo[3,2-b]pyridin-1-yl)(3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-((3,3-dimethylmorpholino)methyl)oxiran-2-yl)methanone;
trans-(3-((7-oxa-4-azaspiro[2.5]octan-4-yl)methyl)oxiran-2-yl)(6-(2,3-difluorophenoxy)-5-methyl-2,3-dihydro-1H-pyrrolo[3,2-b]pyridin-1-yl)methanone;
3-((5-methyl-1-((2R,3S)-3-methyloxirane-2-carbonyl)-2,3-dihydro-1H-pyrrolo[3,2-b]pyridin-6-yl)oxy)benzonitrile;
(6-(benzo[d][1,3]dioxol-5-yloxy)-5-methyl-2,3-dihydro-1H-pyrrolo[3,2-b]pyridin-1-yl)((2R,3S)-3-methyloxiran-2-yl)methanone;
(6-(4-fluoro-3-hydroxyphenoxy)-5-methyl-2,3-dihydro-1H-pyrrolo[3,2-b]pyridin-1-yl)((2R,3S)-3-methyloxiran-2-yl)methanone;
(6-(3-fluoro-4-hydroxyphenoxy)-5-methyl-2,3-dihydro-1H-pyrrolo[3,2-b]pyridin-1-yl)((2R,3S)-3-methyloxiran-2-yl)methanone;
trans-(6-(3-chloro-4-fluorophenoxy)-5-methyl-2,3-dihydro-1H-pyrrolo[3,2-b]pyridin-1-yl)(3-methyloxiran-2-yl)methanone;
(6-(3-chloro-4-fluorophenoxy)-5-methyl-2,3-dihydro-1H-pyrrolo[3,2-b]pyridin-1-yl)((2R,3S)-3-methyloxiran-2-yl)methanone;
(6-(4-fluorophenoxy)-5-methyl-2,3-dihydro-1H-pyrrolo[3,2-b]pyridin-1-yl)((2R,3S)-3-methyloxiran-2-yl)methanone;
(6-(3,4-difluorophenoxy)-5-methyl-2,3-dihydro-1H-pyrrolo[3,2-b]pyridin-1-yl)((2R,3S)-3-methyloxiran-2-yl)methanone;
trans-(6-(3-chloro-4-fluorophenoxy)-5-methyl-2,3-dihydro-1H-pyrrolo[3,2-b]pyridin-1-yl)(3-ethyloxiran-2-yl)methanone;
(6-(3-chloro-4-fluorophenoxy)-5-methyl-2,3-dihydro-1H-pyrrolo[3,2-b]pyridin-1-yl)((2R,3S)-3-ethyloxiran-2-yl)methanone;
trans-3-((1-(3-(difluoromethyl)oxirane-2-carbonyl)-5-methyl-2,3-dihydro-1H-pyrrolo[3,2-b]pyridin-6-yl)oxy)benzonitrile;
trans-(3-(difluoromethyl)oxiran-2-yl)(6-(3-fluorophenoxy)-5-methyl-2,3-dihydro-1H-pyrrolo[3,2-b]pyridin-1-yl)methanone;
((2R,3R)-3-(difluoromethyl)oxiran-2-yl)(6-(3-fluorophenoxy)-5-methyl-2,3-dihydro-1H-pyrrolo[3,2-b]pyridin-1-yl)methanone;
trans-(3-(difluoromethyl)oxiran-2-yl)(6-(3,4-difluorophenoxy)-5-methyl-2,3-dihydro-1H-pyrrolo[3,2-b]pyridin-1-yl)methanone;
trans-(3-(difluoromethyl)oxiran-2-yl)(6-(4-fluorophenoxy)-5-methyl-2,3-dihydro-1H-pyrrolo[3,2-b]pyridin-1-yl)methanone;
((2R,3R)-3-(difluoromethyl)oxiran-2-yl)(6-(3,4-difluorophenoxy)-5-methyl-2,3-dihydro-1H-pyrrolo[3,2-b]pyridin-1-yl)methanone;
trans-3-(6-(2,3-difluorophenoxy)-5-methyl-2,3-dihydro-1H-pyrrolo[3,2-b]pyridine-1-carbonyl)-N,N-dimethyloxirane-2-carboxamide;
trans-3-(6-(3,4-difluorophenoxy)-5-methyl-2,3-dihydro-1H-pyrrolo[3,2-b]pyridine-1-carbonyl)-N,N-dimethyloxirane-2-carboxamide;
(2R,3R)-3-(6-(3,4-difluorophenoxy)-5-methyl-2,3-dihydro-1H-pyrrolo[3,2-b]pyridine-1-carbonyl)-N,N-dimethyloxirane-2-carboxamide;
trans-3-(6-(3-fluorophenoxy)-5-methyl-2,3-dihydro-1H-pyrrolo[3,2-b]pyridine-1-carbonyl)-N,N-dimethyloxirane-2-carboxamide;
(2S,3S)-3-(6-(3-fluorophenoxy)-5-methyl-2,3-dihydro-1H-pyrrolo[3,2-b]pyridine-1-carbonyl)-N,N-dimethyloxirane-2-carboxamide;
trans-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide;
(2R,3R)-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide;
trans-(6-((3,4-difluorophenyl)amino)-5-methyl-2,3-dihydro-1H-pyrrolo[3,2-b]pyridin-1-yl)(3-(oxetan-3-ylmethyl)oxiran-2-yl)methanone;
trans-(6-((3-chloro-4-fluorophenyl)(methyl)amino)-5-methyl-2,3-dihydro-1H-pyrrolo[3,2-b]pyridin-1-yl)(3-methyloxiran-2-yl)methanone;
trans-(6-((3-chloro-4-fluorophenyl)amino)-5-methyl-2,3-dihydro-1H-pyrrolo[3,2-b]pyridin-1-yl)(3-methyloxiran-2-yl)methanone;
(6-((3-chloro-4-fluorophenyl)amino)-5-methyl-2,3-dihydro-1H-pyrrolo[3,2-b]pyridin-1-yl)((2R,3S)-3-methyloxiran-2-yl)methanone;
(6-(3-chloro-4-fluorophenoxy)-5-methoxy-2,3-dihydro-1H-pyrrolo[3,2-b]pyridin-1-yl)((2R,3S)-3-methyloxiran-2-yl)methanone;
(6-(3-chloro-4-fluorophenoxy)-5-fluoro-2,3-dihydro-1H-pyrrolo[3,2-b]pyridin-1-yl)((2R,3S)-3-methyloxiran-2-yl)methanone;
(5-chloro-6-(3-chloro-4-fluorophenoxy)-2,3-dihydro-1H-pyrrolo[3,2-b]pyridin-1-yl)((2R,3S)-3-methyloxiran-2-yl)methanone;
trans-(6-(3-chloro-4-fluorobenzyl)-3,3,5-trimethyl-2,3-dihydro-1H-pyrrolo[3,2-b]pyridin-1-yl)( 3-methyloxiran-2-yl)methanone;
(6-(3-chloro-4-fluorobenzyl)-3,3,5-trimethyl-2,3-dihydro-1H-pyrrolo[3,2-b]pyridin-1-yl)((2R,3S)-3-methyloxiran-2-yl)methanone;
trans-(6-(3-chloro-4-fluorobenzyl)-3,3-dimethyl-2,3-dihydro-1H-pyrrolo[3,2-b]pyridin-1-yl)( 3-methyloxiran-2-yl)methanone;
(6-(3-chloro-4-fluorobenzyl)-3,3-dimethyl-2,3-dihydro-1H-pyrrolo[3,2-b]pyridin-1-yl)(oxiran-2-yl)methanone;
trans-(6'-(3-chloro-4-fluorobenzyl)-5'-methylspiro[cyclopropane-1,3'-pyrrolo[3,2-b]pyridin]-1'(2'H)-yl)(3-methyloxiran-2-yl)methanone;
trans-(6-(3-fluorophenoxy)-2,2,5-trimethyl-2,3-dihydro-1H-pyrrolo[3,2-b]pyridin-1-yl)(3-methyloxiran-2-yl)methanone;
trans-(6-(3-chloro-4-fluorophenoxy)-5-methyl-1H-pyrrolo[3,2-b]pyridin-1-yl)(3-methyloxiran-2-yl)methanone;
(6-(3-chloro-4-fluorophenoxy)-5-methyl-1H-pyrrolo[3,2-b]pyridin-1-yl)((2R,3S)-3-methyloxiran-2-yl)methanone;
(6-(3-chloro-4-fluorophenoxy)-3,3,5-trimethyl-2,3-dihydro-1H-pyrrolo[3,2-b]pyridin-1-yl)((2R,3S)-3-methyloxiran-2-yl)methanone; or
(6'-(4-fluorophenoxy)-5'-methylspiro[cyclopropane-1,3'-pyrrolo[3,2-b]pyridin]-1'(2'H)-yl)((2R,3S)-3-methyloxiran-2-yl)methanone.

The various functional groups and substituents making up the compounds of the formula (I) are typically chosen such that the molecular weight of the compound of the formula (I) does not exceed 1000. More usually, the molecular weight of the compound will be less than 900, for example less than 800, or less than 750, or less than 700, or less than 650. More preferably, the molecular weight is less than 600 and, for example, is 550 or less.

A suitable pharmaceutically acceptable salt of a compound of the invention is, for example, an acidaddition salt of a compound of the invention which is sufficiently basic, for example, an acidaddition salt with, for example, an inorganic or organic acid, for example hydrochloric, hydrobromic, sulfuric, phosphoric, trifluoroacetic, formic, citric methane sulfonate or maleic acid. In addition, a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a pharmaceutically acceptable cation, for example a salt with methylamine, dimethylamine, trimethylamine, piperidine, morpholine or tris(2hydroxyethyl)amine.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are nonsuperimposable mirror images of each other are termed "enantiomers". When a compound has an asymmetric center-, for example, it is bonded to four different groups, a pair of enantiomers is possible. An enantiomer can be characterized by the absolute configuration of its asymmetric center and is described by the R and S sequencing rules of Cahn and Prelog, or by the manner in which the molecule rotates the plane of polarized light and designated as dextrorotatory or -levorotatory- (i.e., as (+) or (-)-isomers respectively). A chiral compound can exist as either individual enantiomer or as a mixture thereof. A mixture containing equal proportions of the enantiomers is called a "racemic mixture".

The compounds of this invention may possess one or more asymmetric centers; such compounds can therefore be produced as individual (R) or (S)stereoisomers or as mixtures thereof. Unless indicated otherwise, the description or naming of a particular compound in the specification and claims is intended to include both individual enantiomers and mixtures, racemic or otherwise, thereof. The methods for the determination of stereochemistry and the separation of stereoisomers are wellknown in the art (see discussion in Chapter 4 of "Advanced Organic Chemistry", 4th edition J. March, John Wiley and Sons, New York, 2001), for example by synthesis from optically active starting materials or by resolution of a racemic form. Some of the compounds of the invention may have geometric isomeric centres (E and Z isomers). It is to be understood that the present invention encompasses all optical, diastereoisomers and geometric isomers and mixtures thereof that possess activity against MutSβ.

The present invention also encompasses compounds of the invention as defined herein which comprise one or more isotopic substitutions. For Example, H may be in any isotopic form, including ¹H, ²H(D), and ³H (T); C may be in any isotopic form, including ¹²C, ¹³C, and ¹⁴C; and O may be in any isotopic form, including ¹⁶O and ¹⁸O; and the like.

It is also to be understood that certain compounds of the formula (I) may exist in solvated as well as unsolvated forms such as, for example, hydrated forms. It is to be understood that the invention encompasses all such solvated forms that possess activity against MutSβ.

It is also to be understood that certain compounds of the formula I may exhibit polymorphism, and that the invention encompasses all such forms that possess activity against MutSβ.

Compounds of the formula I may exist in a number of different tautomeric forms and references to compounds of the formula I include all such forms. For the avoidance of doubt, where a compound can exist in one of several tautomeric forms, and only one is specifically described or shown, all others are nevertheless embraced by formula I. Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

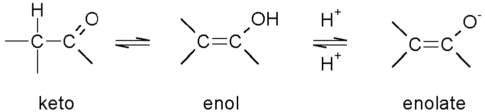

Compounds of the formula I containing an amine function may also form N-oxides. A reference herein to a compound of the formula I that contains an amine function also includes the N-oxide. Where a compound contains several amine functions, one or more than one nitrogen atom may be oxidised to form an N-oxide. Particular Examples of N-oxides are the N-oxides of a *tertiary* amine or a nitrogen atom of a nitrogen-containing heterocycle. N-Oxides can be formed by treatment of the corresponding amine with an oxidizing agent such as hydrogen peroxide or a per-acid (e.g. a peroxycarboxylic acid), see for example Advanced Organic Chemistry, by Jerry March, 4th Edition, Wiley Interscience, pages. More particularly, N-oxides can be made by the procedure of L. W. Deady (Syn. Comm. 1977, 7, 509-514) in which the amine compound is reacted with m-chloroperoxybenzoic acid (mCPBA), for example, in an inert solvent such as dichloromethane.

The compounds of formula (I) may be administered in the form of a prodrug which is broken down in the human or animal body to release a compound of the invention. A pro-drug may be used to alter the physical properties and/or the pharmacokinetic properties of a compound of the invention. A pro-drug can be formed when the compound of the invention contains a suitable group or substituent to which a property-modifying group can be attached. Examples of pro-drugs include *in vivo* cleavable ester derivatives that may be formed at a carboxy group or a hydroxy group in a compound of the formula (I) and in-vivo cleavable amide derivatives that may be formed at a carboxy group or an amino group in a compound of the formula (I).

Accordingly, the present invention includes those compounds of the formula (I) as defined hereinbefore when made available by organic synthesis and when made available within the human or animal body by way of cleavage of a pro-drug thereof. Accordingly, the present invention includes those compounds of the formula I that are produced by organic synthetic means and also such compounds that are produced in the human or animal body by way of metabolism of a precursor compound, that is a compound of the formula (I) may be a synthetically-produced compound or a metabolically-produced compound.

A suitable pharmaceutically acceptable pro-drug of a compound of the formula (I) is one that is based on reasonable medical judgement as being suitable for administration to the human or animal body without undesirable pharmacological activities and without undue toxicity.

Various forms of pro-drug have been described, for example in the following documents :-
a) Methods in Enzymology, Vol. 42, p. 309-396, edited by K. Widder, et al. (Academic Press, 1985);
b) Design of Pro-drugs, edited by H. Bundgaard, (Elsevier, 1985);
c) A Textbook of Drug Design and Development, edited by Krogsgaard-Larsen and H. Bundgaard, Chapter 5 "Design and Application of Pro-drugs", by H. Bundgaard p. 113-191 (1991);
d) H. Bundgaard, Advanced Drug Delivery Reviews, 8, 1-38 (1992);
e) H. Bundgaard, et al., Journal of Pharmaceutical Sciences, 77, 285 (1988);
f) N. Kakeya, et al., Chem. Pharm. Bull., 32, 692 (1984);
g) T. Higuchi and V. Stella, "Pro-Drugs as Novel Delivery Systems", A.C.S. Symposium Series, Volume 14; and
h) E. Roche (editor), "Bioreversible Carriers in Drug Design", Pergamon Press, 1987.

A suitable pharmaceutically acceptable pro-drug of a compound of the formula I that possesses a carboxy group is, for example, an *in vivo* cleavable ester thereof. An *in vivo* cleavable ester of a compound of the formula I containing a carboxy group is, for example, a pharmaceutically acceptable ester which is cleaved in the human or animal body to produce the parent acid. Suitable pharmaceutically acceptable esters for carboxy include C1-6alkyl esters such as methyl, ethyl and *tert-*butyl, C1-6alkoxymethyl esters such as methoxymethyl esters, C1-6alkanoyloxymethyl esters such as pivaloyloxymethyl esters, 3-phthalidyl esters, C3-8cycloalkylcarbonyloxy- C1-6alkyl esters such as cyclopentylcarbonyloxymethyl and 1-cyclohexylcarbonyloxyethyl esters, 2-oxo-1,3-dioxolenylmethyl esters such as 5-methyl-2-oxo-1,3-dioxolen-4-ylmethyl esters and C1-6alkoxycarbonyloxy- C1-6alkyl esters such as methoxycarbonyloxymethyl and 1-methoxycarbonyloxyethyl esters.

A suitable pharmaceutically acceptable pro-drug of a compound of the Formula (I) that possesses a hydroxy group is, for example, an *in vivo* cleavable ester or ether thereof. An *in vivo* cleavable ester or ether of a compound of the formula I containing a hydroxy group is, for example, a pharmaceutically acceptable ester or ether which is cleaved in the human or animal body to produce the parent hydroxy compound. Suitable pharmaceutically acceptable ester forming groups for a hydroxy group include inorganic esters such as phosphate esters (including phosphoramidic cyclic esters). Further suitable pharmaceutically acceptable ester forming groups for a hydroxy group include C1-10alkanoyl groups such as acetyl, benzoyl, phenylacetyl and substituted benzoyl and phenylacetyl groups, C1-10alkoxycarbonyl groups such as ethoxycarbonyl, N,N -(C1-6)2carbamoyl, 2-dialkylaminoacetyl and 2-carboxyacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl. Suitable pharmaceutically acceptable ether forming groups for a hydroxy group include a-acyloxyalkyl groups such as acetoxymethyl and pivaloyloxymethyl groups.

A suitable pharmaceutically acceptable pro-drug of a compound of the formula (I) that possesses a carboxy group is, for example, an *in vivo* cleavable amide thereof, for example an amide formed with an amine such as ammonia, a C1-4alkylamine such as methylamine, a (C1-4alkyl)2amine such as dimethylamine, N-ethyl-N-methylamine or diethylamine, a C1-4alkoxy- C2-4alkylamine such as 2-methoxyethylamine, a phenyl-C1-4alkylamine such as benzylamine and amino acids such as glycine or an ester thereof.

A suitable pharmaceutically acceptable pro-drug of a compound of the formula I that possesses an amino group is, for example, an *in vivo* cleavable amide derivative thereof. Suitable pharmaceutically acceptable amides from an amino group include, for example an amide formed with C1-10alkanoyl groups such as an acetyl, benzoyl, phenylacetyl and substituted benzoyl and phenylacetyl groups. Examples of ring substituents on the phenylacetyl and benzoyl groups include aminomethyl, N-alkylaminomethyl, N,N-dialkylaminomethyl, morpholinomethyl, piperazin-1-ylmethyl and 4-(C1-4alkyl)piperazin-1-ylmethyl.

The *in vivo* effects of a compound of the formula (I) may be exerted in part by one or more metabolites that are formed within the human or animal body after administration of a compound of the formula (I). As stated hereinbefore, the *in vivo* effects of a compound of the formula (I) may also be exerted by way of metabolism of a precursor compound (a pro-drug. Though the present invention may relate to any compound or particular group of compounds defined herein by way of optional, preferred or suitable features or otherwise in terms of particular embodiments, the present invention may also relate to any compound or particular group of compounds that specifically excludes said optional, preferred or suitable features or particular embodiments.

Suitably, the present invention excludes any individual compounds not possessing the biological activity defined herein.

### Synthesis

The compounds of the present invention can be prepared by any suitable technique known in the art. Particular processes for the preparation of the compounds of the invention are described in the Example section below.

In the description of the synthetic methods described herein and in any referenced synthetic methods that are used to prepare the starting materials, it is to be understood that all proposed reaction conditions, including choice of solvent, reaction atmosphere, reaction temperature, duration of the experiment and workup procedures, can be selected by a person skilled in the art.

It is understood by one skilled in the art of organic synthesis that the functionality present on various portions of the molecule must be compatible with the reagents and reaction conditions utilised.

It will be appreciated that during the synthesis of the compounds of the invention in the processes defined herein, or during the synthesis of certain starting materials, it may be desirable to protect certain substituent groups to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons). Protecting groups may be removed by any convenient method described in the literature or known to the skilled chemist as appropriate for the removal of the protecting group in question, such methods being chosen so as to effect removal of the protecting group with the minimum disturbance of groups elsewhere in the molecule.

Thus, if reactants include, for example, groups such as amino, carboxy or hydroxy it may be desirable to protect the group in some of the reactions mentioned herein.

By way of example, a suitable protecting group for an amino or alkylamino group is, for example, an acyl group, for example an alkanoyl group such as acetyl, an alkoxycarbonyl group, for example a methoxycarbonyl, ethoxycarbonyl or tbutoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed by, for example, hydrolysis with a suitable base such as an alkali metal hydroxide, for example lithium or sodium hydroxide. Alternatively an acyl group such as a tertbutoxycarbonyl group may be removed, for example, by treatment with a suitable acid as hydrochloric, sulfuric or phosphoric acid or trifluoroacetic acid and an arylmethoxycarbonyl group such as a benzyloxycarbonyl group may be removed, for example, by hydrogenation over a catalyst such as palladiumoncarbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium, sodium hydroxide or ammonia. Alternatively, an arylmethyl group such as a benzyl group may be removed, for example, by hydrogenation over a catalyst such as palladiumoncarbon.

A suitable protecting group for a carboxy group is, for example, an esterifying group, for example a methyl or an ethyl group which may be removed, for example, by hydrolysis with a base such as sodium hydroxide, or for example a tbutyl group which may be removed, for example, by treatment with an acid, for example an organic acid such as trifluoroacetic acid, or for example a benzyl group which may be removed, for example, by hydrogenation over a catalyst such as palladiumoncarbon.

Resins may also be used as a protecting group.

The methodology employed to synthesise a compound of formula (I) will vary depending on the nature of R₁, R₂, R₃, R₄, R₅, X, Y, Z and bond a and any substituent groups associated therewith. Suitable processes for their preparation are described further in the accompanying Examples.

Once a compound of formula (I) has been synthesised by any one of the processes defined herein, the processes may then further comprise one or more of the additional steps of:
(i) removing any protecting groups present;
(ii) converting the compound formula (I) into another compound of formula (I);
(iii) forming a pharmaceutically acceptable salt, hydrate or solvate of the compound of formula I; and/or
(iv) forming a prodrug of the compound of formula I.

An example of (ii) above is when a compound of formula (I) is synthesised and then one or more of the groups of R₁, R₂, R₃, R₄, R₅, X, Y, Z and bond a may be further reacted to change the nature of the group and provide an alternative compound of formula (I).

The resultant compounds of formula (I) can be isolated and purified using techniques well known in the art.

### Biological Activity

The biological assay described in the example sectionmay be used to measure the pharmacological effects of the compounds of the present invention.

Although the pharmacological properties of the compounds of formula I vary with structural change, as expected, the compounds of the invention were found to be active in the assay described in the Biological Examples.

In general, in terms of MutSβ inhibitory activity, preferred compounds of the invention demonstrate an IC₅₀ of 10 µM or less in the assay described in Biological Example A, with more preferred compounds of the invention demonstrating an IC₅₀ of 1 µM or less and the most preferred compounds of the invention demonstrating an IC₅₀ of 0.5 µM or less.

In general, in terms of activity, preferred compounds of the invention demonstrate a minimum effective concentration of 1 µM or less in the assay described in Biological Example B, with more preferred compounds of the invention demonstrating a minimum effective concentration of 0.5 µM or less and the most preferred compounds of the invention demonstrating a minimum effective concentration of 0.1 µM or less.

### Pharmaceutical Compositions

According to a further aspect of the invention there is provided a pharmaceutical composition which comprises a compound of the invention as defined hereinbefore, or a pharmaceutically acceptable salt, hydrate or solvate thereof, in association with a pharmaceutically acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular, intraperitoneal or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

An effective amount of a compound of the present invention for use in therapy is an amount sufficient to treat or prevent a proliferative condition referred to herein, slow its progression and/or reduce the symptoms associated with the condition.

The amount of active ingredient that is combined with one or more excipients to produce a single dosage form will necessarily vary depending upon the individual treated and the particular route of administration. For Example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the formula I will naturally vary according to the nature and severity of the conditions, the age and sex of the animal or patient and the route of administration, according to well-known principles of medicine.

In using a compound of the invention for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a compound of this invention.

### Therapeutic Uses and Applications

According to a further aspect of the present invention, there is provided a compound, or a pharmaceutically acceptable salt, hydrate or solvate thereof, or a pharmaceutical composition as defined herein, for use in therapy.

According to a further aspect of the present invention, there is provided a compound, or a pharmaceutically acceptable salt, hydrate or solvate thereof, or a pharmaceutical composition as defined herein, for use in the treatment of any of the diseases discussed herein.

The present invention provides compounds that function as MutSβ inhibitors. Thus, the compounds of the invention can be used to inhibit somatic expansion of simple nucleotide repeats associated with MutSβ activity. The compounds of the present invention therefore have particular application in treating, preventing, or delaying the onset of a repeat expansion disease or disorder in a subject.

MutSβ activity is implicated in the somatic instability of expanded simple nucleotide repeats, which leads to further expansion. The inhibition of MutSβ activity may therefore allow the treatment of diseases related to somatic expansion of expanded simple nucleotide repeats, such as repeat expansion diseases or disorders. Thus, by virtue of their activity against MutSβ, the compounds of the present invention may be used as an agent to inhibit somatic expansion in the treatment, prevention, or delaying of the onset of repeat expansion diseases or disorders.

In the present invention, it will be understood that reference to inhibition of somatic expansion encompasses reducing or preventing somatic instability of expanded simple nucleotide repeats, and also encompasses the slowing or stopping of somatic expansion of expanded simple nucleotide repeats.

According to a further aspect of the present invention, there is provided a method of inhibiting MutSβ activity *in vitro, ex vivo* or *in vivo,* said method comprising contacting a cell with an effective amount of a compound or a pharmaceutically acceptable salt, hydrate or solvate thereof as defined herein. Suitably, the method comprises the selective inhibition of MutSβ activity *in vitro, ex vivo* or *in vivo.*

According to a further aspect of the present invention, there is provided a compound, or a pharmaceutically acceptable salt, hydrate or solvate thereof, as defined herein for use in the inhibition of MutSβ activity, e.g. in cellular systems. The inhibition may be the selective inhibition of MutSβ activity.

According to a further aspect of the present invention, there is provided a use of a compound, or a pharmaceutically acceptable salt, hydrate or solvate thereof, as defined herein in the manufacture of a medicament for the inhibition of MutSβ activity, e.g. in cellular systems.

According to a further aspect of the present invention, there is provided a method of inhibiting somatic expansion of expanded simple nucleotide repeats, said method comprising administering to said subject a therapeutically effective amount of a compound or a pharmaceutically acceptable salt, hydrate or solvate thereof as defined herein, or a pharmaceutical composition as defined herein. The method may be performed in a subject or in a cellular assay system.

According to a further aspect of the present invention, there is provided a compound, or a pharmaceutically acceptable salt, hydrate or solvate thereof, or a pharmaceutical composition as defined herein for use in inhibiting somatic expansion of expanded simple nucleotide repeats in a subject (e.g. in the treatment of a repeat expansion disease or disorder).

According to a further aspect of the present invention, there is provide thed use of a compound, or a pharmaceutically acceptable salt, hydrate or solvate thereof, as defined herein in the manufacture of a medicament for inhibiting somatic expansion of expanded simple nucleotide repeats in a subject (e.g. in the treatment of a repeat expansion disease or disorder).

According to a further aspect of the present invention, there is provided the use of a compound, or a pharmaceutically acceptable salt, hydrate or solvate thereof in inhibiting somatic expansion of expanded simple nucleotide repeats in a cellular assay system.

According to a further aspect of the present invention, there is provided a method of treating a disease or condition in which MutSβ activity is implicated in a patient in need of such treatment, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a compound, or a pharmaceutically acceptable salt, hydrate or solvate thereof, as defined herein for use in the treatment of a disease or condition in which MutSβ activity is implicated.

According to a further aspect of the present invention, there is provided a use of a compound, or a pharmaceutically acceptable salt, hydrate or solvate thereof, as defined herein in the manufacture of a medicament for the treatment of a disease or condition in which MutSβ activity is implicated.

The disease or condition in which MutSβ activity is implicated may be a disease related to somatic expansion of expanded simple nucleotide repeats, such as a repeat expansion disease or disorder.

According to a further aspect of the present invention, there is provided a method of treating a repeat expansion disease or disorder in a patient, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a compound, or a pharmaceutically acceptable salt, hydrate or solvate thereof, or a pharmaceutical composition as defined herein for use in the treatment of a repeat expansion disease or disorder.

According to a further aspect of the present invention, there is provided the use of a compound, or a pharmaceutically acceptable salt, hydrate or solvate thereof, as defined herein in the manufacture of a medicament for the treatment of a repeat expansion disease or disorder.

Repeat expansion diseases (REDs) are caused by pathogenic expansions of simple nucleotide repeats within coding and non-coding regions of different genes. Repeat expansion diseases include but are not limited to: benign adult familial myoclonic epilepsy (BAFME); brachydactyly and cleidocranial dysplasia (BCCD); blepharophimosis, ptosis and epicanthus inversus (BPES); Baratela-Scott syndrome (BSS); cerebellar ataxia, neuropathy and vestibular areflexia syndrome (CANVAS); congenital central hypoventilation syndrome (CCHS); dentatorubropallidoluysian atrophy (DRPLA); early infantile epileptic encephalopathy type 1 (EIEE1); familial adult myoclonic epilepsy (FAME); Fuch's endothelial corneal dystrophy type 3 (FECD3); Dentatorubral pallidoluysian atrophy; fragile XE syndrome (FRAXE);; Friedreich ataxia (FRDA) frontotemporal dementia/amyotrophic lateral sclerosis (FTD/ALS); fragile X-associated premature ovarian infertility (FXPOI); fragile X syndrome (FXS); fragile X-associated tremor ataxia syndrome (FXTAS); global development delay, progressive ataxia and elevated glutamine (GDPAG); Huntington's disease (HD); Huntington disease-like 2 (HDL2); hand-foot-genital syndrome (HFGS); holoprosencephaly type 5 (HPE5); mental retardation with isolated growth hormone deficiency (MRGH); myotonic dystrophy type 1 (DM1); myotonic dystrophy type 2 (DM2); progressive myoclonus epilepsy type 1 or Unverricht-Lundborg diseae (EPM1); neuronal intranuclear inclusion disease (NIID); oculopharyngodistal myopathy type 1 (OPDM1); oculopharyngodistal myopathy type 2 (OPDM2); oculopharyngeal myopathy with leukoencephalopathy type 1 (OPML1); spinobulbar muscular atrophy (SBMA); spinocerebellar ataxias type 1, 2, 3, 6, 7, 8, 10, 12, 17, 27B, 31, 36, 37 (SCA1, SCA2, SCA3, SCA6, SCA7, SCA8, SCA10, SCA12, SCA17, SCA31, SCA36, SCA37); synpolydactyly (SPD); X-linked dystonia parkinsonism (XDP); X-linked mental retardation and abnormal genitalia (XLAG); and X-linked mental retardation with or without growth hormone deficiency (XLMR, XLMRGHD).

The repeat expansion disease or disorder may be a coding repeat expansion disease, such as brachydactyly and cleidocranial dysplasia (BCCD); blepharophimosis, ptosis and epicanthus inversus (BPES); cleidocranial dysplasia (CCD); congenital central hypoventilation syndrome (CCHS); dentatorubropallidoluysian atrophy (DRPLA); early infantile epileptic encephalopathy type 1 (EIEE1); Huntington's disease (HD); Huntington disease-like 2 (HDL2); hand-foot-genital syndrome (HFGS); holoprosencephaly type 5 (HPE5); mental retardation with isolated growth hormone deficiency (MRGH); oculopharyngeal muscular dystrophy (OPMD); spinobulbar muscular atrophy (SBMA); spinocerebellar ataxias type 1, 2, 3, 6, 7, 17 (SCA1, SCA2, SCA3, SCA6, SCA7, SCA17); synpolydactyly (SPD); X-linked mental retardation and abnormal genitalia (XLAG); X-linked mental retardation with or without growth hormone deficiency (XLMR, XLMRGHD).

The repeat expansion disease or disorder may be a non-coding repeat expansion diseases include but are not limited to benign adult familial myoclonic epilepsy (BAFME); Baratela-Scott syndrome (BSS); cerebellar ataxia, neuropathy and vestibular areflexia syndrome (CANVAS); myotonic dystrophy type 1 (DM1); myotonic dystrophy type 2 (DM2); progressive myoclonus epilepsy type 1 or Unverricht-Lundborg diseae (EPM1); familial adult myoclonic epilepsy (FAME); Fuch's endothelial corneal dystrophy type 3 (FECD3); fragile XE syndrome (FRAXE);; Friedreich ataxia (FRDA) frontotemporal dementia/amyotrophic lateral sclerosis (FTD/ALS); fragile X-associated premature ovarian infertility (FXPOI); fragile X syndrome (FXS); fragile X-associated tremor ataxia syndrome (FXTAS); global development delay, progressive ataxia and elevated glutamine (GDPAG); neuronal intranuclear inclusion disease (NIID); oculopharyngodistal myopathy type 1 (OPDM1); oculopharyngodistal myopathy type 2 (OPDM2); oculopharyngeal myopathy with leukoencephalopathy type 1 (OPML1), spinocerebellar ataxias types 8, 10, 12, 27B, 31, 36, 37 (SCA8, SCA10, SCA12, SCA27B, SCA31, SCA36, SCA37); X-linked dystonia parkinsonism (XDP).

In certain embodiments, the repeat expansion disease or disorder is a triplet repeat disease or disorder, such as dentatorubropallidoluysian atrophy (DRPLA); Fuch's endothelial corneal dystrophy type 3 (FECD3); fragile XE syndrome (FRAXE); Friedreich ataxia (FRDA); fragile X-associated premature ovarian infertility (FXPOI); fragile X syndrome (FXS); fragile X-associated tremor ataxia syndrome (FXTAS); Huntington's disease (HD); Huntington disease-like 2 (HDL2); myotonic dystrophy type 1 (DM1); neuronal intranuclear inclusion disease (NIID)spinobulbar muscular atrophy (SBMA); spinocerebellar ataxias type 1, 2, 3, 6, 7, 8, 17, (SCA1, SCA2, SCA3, SCA6, SCA7, SCA8, SCA17). Suitably, the triplet repeat disease or disorder is Huntington's disease (HD).

According to a further aspect of the present invention, there is provided a method of treating a triplet repeat disease or disorder in a patient, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a compound, or a pharmaceutically acceptable salt, hydrate or solvate thereof, or a pharmaceutical composition as defined herein for use in the treatment of a triplet repeat disease or disorder.

According to a further aspect of the present invention, there is provided the use of a compound, or a pharmaceutically acceptable salt, hydrate or solvate thereof, as defined herein in the manufacture of a medicament for the treatment of a triplet repeat disease or disorder.

According to a further aspect of the present invention, there is provided a method of treating Huntington's disease in a patient, said method comprising administering to said patient a therapeutically effective amount of a compound or a pharmaceutically acceptable salt, hydrate or solvate thereof as defined herein, or a pharmaceutical composition as defined herein.

According to a further aspect of the present invention, there is provided a compound or a pharmaceutically acceptable salt, hydrate or solvate thereof as defined herein, or a pharmaceutical composition as defined herein, for use in the treatment of Huntington's disease.

According to a further aspect of the present invention, there is provided the use of a compound, or a pharmaceutically acceptable salt, hydrate or solvate thereof, as defined herein in the manufacture of a medicament for the treatment of Huntington's disease.

Suitably, the inhibition of somatic expansion of expanded simple nucleotide repeats allows the treatment of a repeat expansion disease or disorder in a subject who has already developed the disorder. The inhibition of somatic expansion of expanded simple nucleotide repeats may also be performed to prevent or slow the development of the disorder, for example in a patient who is carrying a disease allele for a repeat expansion disease or disorder. For example, the patient may have had a diagnostic test to say they are a repeat expansion disease or disorder gene carrier (for example, a Huntington Disease gene carrier), but who have not yet exhibited any signs or symptoms.

According to a further aspect of the present invention, there is provided a method for the inhibition of somatic expansion of expanded simple nucleotide repeats in a subject carrying a disease allele for a repeat expansion disease or disorder, said method comprising administering to said subject a therapeutically effective amount of a compound or a pharmaceutically acceptable salt, hydrate or solvate thereof as defined herein, or a pharmaceutical composition as defined herein.

According to a further aspect of the present invention, there is provided a compound, or a pharmaceutically acceptable salt, hydrate or solvate thereof, or a pharmaceutical composition as defined herein for use in the inhibition of somatic expansion of expanded simple nucleotide repeats in a subject carrying a disease allele for a repeat expansion disease or disorder.

According to a further aspect of the present invention, there is provided the use of a compound, or a pharmaceutically acceptable salt, hydrate or solvate thereof, as defined herein in the manufacture of a medicament for the inhibition of somatic expansion of expanded simple nucleotide repeats in a subject carrying a disease allele for a repeat expansion disease or disorder.

The patient or subject to be treated is suitably a mammal, and more suitably a human.

### Routes of Administration

The compounds of the invention or pharmaceutical compositions comprising these compounds may be administered to a subject by any convenient route of administration, whether systemically/ peripherally or topically (i.e., at the site of desired action).

Routes of administration include, but are not limited to, oral (e.g, by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### Gene Editing

The compounds of the present invention may also find use in gene editing applications. In particular, the compounds are potentially useful for increasing the efficiency of gene editing techniques, especially gene editing techniques that are susceptibe to reduced efficiency due to host cell MMR factors.

It will be appreciated that the gene editing techniques defined herein may be carried *in vivo, ex vivo* or *in vitro.*

According to a further aspect of the present invention, there is provided a method of increasing the efficiency of a gene editing technique, the method comprising conducting the gene editing technique in the presence of a compound as defined herein, or a pharmaceutically acceptable salt, hydrate or solvate thereof. The efficiency of the gene editing technique may be increased by reducing the error rate.

According to a further aspect of the present invention, there is also provided a method of reducing the error rate of a gene editing technique, the method comprising conducting the gene editing technique in the presence of a compound as defined herein, or a pharmaceutically acceptable salt, hydrate or solvate thereof.

Suitably, the gene editing technique referred to herein may be any suitable gene editing technique in which host cell mis-match repair (MMR) factors are known to decrease the efficiency of the gene editing. Suitable gene editing techniques include homology-directed repair (HDR), non-homologous end joining (NHEJ), ZFN (zinc finger nucleases), TALENs (Transcription activator like effector nucleases), CRISPR-Cas9 nucleases, CRISPR-Cas12 nucleases, base editing (fusion of catalytically dead dCas9 enzymes to DNA deaminases which enable single nucleotide substitutions), prime editing (fusion of a Cas9 nickase to a reverse transcriptase), PASTE (programmable addition via site-specific targeting elements which leverages prime editing to insert AttB sites and then facilitate serine integrase proteins to insert larger sequences), or any editing method using a single-stranded oligonucleotide (ssODN) as a template including nuclease-free editing methods.

According to a further aspect of the present invention, there is provided a method of prime editing, the method comprising contacting a cell with: (1) a Cas9 nickase to nick a non-target strand; (2) a prime editing single guide RNA (pegRNA) to bind the target strand; (3) a reverse transcriptase to reverse transcribe from the 3' end of the non-target strand according to the information provided by the pegRNA; and (4) a compound as defined herein, or a pharmaceutically acceptable salt, hydrate or solvate thereof.

According to a further aspect of the present invention, there is provided the use of a compound, or a pharmaceutically acceptable salt, hydrate or solvate thereof, as defined herein for gene editing *in vitro, ex vivo or in vivo.*

According to a further aspect of the present invention, there is provided a compound, or a pharmaceutically acceptable salt, hydrate or solvate thereof, as defined herein for use in gene editing *in vitro, ex vivo or in vivo.*

According to a further aspect of the present invention, there is provided a compound, or a pharmaceutically acceptable salt, hydrate or solvate thereof, as defined herein, or a pharmaceutical composition as defined herein, for use in prime editing. According to a further aspect of the present invention, there is provided a compound, or a pharmaceutically acceptable salt, hydrate or solvate thereof, as defined herein, or a pharmaceutical composition as defined herein, for use in enhancing the efficiency of prime editing. Suitably, the compound of the invention or a pharmaceutically acceptable salt, hydrate or solvate thereof, is administered in combination with: (1) a Cas9 nickase to nick a non-target strand; (2) a prime editing single guide RNA (pegRNA) to bind the target strand; (3) a reverse transcriptase to reverse transcribe from the 3' end of the non-target strand according to the information provided by the pegRNA.

According to a further aspect of the present invention, there is provided the use a compound, or a pharmaceutically acceptable salt, hydrate or solvate thereof, as defined herein, for prime editing. According to a further aspect of the present invention, there is provided the use of a compound, or a pharmaceutically acceptable salt, hydrate or solvate thereof, as defined herein, or a pharmaceutical composition as defined herein, for enhancing the efficiency of prime editing. Suitably, the compound of the invention or a pharmaceutically acceptable salt, hydrate or solvate thereof, is administered in combination with: (1) a Cas9 nickase to nick a non-target strand; (2) a prime editing single guide RNA (pegRNA) to bind the target strand; (3) a reverse transcriptase to reverse transcribe from the 3' end of the non-target strand according to the information provided by the pegRNA.

According to a further aspect of the present invention, there is provided a compound, or a pharmaceutically acceptable salt, hydrate or solvate thereof, as defined herein for use as an agent to enhance the efficiency of prime editors.

According to a further aspect of the present invention, there is provided the use of a compound, or a pharmaceutically acceptable salt, hydrate or solvate thereof, as defined herein as an agent to enhance the efficiency of prime editors.

According to a further aspect of the present invention, there is provided a method to enhance the efficiency of prime editors, the method comprising contacting a cell with a compound, or a pharmaceutically acceptable salt, hydrate or solvate thereof, as defined herein, or a pharmaceutical composition as defined herein, and conducting the gene editing in the presence of said compound.

According to a further aspect of the present invention, there is provided a compound, or a pharmaceutically acceptable salt, hydrate or solvate thereof, as defined herein for use as an agent to reduce off-target editing (errors) in prime editing.

According to a further aspect of the present invention, there is provided the use of a compound, or a pharmaceutically acceptable salt, hydrate or solvate thereof, as defined herein as an agent to reduce off-target editing (errors) in prime editing. Suitably, the use is in cellular systems.

According to a further aspect of the present invention, there is provided a method to enhance efficiency and reduce off-target editing (errors) in gene editing (e.g. prime editing), the method comprising contacting a cell with a compound, or a pharmaceutically acceptable salt, hydrate or solvate thereof, as defined herein, or a pharmaceutical composition as defined herein, and conducting the gene editing in the presence of said compound.

According to a further aspect of the present invention, there is provided a compound, or a pharmaceutically acceptable salt, hydrate or solvate thereof, as defined herein for use as an agent to both enhance efficiency and reduce off-target editing (errors) in gene editing (e.g. prime editing).

According to a further aspect of the present invention, there is provided the use of a compound, or a pharmaceutically acceptable salt, hydrate or solvate thereof, as defined herein as an agent to both enhance efficiency and reduce off-target editing (errors) in gene editing (e.g. prime editing). Suitably, the use is in cellular systems.

According to a further aspect of the present invention, there is provided a method to enhance the efficiency of prime editor therapeutics *in vivo* or *ex vivo* (e.g. allogeneic editing of hematopoietic cells), the method comprising contacting a cell with a compound, or a pharmaceutically acceptable salt, hydrate or solvate thereof, as defined herein, or a pharmaceutical composition as defined herein, and conducting the gene editing in the presence of said compound.

According to a further aspect of the present invention, there is provided a compound, or a pharmaceutically acceptable salt, hydrate or solvate thereof, as defined herein for use as an agent to enhance the efficiency of prime editor therapeutics *ex vivo* (e.g. allogeneic editing of hematopoietic cells).

According to a further aspect of the present invention, there is provided the use of a compound, or a pharmaceutically acceptable salt, hydrate or solvate thereof, as defined herein as an agent to enhance the efficiency of prime editor therapeutics *ex vivo* (e.g. allogeneic editing of hematopoietic cells).

According to a further aspect of the present invention, there is provided a compound, or a pharmaceutically acceptable salt, hydrate or solvate thereof, as defined herein for use as an agent to enhance the efficiency of prime editor therapeutics *in vivo.*

In certain embodiments, the gene is in a cell, e.g. a mammalian cell, e.g. a human cell.

In certain embodiments, the cell is in a subject or patient, e.g. the subject is human.

In certain embodiments, the cell is present *in vitro, ex vivo* or *in vivo.*

In certain embodiments, the cell is in a cellular assay.

According to a further aspect of the present invention, there is provided the use of a compound, or a pharmaceutically acceptable salt, hydrate or solvate thereof, as defined herein as an agent to inhibit mismatch repair *in vitro, ex vivo* or *in vivo,* for example in cellular systems.

According to a further aspect of the present invention, there is provided the use of a compound, or a pharmaceutically acceptable salt, hydrate or solvate thereof, as defined herein in testing an agent for the ability to inhibit ATP hydrolysis by MutSβ in the presence or absence of a suitable heteroduplex DNA substrate.

According to a further aspect of the present invention, there is provided the use of a compound, or a pharmaceutically acceptable salt, hydrate or solvate thereof, as defined herein, in testing an agent for the ability to inhibit ATP-dependent recruitment of MutLα or MutLβ or MutLγ by MutSβ.

### Combination Therapies

The present invention provides methods for the treatment of certain diseases or disorders through the inhibition of MutSβ with a compound as defined herein. The compounds as defined herein may be administered either as single agents or in combination with one or more additional agents.

The compounds as defined herein may be administered either as single agents or in combination with one or more disease modification therapies for Huntington's Disease. Suitable disease modification therapies for Huntington's disease include, but are not limited to RNA interference approaches (such as tominersen, WVE-003, AMT-130, BV-101, VO-659, SPK-miHTT, ALN-HTT02), mis-splicing (PTC-518) and antibody therapeutics (INT-41, NI-302, C6-17, VTx-003, ATLX-1095).

The compounds as defined herein may be administered either as single agents or in combination with one or more Huntingtin lowering therapies. Huntingtin lowering therapies may include approaches such as RNAi, miRNA (such as AMT-130), antisense oligonucleotides (such as tominersen), and small-molecule splicing modulators (such as PTC-518); and novel methods to clear the mHTT protein, such as proteolysis-targeting chimeras.

The present invention also provides methods for the treatment of repeat expansion diseases or disorders through the inhibition of MutSβ with a compound as defined herein. The compounds as defined herein may be administered either as single agents or in combination with one or more Huntingtin lowering therapies.

Thus, there is provided a combination comprising a compound or a pharmaceutically acceptable salt, hydrate or solvate thereof as defined herein, and one or more Huntingtin lowering therapies.

According to a further aspect of the present invention, there is provided a method of treating a repeat expansion disease or disorder in a patient, said method comprising administering to said patient a therapeutically effective amount of a compound or a pharmaceutically acceptable salt, hydrate or solvate thereof as defined herein, or a pharmaceutical composition as defined herein, and one or more Huntingtin lowering therapies.

According to a further aspect of the present invention, there is provided a compound or a pharmaceutically acceptable salt, hydrate or solvate thereof as defined herein, or a pharmaceutical composition as defined herein, for use in the treatment of a repeat expansion disease or disorder, wherein the compound or pharmaceutical composition is administered in combination with one or more Huntingtin lowering therapies.

According to a further aspect of the present invention, there is provided the use of a compound, or a pharmaceutically acceptable salt, hydrate or solvate thereof, as defined herein in the manufacture of a medicament for the treatment of a repeat expansion disease or disorder, wherein the compound or pharmaceutical composition is administered in combination with one or more Huntingtin lowering therapies.

The repeat expansion disease or disorder may be selected from any of those described herein, e.g. a triplet repeat disease or disorder.

Suitably, the triplet repeat disease or disorder is Huntington's disease (HD).

Thus, there is provided a combination comprising a compound or a pharmaceutically acceptable salt, hydrate or solvate thereof as defined herein, and one or more disease modification therapies for Huntington's Disease. Suitable disease modification therapies for Huntington's disease include, but are not limited to RNA interference approaches (such as tominersen, WVE-003, AMT-130, BV-101, VO-659, SPK-miHTT, ALN-HTT02), mis-splicing (PTC-518) and antibody therapeutics (INT-41, NI-302, C6-17, VTx-003, ATLX-1095).

According to a further aspect of the present invention, there is provided a method of treating Huntington's disease in a patient, said method comprising administering to said patient a therapeutically effective amount of a compound or a pharmaceutically acceptable salt, hydrate or solvate thereof as defined herein, or a pharmaceutical composition as defined herein, and one or more disease modification therapies for Huntington's disease.

According to a further aspect of the present invention, there is provided a compound or a pharmaceutically acceptable salt, hydrate or solvate thereof as defined herein, or a pharmaceutical composition as defined herein, for use in the treatment of Huntington's disease, wherein the compound or pharmaceutical composition is administered in combination with one or more disease modification therapies for Huntington's disease.

According to a further aspect of the present invention, there is provided the use of a compound, or a pharmaceutically acceptable salt, hydrate or solvate thereof, as defined herein in the manufacture of a medicament for the treatment of Huntington's disease, wherein the compound or pharmaceutical composition is administered in combination with one or more disease modification therapies for Huntington's disease.

The compounds as defined herein may be administered either as single agents or in combination with one or more Huntingtin lowering therapies, particularly in the treatment of Huntington's disease. Huntingtin lowering therapies may include approaches such as RNAi, miRNA (such as AMT-130), antisense oligonucleotides (such as tominersen), and small-molecule splicing modulators (such as PTC-518); and methods to clear the mHTT protein, such as proteolysis-targeting chimeras.

According to a further aspect of the present invention, there is provided a method of treating Huntington's disease in a patient, said method comprising administering to said patient a therapeutically effective amount of a compound or a pharmaceutically acceptable salt, hydrate or solvate thereof as defined herein, or a pharmaceutical composition as defined herein, and one or more Huntingtin lowering therapies.

According to a further aspect of the present invention, there is provided a compound or a pharmaceutically acceptable salt, hydrate or solvate thereof as defined herein, or a pharmaceutical composition as defined herein, for use in the treatment of Huntington's disease, wherein the compound or pharmaceutical composition is administered in combination with one or more Huntingtin lowering therapies.

According to a further aspect of the present invention, there is provided the use of a compound, or a pharmaceutically acceptable salt, hydrate or solvate thereof, as defined herein in the manufacture of a medicament for the treatment of Huntington's disease, wherein the compound or pharmaceutical composition is administered in combination with one or more Huntingtin lowering therapies.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial effect of the combination.

According to a further aspect of the invention there is provided a pharmaceutical composition which comprises a compound of the invention, or a pharmaceutically acceptable salt, hydrate or solvate thereof, in combination with an anti-tumour agent (optionally selected from one listed herein above), in association with a pharmaceutically acceptable diluent or carrier.

### EXAMPLES

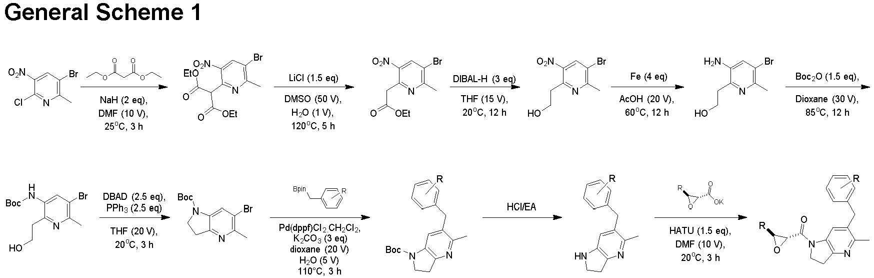

### Example 5 -Step 1: Procedure for preparation of 1b

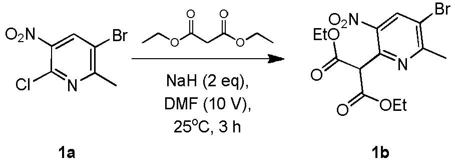

To a solution of diethyl propanedioate (6.369 g, 39.8 mmol, 2 eq) in *N,N-*dimethylformamide (50 mL) was slowly added sodium hydride (1.59 g, 39.8 mmol, 60% purity, 2 eq) at 0 °C at 15 min and the mixture was stirred at 25 °C for 0.5 h. **1a** (5 g, 19.9 mmol, 1 eq) was added to the above solution over 10 min at 0 °C and the reaction mixture was stirred at 25°C for 3 h. LCMS showed the starting material was consumed and 42% of the desired mass was detected. The reaction mixture was diluted with water (100 mL) and extracted with ethyl acetate (3 × 100 mL). The combined organic layers were washed with brine (200 mL), dried over sodium sulfate, filtered and the filtrate was concentrated under reduced pressure to give **1b** (diethyl 2-(5-bromo-6-methyl-3-nitro-2-pyridyl)propanedioate) (650 mg, yield 87.13%) as a yellow oil. LCMS (ESI+): found m/z: 374.0.

### Step 2: Procedure for preparation of 1c.

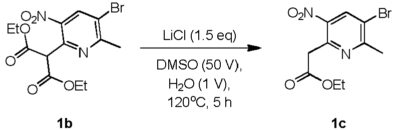

A mixture of 1b (6.7 g, 17.86 mmol, 1 eq), lithium chloride (1.14 g, 26.79 mmol, 1.5 eq) in dimethyl sulfoxide (335 mL) and water (7 mL) was stirred at 120°C for 5 h. LCMS showed the starting material was consumed and 48% of the desired mass was detected. The reaction mixture was diluted with water (200 mL) and extracted with ethyl acetate (3 × 100 mL), the combined organic layers were washed with brine (200 mL), dried over sodium sulfate, filtered and the filtrate was concentrated under reduced pressure to give a residue. The residue was purified by column chromatography (petroleum ether/ethyl acetate = 10/1) to give **1c** (ethyl 2-(5-bromo-6-methyl-3-nitro-2-pyridyl) acetate (5.3 g, yield 97.91%) as yellow oil. **¹H NMR** (400 MHz, DMSO-*d*₆) δ ppm 8.76 (s, 1 H) 4.18 (s, 2 H) 4.09 - 4.11 (m, 2 H) 2.68 (s, 3 H) 1.19 (s, 3 H). **LCMS (ESI+):** found m/z: 302.0.

### Step 3: Procedure for preparation of 1d

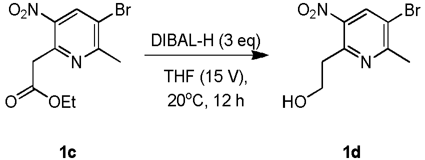

To a solution of **1c** (3.44 g, 11.35 mmol, 1 eq) in tetrahydrofuran (50 mL) was added diisobutylaluminum hydride (1 M, 34.05 mL, 3 eq) at 0°C under N₂ atmosphere. The mixture was stirred at 20°C for 3 h under a N₂ atmosphere. LCMS showed 65% of the desired mass was detected. The reaction mixture was added to water (50 mL) at 0°C, and then 15% aqueous sodium hydroxide (50 mL) and 100 mL of water were added slowly, and the mixture was stirred at 25°C for 1 h. After extraction with ethyl acetate (3 × 100 mL), the organic fraction was dried over sodium sulfate, filtered and the filtrate was concentrated under reduced pressure to give a residue. The residue was purified by column chromatography (petroleum ether/ethyl acetate = 1/1) to give **1d** (2-(5-bromo-6-methyl-3-nitro-2-pyridyl)ethanol)(1.6 g, yield 54.00%) as a yellow oil. **¹H NMR** (400 MHz, DMSO-*d*₆) δ ppm 7.21 (s, 1 H) 5.19 (s, 2 H) 4.71 (t, *J* = 5.2 Hz, 1 H) 3.75 (td, *J* = 6.8, 5.6 Hz, 2 H) 2.77 (t, *J* = 6.8 Hz, 2 H) 2.42 (s, 3 H). LCMS (ESI+): m/z: 230.0

### Step 4: Procedure for preparation of 1e

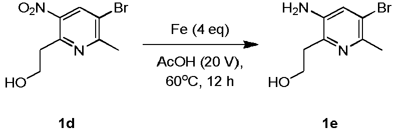

A mixture of **1d** (1.6 g, 6.13 mmol, 1 eq) and iron (1.37 g, 24.51 mmol, 4 eq) in acetic acid (32 mL) was stirred at 60°C for 12 h. LCMS showed the starting material was consumed and 61% of the desired mass was detected. The reaction mixture was added to 50 mL of saturated aqueous sodium bicarbonate at 20°C. The reaction mixture was extracted with ethyl acetate (3 × 50 mL). The combined organic layers were washed with brine (100 mL), dried over sodium sulfate, filtered and the filtrate was concentrated under reduced pressure to give **1e** (2-(3-amino-5-bromo-6-methyl-2-pyridyl)ethanol) (1.3 g, yield 91.79%) as a yellow oil. **¹H NMR** (400 MHz, DMSO-*d*₆) δ ppm 7.21 (s, 1H), 5.19 (s, 2H), 4.71 (t, *J =* 5.2 Hz, 1H), 3.75 (dt, *J* = 5.6, 6.8 Hz, 2H), 2.77 (t, *J =* 6.8 Hz, 2H), 2.42 (s, 3H). **LCMS (ESI+):** m/z: 431.0.

### Step 5: Procedure for preparation of 1f

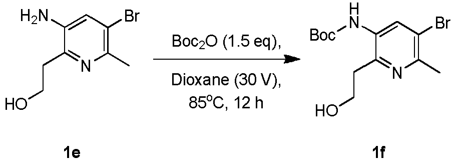

A mixture of **1e** (1.3 g, 5.63 mmol, 1 eq) and di-tert-butyl dicarbonate (1.84 g, 8.44 mmol, 1.94 mL, 1.5 eq) in dioxane (40 mL) was stirred at 85°C for 12 h. LCMS showed the starting material was consumed and 91% of the desired mass was detected. The reaction mixture was quenched by the addition water (50 mL), and extracted with ethyl acetate (3 × 20 mL).The combined organic layers were washed with brine (100 mL), dried over sodium sulfate, filtered and the filtrate was concentrated under reduced pressure to give a residue. The residue was purified by column chromatography (petroleum ether/ethyl acetate = 2/1) to give **1f** (tert-butyl N-[5-bromo-2-(2-hydroxyethyl)-6-methyl-3-pyridyl]carbamate) (0.6 g, yield 32.2%) as a yellow oil. **¹H NMR** (400 MHz, DMSO-*d*₆) δ ppm 8.95 (br s, 1 H) 8.03 (br s, 1 H) 5.03 (br s, 1 H) 3.63 - 3.80 (m, 2 H) 2.85 (br t, *J* = 6.4 Hz, 2 H) 1.46 (s, 9 H). **LCMS (ESI+):** m/z: 330.1.

### Step 6: Procedure for preparation of 1g

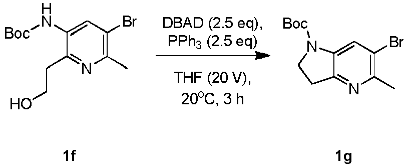

To a solution of **1f** (680 mg, 2.05 mmol, 1 eq), triphenylphosphine (1.35 g, 5.13 mmol, 2.5 eq) in tetrahydrofuran (15 mL) was added di-tert-butyl azodicarboxylate (1.18 g, 5.13 mmol, 2.5 eq) at 20°C. The mixture was stirred at 20°C for 3 h under a N₂ atmosphere. LCMS showed 27% of the desired product mass was detected. The reaction mixture was diluted with water (20 mL) and extracted with ethyl acetate (3 × 20 mL). The combined organic layers were washed with brine (100 mL), dried over sodium sulfate, filtered and the filtrate was concentrated under reduced pressure to give a residue. The residue was purified by column chromatography (petroleum ether/ethyl acetate = 4/1) to give **1g** (tert-butyl 6-bromo-5-methyl-2,3-dihydropyrrolo[3,2-b]pyridine-1-carboxylate) (1.21 g, yield 65.86%) as a yellow oil. **¹H NMR** (400 MHz, DMSO-*d*₆) δ ppm 7.96 (br d, *J = 8.8* Hz, 1 H) 3.94 (br s, 2 H) 3.08 (t, *J = 8.8* Hz, 2 H) 2.47 (s, 3 H) 1.50 (br s, 9 H). **LCMS (ESI+):** m/z: 312.0.

### Step 7: Procedure for preparation of 1h

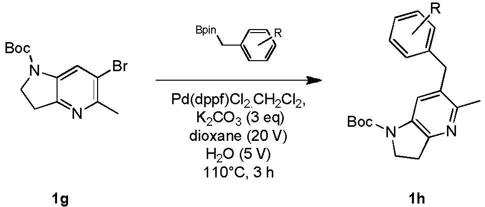

A mixture of **1g** (725.60 mg, 2.68 mmol, 1.2 eq), tert-butyl 6-bromo-5-methyl-2,3-dihydropyrrolo [3,2-b] pyridine-1-carboxylate (700 mg, 2.24 mmol, 1 eq), cis-dichloro-1,1'-bis(diphenylphosphino)ferrocene palladium(II) (182.52 mg, 223.51 µmol, 0.1 eq), potassium carbonate (926.70 mg, 6.71 mmol, 3 eq) in dioxane (14 mL) and water (3 mL) was degassed and purged with N₂ for 3 times. The mixture was stirred at 110°C for 3 h under a N₂ atmosphere. LCMS showed the starting material was consumed and 59% of the desired mass was detected. The reaction was quenched with water (20 mL) and extracted with ethyl acetate (40 mL). The organic layer was dried over sodium sulfate, filtered and concentrated under reduced pressure to give a residue. The residue was purified by column chromatography (SiO₂, petroleum ether/ethyl acetate=1/0 to 10/1) to give **1h** (tert-butyl 6-[(3-chloro-4-fluorophenyl)methyl]-5-methyl-2,3-dihydropyrrolo[3,2-b]pyridine-1-carboxylate) (250 mg, yield 29.68%) as a yellow oil. **¹H NMR** (400 MHz, DMSO-*d*₆) δ ppm 7.93 - 6.87 (m, 4H), 3.97 - 3.84 (m, 4H), 3.07 (br t, *J* = 8.4 Hz, 2H), 2.43 - 2.28 (m, 3H), 1.55 - 1.31 (m, 9H). **LCMS (ESI+):** m/z: 377.0.

### Step 8: Procedure for preparation of 1i

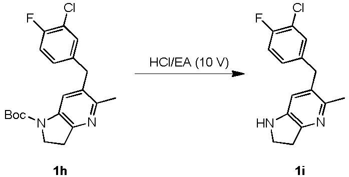

To a solution of **1h** (100 mg, 265.36 µmol, 1 eq) in ethyl acetate (0.5 mL) was added hydrochloric acid / ethyl acetate (2 M, 1.33 mL, 10 eq) in portions at 0°C and the mixture was stirred at 25°C for 12 h. LCMS showed 59% of the desired mass was detected. The reaction mixture was concentrated under reduced pressure to give **1i** (6-[(3-chloro-4-fluorophenyl)methyl]-5-methyl-2,3-dihydro-1H-pyrrolo[3,2-b]pyridine) (250 mg, yield 85.11%) as a yellow solid. **¹H NMR** (400 MHz, DMSO-*d*₆) δ ppm 7.46 - 7.34 (m, 2H), 7.24 - 7.18 (m, 1H), 7.14 (s, 1H), 4.00 (s, 2H), 3.70 - 3.67 (m, 2H), 3.32 (s, 2H), 2.43 (s, 3H). **LCMS (ESI+):** m/z: 277.0.

### Step 7: Procedure for preparation of Example 5

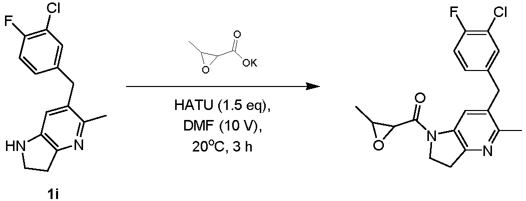

To a solution of *trans*-(3-methyloxirane-2-carbonyl)oxypotassium (75.98 mg, 542.03 µmol, 1.5 eq), 6-[(3-chloro-4-fluoro-phenyl)methyl]-5-methyl-2,3-dihydro-1H-pyrrolo[3,2-b]pyridine (100 mg, 361.36 µmol, 1 eq) in N,N-dimethylformamide (2 mL) was added O-(7-azabenzotriazol-1-yl)-N,N,N,N-tetramethyluronium hexafluorophosphate (206.10 mg, 542.03 µmol, 1.5 eq) andthe mixture was stirred at 20°C for 2 hrs. LCMS showed the starting material was detected with 75% of the desired mass. The reaction was quenched with water (20 mL) and extracted with ethyl acetate (3 x 10 mL). The organic layer was dried over Na₂SO₄, filtered and concentrated under reduced pressure to give a residue. The residue was purified by preparative HPLC (column: CD24-XPT C18 150 × 25 × 7um;mobile phase: [water(FA)-CH₃CN]; gradient:26%-46% B over 25 min) to give **Example 5** [6-[(3-chloro-4-fluorophenyl)methyl]-5-methyl-2,3-dihydropyrrolo[3,2-b]pyridin-1-yl]-*trans*-(3-methyloxiran-2-yl)methanone (52 mg, yield 19.94%) as a white soild. **¹H NMR** (400 MHz, DMSO-*d*₆) δ ppm 8.01 (s, 1H), 7.41 - 7.28 (m, 2H), 7.12 (dt, *J* = 2.4, 5.3 Hz, 1H), 4.45 - 4.18 (m, 2H), 3.98 (s, 2H), 3.65 (d, *J =* 1.6 Hz, 1H), 3.23 (br t, *J =* 8.4 Hz, 2H), 3.17 - 3.13 (m, 1H), 2.36 (s, 3H), 1.35 (d, *J =* 5.2 Hz, 3H). **LCMS (ESI+):** m/z: 361.2.

**Example 5** was separated by SFC (column: DAICEL CHIRALPAK AD(250mm*30mm, 10um);mobile phase: [CO₂-MeOH(0.1%NH₃H₂O)]; B%:45%, isocratic elution mode) to obtain **Example 6** (6-(3-chloro-4-fluorobenzyl)-5-methyl-2,3-dihydro-1H-pyrrolo[3,2-b]pyridin-1-yl)((2R,3S)-3-methyloxiran-2-yl)methanone.

The following examples were prepared in a similar fashion using **Scheme 1.**

| Example | Structure | Chemical name | LC-MS method | LC-MS retention time (mins) | Mass found (M+H) ⁺ |
|---|---|---|---|---|---|
| 1 | 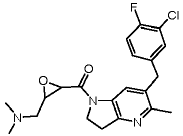 | *Trans*-(6-(3-chloro-4-fluorobenzyl)-5-methyl-2,3-dihydro-1H-pyrrolo[3,2-b]pyridin-1-yl)(3-((dimethylamino)methyl) oxiran-2-yl)methanone | H | 2.641 | 404.1 |
| 2 | 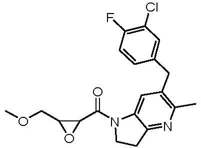 | *Trans-*(6-(3-chloro-4-fluorobenzyl)-5-methyl-2,3-dihydro-1H-pyrrolo[3,2-b]pyridin-1-yl)(3-(methoxymethyl)oxiran-2-yl)methanone | I | 2.596 | 391.2 |
| 3 | 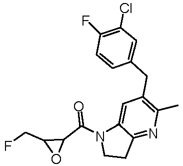 | *Trans-*(6-(3-chloro-4-fluorobenzyl)-5-methyl-2,3-dihydro-1H-pyrrolo[3,2-b]pyridin-1-yl)(3-(fluoromethyl)oxiran-2-yl)methanone | E | 1.764 | 379.1 |
| 4 | 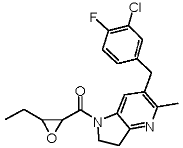 | *Trans*-(6-(3-chloro-4-fluorobenzyl)-5-methyl-2,3-dihydro-1H-pyrrolo[3,2-b]pyridin-1-yl)(3-ethyloxiran-2-yl)methanone | D | 2.054 | 375.1 |
| 5 | 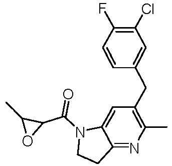 | *Trans*-(6-(3-chloro-4-fluorobenzyl)-5-methyl-2,3-dihydro-1H-pyrrolo[3,2-b]pyridin-1-yl)(3-methyloxiran-2-yl)methanone | D | 1.998 | 361.2 |
| 6 | 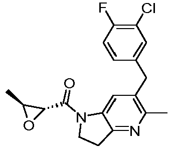 | (6-(3-chloro-4-fluorobenzyl)-5-methyl-2,3-dihydro-1H-pyrrolo[3,2-b]pyridin-1-yl)((2R,3S)-3-methyloxiran-2-yl)methanone | D | 1.995 | 361.2 |
| 7 | 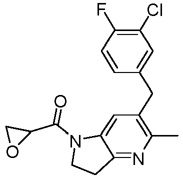 | (6-(3-chloro-4-fluorobenzyl)-5-methyl-2,3-dihydro-1H-pyrrolo[3,2-b]pyridin-1-yl)(oxiran-2-yl)methanone | T | 2.080 | 365.2 |
| 8 | 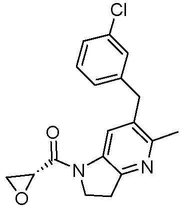 | (R)-(6-(3-chlorobenzyl)-5-methyl-2,3-dihydro-1H-pyrrolo[3,2-b]pyridin-1-yl)(oxiran-2-yl)methanone | H | 2.648 | 329.1 |

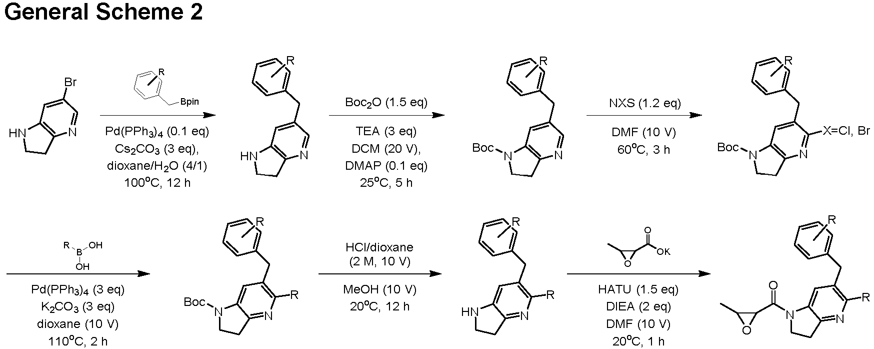

### Example 11-Step 1: Procedure for preparation of 2b

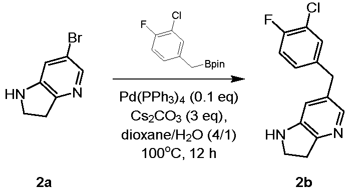

A mixture of **2a** (3 g, 15.1 mmol, 1.0 eq) , 2-[(3-chloro-4-fluoro-phenyl)methyl]-4,4,5,5-tetramethyl-1,3,2-dioxaborolane (8.16 g, 30.2 mmol, 2.0 eq), tetrakis(triphenylphosphane) palladium (1.74 g, 1.51 mmol, 0.1 eq), cesium carbonate (14.7 g, 45.2 mmol, 3.0 eq) in dioxane (24 mL) and water (6 mL) was degassed and purged with nitrogen 3 times, and then the mixture was stirred at 100°C for 12 hrs under a nitrogen atmosphere. LCMS showed 56% of the desired mass was detected. The reaction mixture was quenched by addition water (20 mL), and then extracted with ethyl acetate (10 mL × 3). The combined organic layers were washed with brine (50 mL), dried over Na₂SO₄, filtered and concentrated under reduced pressure to give a residue. The residue was purified by preparative HPLC (Waters Xbridge BEH C18 150*40mm*10um; mobile phase: [H₂O (10mM NH₄HCO₃)-ACN]; gradient: 30%-60% B over 10.0 min) to give **2b** (6-(3-chloro-4-fluorobenzyl)-2,3-dihydro-1H-pyrrolo[3,2-b]pyridine) (1.6 g, yield 40%) as a yellow solid. **¹H NMR** (400 MHz, DMSO-*d*₆) δ ppm 7.57 (s, 1 H), 7.44 (dd, *J* = 7.2, 1.6 Hz, 1 H), 7.16 - 7.36 (m, 2 H), 6.53 (d, *J* = 1.2 Hz, 1 H), 5.70 (s, 1 H), 3.77 (s, 2 H), 3.40 - 3.48 (m, 2 H), 2.89 (t, *J =* 8.4 Hz, 2 H). **LCMS (ESI+):** m/z: 263.1.

### Step 2: Procedure for preparation of 2c

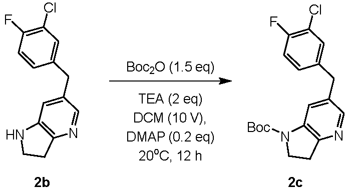

To a solution of **2b** (1.3 g, 4.95 mmol, 1.0 eq) in dichloromethane (13 mL) was added triethylamine (1.00 g, 9.90 mmol, 1.38 mL, 2.0 eq) and *N,N-*dimethylpyridin-4-amine (121 mg, 990 µmol, 0.2 eq) and di-tert-butyl dicarbonate (1.62 g, 7.42 mmol, 1.71 mL, 1.5 eq). The mixture was stirred at 20°C for 12 h. LCMS showed 49% of the desired mass was detected. The reaction mixture was concentrated under reduced pressure to give a residue. The residue was purified by column chromatography (petroleum ether/ethyl acetate = 20/1) to give **2c** (tert-butyl 6-(3-chloro-4-fluorobenzyl)-2,3-dihydro-1*H*-pyrrolo[3,2-*b*]pyridine-1-carboxylate) (1.0 g, yield 55%) as a yellow solid. **¹H NMR** (400 MHz, DMSO-*d*₆) δ ppm 8.01 (d, *J =* 1.6 Hz, 1 H), 7.50 (d, *J* = 6.8 Hz, 1 H), 7.17 - 7.42 (m, 3 H), 3.85 - 3.98 (m, 4 H), 3.09 (t, *J = 8.8* Hz, 2 H), 1.46 (s, 9 H). **LCMS (ESI+):** m/z: 363.1.

### Step 3: Procedure for preparation of 2d

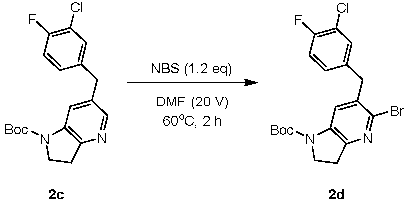

To a solution of **2c** (1.0 g, 2.76 mmol, 1.0 eq) in *N,N*-dimethylformamide (20 mL) was added 1-bromopyrrolidine-2,5-dione (589 mg, 3.31 mmol, 1.2 eq). The mixture was stirred at 60°C for 2 h. LCMS showed the starting material was consumed and 90% of the desired mass was detected. The reaction mixture was quenched by the addition of water (20 mL) and then extracted with ethyl acetate (3 x 10 mL). The combined organic layers were washed with brine (50 mL), dried over Na₂SO₄, filtered and concentrated under reduced pressure to give a residue. The residue was purified by column chromatography (petroleum ether/ethyl acetate = 20/1) to give **2d** (tert-butyl 5-bromo-6-(3-chloro-4-fluorobenzyl)-2,3-dihydro-1*H*-pyrrolo[3,2-*b*]pyridine-1-carboxylate) (800 mg, yield 66%) as a yellow solid. **¹H NMR** (400 MHz, DMSO-*d*₆) δ ppm 7.05 - 8.00 (m, 4 H), 4.01 - 4.05 (m, 2 H), 3.93 (s, 2 H), 3.12 (t, *J =* 8.8 Hz, 2 H), 1.44 (s, 9 H). LCMS (ESI+): m/z: 441.0.

### Step 4: Procedure for preparation of 2e

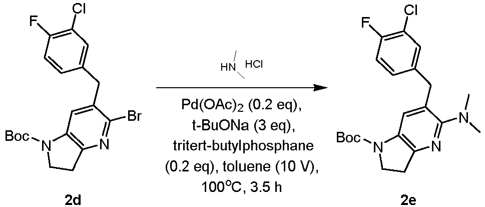

To a solution of **2d** (50 mg, 1 eq) and dimethylamine hydrochloride (11.08 mg, 1.2 eq) in toluene (0.5 mL) was added sodium tert-butoxide (32.63 mg, 3 eq), palladium acetate (5.08 mg, 0.2 eq) and tritert-butylphosphane (45.80 mg, 53.14 µL, 0.2 eq) under N₂. The mixture was stirred at 100°C for 3.5 h under a N₂ atmosphere. LCMS showed all of the starting material was consumed and 42% of the desired mass was detected. The reaction was filtered and the filtrate was concentrated to give a residue, which was purified by column chromatography on silica gel (petroleum ether/ethyl acetate = 7/1) to give **2e** (tert-butyl 6-[(3-chloro-4-fluoro-phenyl)methyl]-5-(dimethylamino)-2,3-dihydropyrrolo[3,2-b]pyridine-1-carboxylate) (10 mg, yield 21.77%) as a white solid. **¹H NMR** (400 MHz, CHLOROFORM-d) δ ppm 7.68 - 7.95 (m, 1 H) 7.15 - 7.26 (m, 1 H) 7.05 (br s, 2 H) 3.92 - 4.04 (m, 4 H) 3.14 (br t, *J* = 8.4 Hz, 2 H) 2.58 - 2.80 (m, 6 H) 1.38 - 1.55 (m, 9 H). **LCMS (ESI+):** m/z: 405.2.

### Step 5: Procedure for preparation of 2f

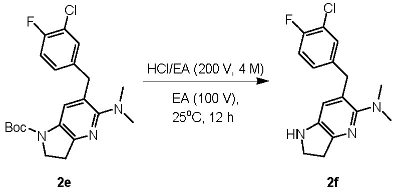

To a solution of **2e** (14 mg, 34.49 µmol, 1 eq) in ethyl acetate (1.4 mL) was added hydrogen chloride/ethyl acetate (4 M, 2.80 mL). The mixture was stirred at 25°C for 12 h. LCMS showed 64% of the desired mass was detected. The reaction mixture was concentrated in vacuum to give 2f (6-[(3-chloro-4-fluoro-phenyl)methyl]-N,N-dimethyl-2,3-dihydro-1H-pyrrolo[3,2-b]pyridin-5-amine) (12 mg, yield 81.33%) as a yellow solid. **¹H NMR** (400 MHz, CHLOROFORM-d) δ ppm 7.68 - 7.95 (m, 1 H) 7.15 - 7.26 (m, 1 H) 7.05 (br s, 2 H) 3.92 - 4.04 (m, 4 H) 3.14 (br t, *J =* 8.4 Hz, 2 H) 2.58 - 2.80 (m, 6 H). **LCMS (ESI+):** m/z: 305.2.

### Step 6: Procedure for preparation of Example 11

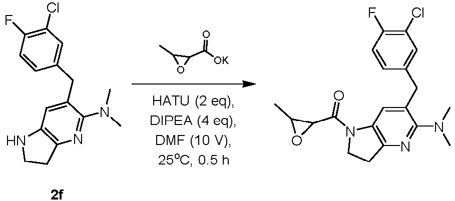

To a solution of *trans*-(3-methyloxirane-2-carbonyl)oxypotassium (9.83 mg, 2 eq) and 2-(3H-[1,2,3]triazolo[4,5-b]pyridin-3-yl)-1,1,3,3-tetramethyluronium hexafluorophosphate (V) (26.66 mg, 2 eq) in N,N-dimethylformamide (0.12 mL) was added **2f** (12 mg, 1 eq, HCl) and diisopropylethylamine (18.13 mg, 24.43 µL, 4 eq) at 25°C under N₂. The mixture was stirred at 25°C for 0.5 h. LCMS showed starting material was consumed and 28% of the desired mass was detected. The reaction mixture was purified by preparative HPLC (column: Phenomenex Luna C18 100 × 30 mm × 5 um; mobile phase: [H₂O (0.2% FA)-MeCN]; gradient: 35%-65% B over 8.0 min) to give **Example 11** ([6-[(3-chloro-4-fluoro-phenyl)methyl]-5-(dimethylamino)-2,3-dihydropyrrolo[3,2-b]pyridin-1-yl]-(3-methyloxiran-2-yl)methanone) (6.68 mg, yield 25.96%) as a white solid. **¹H NMR** (400 MHz, DMSO-*d*₆) δ ppm 7.87 (s, 1 H) 7.29 - 7.41 (m, 2 H) 7.08 - 7.18 (m, 1 H) 4.29 - 4.40 (m, 1 H) 4.20 (q, *J*=9.05 Hz, 1 H) 3.98 (s, 2 H) 3.60 (d, *J* = 2.0 Hz, 1 H) 3.18 (br t, *J* = 8.4 Hz, 2 H) 3.11 (br dd, *J* = 5.2, 1.88 Hz, 1 H) 2.70 (s, 6 H) 1.33 (d, *J* = 5.2 Hz, 3 H). **LCMS (ESI+):** m/z: 389.1.

The following examples were prepared in a similar fashion using **Scheme 2.**

| Example | Structure | Chemical name | LC-MS method | LC-MS retention time (mins) | Mass found (M+H) ⁺ |
|---|---|---|---|---|---|
| 9 | 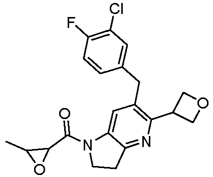 | *Trans*-(6-(3-chloro-4-fluorobenzyl)-5-(oxetan-3-yl)-2,3-dihydro-1H-pyrrolo[3,2-b]pyridin-1-yl)(3-methyloxiran-2-yl)methanone | D | 2.368 | 403.2 |
| 10 | 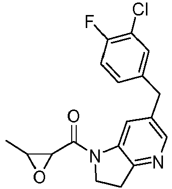 | *Trans*-(6-(3-chloro-4-fluorobenzyl)-2,3-dihydro-1H-pyrrolo[3,2-b]pyridin-1-yl)(3-methyloxiran-2-yl)methanone | D | 2.215 | 347.2 |
| 11 | 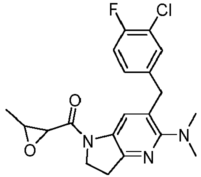 | *Trans*-(6-(3-chloro-4-fluorobenzyl)-5-(dimethylamino)-2,3-dihydro-1H-pyrrolo[3,2-b]pyridin-1-yl)(3-methyloxiran-2-yl)methanone | E | 1.905 | 390.1 |
| 12 | 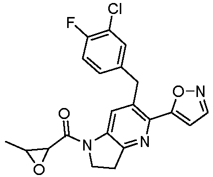 | *Trans*-(6-(3-chloro-4-fluorobenzyl)-5-(isoxazol-5-yl)-2,3-dihydro-1H-pyrrolo[3,2-b]pyridin-1-yl)(3-methyloxiran-2-yl)methanone | D | 2.474 | 414.2 |
| 13 | 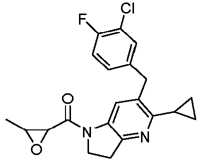 | *Trans*-(6-(3-chloro-4-fluorobenzyl)-5-cyclopropyl-2,3-dihydro-1H-pyrrolo[3,2-b]pyridin-1-yl)(3-methyloxiran-2-yl)methanone | D | 2.301 | 387.2 |
| 14 | 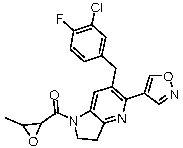 | *Trans*-(6-(3-chloro-4-fluorobenzyl)-5-(isoxazol-4-yl)-2,3-dihydro-1H-pyrrolo[3,2-b]pyridin-1-yl)(3-methyloxiran-2-yl)methanone | D | 2.443 | 414.2 |
| 15 | 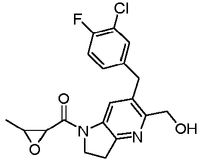 | *Trans*-(6-(3-chloro-4-fluorobenzyl)-5-(hydroxymethyl)-2,3-dihydro-1H-pyrrolo[3,2-b]pyridin-1-yl)(3-methyloxiran-2-yl)methanone | D | 2.016 | 377.2 |
| 16 | 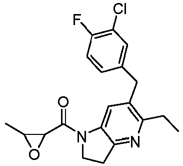 | *Trans*-(6-(3-chloro-4-fluorobenzyl)-5-ethyl-2,3-dihydro-1H-pyrrolo[3,2-b]pyridin-1-yl)(3-methyloxiran-2-yl)methanone | D | 2.158 | 375.2 |
| 17 | 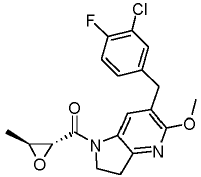 | (6-(3-chloro-4-fluorobenzyl)-5-methoxy-2,3-dihydro-1H-pyrrolo[3,2-b]pyridin-1-yl)((2R,3S)-3-methyloxiran-2-yl)methanone | E | 2.307 | 377.1 |
| 18 | 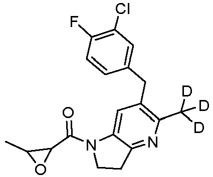 | *Trans*-(6-(3-chloro-4-fluorobenzyl)-5-(methyl-d3)-2,3-dihydro-1H-pyrrolo[3,2-b]pyridin-1-yl)(3-methyloxiran-2-yl)methanone | D | 2.123 | 364.2 |
| 19 | 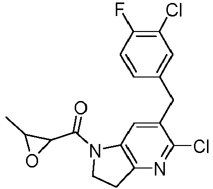 | *Trans*-(5-chloro-6-(3-chloro-4-fluorobenzyl)-2,3-dihydro-1H-pyrrolo[3,2-b]pyridin-1-yl)(3-methyloxiran-2-yl)methanone | D | (2.425 | 381.0 |
| 20 | 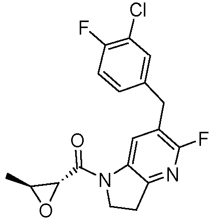 | (6-(3-chloro-4-fluorobenzyl)-5-fluoro-2,3-dihydro-1H-pyrrolo[3,2-b]pyridin-1-yl)((2R,3S)-3-methyloxiran-2-yl)methanone | E | 2.214 | 365.1 |
| 21 | 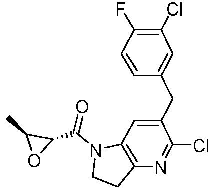 | (5-chloro-6-(3-chloro-4-fluorobenzyl)-2,3-dihydro-1H-pyrrolo[3,2-b]pyridin-1-yl)((2R,3S)-3-methyloxiran-2-yl)methanone | E | 2.276 | 381.0 |

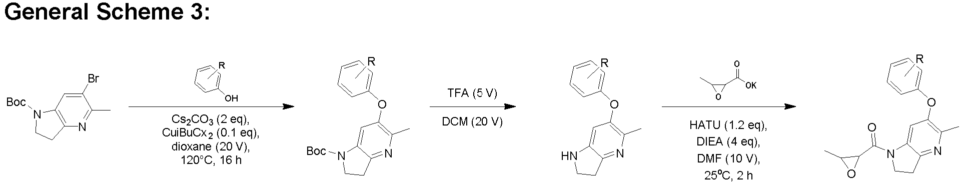

### Example 147 - Step 1: Procedure for preparation of 3a

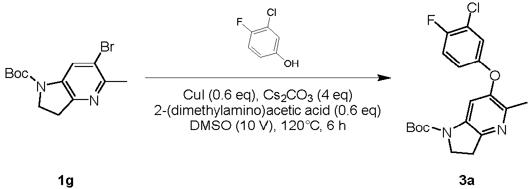

To a solution of **1g** (0.3 g, 1 eq) and 3-chloro-4-fluoro-phenol (280.75 mg, 2 eq) in dimethyl sulfoxide (3 mL) was added cesium carbonate (1.25 g, 4 eq), 2-(dimethylamino)acetic acid (59.27 mg, 0.6 eq) and copper(I) iodide (109.46 mg, 0.6 eq) under a N₂ atmosphere. The reaction mixture was stirred at 120°C for 6 hrs. LCMS showed 15% of desired mass was detected. The reaction mixture was quenched by the addition of water (10 mL), and then extracted with ethyl acetate (3 x 10 mL). The combined organic layers were washed with brine (50 mL), dried over Na₂SO₄, filtered and concentrated under reduced pressure to give a residue. The residue was purified by preparative HPLC (column: CD25-XPT PHS C18 150*25*10um;mobile phase: [water(FA)-CH₃CN];gradient:60%-90% B over 10 min) to give 3a (tert-butyl 6-(3-chloro-4-fluoro-phenoxy)-5-methyl-2,3-dihydropyrrolo[3,2-b]pyridine-1-carboxylate) (25 mg, yield 6.55%) as a brown solid. **¹H NMR** (400 MHz, DMSO-*d*₆) δ ppm 7.59 - 6.72 (m, 4H), 3.95 (br s, 2H), 3.10 (br s, 2H), 2.31 (br s, 3H), 1.47 - 1.30 (m, 9H). **LCMS (ESI+):** m/z: 379.2

### Step 2: Procedure for preparation of 3b.

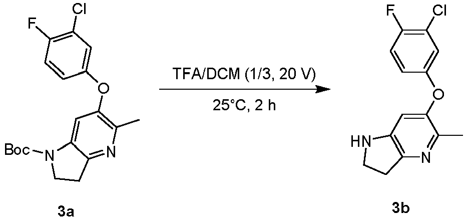

To a solution of **3a** (0.04 g, 105.59 µmol, 1 eq) in dichloromethane (0.6 mL) was added trifluoroacetic acid (307.00 mg, 2.69 mmol, 25.50 eq). The reaction mixture was stirred at 25°C for 2 hrs. LCMS showed the starting material was consumed and 95% of the desired mass was detected. The reaction mixture was concentrated under reduced pressure to give **3b** (6-(3-chloro-4-fluoro-phenoxy)-5-methyl-2,3-dihydro-1H-pyrrolo[3,2-b]pyridine) (0.03 g, yield 91.75%) as a brown oil. **¹H NMR** (400 MHz, METHANOL-*d*₄) δ ppm 7.33 (t, *J =* 8.8 Hz, 1H), 7.28 (dd, *J* = 2.8, 6.2 Hz, 1H), 7.08 (td, *J* = 3.2, 9.2 Hz, 1H), 6.83 (s, 1H), 3.84 (t, *J = 8.8* Hz, 2H), 3.37 (t, *J* = 8.8 Hz, 2H), 2.46 (s, 3H). **LCMS (ESI+):** m/z: 279.1

### Step 3: Procedure for preparation of Example 147

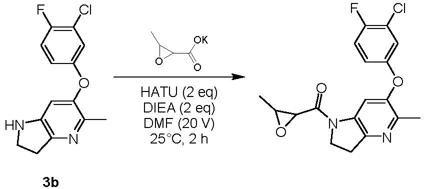

To a solution of *trans*-(3-methyloxirane-2-carbonyl)oxypotassium (30.18 mg, 215.28 µmol, 2 eq) and **3b** (0.03 g, 107.64 µmol, 1 eq) in N,N-dimethylformamide (0.6 mL) was added 2-(3H-[1,2,3]triazolo[4,5-b]pyridin-3-yl)-1,1,3,3-tetramethyluronium hexafluorophosphate(V) (81.86 mg, 215.28 µmol, 2 eq) and N-ethyl-N-isopropylpropan-2-amine (27.82 mg, 215.28 µmol, 2 eq). The reaction mixture was stirred at 25°C for 2 h. LCMS showed the starting material was consumed and 92% of the desired mass was detected. The reaction mixture was quenched by the addition of water (2 mL) and then extracted with ethyl acetate (3 x 2 mL). The combined organic layers were washed with brine (10 mL), dried over Na₂SO₄, filtered and concentrated under reduced pressure to give a residue. The residue was purified by preparative HPLC (column: CD25-XPT PHS C18 150*25*10um;mobile phase: [water(FA)-ACN];gradient:35%-65% B over 10 min) to give **Example 147** ([6-(3-chloro-4-fluoro-phenoxy)-5-methyl-2,3-dihydropyrrolo[3,2-b]pyridin-1-yl]-(3-methyloxiran-2-yl)methanone) (21.35 mg, yield 54.56%) as a white solid. **¹H NMR** (400 MHz, DMSO-*d*₆) δ ppm 7.73 (s, 1H), 7.43 (t, *J =* 9.2 Hz, 1H), 7.23 (dd, *J =* 2.8, 6.0 Hz, 1H), 6.97 (td, *J =* 3.6, 9.2 Hz, 1H), 4.48 - 4.39 (m, 1H), 4.33 - 4.25 (m, 1H), 3.65 (d, *J* = 1.6 Hz, 1H), 3.24 (t, *J =* 8.8 Hz, 2H), 3.16 - 3.11 (m, 1H), 2.33 (s, 3H), 1.34 (d, *J =* 5.2 Hz, 3H). **LCMS (ESI+):** m/z 363.1.

**Example 147** was separated by SFC (column: DAICEL CHIRALPAK AD(250mm*30mm,10um);mobile phase: [CO₂-EtOH];B%:50%, isocratic elution mode) to obtain **Example 148** [(2R,3S)-3-(azetidin-1-ylmethyl)oxiran-2-yl]-[6-(3-chloro-4-fluoro-phenoxy)-5-methyl-2,3-dihydropyrrolo[3,2-b]pyridin-1-yl]methanone.

The following examples were prepared using **Scheme 3.**

| Example | Structure | Chemical name | LC-MS method | LC-MS retention time (mins) | Mass found (M+H) ⁺ |
|---|---|---|---|---|---|
| 22 | 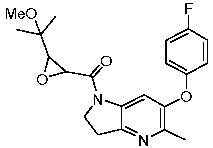 | *Trans*-(6-(4-fluorophenoxy)-5-methyl-2,3-dihydro-1H-pyrrolo[3,2-b]pyridin-1-yl)(3-(2-methoxypropan-2-yl)oxiran-2-yl)methanone | Q | 3.468 | 387.3 |
| 23 | 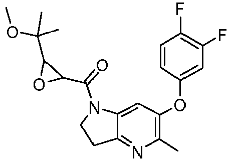 | *Trans*-(6-(3,4-difluorophenoxy)-5-methyl-2,3-dihydro-1H-pyrrolo[3,2-b]pyridin-1-yl)(3-(2-methoxypropan-2-yl)oxiran-2-yl)methanone | O | 2.895 | 405.3 |
| 24 | 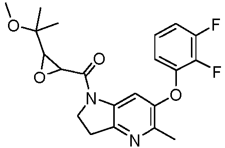 | *Trans*-(6-(2,3-difluorophenoxy)-5-methyl-2,3-dihydro-1H-pyrrolo[3,2-b]pyridin-1-yl)(3-(2-methoxypropan-2-yl)oxiran-2-yl)methanone | E | 2.053 | 405.1 |
| 25 | 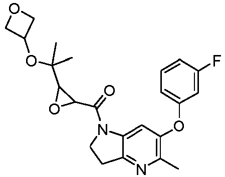 | *Trans*-(6-(3-fluorophenoxy)-5-methyl-2,3-dihydro-1H-pyrrolo[3,2-b]pyridin-1-yl)(3-(2-(oxetan-3-yloxy)propan-2-yl)oxiran-2-yl)methanone | S | 2.025 | 429.3 |
| 26 | 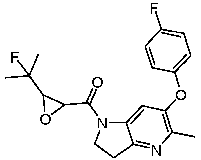 | *Trans*-(6-(4-fluorophenoxy)-5-methyl-2,3-dihydro-1H-pyrrolo[3,2-b]pyridin-1-yl)(3-(2-fluoropropan-2-yl)oxiran-2-yl)methanone | D | 2.343 | 375.3 |
| 27 | 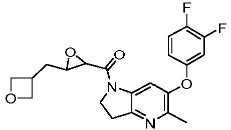 | *Trans*-(6-(3,4-difluorophenoxy)-5-methyl-2,3-dihydro-1H-pyrrolo[3,2-b]pyridin-1-yl)(3-(oxetan-3-ylmethyl)oxiran-2-yl)methanone | B | 2.124 | 403.2 |
| 28 | 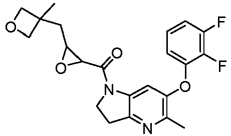 | *Trans*-(6-(2,3-difluorophenoxy)-5-methyl-2,3-dihydro-1H-pyrrolo[3,2-b]pyridin-1-yl)(3-((3-methyloxetan-3-yl)methyl)oxiran-2-yl)methanone | D | 2.372 | 417.3 |
| 29 | 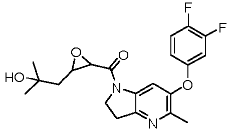 | *Trans*-(6-(3,4-difluorophenoxy)-5-methyl-2,3-dihydro-1H-pyrrolo[3,2-b]pyridin-1-yl)(3-(2-hydroxy-2-methylpropyl)oxiran -2-yl)methanone | D | 2.182 | 405.2 |
| 30 | 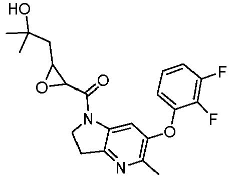 | *Trans*-(6-(2,3-difluorophenoxy)-5-methyl-2,3-dihydro-1H-pyrrolo[3,2-b]pyridin-1-yl)(3-(2-hydroxy-2-methylpropyl)oxiran -2-yl)methanone | E | 1.837 | 405.1 |
| 31 | 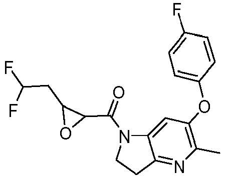 | *Trans*-(3-(2,2-difluoroethyl)oxiran-2-yl)(6-(4-fluorophenoxy)-5-methyl-2,3-dihydro-1H-pyrrolo[3,2-b]pyridin-1-yl)methanone | E | 1.956 | 379.1 |
| 32 | 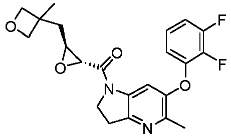 | (6-(2,3-difluorophenoxy)-5-methyl-2,3-dihydro-1H-pyrrolo[3,2-b]pyridin-1-yl)((2R,3S)-3-((3-methyloxetan-3-yl)methyl)oxiran-2-yl)methanone | E | 1.945 | 417.0 |
| 33 | 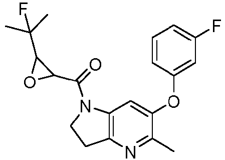 | *Trans*-(6-(3-fluorophenoxy)-5-methyl-2,3-dihydro-1H-pyrrolo[3,2-b]pyridin-1-yl)(3-(2-fluoropropan-2-yl)oxiran-2-yl)methanone | B | 2.311 | 375.0 |
| 34 | 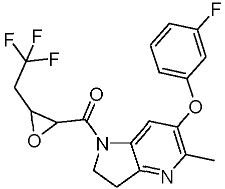 | *Trans*-(6-(3-fluorophenoxy)-5-methyl-2,3-dihydro-1H-pyrrolo[3,2-b]pyridin-1-yl)(3-(2,2,2-trifluoroethyl)oxiran-2-yl)methanone | B | 2.140 | 355.1 |
| 35 | 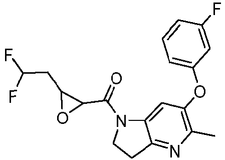 | *Trans*-(3-(2,2-difluoroethyl)oxiran-2-yl)(6-(3-fluorophenoxy)-5-methyl-2,3-dihydro-1H-pyrrolo[3,2-b]pyridin-1-yl)methanone | E | 2.002 | 379.0 |
| 36 | 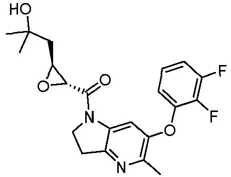 | (6-(2,3-difluorophenoxy)-5-methyl-2,3-dihydro-1H-pyrrolo[3,2-b]pyridin-1-yl)((2R,3S)-3-(2-hydroxy-2-methylpropyl)oxiran -2-yl)methanone | E | 1.834 | 405.1 |
| 37 | 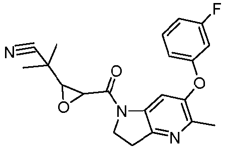 | *Trans*-2-(3-(6-(3-fluorophenoxy)-5-methyl-2,3-dihydro-1H-pyrrolo[3,2-b]pyridine-1-carbonyl)oxiran-2-yl)-2-methylpropanenitril e | D | 2.369 | 382.3 |
| 38 | 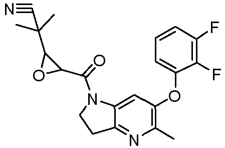 | *Trans*-2-(3-(6-(2,3-difluorophenoxy)-5-methyl-2,3-dihydro-1H-pyrrolo[3,2-b]pyridine-1-carbonyl)oxiran-2-yl)-2-methylpropanenitril e | E | 2.092 | 400.1 |
| 39 | 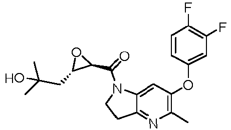 | (6-(3,4-difluorophenoxy)-5-methyl-2,3-dihydro-1H-pyrrolo[3,2-b]pyridin-1-yl)((2R,3S)-3-(2-hydroxy-2-methylpropyl)oxiran -2-yl)methanone | E | 1.665 | 448.3 |
| 40 | 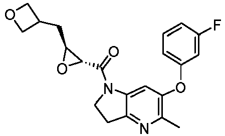 | (6-(3-fluorophenoxy)-5-methyl-2,3-dihydro-1H-pyrrolo[3,2-b]pyridin-1-yl)((2R,3S)-3-(oxetan-3-ylmethyl)oxiran-2-yl)methanone | E | 1.789 | 385.1 |
| 41 | 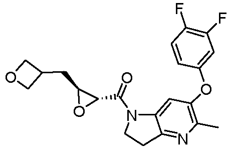 | (6-(3,4-difluorophenoxy)-5-methyl-2,3-dihydro-1H-pyrrolo[3,2-b]pyridin-1-yl)((2R,3S)-3-(oxetan-3-ylmethyl)oxiran-2-yl)methanone | D | 2.248 | 403.3 |
| 42 | 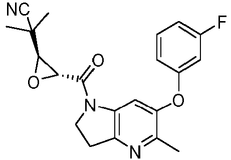 | 2-((2S,3R)-3-(6-(3-fluorophenoxy)-5-methyl-2,3-dihydro-1H-pyrrolo[3,2-b]pyridine-1-carbonyl)oxiran-2-yl)-2-methylpropanenitril e | E | 2.041 | 382.1 |
| 43 | 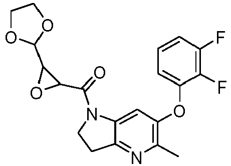 | *Trans*-(3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone | D | 2.322 | 405.3 |
| 44 | 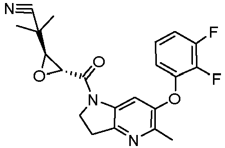 | 2-((2S,3R)-3-(6-(2,3-difluorophenoxy)-5-methyl-2,3-dihydro-1H-pyrrolo[3,2-b]pyridine-1-carbonyl)oxiran-2-yl)-2-methylpropanenitril e | E | 2.088 | 400.0 |
| 45 | 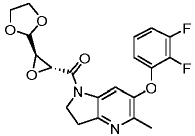 | ((2R,3R)-3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone | B | 2.856 | 405.3 |
| 46 | 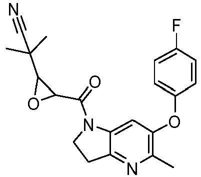 | *Trans*-2-(3-(6-(4-fluorophenoxy)-5-methyl-2,3-dihydro-1H-pyrrolo[3,2-b]pyridine-1-carbonyl)oxiran-2-yl)-2-methylpropanenitril e | D | 2.325 | 382.3 |
| 47 | 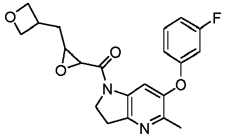 | *Trans*-(6-(3-fluorophenoxy)-5-methyl-2,3-dihydro-1H-pyrrolo[3,2-b]pyridin-1-yl)(3-(oxetan-3-ylmethyl)oxiran-2-yl)methanone | B | 2.067 | 385.2 |
| 48 | 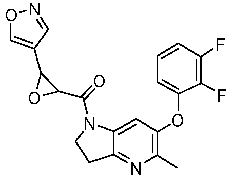 | *Trans*-(6-(2,3-difluorophenoxy)-5-methyl-2,3-dihydro-1H-pyrrolo[3,2-b]pyridin-1-yl)(3-(isoxazol-4-yl)oxiran-2-yl)methanone | D | 2.675 | 400.2 |
| 49 | 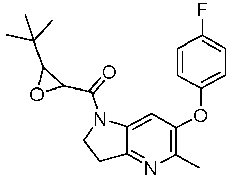 | *Trans*-(3-(tert-butyl)oxiran-2-yl)(6-(4-fluorophenoxy)-5-methyl-2,3-dihydro-1H-pyrrolo[3,2-b]pyridin-1-yl)methanone | B | 2.392 | 371.2 |
| 50 | 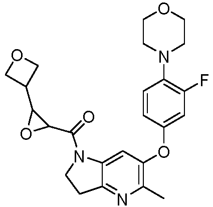 | *Trans*-(6-(3-fluoro-4-morpholinophenoxy )-5-methyl-2,3-dihydro-1H-pyrrolo[3,2-b]pyridin-1-yl)(3-(oxetan-3-yl)oxiran-2-yl)methanone | Q | 2.919 | 456.3 |
| 51 | 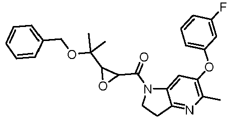 | *Trans*-(3-(2-(benzyloxy)propan-2-yl)oxiran-2-yl)(6-(3-fluorophenoxy)-5-methyl-2,3-dihydro-1H-pyrrolo[3,2-b]pyridin-1-yl)methanone | D | 2.723 | 463.4 |
| 52 | 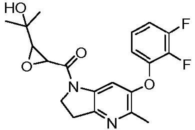 | *Trans*-(6-(2,3-difluorophenoxy)-5-methyl-2,3-dihydro-1H-pyrrolo[3,2-b]pyridin-1-yl)(3-(2-hydroxypropan-2-yl)oxiran-2-yl)methanone | H | 2.644 | 391.1 |
| 53 | 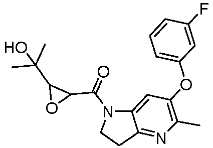 | *Trans*-(6-(3-fluorophenoxy)-5-methyl-2,3-dihydro-1H-pyrrolo[3,2-b]pyridin-1-yl)(3-(2-hydroxypropan-2-yl)oxiran-2-yl)methanone | Q | 3.129 | 373.2 |
| 54 | 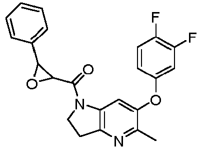 | *Trans*-(6-(3,4-difluorophenoxy)-5-methyl-2,3-dihydro-1H-pyrrolo[3,2-b]pyridin-1-yl)(3-phenyloxiran-2-yl)methanone | K | 2.696 | 409.3 |
| 55 | 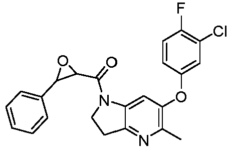 | *Trans*-(6-(3-chloro-4-fluorophenoxy)-5-methyl-2,3-dihydro-1H-pyrrolo[3,2-b]pyridin-1-yl)(3-phenyloxiran-2-yl)methanone | D | 2.723 | 425.2 |
| 56 | 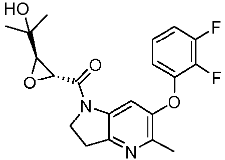 | (6-(2,3-difluorophenoxy)-5-methyl-2,3-dihydro-1H-pyrrolo[3,2-b]pyridin-1-yl)((2R,3R)-3-(2-hydroxypropan-2-yl)oxiran-2-yl)methanone | H | 2.629 | 391.1 |
| 57 | 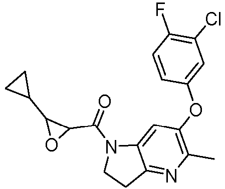 | *Trans*-(6-(3-chloro-4-fluorophenoxy)-5-methyl-2,3-dihydro-1H-pyrrolo[3,2-b]pyridin-1-yl)(3-cyclopropyloxiran-2-yl)methanone | M | 2.185 | 389.2 |
| 58 | 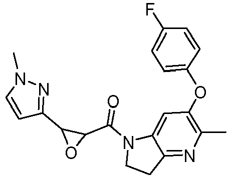 | *Trans*-(6-(4-fluorophenoxy)-5-methyl-2,3-dihydro-1H-pyrrolo[3,2-b]pyridin-1-yl)(3-(1-methyl-1H-pyrazol-3-yl)oxiran-2-yl)methanone | H | 2.661 | 395.1 |
| 59 | 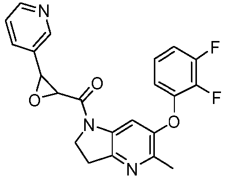 | *Trans*-(6-(2,3-difluorophenoxy)-5-methyl-2,3-dihydro-1H-pyrrolo[3,2-b]pyridin-1-yl)(3-(pyridin-3-yl)oxiran-2-yl)methanone | H | 2.726 | 410.1 |
| 60 | 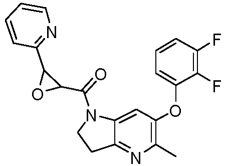 | *Trans*-(6-(2,3-difluorophenoxy)-5-methyl-2,3-dihydro-1H-pyrrolo[3,2-b]pyridin-1-yl)(3-(pyridin-2-yl)oxiran-2-yl)methanone | N | 2.978 | 410.2 |
| 61 | 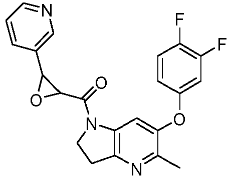 | *Trans*-(6-(3,4-difluorophenoxy)-5-methyl-2,3-dihydro-1H-pyrrolo[3,2-b]pyridin-1-yl)(3-(pyridin-3-yl)oxiran-2-yl)methanone | K | 2.539 | 410.2 |
| 62 | 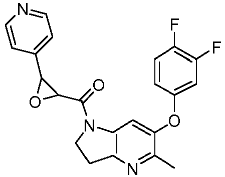 | *Trans*-(6-(3,4-difluorophenoxy)-5-methyl-2,3-dihydro-1H-pyrrolo[3,2-b]pyridin-1-yl)(3-(pyridin-4-yl)oxiran-2-yl)methanone | D | 1.987 | 410.3 |
| 63 | 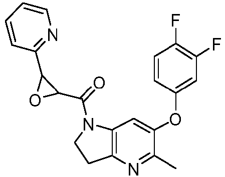 | *Trans*-(6-(3,4-difluorophenoxy)-5-methyl-2,3-dihydro-1H-pyrrolo[3,2-b]pyridin-1-yl)(3-(pyridin-2-yl)oxiran-2-yl)methanone | D | 2.343 | 410.3 |
| 64 | 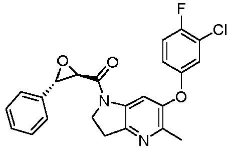 | (6-(3-chloro-4-fluorophenoxy)-5-methyl-2,3-dihydro-1H-pyrrolo[3,2-b]pyridin-1-yl)((2R,3S)-3-phenyloxiran-2-yl)methanone | E | 2.373 | 425.2 |
| 65 | 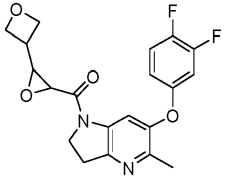 | *Trans*-(6-(3,4-difluorophenoxy)-5-methyl-2,3-dihydro-1H-pyrrolo[3,2-b]pyridin-1-yl)(3-(oxetan-3-yl)oxiran-2-yl)methanone | B | 2.085 | 389.2 |
| 66 | 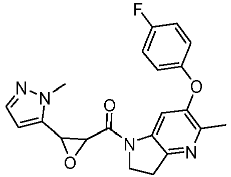 | *Trans*-(6-(4-fluorophenoxy)-5-methyl-2,3-dihydro-1H-pyrrolo[3,2-b]pyridin-1-yl)(3-(1-methyl-1H-pyrazol-5-yl)oxiran-2-yl)methanone | E | 1.849 | 395.0 |
| 67 | 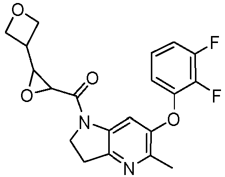 | *Trans*-(6-(2,3-difluorophenoxy)-5-methyl-2,3-dihydro-1H-pyrrolo[3,2-b]pyridin-1-yl)(3-(oxetan-3-yl)oxiran-2-yl)methanone | E | 1.812 | 389.0 |
| 68 | 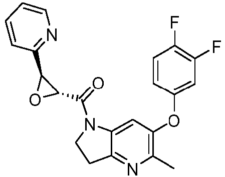 | (6-(3,4-difluorophenoxy)-5-methyl-2,3-dihydro-1H-pyrrolo[3,2-b]pyridin-1-yl)((2R,3S)-3-(pyridin-2-yl)oxiran-2-yl)methanone | U | 2.928 | 410.2 |
| 69 | 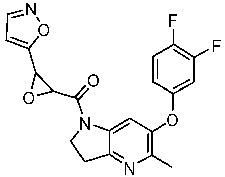 | *Trans*-(6-(3,4-difluorophenoxy)-5-methyl-2,3-dihydro-1H-pyrrolo[3,2-b]pyridin-1-yl)(3-(isoxazol-5-yl)oxiran-2-yl)methanone | E | 2.026 | 400.1 |
| 70 | 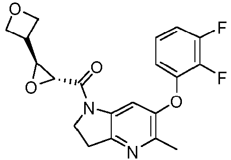 | (6-(2,3-difluorophenoxy)-5-methyl-2,3-dihydro-1H-pyrrolo[3,2-b]pyridin-1-yl)((2R,3S)-3-(oxetan-3-yl)oxiran-2-yl)methanone | E | 1.812 | 389.0 |
| 71 | 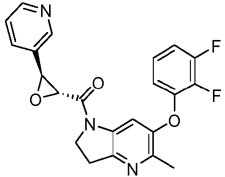 | (6-(2,3-difluorophenoxy)-5-methyl-2,3-dihydro-1H-pyrrolo[3,2-b]pyridin-1-yl)((2R,3S)-3-(pyridin-3-yl)oxiran-2-yl)methanone | H | 2.724 | 410.1 |
| 72 | 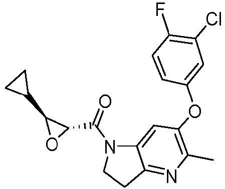 | (6-(3-chloro-4-fluorophenoxy)-5-methyl-2,3-dihydro-1H-pyrrolo[3,2-b]pyridin-1-yl)((2R,3S)-3-cyclopropyloxiran-2-yl)methanone | H | 3.007 | 389.1 |
| 73 | 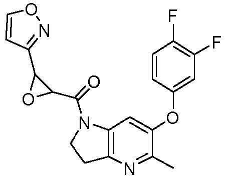 | (6-(3,4-difluorophenoxy)-5-methyl-2,3-dihydro-1H-pyrrolo[3,2-b]pyridin-1-yl)(3-(isoxazol-3-yl)oxiran-2-yl)methanone | E | 2.018 | 400.1 |
| 74 | 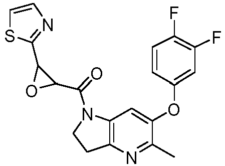 | *Trans*-(6-(3,4-difluorophenoxy)-5-methyl-2,3-dihydro-1H-pyrrolo[3,2-b]pyridin-1-yl)(3-(isoxazol-5-yl)oxiran-2-yl)methanone | E | 2.013 | 416.1 |
| 75 | 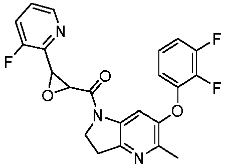 | *Trans*-(6-(2,3-difluorophenoxy)-5-methyl-2,3-dihydro-1H-pyrrolo[3,2-b]pyridin-1-yl)(3-(3-fluoropyridin-2-yl)oxiran-2-yl)methanone | O | 2.870 | 428.2 |
| 76 | 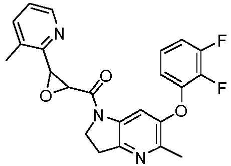 | *Trans*-(6-(2,3-difluorophenoxy)-5-methyl-2,3-dihydro-1H-pyrrolo[3,2-b]pyridin-1-yl)(3-(3-methylpyridin-2-yl)oxiran-2-yl)methanone | Q | 3.127 | 424.3 |
| 77 | 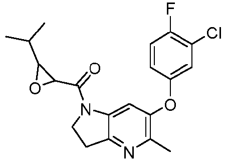 | *Trans*-(6-(3-chloro-4-fluorophenoxy)-5-methyl-2,3-dihydro-1H-pyrrolo[3,2-b]pyridin-1-yl)(3-isopropyloxiran-2-yl)methanone | D | 2.605 | 391.2 |
| 78 | 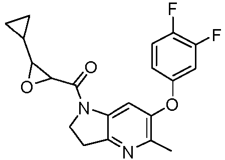 | *Trans*-(3-cyclopropyloxiran-2-yl)(6-(3,4-difluorophenoxy)-5-methyl-2,3-dihydro-1H-pyrrolo[3,2-b]pyridin-1-yl)methanone | J | 3.173 | 373.3 |
| 79 | 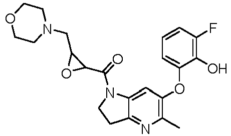 | *Trans*-(6-(3-fluoro-2-hydroxyphenoxy)-5-methyl-2,3-dihydro-1H-pyrrolo[3,2-b]pyridin-1-yl)(3-(morpholinomethyl) oxiran-2-yl)methanone | Q | 2.267 | 430.3 |
| 80 | 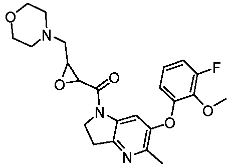 | *Trans*-(6-(3-fluoro-2-methoxyphenoxy)-5-methyl-2,3-dihydro-1H-pyrrolo[3,2-b]pyridin-1-yl)(3-(morpholinomethyl) oxiran-2-yl)methanone | D | 1.756 | 444.3 |
| 81 | 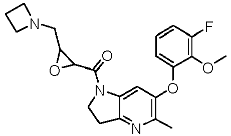 | *Trans*-(3-(azetidin-1-ylmethyl)oxiran-2-yl)(6-(3-fluoro-2-methoxyphenoxy)-5-methyl-2,3-dihydro-1H-pyrrolo[3,2-b]pyridin-1-yl)methanone | P | 3.247 | 414.3 |
| 82 | 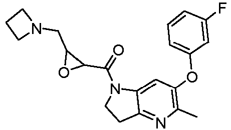 | *Trans*-(3-(azetidin-1-ylmethyl)oxiran-2-yl)(6-(3-fluorophenoxy)-5-methyl-2,3-dihydro-1H-pyrrolo[3,2-b]pyridin-1-yl)methanone | E | 1.539 | 384.1 |
| 83 | 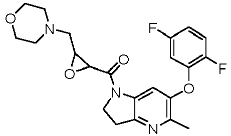 | *Trans*-(6-(2,5-difluorophenoxy)-5-methyl-2,3-dihydro-1H-pyrrolo[3,2-b]pyridin-1-yl)(3-(morpholinomethyl) oxiran-2-yl)methanone | D | 1.791 | 432.3 |
| 84 | 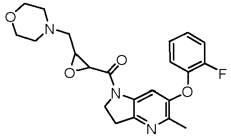 | *Trans*-(6-(2-fluorophenoxy)-5-methyl-2,3-dihydro-1H-pyrrolo[3,2-b]pyridin-1-yl)(3-(morpholinomethyl) oxiran-2-yl)methanone | D | 1.729 | 414.4 |
| 85 | 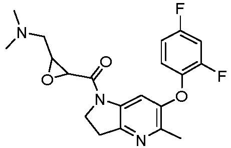 | *Trans*-(6-(2,4-difluorophenoxy)-5-methyl-2,3-dihydro-1H-pyrrolo[3,2-b]pyridin-1-yl)(3-((dimethylamino)me thyl)oxiran-2-yl)methanone | H | 2.605 | 390.1 |
| 86 | 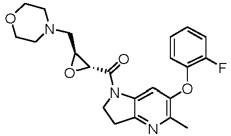 | (6-(2-fluorophenoxy)-5-methyl-2,3-dihydro-1H-pyrrolo[3,2-b]pyridin-1-yl)((2R,3S)-3-(morpholinomethyl) oxiran-2-yl)methanone | H | 2.514 | 414.2 |
| 87 | 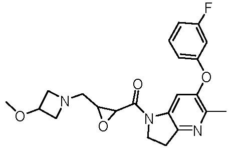 | *Trans*-(6-(3-fluorophenoxy)-5-methyl-2,3-dihydro-1H-pyrrolo[3,2-b]pyridin-1-yl)(3-((3-methoxyazetidin-1-yl)methyl)oxiran-2-yl)methanone | E | 1.581 | 414.1 |
| 88 | 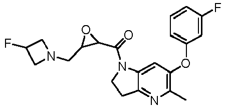 | *Trans*-(3-((3-fluoroazetidin-1-yl)methyl)oxiran-2-yl)(6-(3-fluorophenoxy)-5-methyl-2,3-dihydro-1H-pyrrolo[3,2-b]pyridin-1-yl)methanone | E | 1.529 | 402.1 |
| 89 | 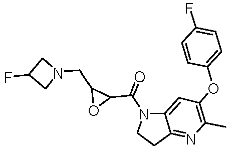 | *Trans*-(3-((3-fluoroazetidin-1-yl)methyl)oxiran-2-yl)(6-(4-fluorophenoxy)-5-methyl-2,3-dihydro-1H-pyrrolo[3,2-b]pyridin-1-yl)methanone | K | 2.358 | 402.3 |
| 90 | 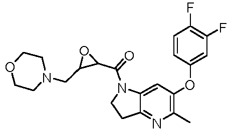 | *Trans*-(6-(3,4-difluorophenoxy)-5-methyl-2,3-dihydro-1H-pyrrolo[3,2-b]pyridin-1-yl)(3-(morpholinomethyl)oxiran-2-yl)methanone | D | 1.878 | 432.2 |
| 91 | 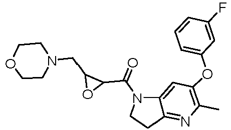 | *Trans*-(6-(3-fluorophenoxy)-5-methyl-2,3-dihydro-1H-pyrrolo[3,2-b]pyridin-1-yl)(3-(morpholinomethyl) oxiran-2-yl)methanone | H | 2.573 | 414.2 |
| 92 | 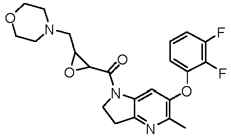 | *Trans*-(6-(2,3-difluorophenoxy)-5-methyl-2,3-dihydro-1H-pyrrolo[3,2-b]pyridin-1-yl)(3-(morpholinomethyl) oxiran-2-yl)methanone | H | 2.601 | 432.1 |
| 93 | 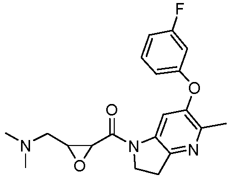 | *Trans*-(3-((dimethylamino)me thyl)oxiran-2-yl)(6-(3-fluorophenoxy)-5-methyl-2,3-dihydro-1H-pyrrolo[3,2-b]pyridin-1-yl)methanone | E | 1.510 | 372.1 |
| 94 | 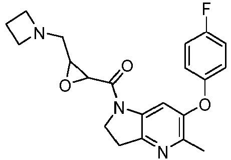 | *Trans*-(3-(azetidin-1-ylmethyl)oxiran-2-yl)(6-(4-fluorophenoxy)-5-methyl-2,3-dihydro-1H-pyrrolo[3,2-b]pyridin-1-yl)methanone | D | 1.790 | 384.2 |
| 95 | 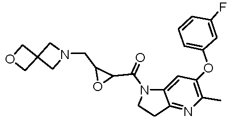 | *Trans*-(3-((2-oxa-6-azaspiro[3.3]heptan -6-yl)methyl)oxiran-2-yl)(6-(3-fluorophenoxy)-5-methyl-2,3-dihydro-1H-pyrrolo[3,2-b]pyridin-1-yl)methanone | E | 1.478 | 426.1 |
| 96 | 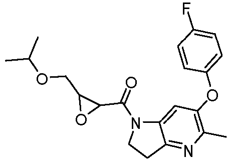 | *Trans*-(6-(4-fluorophenoxy)-5-methyl-2,3-dihydro-1H-pyrrolo[3,2-b]pyridin-1-yl)(3-(isopropoxymethyl)oxiran-2-yl)methanone | K | 2.531 | 387.2 |
| 97 | 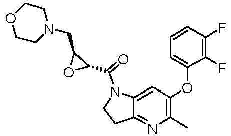 | (6-(2,3-difluorophenoxy)-5-methyl-2,3-dihydro-1H-pyrrolo[3,2-b]pyridin-1-yl)((2R,3S)-3-(morpholinomethyl) oxiran-2-yl)methanone | H | 2.601 | 432.1 |
| 98 | 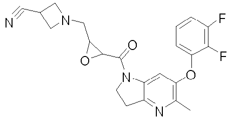 | *Trans*-1-((3-(6-(2,3-difluorophenoxy)-5-methyl-2,3-dihydro-1H-pyrrolo[3,2-b]pyridine-1-carbonyl)oxiran-2-yl)methyl)azetidine-3-carbonitrile | H | 2.653 | 427.2 |
| 99 | 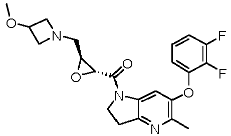 | (6-(2,3-difluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone | H | 2.620 | 432.2 |
| 100 | 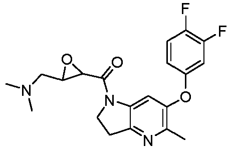 | *Trans*-(6-(3,4-difluorophenoxy)-5-methyl-2,3-dihydro-1H-pyrrolo[3,2-b]pyridin-1-yl)(3-((dimethylamino)me thyl)oxiran-2-yl)methanone | Q | 2.720 | 390.1 |
| 101 | 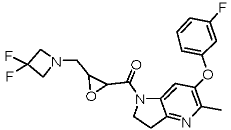 | *Trans*-(3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone | H | 2.788 | 420.1 |
| 102 | 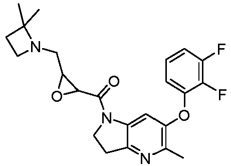 | *Trans*-(6-(2,3-difluorophenoxy)-5-methyl-2,3-dihydro-1H-pyrrolo[3,2-b]pyridin-1-yl)(3-((2,2-dimethylazetidin-1-yl)methyl)oxiran-2-yl)methanone | H | 2.827 | 430.2 |
| 103 | 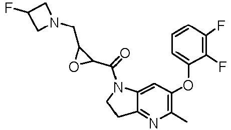 | *Trans*-(6-(2,3-difluorophenoxy)-5-methyl-2,3-dihydro-1H-pyrrolo[3,2-b]pyridin-1-yl)(3-((3-fluoroazetidin-1-yl)methyl)oxiran-2-yl)methanone | E | 1.586 | 420.1 |
| 104 | 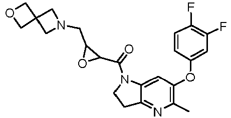 | *Trans*-(3-((2-oxa-6-azaspiro[3.3]heptan -6-yl)methyl)oxiran-2-yl)(6-(3,4-difluorophenoxy)-5-methyl-2,3-dihydro-1H-pyrrolo[3,2-b]pyridin-1-yl)methanone | Q | 2.729 | 444.3 |
| 105 | 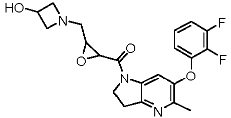 | *Trans*-(6-(2,3-difluorophenoxy)-5-methyl-2,3-dihydro-1H-pyrrolo[3,2-b]pyridin-1-yl)(3-((3-hydroxyazetidin-1-yl)methyl)oxiran-2-yl)methanone | H | 2.455 | 418.2 |
| 106 | 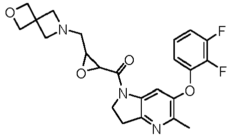 | *Trans*-(3-((2-oxa-6-azaspiro[3.3]heptan -6-yl)methyl)oxiran-2-yl)(6-(2,3-difluorophenoxy)-5-methyl-2,3-dihydro-1H-pyrrolo[3,2-b]pyridin-1-yl)methanone | H | 2.543 | 444.1 |
| 107 | 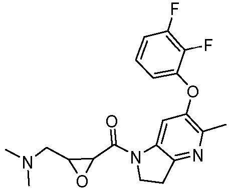 | *Trans*-(6-(2,3-difluorophenoxy)-5-methyl-2,3-dihydro-1H-pyrrolo[3,2-b]pyridin-1-yl)(3-((dimethylamino)me thyl)oxiran-2-yl)methanone | E | 1.577 | 390.1 |
| 108 | 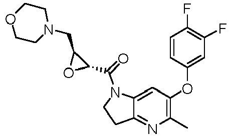 | (6-(3,4-difluorophenoxy)-5-methyl-2,3-dihydro-1H-pyrrolo[3,2-b]pyridin-1-yl)((2R,3S)-3-(morpholinomethyl)oxiran-2-yl)methanone | K | 2.291 | 432.3 |
| 109 | 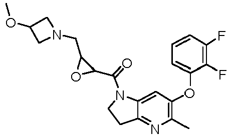 | *Trans*-(6-(2,3-difluorophenoxy)-5-methyl-2,3-dihydro-1H-pyrrolo[3,2-b]pyridin-1-yl)(3-((3-methoxyazetidin-1-yl)methyl)oxiran-2-yl)methanone | H | 2.623 | 432.2 |
| 110 | 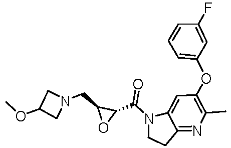 | (6-(3-fluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone | E | 1.584 | 414.1 |
| 111 | 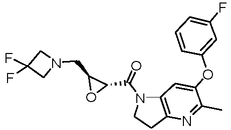 | ((2R,3S)-3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone | H | 2.783 | 420.1 |
| 112 | 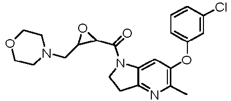 | *Trans*-(6-(3-chlorophenoxy)-5-methyl-2,3-dihydro-1H-pyrrolo[3,2-b]pyridin-1-yl)(3-(morpholinomethyl) oxiran-2-yl)methanone | D | 1.875 | 430.2 |
| 113 | 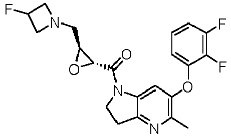 | (6-(2,3-difluorophenoxy)-5-methyl-2,3-dihydro-1H-pyrrolo[3,2-b]pyridin-1-yl)((2R,3S)-3-((3-fluoroazetidin-1-yl)methyl)oxiran-2-yl)methanone | E | 1.605 | 420.0 |
| 114 | 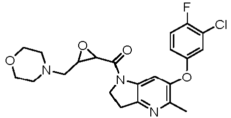 | *Trans*-(6-(3-chloro-4-fluorophenoxy)-5-methyl-2,3-dihydro-1H-pyrrolo[3,2-b]pyridin-1-yl)(3-(morpholinomethyl)oxiran-2-yl)methanone | E | 1.649 | 448.2 |
| 115 | 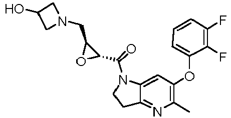 | (6-(2,3-difluorophenoxy)-5-methyl-2,3-dihydro-1H-pyrrolo[3,2-b]pyridin-1-yl)((2R,3S)-3-((3-hydroxyazetidin-1-yl)methyl)oxiran-2-yl)methanone | H | 2.458 | 418.1 |
| 116 | 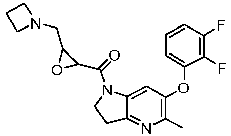 | *Trans*-(3-(azetidin-1-ylmethyl)oxiran-2-yl)(6-(2,3-difluorophenoxy)-5-methyl-2,3-dihydro-1H-pyrrolo[3,2-b]pyridin-1-yl)methanone | E | 1.592 | 402.1 |
| 117 | 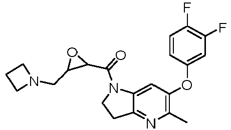 | *Trans*-(3-(azetidin-1-ylmethyl)oxiran-2-yl)(6-(3,4-difluorophenoxy)-5-methyl-2,3-dihydro-1H-pyrrolo[3,2-b]pyridin-1-yl)methanone | D | 1.774 | 402.1 |
| 118 | 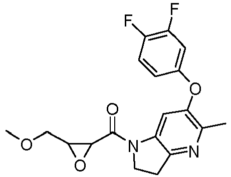 | *Trans*-(6-(3,4-difluorophenoxy)-5-methyl-2,3-dihydro-1H-pyrrolo[3,2-b]pyridin-1-yl)(3-(methoxymethyl)oxi ran-2-yl)methanone | B | 2.159 | 377.2 |
| 119 | 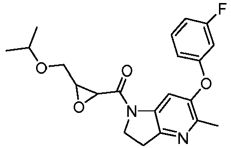 | *Trans*-(6-(3-fluorophenoxy)-5-methyl-2,3-dihydro-1H-pyrrolo[3,2-b]pyridin-1-yl)(3-(isopropoxymethyl) oxiran-2-yl)methanone | E | 2.065 | 387.2 |
| 120 | 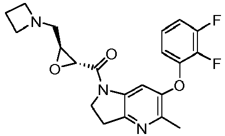 | ((2R,3S)-3-(azetidin-1-ylmethyl)oxiran-2-yl)(6-(2,3-difluorophenoxy)-5-methyl-2,3-dihydro-1H-pyrrolo[3,2-b]pyridin-1-yl)methanone | E | 1.594 | 402.2 |
| 121 | 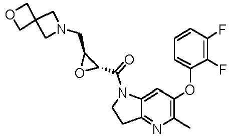 | ((2R,3S)-3-((2-oxa-6-azaspiro[3.3]heptan -6-yl)methyl)oxiran-2-yl)(6-(2,3-difluorophenoxy)-5-methyl-2,3-dihydro-1H-pyrrolo[3,2-b]pyridin-1-yl)methanone | H | 2.547 | 444.2 |
| 122 | 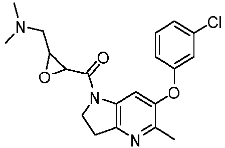 | *Trans*-(6-(3-chlorophenoxy)-5-methyl-2,3-dihydro-1H-pyrrolo[3,2-b]pyridin-1-yl)(3-((dimethylamino)me thyl)oxiran-2-yl)methanone | R | 2.915 | 388.1 |
| 123 | 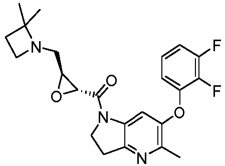 | (6-(2,3-difluorophenoxy)-5-methyl-2,3-dihydro-1H-pyrrolo[3,2-b]pyridin-1-yl)((2R,3S)-3-((2,2-dimethylazetidin-1-yl)methyl)oxiran-2-yl)methanone | E | 1.655 | 430.2 |
| 124 | 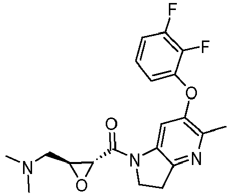 | (6-(2,3-difluorophenoxy)-5-methyl-2,3-dihydro-1H-pyrrolo[3,2-b]pyridin-1-yl)((2R,3S)-3-((dimethylamino)me thyl)oxiran-2-yl)methanone | E | 1.566 | 390.1 |
| 125 | 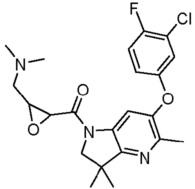 | *Trans*-(6-(3-chloro-4-fluorophenoxy)-3,3,5-trimethyl-2,3-dihydro-1H-pyrrolo[3,2-b]pyridin-1-yl)(3-((dimethylamino)me thyl)oxiran-2-yl)methanone | E | 1.822 | 434.3 |
| 126 | 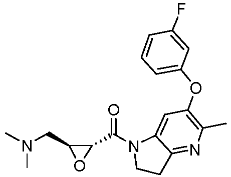 | ((2R,3S)-3-((dimethylamino)me thyl)oxiran-2-yl)(6-(3-fluorophenoxy)-5-methyl-2,3-dihydro-1H-pyrrolo[3,2-b]pyridin-1-yl)methanone | E | 1.527 | 372.1 |
| 127 | 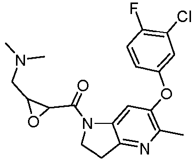 | *Trans*-(6-(3-chloro-4-fluorophenoxy)-5-methyl-2,3-dihydro-1H-pyrrolo[3,2-b]pyridin-1-yl)(3-((dimethylamino)me thyl)oxiran-2-yl)methanone | H | 2.769 | 405.1 |
| 128 | 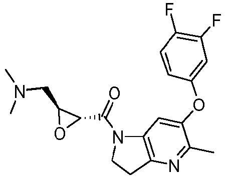 | (6-(3,4-difluorophenoxy)-5-methyl-2,3-dihydro-1H-pyrrolo[3,2-b]pyridin-1-yl)((2R,3S)-3-((dimethylamino)me thyl)oxiran-2-yl)methanone | D | 1.859 | 390.3 |
| 129 | 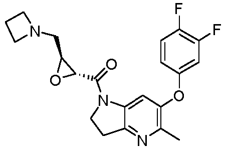 | ((2R,3S)-3-(azetidin-1-ylmethyl)oxiran-2-yl)(6-(3,4-difluorophenoxy)-5-methyl-2,3-dihydro-1H-pyrrolo[3,2-b]pyridin-1-yl)methanone | B | 1.754 | 402.0 |
| 130 | 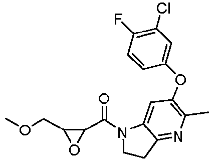 | *Trans*-(6-(3-chloro-4-fluorophenoxy)-5-methyl-2,3-dihydro-1H-pyrrolo[3,2-b]pyridin-1-yl)(3-(methoxymethyl)oxi ran-2-yl)methanone | E | 1.992 | 393.1 |
| 131 | 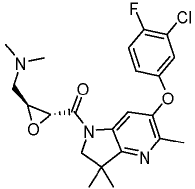 | (6-(3-chloro-4-fluorophenoxy)-3,3,5-trimethyl-2,3-dihydro-1H-pyrrolo[3,2-b]pyridin-1-yl)((2R,3S)-3-((dimethylamino)me thyl)oxiran-2-yl)methanone | E | 1.824 | 434.2 |
| 132 | 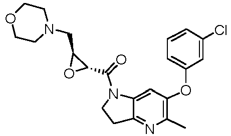 | (6-(3-chlorophenoxy)-5-methyl-2,3-dihydro-1H-pyrrolo[3,2-b]pyridin-1-yl)((2R,3S)-3-(morpholinomethyl) oxiran-2-yl)methanone | B | 1.830 | 430.4 |
| 133 | 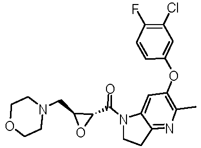 | (6-(3-chloro-4-fluorophenoxy)-5-methyl-2,3-dihydro-1H-pyrrolo[3,2-b]pyridin-1-yl)((2R,3S)-3-(morpholinomethyl) oxiran-2-yl)methanone | E | 1.665 | 448.3 |
| 134 | 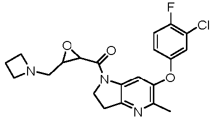 | *Trans*-(3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone | H | 2.746 | 418.1 |
| 135 | 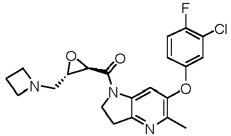 | ((2R,3S)-3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone | H | 2.778 | 418.1 |
| 136 | 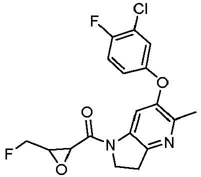 | *Trans*-(6-(3-chloro-4-fluorophenoxy)-5-methyl-2,3-dihydro-1H-pyrrolo[3,2-b]pyridin-1-yl)(3-(fluoromethyl)oxiran -2-yl)methanone | E | 2.051 | 381.1 |
| 137 | 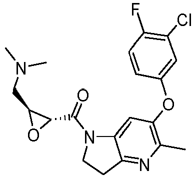 | (6-(3-chloro-4-fluorophenoxy)-5-methyl-2,3-dihydro-1H-pyrrolo[3,2-b]pyridin-1-yl)((2R,3S)-3-((dimethylamino)me thyl)oxiran-2-yl)methanone | H | 2.741 | 406.1 |
| 138 | 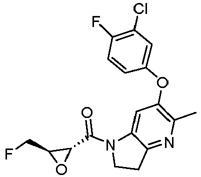 | (6-(3-chloro-4-fluorophenoxy)-5-methyl-2,3-dihydro-1H-pyrrolo[3,2-b]pyridin-1-yl)((2R,3R)-3-(fluoromethyl)oxiran -2-yl)methanone | E | 2.067 | 381.2 |
| 139 | 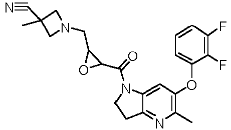 | *Trans*-1-((3-(6-(2,3-difluorophenoxy)-5-methyl-2,3-dihydro-1H-pyrrolo[3,2-b]pyridine-1-carbonyl)oxiran-2-yl)methyl)-3-methylazetidine-3-carbonitrile | E | 1.640 | 441.1 |
| 140 | 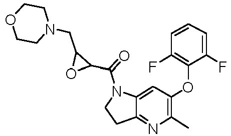 | *Trans*-(6-(2,6-difluorophenoxy)-5-methyl-2,3-dihydro-1H-pyrrolo[3,2-b]pyridin-1-yl)(3-(morpholinomethyl) oxiran-2-yl)methanone | B | 2.409 | 432.4 |
| 141 | 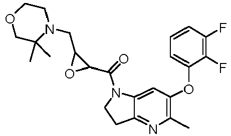 | *Trans*-(6-(2,3-difluorophenoxy)-5-methyl-2,3-dihydro-1H-pyrrolo[3,2-b]pyridin-1-yl)(3-((3,3-dimethylmorpholino )methyl)oxiran-2-yl)methanone | H | 2.826 | 460.2 |
| 142 | 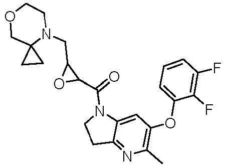 | *Trans*-(3-((7-oxa-4-azaspiro[2.5]octan-4-yl)methyl)oxiran-2-yl)(6-(2,3-difluorophenoxy)-5-methyl-2,3-dihydro-1H-pyrrolo[3,2-b]pyridin-1-yl)methanone | H | 2.742 | 458.2 |
| 143 | 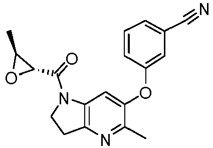 | 3-((5-methyl-1-((2R,3S)-3-methyloxirane-2-carbonyl)-2,3-dihydro-1H-pyrrolo[3,2-b]pyridin-6-yl)oxy)benzonitrile | D | 2.081 | 336.1 |
| 144 | 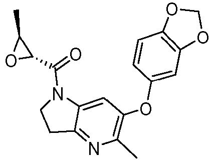 | (6-(benzo[d][1,3]dioxol -5-yloxy)-5-methyl-2,3-dihydro-1H-pyrrolo[3,2-b]pyridin-1-yl)((2R,3S)-3-methyloxiran-2-yl)methanone | B | 2.140 | 355.1 |
| 145 | 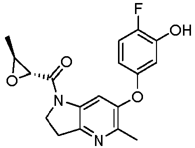 | (6-(4-fluoro-3-hydroxyphenoxy)-5-methyl-2,3-dihydro-1H-pyrrolo[3,2-b]pyridin-1-yl)((2R,3S)-3-methyloxiran-2-yl)methanone | L | 2.076 | 345.3 |
| 146 | 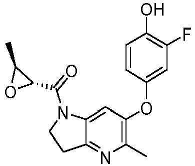 | (6-(3-fluoro-4-hydroxyphenoxy)-5-methyl-2,3-dihydro-1H-pyrrolo[3,2-b]pyridin-1-yl)((2R,3S)-3-methyloxiran-2-yl)methanone | D | 1.843 | 345.2 |
| 147 | 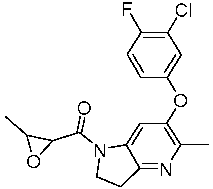 | *Trans*-(6-(3-chloro-4-fluorophenoxy)-5-methyl-2,3-dihydro-1H-pyrrolo[3,2-b]pyridin-1-yl)(3-methyloxiran-2-yl)methanone | D | 2.321 | 363.2 |
| 148 | 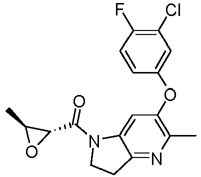 | (6-(3-chloro-4-fluorophenoxy)-5-methyl-2,3-dihydro-1H-pyrrolo[3,2-b]pyridin-1-yl)((2R,3S)-3-methyloxiran-2-yl)methanone | D | 2.366 | 363.2 |
| 149 | 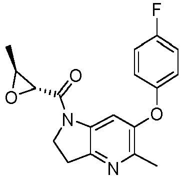 | (6-(4-fluorophenoxy)-5-methyl-2,3-dihydro-1H-pyrrolo[3,2-b]pyridin-1-yl)((2R,3S)-3-methyloxiran-2-yl)methanone | Q | 3.129 | 329.2 |
| 150 | 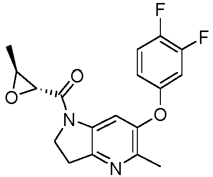 | (6-(3,4-difluorophenoxy)-5-methyl-2,3-dihydro-1H-pyrrolo[3,2-b]pyridin-1-yl)((2R,3S)-3-methyloxiran-2-yl)methanone | L | 2.379 | 347.3 |
| 151 | 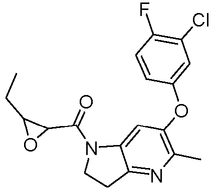 | *Trans*-(6-(3-chloro-4-fluorophenoxy)-5-methyl-2,3-dihydro-1H-pyrrolo[3,2-b]pyridin-1-yl)(3-ethyloxiran-2-yl)methanone | D | 2.524 | 377.1 |
| 152 | 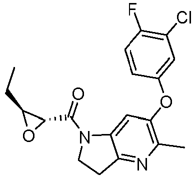 | (6-(3-chloro-4-fluorophenoxy)-5-methyl-2,3-dihydro-1H-pyrrolo[3,2-b]pyridin-1-yl)((2R,3S)-3-ethyloxiran-2-yl)methanone | E | 2.136 | 377.2 |
| 153 | 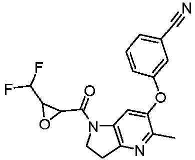 | *Trans*-3-((1-(3-(difluoromethyl)oxir ane-2-carbonyl)-5-methyl-2,3-dihydro-1H-pyrrolo[3,2-b]pyridin-6-yl)oxy)benzonitrile | D | 2.266 | 372.1 |
| 154 | 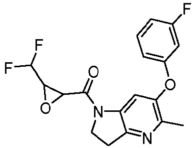 | *Trans*-(3-(difluoromethyl)oxir an-2-yl)(6-(3-fluorophenoxy)-5-methyl-2,3-dihydro-1H-pyrrolo[3,2-b]pyridin-1-yl)methanone | D | 2.422 | 365.2 |
| 155 | 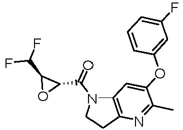 | ((2R,3R)-3-(difluoromethyl)oxir an-2-yl)(6-(3-fluorophenoxy)-5-methyl-2,3-dihydro-1H-pyrrolo[3,2-b]pyridin-1-yl)methanone | D | 2.327 | 365.2 |
| 156 | 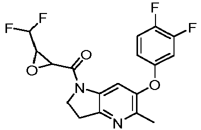 | *Trans*-(3-(difluoromethyl)oxir an-2-yl)(6-(3,4-difluorophenoxy)-5-methyl-2,3-dihydro-1H-pyrrolo[3,2-b]pyridin-1-yl)methanone | D | 2.392 | 383.2 |
| 157 | 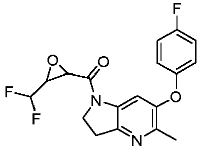 | *Trans*-(3-(difluoromethyl)oxir an-2-yl)(6-(4-fluorophenoxy)-5-methyl-2,3-dihydro-1H-pyrrolo[3,2-b]pyridin-1-yl)methanone | B | 2.229 | 365.2 |
| 158 | 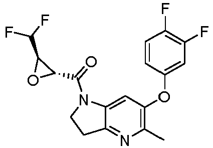 | ((2R,3R)-3-(difluoromethyl)oxir an-2-yl)(6-(3,4-difluorophenoxy)-5-methyl-2,3-dihydro-1H-pyrrolo[3,2-b]pyridin-1-yl)methanone | D | 2.471 | 383.2 |
| 159 | 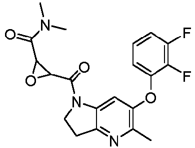 | *Trans*-3-(6-(2,3-difluorophenoxy)-5-methyl-2,3-dihydro-1H-pyrrolo[3,2-b]pyridine-1-carbonyl)-N,N-dimethyloxirane-2-carboxamide | H | 2.585 | 404.1 |
| 160 | 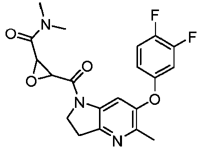 | *Trans*-3-(6-(3,4-difluorophenoxy)-5-methyl-2,3-dihydro-1H-pyrrolo[3,2-b]pyridine-1-carbonyl)-N,N-dimethyloxirane-2-carboxamide | D | 2.143 | 404.3 |
| 161 | 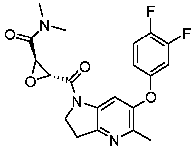 | (2R,3R)-3-(6-(3,4-difluorophenoxy)-5-methyl-2,3-dihydro-1H-pyrrolo[3,2-b]pyridine-1-carbonyl)-N,N-dimethyloxirane-2-carboxamide | E | 1.795 | 404.1 |
| 162 | 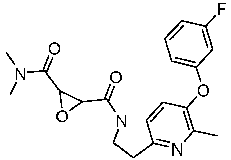 | *Trans*-3-(6-(3-fluorophenoxy)-5-methyl-2,3-dihydro-1H-pyrrolo[3,2-b]pyridine-1-carbonyl)-N,N-dimethyloxirane-2-carboxamide | D | 2.087 | 386.2 |
| 163 | 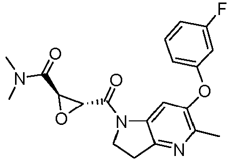 | (2R,3R)-3-(6-(3-fluorophenoxy)-5-methyl-2,3-dihydro-1H-pyrrolo[3,2-b]pyridine-1-carbonyl)-N,N-dimethyloxirane-2-carboxamide | E | 1.735 | 386.1 |
| 164 | 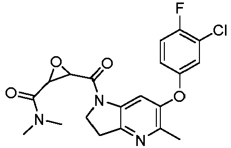 | *Trans*-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide | Q | 3.280 | 420.3 |
| 165 | 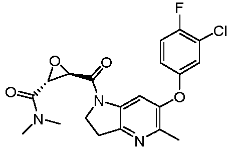 | (2R,3R)-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide | Q | 3.339 | 420.1 |

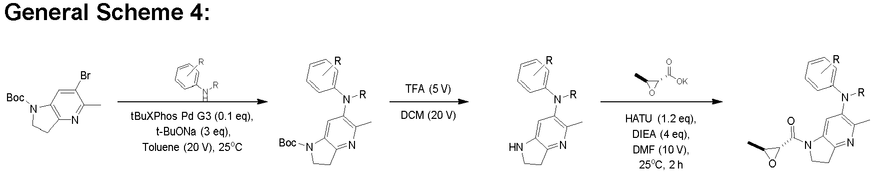

### Example 169 -Step 1: Procedure for preparation of 4a

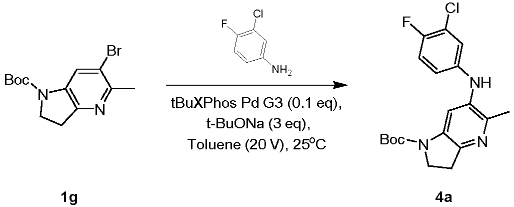

A mixture of **1g** (0.3 g, 957.89 µmol, 1 eq), 3-chloro-4-fluoro-aniline (111.55 mg, 766.31 µmol, 0.8 *eq*), tBuXPhos Pd G₃ (76.09 mg, 95.79 µmol, 0.1 *eq*), sodium tert-butoxide (276.17 mg, 2.87 mmol, 3 eq) in toluene (6 mL) was degassed and purged with N₂ for 3 times, and then the mixture was stirred at 25°C for 2 hrs under a N₂ atmosphere. LCMS showed the starting material was consumed and the desired product mass was detected. The reaction mixture was diluted with water (20 mL) and extracted with ethyl acetate (50 mL × 3). The combined organic layers were washed with brine (60 mL), dried over sodium sulfate, filtered and concentrated in vacuum to give a residue. The residue was purified by preparative HPLC (column: Phenomenex Luna C18 100*30mm*5um;mobile phase: [H₂O (0.1% TFA)-ACN];gradient:30%-60% B over 8.0 min) to give **4a** (tert-butyl 6-(3-chloro-4-fluoro-anilino)-5-methyl-2,3-dihydropyrrolo[3,2-b]pyridine-1-carboxylate) (100 mg, 27.63% yield) as a white solid. **¹H NMR** (400 MHz, DMSO-*d*₆) δ ppm 8.47 - 8.74 (m, 2 H) 7.73 - 8.32 (m, 1 H) 6.77 - 7.21 (m, 1 H) 3.96 (br s, 1 H) 3.15 (br t, J=8.13 Hz, 1 H) 2.52 (br s, 3 H) 2.37 (br s, 2 H) 1.39 (s, 9 H). **LCMS (ESI+):** m/z: 377.1.

### Step 2: Procedure for preparation of 4b

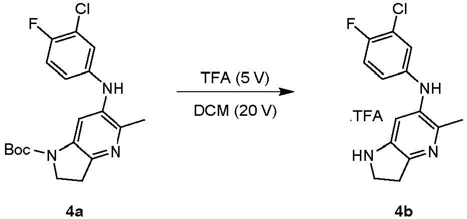

A mixture of **4a** (100 mg, 264.66 µmol, 1 eq), trifluoroacetic acid (767.50 mg, 6.73 mmol, 500.00 µL, 25.43 eq) in dichloromethane (2 mL) was stirred at 25°C for 1 h under a N₂ atmosphere. LCMS showed 80% of the desired product mass was detected. The reaction mixture was concentrated under reduce pressure to give **4b** (N-(3-chloro-4-fluoro-phenyl)-5-methyl-2,3-dihydro-1H-pyrrolo[3,2-b]pyridin-6-amine) (80 mg, 87.07% yield) as a yellow oil, which was used in the next step directly without purification. **¹H NMR** (400 MHz, DMSO-*d*₆) δ ppm 8.47 - 8.74 (m, 2 H) 7.73 - 8.32 (m, 1 H) 6.77 - 7.21 (m, 1 H) 3.96 (br s, 1 H) 3.15 (br t, *J* = 8.0 Hz, 1 H) 2.52 (br s, 3 H) 2.37 (br s, 2 H). **LCMS (ESI+):** m/z: 243.0 RT: 1.038 min

### Step 3: Procedure for preparation of Example 169

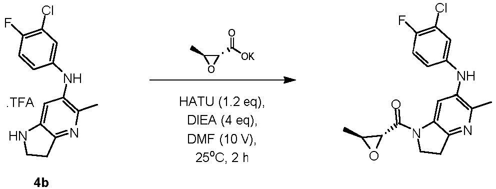

A mixture of **4b** (0.07 g, 252.05 µmol, 1 eq), [(2*R*,3*S*)-3-methyloxirane-2-carbonyl]oxypotassium (45.93 mg, 327.66 µmol, 1.3 *eq*), 2-(3H-[1,2,3]triazolo[4,5-b]pyridin-3-yl)-1,1,3,3-tetramethyluronium hexafluorophosphate(V) (143.75 mg, 378.07 µmol, 1.5 eq), N-ethyl-N-isopropylpropan-2-amine (130.30 mg, 1.01 mmol, 175.61 µL, 4 *eq*) in N,N-dimethylformamide (1 mL) was stirred at 25°C for 1 h under a N₂ atmosphere. LCMS showed the starting material was consumed and the desired product mass was detected. The residue was diluted with water (5 mL) and extracted with ethyl acetate (3 mL × 3). The combined organic layers were washed with aqueous sodium chloride (10 mL), dried over sodium sulfate, filtered and concentrated under reduced pressure to give a residue. The residue was purified by preparative HPLC (column: Phenomenex Luna C18 100*30mm*5um;mobile phase: [H₂O(0.2% FA)-ACN];gradient:15%-45% B over 8.0 min) to give **Example 169** [6-(3-chloro-4-fluoro-anilino)-5-methyl-2,3-dihydropyrrolo[3,2-b]pyridin-1-yl]-(3-methyloxiran-2-yl)methanone (11.11 mg, 11.73% yield) as a white solid. **¹H NMR** (400 MHz, DMSO-*d*₆) δ ppm 8.04 (s, 1 H) 7.75 (s, 1 H) 7.24 (t, *J* = 9.2 Hz, 1 H) 6.89 (dd, *J* = 6.4, 2.75 Hz, 1 H) 6.77 (dt, *J* = 8.8, 3.44 Hz, 1 H) 4.22 - 4.46 (m, 2 H) 3.65 (d, *J* = 2.0 Hz, 1 H) 3.20 (br t, *J* = 8.4 Hz, 2 H) 3.12 - 3.17 (m, 1 H) 2.33 (s, 3 H) 1.35 (d, *J* = 5.2 Hz, 3 H). **LCMS (ESI+):** m/z: 361.1 RT: 1.646 min.

The following examples were prepared using **Scheme 4.**

| Example | Structure | Chemical name | LC-MS method | LC-MS retention time (mins) | Mass found (M+H) ⁺ |
|---|---|---|---|---|---|
| 166 | 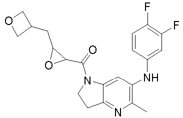 | *Trans*-(6-((3,4-difluorophenyl)amino)-5-methyl-2,3-dihydro-1 H-pyrrolo[3,2-b]pyridin-1-yl)(3-(oxetan-3-ylmethyl)oxiran-2-yl)methanone (Racemic) | L | 2.181 | 402.2 |
| 167 | 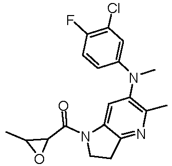 | *Trans*-(6-((3-chloro-4-fluorophenyl)(methyl)a mino)-5-methyl-2,3-dihydro-1 H-pyrrolo[3,2-b]pyridin-1-yl)(3-methyloxiran-2-yl)methanone (Racemic) | E | 1.936 | 376.1 |
| 168 | 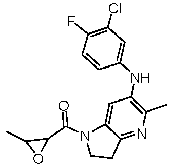 | *Trans*-(6-((3-chloro-4-fluorophenyl)amino)-5-methyl-2,3-dihydro-1H-pyrrolo[3,2-b]pyridin-1-yl)(3-methyloxiran-2-yl)methanone (Racemic) | E | 1.644 | 362.1 |
| 169 | 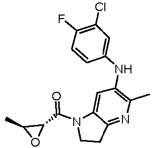 | (6-((3-chloro-4-fluorophenyl)amino)-5-methyl-2,3-dihydro-1H-pyrrolo[3,2-b]pyridin-1-yl)((2R,3S)-3-methyloxiran-2-yl)methanone | E | 1.659 | 362.1 |

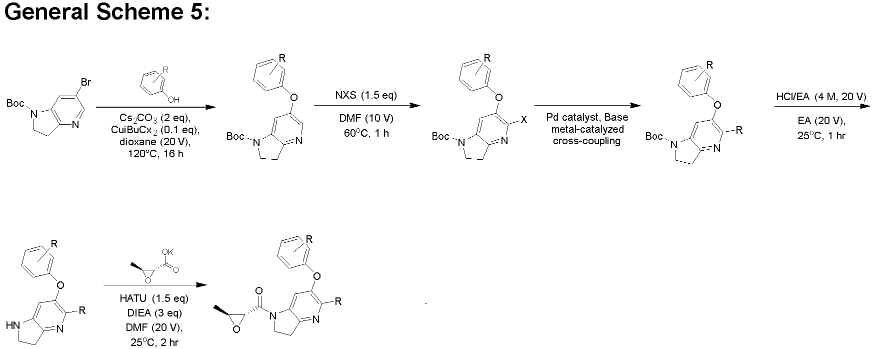

### Example 170-Step 1: Procedure for preparation of 5b

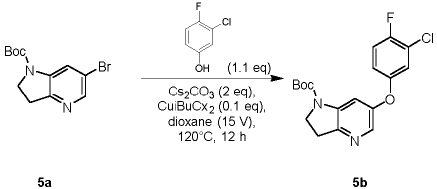

To a solution of **5a** (2 g, 6.69 mmol, 1 eq) , 3-chloro-4-fluoro-phenol (1.18 g, 8.02 mmol, 1.2 eq) and cesium carbonate (4.36 g, 13.37 mmol, 2 eq) in dioxane (20 mL) was added bis[(1E)-2-methyl-1-(2-oxocyclohexylidene)propoxy]copper (266.07 mg, 668.53 µmol, 0.1 eq) under N₂. The reaction mixture was stirred at 120°C for 16 h. LCMS showed 56% of desired mass was detected. The reaction mixture was diluted with water 30 mL and extracted with ethyl acetate (10 mL x 3). The combined organic layers were washed with brine, dried over anhydrous sodium sulfate, filtered and concentrated under reduced pressure to give a residue. The residue was purified by column chromatography (petroleum ether: ethyl acetate=1/1) to give **5b** (tert-butyl 6-(3-chloro-4-fluoro-phenoxy)-2,3-dihydropyrrolo[3,2-b]pyridine-1-carboxylate) (1.6 g, 62.33% yield) as a white solid. **¹H NMR** (400 MHz, DMSO-*d*₆) δ ppm 7.87 (d, *J* = 2.0 Hz, 1H), 7.61 - 6.94 (m, 4H), 4.03 - 3.92 (m, 2H), 3.11 (br t, *J* = 8.4 Hz, 2H), 1.44 (br s, 9H). **LCMS (ESI+):** m/z: 365.2.

### Step 2: Procedure for preparation of 5c

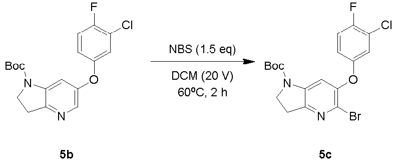

To a solution of **5b** (1.6 g, 4.39 mmol, 1 eq) in N,N-dimethylformamide (32 mL) was adde N-bromosuccinimide (1.17 g, 6.58 mmol, 1.5 eq). The reaction mixture was stirred at 60°C for 2 h. LCMS showed 74% of desired mass was detected. The reaction mixture was diluted with water (30 mL) and extracted with ethyl acetate (10 mL x 3). The combined organic layers were washed with brine, dried over anhydrous sodium sulfate, filtered and concentrated under reduced pressure to give a residue. The residue was purified by column chromatography (petroleum ether: ethyl acetate=5/1) to give **5c** (tert-butyl 5-bromo-6-(3-chloro-4-fluoro-phenoxy)-2,3-dihydropyrrolo[3,2-b]pyridine-1-carboxylate) (1.5 g, 73.23% yield) as a white solid. **¹H NMR** (400 MHz, DMSO-*d*₆) δ ppm 7.66 - 6.90 (m, 4H), 3.98 (br s, 2H), 3.14 (br s, 2H), 1.50 - 1.24 (m, 9H). **LCMS (ESI+):** m/z: 443.0.

### Step 3: Procedure for preparation of 5d

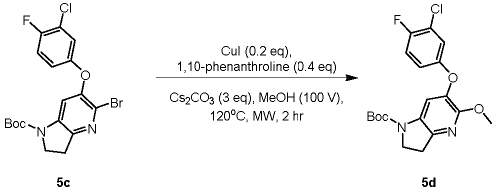

A mixture of **5c** (0.2 g, 450.76 µmol, 1 eq), copper(i) iodide (17.17 mg, 90.15 µmol, 0.2 eq), 1,10-phenanthroline monohydrate (35.74 mg, 180.30 µmol, 0.4 eq) and cesium carbonate (440.60 mg, 1.35 mmol, 3 eq) in methanol (20 mL) was degassed and purged with N₂ 3 times. The mixture was stirred at 120°C for 2 h in microwave under N₂ atmosphere. LCMS showed the starting material was consumed and 37% of the desired mass was detected. After cooling to 25°C, the combined mixture was filtered through a Celite pad and rinsed with dichloromethane (3 × 30 mL). The filtrate was concentrated to afford the crude product. The residue was purified by column chromatography (petroleum ether/ethyl acetate = 9/1) to give **5d** (tert-butyl 6-(3-chloro-4-fluoro-phenoxy)-5-methoxy-2,3-dihydropyrrolo[3,2-b]pyridine-1-carboxylate) (0.05 g, 28.09%yield) as a white solid. **¹H NMR** (400 MHz, METHANOL-*d₄*) δ ppm 6.57 - 8.00 (m, 4 H) 4.03 (br s, 2 H) 3.87 (br s, 3 H) 3.14 (br t, *J = 8.4* Hz, 2 H) 1.44 - 1.57 (m, 9 H). **LCMS (ESI+):** m/z: 393.8.

### Step 4: Procedure for preparation of 5e.

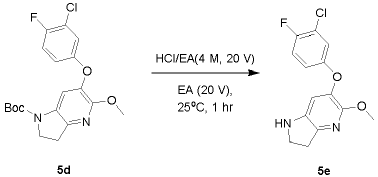

To a mixture of **5d** (0.05 g, 126.64 µmol, 1 eq) in ethyl acetate (1 mL) was added HCl/ ethyl acetate (4 M, 1 mL, 15.31 eq). The mixture was stirred at 25°C for 1 h. LCMS showed the starting material was consumed and 63% of the desired mass was detected. The reaction mixture was evaporated under a stream of N₂ to give **5e** (6-(3-chloro-4-fluoro-phenoxy)-5-methoxy-2,3-dihydro-1H-pyrrolo[3,2-b]pyridine) (0.05 g, 95.38% yield,) as a white solid, which was used to the next step directly without purification. **¹H NMR** (400 MHz, METHANOL-*d₄*) δ ppm 6.57 - 8.00 (m, 4 H) 4.03 (br s, 2 H) 3.87 (br s, 3 H) 3.14 (br t, *J =* 8.4 Hz, 2 H). **LCMS (ESI+):** m/z: 294.1.

### Step 5: Procedure for preparation of Compound 170

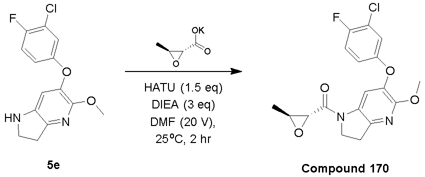

A mixture of **5e** (0.05 g, 150.98 µmol, 1 eq, HCl salt), [(2*R*,3*S*)-3-methyloxirane-2-carbonyl]oxypotassium (25.40 mg, 181.18 µmol, 1.2 eq), 2-(3H-[1,2,3]triazolo[4,5-b]pyridin-3-yl)-1,1,3,3-tetramethyluronium hexafluorophosphate(V) (86.11 mg, 226.47 µmol, 1.5 eq), N,N-diisopropylethylamine (58.54 mg, 452.94 µmol, 78.89 µL, 3 eq) in N,N-dimethylformamide (1 mL) was stirred at 20°C for 2 hrs. LCMS showed 33% of the desired mass was detected. The residue was quenched with water (10 mL) and extracted with ethyl acetate (3 mL × 3). The combined organic layers were washed with aqueous sodium chloride (10 mL), dried over sodium sulfate, filtered and concentrated under reduced pressure to give a residue. The residue was purified by pre-HPLC (column: WePure Biotech XP tC18 150 × 40 × 7 um; mobile phase: [H₂O(10mM NH₄HCO₃)-CH₃CN]; gradient: 35%-70% B over 8.0 min]; gradient: 40%-70% B over 8.0 min) to give **Example 170** ([6-(3-chloro-4-fluoro-phenoxy)-5-methoxy-2,3-dihydropyrrolo[3,2-b]pyridin-1-yl]-[(2R,3S)-3-methyloxiran-2-yl]methanone) (24.04 mg, 42.04% yield) as a white solid. **¹H NMR** (400 MHz, CHLOROFORM-d) δ ppm 8.11 (s, 1 H) 7.07 (t, *J* = 8.8 Hz, 1 H) 6.94 (dd, *J* = 6.0, 2.94 Hz, 1 H) 6.83 (dt, *J* = 9.2, 3.38 Hz, 1 H) 4.36 - 4.46 (m, 1 H) 4.27 - 4.36 (m, 1 H) 3.95 (s, 3 H) 3.27 - 3.38 (m, 4 H) 1.46 (d, *J* = 5.2 Hz, 3 H). **LCMS (ESI+):** m/z: 378.1

The following examples were also prepared in a similar fashion using **Scheme 5.**

| Example | Structure | Chemical name | LC-MS method | LC-MS retention time (mins) | Mass found (M+H) ⁺ |
|---|---|---|---|---|---|
| 170 | 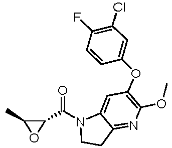 | (6-(3-chloro-4-fluorophenoxy)-5-methoxy-2,3-dihydro-1H-pyrrolo[3,2-b]pyridin-1-yl)((2R,3S)-3-methyloxiran-2-yl)methanone | H | 2.973 | 379.1 |
| 171 | 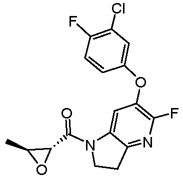 | (6-(3-chloro-4-fluorophenoxy)-5-fluoro-2,3-dihydro-1H-pyrrolo[3,2-b]pyridin-1-yl)((2R,3S)-3-methyloxiran-2-yl)methanone | H | 2.995 | 367.0 |
| 172 | 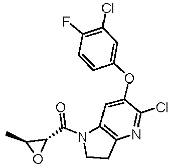 | (5-chloro-6-(3-chloro-4-fluorophenoxy)-2,3-dihydro-1H-pyrrolo[3,2-b]pyridin-1-yl)((2R,3S)-3-methyloxiran-2-yl)methanone | E | 2.257 | 383.1 |

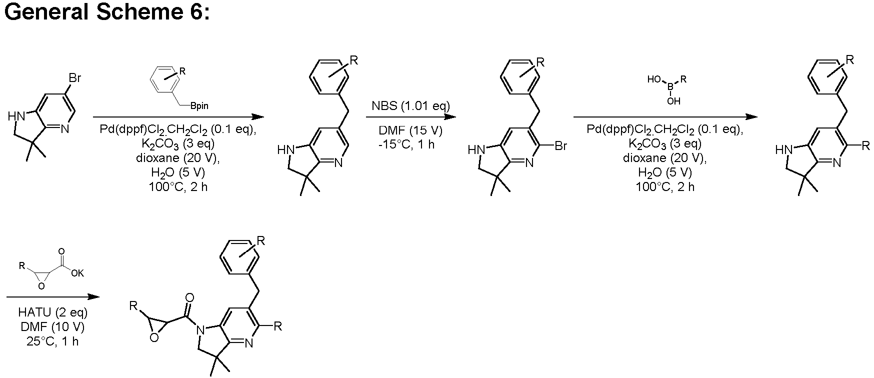

### Example 174 -Step 1: Procedure for preparation of 6b

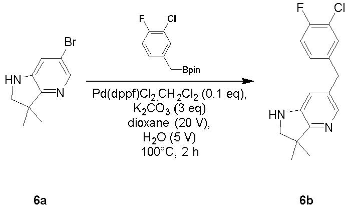

To a solution of **6a** (250 mg, 1.10 mmol, 1 eq) in dioxane (5 mL) and water (1.25 mL) was added 2-[(3-chloro-4-fluoro-phenyl)methyl]-4,4,5,5-tetramethyl-1,3,2-dioxaborolane (446.72 mg, 1.65 mmol, 1.5 eq), potassium carbonate (456.42 mg, 3.30 mmol, 3 eq) and Pd(dppf)Cl₂ (89.90 mg, 110.08 µmol, 0.1 eq). The mixture was stirred at 100°C for 2 h under nitrogen. LCMS showed the starting material was consumed and 54% of the desired mass was detected. The reaction mixture was quenched with water (10 mL) and extracted with ethyl acetate (3 × 10 mL). The combined organic layer was dried over sodium sulfate, filtered and the filtrate was concentrated under reduced pressure to give a residue. The residue was purified by column chromatography on silica gel (petroleum ether/ethyl acetate = 5/1) to give **6b** (6-[(3-chloro-4-fluoro-phenyl)methyl]-3,3-dimethyl-1,2-dihydropyrrolo[3,2-b]pyridine) (350 mg, yield 54.67 %) as a yellow solid. **¹H NMR** (400 MHz, CHLOROFORM-d) δ ppm 7.77 (s, 1 H) 7.18 - 7.23 (m, 1 H) 6.96 - 7.09 (m, 2 H) 6.55 (d, *J = 1.2* Hz, 1 H) 3.80 (s, 2 H) 3.31 - 3.46 (m, 2 H) 1.32 - 1.39 (m, 6 H). **LCMS (ESI+):** m/z: 290.1.

### Step 2: Procedure for preparation of 6c.

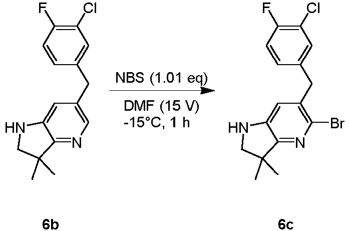

To a solution of **6b** (300 mg, 1.03 mmol, 1 eq) in N,N-dimethylformamide (4.5 mL) was added 1-bromopyrrolidine-2,5-dione (185.47 mg, 1.04 mmol, 1.01 eq) in N,N-dimethylformamide (0.5 mL) at -15°C. The mixture was stirred at -15°C for 1 h. LCMS showed the starting material was consumed and 65% of the desired mass was detected. The reaction mixture was quenched with water (15 mL) and extracted with ethyl acetate (3 × 10 mL). The combined organic layer was dried over sodium sulfate, filtered and the filtrate was concentrated under reduced pressure to give a residue. The residue was purified by column chromatography on silica gel (petroleum ether/ethyl acetate = 6/1) to give **6c** (5-bromo-6-[(3-chloro-4-fluoro-phenyl)methyl]-3,3-dimethyl-1,2-dihydropyrrolo[3,2-b]pyridine) (250 mg, yield 65.55%) as a brown solid. **¹H NMR** (400 MHz, CHLOROFORM-d) δ ppm 7.15 (d, *J =* 7.2 Hz, 1 H) 6.95 - 7.03 (m, 2 H) 6.47 (s, 1 H) 3.85 (s, 2 H) 3.32 (s, 2 H) 1.28 (s, 6 H). **LCMS (ESI+):** m/z: 368.0.

### Step 3: Procedure for preparation of 6d

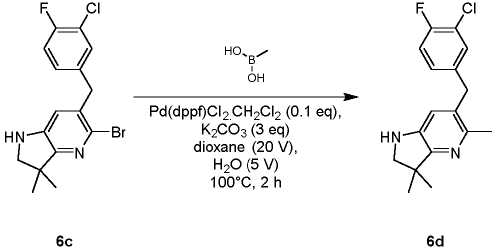

To a solution of **6c** (200 mg, 1 eq) in dioxane (4 mL) and water (1 mL) was added methylboronic acid (42.10 mg, 1.3 eq), potassium carbonate (224.32 mg, 3 eq) and Pd(dppf)Cl₂ (44.18 mg, 54.10 µmol, 0.1 eq). The mixture was stirred at 100°C for 2 h under nitrogen. LCMS showed the starting material was consumed and 65% of the desired mass was detected. The reaction mixture was quenched with water (5 mL) and extracted with ethyl acetate (3 × 5 mL). The combined organic layer was dried over sodium sulfate, filtered and the filtrate was concentrated under reduced pressure to give a residue. The residue was purified by column chromatography on silica gel (petroleum ether/ethyl acetate = 5/1) to give **6d** (6-[(3-chloro-4-fluoro-phenyl)methyl]-3,3,5-trimethyl-1,2-dihydropyrrolo[3,2-b]pyridine) (110 mg, yield 63.53%) as a brown solid. **¹H NMR** (400 MHz, CHLOROFORM-*d*) δ ppm 7.01 - 7.12 (m, 1 H) 6.94 - 7.01 (m, 1 H) 6.87 - 6.94 (m, 1 H) 6.44 - 6.54 (m, 1 H) 3.74 (s, 2 H) 3.29 (s, 2 H) 2.32 (s, 3 H) 1.29 (s, 6 H). **LCMS (ESI+):** m/z 304.1

### Step 4: Procedure for preparation of Compound 174

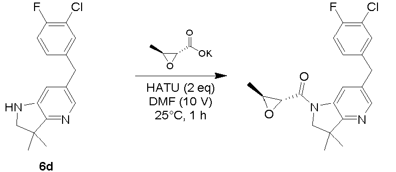

To a solution of [(2*R*,3*S*)-3-methyloxirane-2-carbonyl]oxypotassium (73.59 mg, 524.95 µmol, 2 eq) in N,N-dimethylformamide (0.8 mL) was added 2-(3H-[1,2,3]triazolo[4,5-b]pyridin-3-yl)-1,1,3,3-tetramethyluronium hexafluorophosphate(V) (199.60 mg, 524.95 µmol, 2 eq) and **6d** (80 mg, 262.48 µmol, 1 eq). The mixture was stirred at 25°C for 1 h. LCMS showed the starting material was consumed and 35% of the desired mass was detected. The reaction mixture was quenched with water (3 mL) and extracted with ethyl acetate (6 mL). The combined organic layers were dried over sodium sulfate, filtered and the filtrate was concentrated under reduced pressure to give a residue. The residue was purified by preparative HPLC (FA condition. column: Phenomenex luna C₁₈ 100 × 40mm × 5 µm; mobile phase: [H₂O (0.2% FA)-ACN]; gradient: 25%-65% B over 8.0 min) to give **Example 174** ([6-[(3-chloro-4-fluoro-phenyl)methyl]-3,3,5-trimethyl-2H-pyrrolo[3,2-b]pyridin-1-yl]-[(2*R*,3*S*)-3-methyloxiran-2-yl)methanone) (27.84 mg, yield24.05%) as a pink solid. **¹H NMR** (400 MHz, CHLOROFORM-*d*) δ ppm 8.29 (s, 1 H) 6.96 - 7.15 (m, 3 H) 4.10 - 4.17 (m, 1 H) 4.02 (d, *J =* 10.2 Hz, 1 H) 3.94 (s, 2 H) 3.30 - 3.42 (m, 2 H) 2.51 (s, 3 H) 1.45 - 1.54 (m, 9 H). **LCMS (ESI+):** m/z 389.1

The following examples were prepared in a similar fashion using **Scheme 6.**

| Example | Structure | Chemical name | LC-MS method | LC-MS retention time (mins) | Mass found (M+H) ⁺ |
|---|---|---|---|---|---|
| 173 | 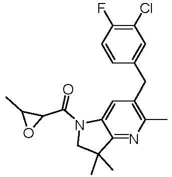 | *Trans-*(6-(3-chloro-4-fluorobenzyl)-3,3,5-trimethyl-2,3-dihydro-1H-pyrrolo[3,2-b]pyridin-1-yl)( 3-methyloxiran-2-yl)methanone (Racemic) | B | 2.552 | 389.1 |
| 174 | 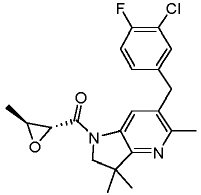 | (6-(3-chloro-4-fluorobenzyl)-3,3,5-trimethyl-2,3-dihydro-1H-pyrrolo[3,2-b]pyridin-1-yl)((2R,3S)-3-methyloxiran-2-yl)methanone | E | 2.012 | 389.1 |
| 175 | 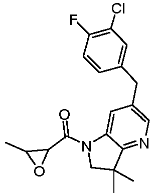 | *Trans*-(6-(3-chloro-4-fluorobenzyl)-3,3-dimethyl-2,3-dihydro-1H-pyrrolo[3,2-b]pyridin-1-yl)( 3-methyloxiran-2-yl)methanone (Racemic) | E | 2.121 | 375.1 |
| 176 | 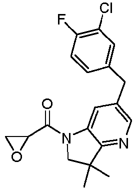 | (6-(3-chloro-4-fluorobenzyl)-3,3-dimethyl-2,3-dihydro-1H-pyrrolo[3,2-b]pyridin-1-yl)(oxiran-2-yl)methanone (Racemic) | I | 2.887 | 361.1 |
| 177 | 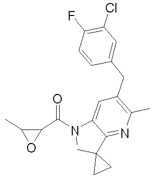 | *Trans*-(6'-(3-chloro-4-fluorobenzyl)-5'-methylspiro[cyclopro pane-1,3'-pyrrolo[3,2-b]pyridin]-1'(2'H)-yl)(3-methyloxiran-2-yl)methanone (Racemic) | E | 2.049 | 387.2 |

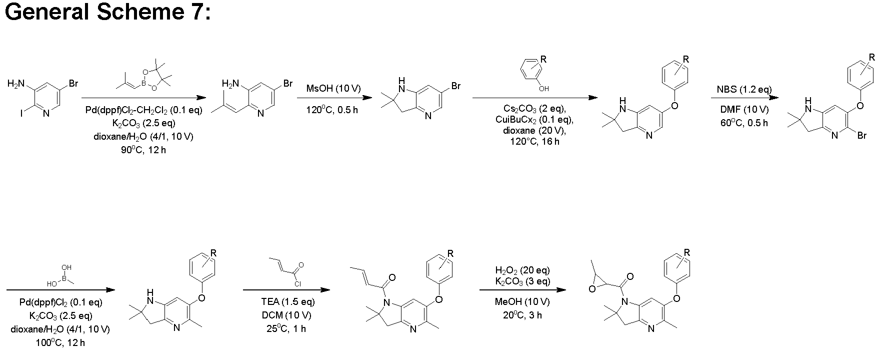

### Example 178-Step 1: Procedure for preparation of 7b

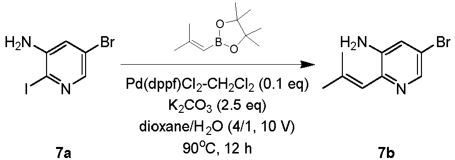

To a solution of **7a** (5 g, 16.73 mmol, 1 eq) and 4,4,5,5-tetramethyl-2-(2-methylprop-1-enyl)-1,3,2-dioxaborolane (4.57 g, 25.09 mmol, 1.5 eq) in water (10 mL) and dioxane (40 mL) was added 1,1-bis(diphenylphosphino)ferrocene-palladium(II)dichloride dichloromethane complex (1.37 g, 1.67 mmol, 0.1 eq) and sodium carbonate (4.43 g, 41.82 mmol, 2.5 eq). The mixture was stirred at 90 °C for 12 h. LCMS showed 30% of desired mass was detected. The reaction mixture was diluted with water (10 mL) and extracted with ethyl acetate 60 mL (20 mL x 3). The combined organic layers were washed with brine (60 mL), dried over sodium sulfate, filtered and concentrated under reduced pressure to give a residue. The residue was purified by silica gel chromatography (petroleum ether/ethyl acetate = 1/2) to give a **7b** (5-bromo-2-(2-methylprop-1-en-1-yl)pyridin-3-amine) (2.5 g, 65.81% yield). **¹H NMR** (400 MHz, DMSO-*d₆*) δ ppm 7.79 (d, *J = 2.0* Hz, 1H), 7.13 (d, *J* = 2.1 Hz, 1H), 6.15 (s, 1H), 5.40 (s, 2H), 1.92 - 1.88 (m, 6H). **LCMS (ESI+):** m/z: 229.0.

### Step 2: Procedure for preparation of 7c

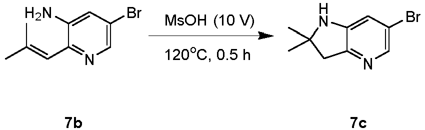

To a solution of **7b** (2.5 g, 11.01 mmol, 1 eq) in methanesulfonic acid (25 mL) was stirred at 120 °C for 0.5 h. LCMS showed the starting material was consumed and 84% of the desired mass was detected. The reaction mixture was quenched by the addition of aqueous sodium carbonate (60 mL) at 0°C, and then diluted with water 50 mL and extracted with ethyl acetate (50 mL x 3). The combined organic layers were washed with brine (200 mL), dried over sodium sulfate, filtered and concentrated under reduced pressure to give a residue. The residue was purified by silica gel chromatography (petroleum ether/ethyl acetate=3/1) to afford **7c** (6-bromo-2,2-dimethyl-2,3-dihydro-1H-pyrrolo[3,2-b]pyridine) (1.7 g, 68% yield). **¹H NMR** (400 MHz, DMSO-*d₆*) δ ppm 7.62 (d, *J* = 2.0 Hz, 1H), 6.77 (d, *J* = 2.0 Hz, 1H), 6.17 (s, 1H), 2.74 (s, 2H), 1.25 (s, 6H). **LCMS (ESI+):** m/z: 227.0.

### Step 3: Procedure for preparation of 7d

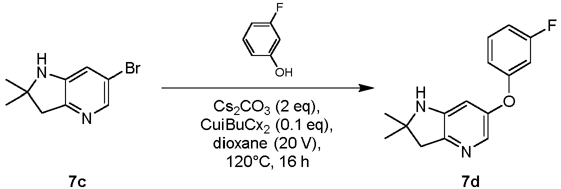

To a solution of **7c** (0.9 g, 3.96 mmol, 1 eq) and 3-fluorophenol (666.39 mg, 5.94 mmol, 548.02 µL, 1.5 eq) in dioxane (15 mL) was added bis[(1Z)-2-methyl-1-(2-oxocyclohexylidene)propoxy]copper (315.45 mg, 792.60 µmol, 0.2 eq) and cesium carbonate (3.87 g, 11.89 mmol, 3 eq) under a N₂ atmosphere. The mixture was stirred at 120°C for 16 h. LCMS showed the starting material was consumed and 75% of the desired mass was detected. The reaction mixture was diluted with water (10 mL) and extracted with ethyl acetate (10 mL x 3). The combined organic layers were washed with brine (50 mL), dried over sodium sulfate, filtered and concentrated under reduced pressure to give a residue. The residue was purified by silica gel chromatography (petroleum ether: ethyl acetate=1/1) to give **7d** (6-(3-fluorophenoxy)-2,2-dimethyl-2,3-dihydro-1H-pyrrolo[3,2-b]pyridine) (700 mg, 68.39% yield). **¹H NMR** (400 MHz, DMSO-*d₆*) δ ppm 7.43 - 7.13 (m, 2H), 6.95 (dt, *J* = 2.4, 8.5 Hz, 1H), 6.90 - 6.79 (m, 2H), 6.33 (d, *J* = 2.4 Hz, 1H), 6.05 (s, 1H), 2.78 (s, 2H), 1.27 (s, 6H). **LCMS (ESI+):** m/z: 259.2.

### Step 4: Procedure for preparation of 7e

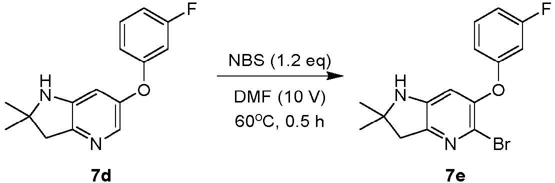

To a solution of **7d** (700 mg, 2.71 mmol, 1 eq) in N,N-dimethylformamide (7 mL) was added N-bromosuccinimide (578.83 mg, 3.25 mmol, 1.2 eq). The mixture was stirred at 60°C for 1 h. LCMS showed the starting material was consumed and 73% of the desired mass was detected. The reaction mixture was diluted with water 10 mL and extracted with ethyl acetate (20 mL x 3). The combined organic layers were washed with brine (60 mL), dried over sodium sulfate, filtered and concentrated under reduced pressure to give a residue. The residue was purified by preparative HPLC (column: CD05-Phenomenex Luna C18 100*40mm*10um;mobile phase: [H₂O(0.225% FA)-ACN]; gradient:46%-76% B over 13.0 min) to give **7e** (5-bromo-6-(3-fluorophenoxy)-2,2-dimethyl-2,3-dihydro-1H-pyrrolo[3,2-b]pyridine) (500 mg, 54.72% yield). **¹H NMR** (400 MHz, DMSO-*d₆*) δ ppm 7.46 - 7.33 (m, 1H), 6.95 (dt, *J* = 2.4, 8.4 Hz, 1H), 6.84 (td, *J* = 2.4, 10.5 Hz, 1H), 6.75 (dd, *J* = 2.4, 8.3 Hz, 1H), 6.46 (s, 1H), 6.22 (s, 1H), 2.81 (s, 2H), 1.27 (s, 6H). **LCMS (ESI+):** m/z: 337.1.

### Step 5: Procedure for preparation of 7f

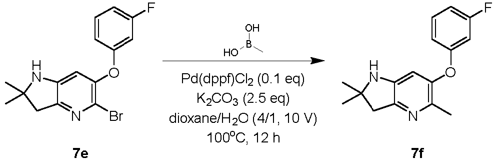

To a solution of **7e** (500 mg, 1.48 mmol, 1 eq) and methylboronic acid (443.82 mg, 7.41 mmol, 5 eq) in water (1 mL) and dioxane (4 mL) was added 1,1-bis(diphenylphosphino)ferrocene-palladium(II)dichloride dichloromethane complex (121.10 mg, 148.29 µmol, 0.1 *eq*) and potassium carbonate (512.35 mg, 3.71 mmol, 2.5 *eq*) under a N₂ atmosphere. The mixture was stirred at 100°C for 12 h. LCMS showed 60% of the desired mass was detected. The reaction mixture was diluted with water (10 mL) and extracted with ethyl acetate (20 mL x 3). The combined organic layers were washed with brine (60 mL), dried over sodium sulfate, filtered and concentrated under reduced pressure to give a residue. The residue was purified by preparative HPLC (column: CD05-Phenomenex Luna C18 100*40mm*10um;mobile phase: [H₂O(0.225% FA)-ACN];gradient:10%-40% B over 13.0 min) to give **7f** (5-bromo-6-(3-fluorophenoxy)-2,2-dimethyl-2,3-dihydro-1H-pyrrolo[3,2-b]pyridine) (500 mg, 54.72% yield). **¹H NMR** (400 MHz, DMSO-*d₆*) δ ppm 7.46 - 7.33 (m, 1H), 6.95 (dt, *J* = 2.4, 8.4 Hz, 1H), 6.84 (td, *J* = 2.4, 10.5 Hz, 1H), 6.75 (dd, *J* = 2.4, 8.3 Hz, 1H), 6.46 (s, 1H), 6.22 (s, 1H), 2.81 (s, 2H), 1.27 (s, 6H). **LCMS (ESI+):** m/z: 337.1.

### Step 6: Procedure for preparation of 7g

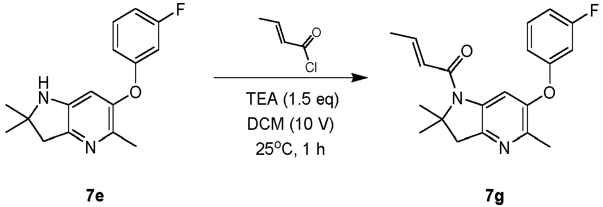

To a solution of **7e** (50 mg, 183.61 µmol, 1 eq) and (E)-but-2-enoyl chloride (28.79 mg, 275.41 µmol, 26.39 µL, 1.5 eq) in dichloromethane (0.5 mL) was added triethylamine (27.87 mg, 275.41 µmol, 38.33 µL, 1.5 eq). The mixture was stirred at 25°C for 1 h. LCMS showed the starting material was consumed and 60% of the desired mass was detected. The residue was diluted with water (2 mL) and extracted with ethyl acetate (3 mL × 3). The combined organic layers were washed with aqueous sodium chloride (10 mL), dried over sodium sulfate, filtered and concentrated under reduced pressure to give a residue. The residue was purified by preparative HPLC (column: CD24-WePure Biotech XPT C18 150*25*7um;mobile phase: [H₂O(10mM NH₄HCO₃)-ACN (0.02%CH₃COOH)];gradient:41%-71% B over 10.0 min) to give **7g** ((E)-1-(6-(3-fluorophenoxy)-2,2,5-trimethyl-2,3-dihydro-1H-pyrrolo[3,2-b]pyridin-1-yl)but-2-en-1-one) (40 mg, 64.00% yield) as a yellow solid. **¹H NMR** (400 MHz, CHLOROFORM-d) δ ppm 8.34 - 6.57 (m, 5H), 6.48 - 5.83 (m, 1H), 5.10 (br d, *J* = 11.6 Hz, 1H), 3.39 (d, *J* = 6.4 Hz, 2H), 3.13 - 3.07 (m, 2H), 2.31 - 2.26 (m, 3H), 1.61 (s, 6H). **LCMS (ESI+):** m/z: 341.3.

### Step 7: Procedure for preparation of 7h

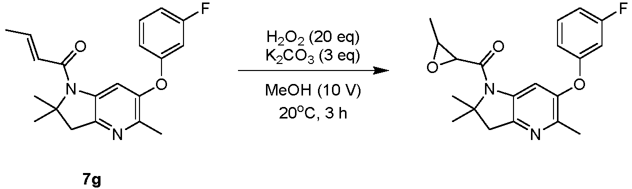

To a solution of **7g** (170 mg, 499.43 µmol, 1 eq) and potassium carbonate (345.12 mg, 2.50 mmol, 5 eq) in methanol (10 mL) was added hydrogen peroxide (1.13 g, 9.99 mmol, 959.76 µL, 30% purity, 20 eq) under N₂. The mixture was stirred at 25°C for 3 h. LCMS showed 52% of the desired mass was detected. The reaction mixture was quenched by the addition of Na₂SO₃ solution (2 mL) at 0°C, and then diluted with further H₂O (2 mL) and extracted with ethyl acetate (2 mL x 3). The combined organic layers were washed with brine (10 mL), dried over sodium sulfate, filtered and concentrated under reduced pressure to give a residue. The residue was purified by preparative HPLC (column: CD01-Phenomenex luna C18 150*25mm* 10um;mobile phase: [H₂O(0.225% FA)-ACN];gradient:33%-63% B over 11.0 min) to give **Example 178** ((6-(3-fluorophenoxy)-2,2,5-trimethyl-2,3-dihydro-1H-pyrrolo[3,2-b]pyridin-1-yl)(*trans*-3-methyloxiran-2-yl)methanone) (61.04 mg, 34.29% yield) as yellow solid. **¹H NMR** (400 MHz, CHLOROFORM-d) δ ppm 7.55 - 7.35 (m, 2H), 6.97 - 6.89 (m, 1H), 6.86 - 6.78 (m, 2H), 3.61 (d, *J* = 2.0 Hz, 1H), 3.20 - 3.10 (m, 3H), 2.33 (s, 3H), 1.67 (s, 3H), 1.61 (s, 3H), 1.23 (d, *J* = 5.2 Hz, 3H). **LCMS (ESI+):** m/z: 357.3.

The following example was prepared using **Scheme 7.**

| Example | Structure | Chemical name | LC-MS method | LC-MS retention time (mins) | Mass found (M+H) ⁺ |
|---|---|---|---|---|---|
| 178 | 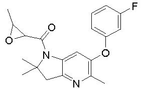 | *Trans*-(6-(3-fluorophenoxy)-2,2,5-trimethyl-2,3-dihydro-1H-pyrrolo[3,2-b]pyridin-1-yl)(3-methyloxiran-2-yl)methanone (Racemic) | K | 2.486 | 357.3 |

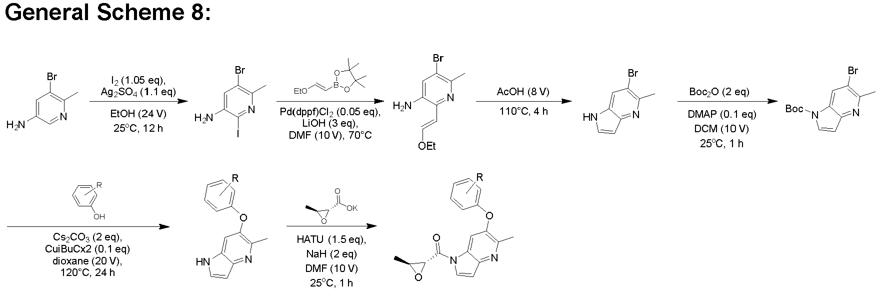

### Example 180-Step 1: Procedure for preparation of 8b

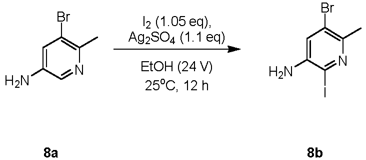

To a solution of **8a** (14 g, 74.85 mmol, 1 eq) in ethanol (336 mL) was added iodine (19.95 g, 78.59 mmol, 15.83 mL, 1.05 eq) and silver sulfate (25.67 g, 82.34 mmol, 13.95 mL, 1.1 eq). The mixture was stirred at 25°C for 5 hrs. LCMS showed the starting material was consumed, and 60% of desired mass was detected. The reaction mixture was filtered and concentrated under reduced pressure to give a residue, which was then diluted with dichloromethane (300 mL) and washed with NaOH 600 mL (3 x 200 mL). The combined organic layers were washed with brine (500 mL), dried over sodium sulfate, filtered and concentrated under reduced pressure to give **8b** (5-bromo-2-iodo-6-methylpyridin-3-amine) (10 g, 42.69% yield) as a black solid. **¹H NMR** (400 MHz, DMSO-*d₆*) δ ppm 7.20 - 7.16 (m, 1H), 5.39 (br s, 2H), 2.35 (s, 3H). **LCMS (ESI+):** m/z: 312.9.

### Step 2: Procedure for preparation of 8c

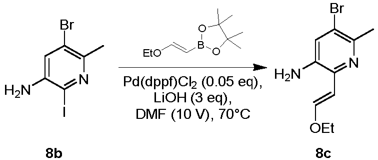

To a solution of **8b** (10 g, 31.96 mmol, 1 eq) and 2-[(E)-2-ethoxyvinyl]-4,4,5,5-tetramethyl-1,3,2-dioxaborolane (9.49 g, 47.93 mmol, 1.5 eq) in N,N-dimethylformamide (140 mL) was added lithium hydroxide (2.30 g, 95.87 mmol, 3 eq) and Pd(dppf)Cl₂ (1.30 g, 1.60 mmol, 0.05 eq) under a N₂ atmosphere. The mixture was stirred at 70°C for 5 h. LCMS showed 39% of the desired mass was detected. The reaction mixture was diluted by water (200 mL), and extracted with ethyl acetate (3 x 100 mL). The combined organic layers were washed with brine (100 mL), dried over sodium sulfate, filtered and concentrated under reduced pressure to give a residue. The residue was purified by silica gel chromatography (petroleum ether/ethyl acetate=1/1) to get **8c** (E)-5-bromo-2-(2-ethoxyvinyl)-6-methylpyridin-3-amine) (5 g, 60.85% yield) as a black solid. **¹H NMR** (400 MHz, DMSO-*d₆*) δ ppm 7.41 (*d*, *J* = 12.0 Hz, 1H), 7.10 (s, 1H), 5.98 (*d*, *J =* 12.0 Hz, 1H), 5.15 (s, 2H), 3.93 (q, *J =* 7.2 Hz, 2H), 2.34 (s, 3H), 1.26 (t, *J = 7.2* Hz, 3H). **LCMS (ESI+):** m/z: 257.0

### Step 3: Procedure for preparation of 8d

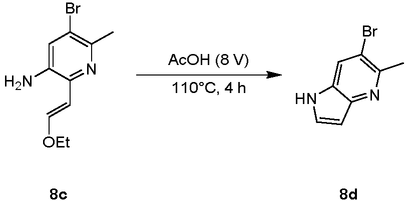

A solution of **8c** (5 g, 7.78 mmol, 1 eq) in acetic acid (40 mL) was stirred at 110 °C for 4 h. LCMS showed the starting material was consumed and 98% of desired mass was detected. The reaction mixture was concentrated under reduced pressure to give a residue, and then diluted with aqueous saturated sodium bicarbonate (200 mL) and extracted with ethyl acetate (3 x 200 mL). The combined organic layers were washed with brine (500 mL), dried over sodium sulfate, filtered and concentrated under reduced pressure to give a residue. The residue was purified by silica gel chromatography (petroleum ether: ethyl acetate=1/2) to give **8d** (6-bromo-5-methyl-1H-pyrrolo[3,2-b]pyridine) (2.4 g, 73.10% yield) as a orange solid. **¹H NMR** (400 MHz, DMSO-*d₆*) δ ppm 11.27 (br s, 1H), 7.98 (s, 1H), 7.60 (t, *J* = 2.8 Hz, 1H), 6.48 (t, *J* = 2.0 Hz, 1H), 2.62 (s, 3H). **LCMS (ESI+):** m/z: 211.0.

### Step 4: Procedure for preparation of 8e

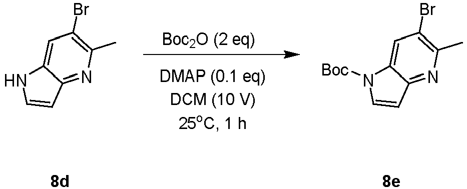

To a solution of **8d** (1.8 g, 4.26 mmol, 1 eq) in dichloromethane (20 mL) was added di-tert-butyl dicarbonate (2.24 g, 5.12 mmol, 1.18 mL, 1.2 eq) and N,N-dimethylpyridin-4-amine (156.28 mg, 639.63 µmol, 0.15 eq). The mixture was stirred at 25°C for 1 h. LCMS showed the starting material was consumed and 98% of the desired mass was detected. The reaction mixture was diluted with water (50 mL) and extracted with dichloromethane (50 mL x 3). The combined organic layers were washed with brine (60 mL), dried over sodium sulfate, filtered and concentrated under reduced pressure to give a residue. The residue was purified by silica gel chromatography (petroleum ether/ethyl acetate=1/1) to give **8e** (5-bromo-6-(tert-butyl 6-bromo-5-methyl-1H-pyrrolo[3,2-b]pyridine-1-carboxylate) (1.8 g, 67.83% yield) as an orange solid. **¹H NMR** (400 MHz, DMSO-*d₆*) δ ppm 8.42 (s, 1H), 7.95 (d, *J* = 4.0 Hz, 1H), 6.77 (d, *J* = 4.0 Hz, 1H), 2.65 (s, 3H), 1.64 (s, 9H). **LCMS (ESI+):** m/z: 311.0.

### Step 5: Procedure for preparation of 8f

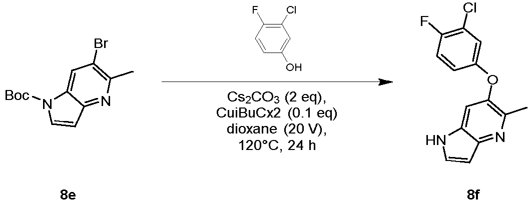

To a solution of **8e** (200 mg, 642.73 µmol, 1 eq) and 3-chloro-4-fluoro-phenol (188.38 mg, 1.29 mmol, 2 eq) in dioxane (4 mL) was added cesium carbonate (418.83 mg, 1.29 mmol, 2 eq) and [(1Z)-2-methyl-1-(2-oxocyclohexylidene)propoxy]-[(1E)-2-methyl-1-(2-oxocyclohexylidene)propoxy]copper (51.16 mg, 128.55 µmol, 0.2 eq) under a N₂ atmosphere. The mixture was stirred at 120 °C for 6 h. LCMS showed the starting material was consumed and 32% of the desired mass was detected. The reaction mixture was diluted with water (10 mL) and extracted with ethyl acetate (20 mL x 3). The combined organic layers were washed with brine (60 mL), dried over sodium sulfate, filtered and concentrated under reduced pressure to give a residue. The residue was purified by preparative HPLC (column: CD24-WePure Biotech XPT C18 150*25*7um;mobile phase: [H₂O(0.1%TFA)-ACN];gradient:7%-37% B over 12.0 min) to give **8f** (6-(3-chloro-4-fluorophenoxy)-5-methyl-1H-pyrrolo[3,2-b]pyridine) (80 mg, 44.98% yield) as a yellow solid. **¹H NMR** (400 MHz, DMSO-*d₆*) δ ppm 12.14 (br s, 1H), 8.15 - 7.91 (m, 2H), 7.53 - 7.30 (m, 2H), 7.10 (td, *J* = 3.6, 9.2 Hz, 1H), 6.72 (br s, 1H), 2.57 (s, 3H). **LCMS (ESI+):** m/z: 277.0.

### Step 6: Procedure for preparation of Compound 180

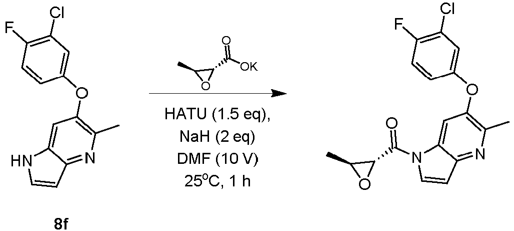

To a solution of **8f** (60 mg, 108.42 µmol, 1 eq) in N,N-dimethylformamide (0.5 mL) was added sodium hydride (17.34 mg, 216.85 µmol, 60% purity, 2 eq) at 0°C under N₂. The mixture was stirred at 25°C for 0.5 h and then the solution was added into the mixture of [(2*R*,3*S*)-3-methyloxirane-2-carbonyl]oxypotassium (60.80 mg, 216.85 µmol, 2 eq) and 2-(3H-[1,2,3]triazolo[4,5-b]pyridin-3-yl)-1,1,3,3-tetramethyluronium hexafluorophosphate(V) (82.45 mg, 216.85 µmol, 2 eq) in N,N-dimethylformamide (0.5 mL) at 0°C under N₂. The reaction mixture was stirred at 25°C for 1 h. The reaction was then quenched with water (2 mL) and extracted with ethyl acetate (3 mL × 3). The combined organic layers were washed with aqueous sodium chloride (10 mL), dried over sodium sulfate, filtered and concentrated under reduced pressure to give a residue. The residue was purified by preparative HPLC (column: CD01-Phenomenex luna C18 150*25*10um;mobile phase: [H₂O(0.225%FA)-ACN];gradient:44%-74% B over 10.0 min) to give **Example 180** (6-(3-chloro-4-fluorophenoxy)-5-methyl-1H-pyrrolo[3,2-b]pyridin-1-yl)-[2*R*,3*S*-methyloxiran-2-yl)methanone) (7.99 mg, 10.21% yield) was obtained as white solid. **¹H NMR** (400 MHz, METHANOL-*d₄*) δ ppm 8.28 - 8.18 (m, 2H), 7.26 (t, *J =* 8.8 Hz, 1H), 7.14 (dd, *J* = 2.8, 6.2 Hz, 1H), 6.95 (td, *J =* 3.6, 9.2 Hz, 1H), 6.87 - 6.81 (m, 1H), 4.14 (d, *J* = 2.0 Hz, 1H), 3.35 - 3.33 (m, 1H), 2.56 (s, 3H), 1.48 (d, *J* = 5.2 Hz, 3H). **LCMS (ESI+):** m/z: 360.8.

The following examples were prepared using **Scheme 8.**

| Example | Structure | Chemical name | LC-MS method | LC-MS retention time (mins) | Mass found (M+H) ⁺ |
|---|---|---|---|---|---|
| 179 | 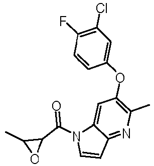 | *Trans*-(6-(3-chloro-4-fluorophenoxy)-5-methyl-1H-pyrrolo[3,2-b]pyridin-1-yl)(3-methyloxiran-2-yl)methanone (Racemic) | D | 2.515 | 361.2 |
| 180 | 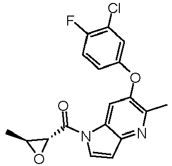 | (6-(3-chloro-4-fluorophenoxy)-5-methyl-1H-pyrrolo[3,2-b]pyridin-1-yl)((2R,3S)-3-methyloxiran-2-yl)methanone | T | 2.576 | 361.2 |

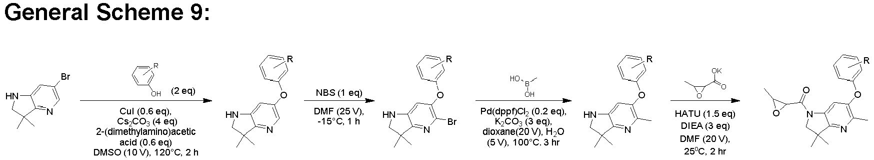

### Example 181-Step 1: Procedure for preparation of 9b

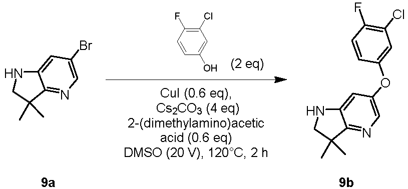

A mixture of 9a (0.4 g, 1.76 mmol, 1 eq), 3-chloro-4-fluoro-phenol (516.24 mg, 3.52 mmol, 2 eq), 2-(dimethylamino)acetic acid (108.98 mg, 1.06 mmol, 0.6 eq), copper(i) iodide (201.27 mg, 1.06 mmol, 0.6 eq) and cesium carbonate (2.30 g, 7.05 mmol, 4 eq) in dimethyl sulfoxide (8 mL) was degassed and purged with N₂ 3 times. The mixture was stirred at 120°C for 2 hrs under a N₂ atmosphere. LCMS showed the starting material was consumed and 27% of the desired mass was detected. After cooling to 25°C, the combined mixture was filtered through a Celite pad and rinsed with dichloromethane (3 × 30 mL). The filtrate was concentrated to afford the crude product The residue was purified by column chromatography (petroleum ether/ethyl acetate = 10/1) to give **9b** (6-(3-chloro-4-fluoro-phenoxy)-3,3-dimethyl-1,2-dihydropyrrolo[3,2-b]pyridine) (0.09 g, 17.46% yield) as a red solid. **¹H NMR** (400 MHz, METHANOL-*d₄*) δ ppm 7.40 (d, J = 2.4 Hz, 1 H) 7.24 (t, J = 8.8 Hz, 1 H) 7.13 (dd, J = 6.0, 3.00 Hz, 1 H) 6.94 - 7.00 (m, 1 H) 6.52 (d, J = 2.4 Hz, 1 H) 3.40 (s, 2 H) 1.32 (s, 6 H). **LCMS (ESI+):** m/z: 291.7.

### Step 2: Procedure for preparation of 9c

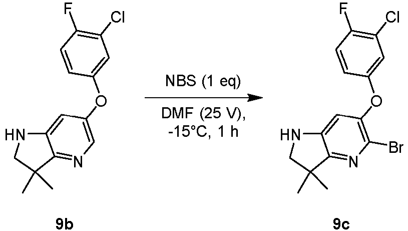

To a solution of 9b (0.09 g, 307.44 µmol, 1 eq) in N,N-dimethylformamide (1.8 mL) was added N-bromosuccinimide (54.72 mg, 307.44 µmol, 1 eq) in N,N-dimethylformamide (0.45 mL). The mixture was stirred at -15°C for 1 hr. LCMS showed no starting material was remaining and 88% of the desired mass was detected. The combined mixture was filtered through a Celite pad and rinsed with dichloromethane (3 × 10 mL). The filtrate was concentrated to afford the crude product. The residue was purified by column chromatography (petroleum ether/ethyl acetate=2/1) to give **9c** (5-bromo-6-(3-chloro-4-fluoro-phenoxy)-3,3-dimethyl-1,2-dihydropyrrolo[3,2-b]pyridine) (0.08 g, 70.02% yield) as a yellow solid. **¹H NMR** (400 MHz, METHANOL-*d₄*) δ ppm 7.23 (t, *J = 8.8* Hz, 1 H) 7.04 (dd, *J* = 6.0, 3.00 Hz, 1 H) 6.88 (dt, *J* = 9.2, 3.42 Hz, 1 H) 6.56 (s, 1 H) 3.41 (s, 2 H) 1.32 (s, 6 H). **LCMS (ESI+):** m/z: 369.6.

### Step 3: Procedure for preparation of 9d

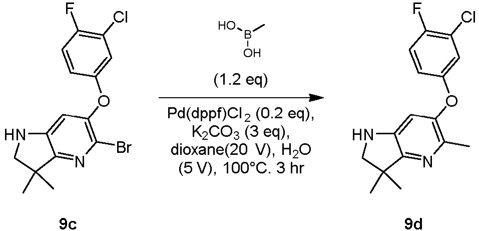

A mixture of **9c** (0.08 g, 215.27 µmol, 1 eq), methylboronic acid (15.46 mg, 258.32 µmol, 1.2 eq), Pd(dppf)Cl₂ (35.16 mg, 43.05 µmol, 0.2 eq), potassium carbonate (89.25 mg, 645.80 µmol, 3 eq) in dioxane (1.6 mL) and H₂O (0.4 mL) was degassed and purged with N₂. The mixture was stirred at 100°C for 3 hrs under a N₂ atmosphere. LCMS showed 54% of the desired mass was detected. The combined mixture was filtered through a Celite pad and rinsed with dichloromethane (3 × 30 mL). The filtrate was concentrated to afford the crude product. The residue was purified by column chromatography (petroleum ether/ethyl acetate = 2/1) to give **9d** (6-(3-chloro-4-fluoro-phenoxy)-3,3,5-trimethyl-1,2-dihydropyrrolo[3,2-b]pyridine) (0.03 g, 45.43% yield) as a white solid. **¹H NMR** (400 MHz, METHANOL-*d₄*) δ ppm 7.21 (t, *J* = 8.8 Hz, 1 H) 6.99 (dd, *J* = 6.0, 3.00 Hz, 1 H) 6.85 (dt, *J* = 9.2, 3.41 Hz, 1 H) 6.51 (s, 1 H) 3.36 (s, 2 H) 2.28 (s, 3 H) 1.33 (s, 6 H). **LCMS (ESI+):** m/z: 306.2

### Step 4: Procedure for preparation of Compound 181

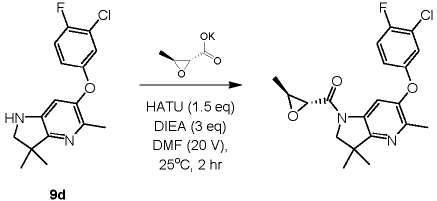

A mixture of **9d** (0.03 g, 97.80 µmol, 1 eq), [(2R,3S)-3-methyloxirane-2-carbonyl]oxypotassium (16.45 mg, 117.35 µmol, 1.2 eq), N,N-diisopropylethylamine (37.92 mg, 293.39 µmol, 51.10 µL, 3 eq) 2-(3H-[1,2,3]triazolo[4,5-b]pyridin-3-yl)-1,1,3,3-tetramethyluronium hexafluorophosphate(V) (55.78 mg, 146.69 µmol, 1.5 eq) in N,N-dimethylformamide (0.6 mL) was degassed and purged with N₂ 3 times. The mixture was stirred at 25°C for 2 hrs under N₂. LCMS showed no starting material remained and 54% of the desired mass was detected. The residue was quenched with water (10 mL) and extracted with ethyl acetate (3 mL × 3). The combined organic layers were washed with aqueous sodium chloride (10 mL), dried over sodium sulfate, filtered and concentrated under reduced pressure to give a residue. The residue was purified by preparative HPLC (column: WePure Biotech XP tC18 150 × 40 × 7 um; mobile phase: [H₂O(10mM NH₄HCO₃)-CH₃CN]; gradient: 45%-75% B over 8.0 min). SFC separation gave **Example 181** ([6-(3-chloro-4-fluoro-phenoxy)-3,3,5-trimethyl-2H-pyrrolo[3,2-b]pyridin-1-yl]-[(2*R*,3*S*)-3-methyloxiran-2-yl]methanone) (17.78 mg, 45.12% yield,) as a yellow solid. **¹H NMR** (400 MHz, CHLOROFORM-d) δ ppm 7.95 (s, 1 H) 7.09 (t, *J* = 8.8 Hz, 1 H) 6.94 (dd, *J* = 6.0, 2.94 Hz, 1 H) 6.80 (dt, *J* = 8.8, 3.33 Hz, 1 H) 4.13 (d, *J* = 10.0 Hz, 1 H) 3.98 - 4.06 (m, 1 H) 3.26 - 3.38 (m, 2 H) 2.45 (s, 3 H) 1.47 (d, *J* = 5.6 Hz, 9 H). **LCMS (ESI+):** m/z: 390.1.

The following examples were prepared in a similar fashion using **Scheme 9.**

| Example | Structure | Chemical name | LC-MS method | LC-MS retention time (mins) | Mass found (M+H) ⁺ |
|---|---|---|---|---|---|
| 181 | 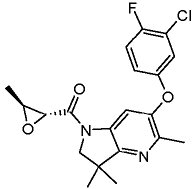 | (6-(3-chloro-4-fluorophenoxy)-3,3,5-trimethyl-2,3-dihydro-1H-pyrrolo[3,2-b]pyridin-1-yl)((2R,3S)-3-methyloxiran-2-yl)methanone | H | 3.164 | 391.1 |
| 182 | 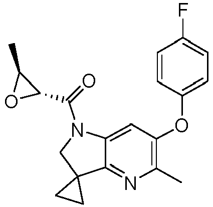 | (6'-(4-fluorophenoxy)-5'-methylspiro[cyclopropan e-1,3'-pyrrolo[3,2-b]pyridin]-1'(2'H)-yl)((2R,3S)-3-methyloxiran-2-yl)methanone | D | 2.470 | 355.2 |

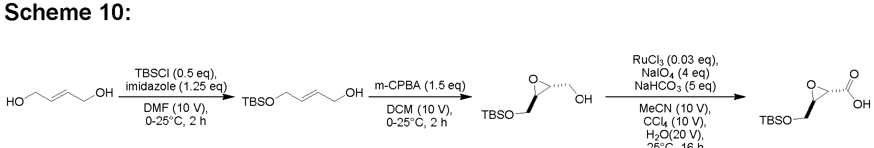

### Step 1: Procedure for preparation of 1B

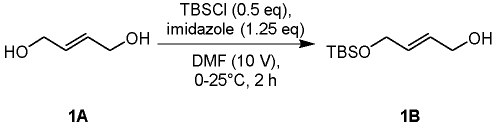

To a solution of **1A** (10 g, 113.50 mmol, 9.35 mL, 1 eq) and imidazole (9.66 g, 141.88 mmol, 1.25 eq) in N,N-dimethylformamide (100 mL) was added tert-butyl chlorodimethylsilane (8.55 g, 56.75 mmol, 6.98 mL, 0.5 eq) dropwise at 0°C. The resulting mixture was stirred at 25°C for 2 h. TLC (petroleum ether/ethyl acetate = 3/1, Rf = 0.5) showed the starting material was consumed and a new major spot generated. The residue was diluted with saturated ammonium chloride solution (200 mL) and extracted with ethyl acetate (3 x 50 mL). The combined organic layers were washed with brine (100 mL), dried over sodium sulfate, filtered and concentrated under reduced pressure to give a residue. The residue was purified by column chromatography (petroleum ether/ethyl acetate = 5/1) to give **1B** ((E)-4-[tert-butyl(dimethyl)silyl]oxybut-2-en-1-ol) (6 g, yield 26.12%) as a colourless oil. **¹H NMR** (400 MHz, CHLOROFORM-*d*) δ ppm 5.74 - 5.96 (m, 2 H) 4.14 - 4.24 (m, 4 H) 0.92 (s, 9 H) 0.09 (s, 6 H).

### Step 2: Procedure for preparation of 1C

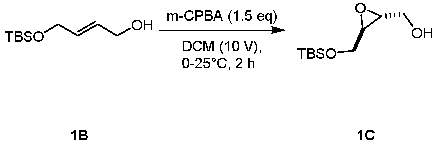

To a solution of **1B** (6 g, 29.65 mmol, 1 eq) in dichloromethane (60 mL) was added 3-chloroperoxybenzoic acid (7.67 g, 44.47 mmol, 1.5 eq) at 0°C. The mixture was stirred at 25°C for 2 h. TLC (petroleum ether/ethyl acetate = 3/1, Rf = 0.5) showed the starting material was consumed and a new major spot was generated. The mixture was filtered, and the filtrate was quenched by the addition of saturated sodium bicarbonate aqueous solution (60 mL) at 0°C, followed by extraction with dichloromethane (3 x 30 mL). The combined organic layers were washed with aqueous sodium chloride (10 mL), dried over sodium sulfate, filtered and concentrated under reduced pressure to give a residue. The residue was purified by column chromatography (petroleum ether/ethyl acetate = 5/1) to give **1C** ([3-[[tert-butyl(dimethyl)silyl]oxymethyl]oxiran-2-yl]methanol) (6 g, yield 92.67%) as a colourless oil. **¹H NMR** (400 MHz, CHLOROFORM-*d*) δ ppm 3.87 - 4.01 (m, 2 H) 3.64 - 3.77 (m, 2 H) 3.06 - 3.22 (m, 2 H) 0.91 (s, 9 H) 0.09 (d, *J = 4.00* Hz, 6 H).

### Step 3: Procedure for preparation of 1D

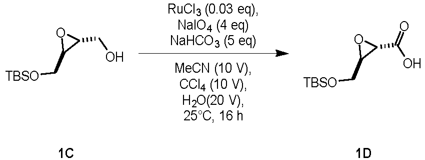

To a solution of **1C** (1 g, 4.58 mmol, 1 eq) in carbon tetrachloride (10 mL), acetonitrile (10 mL) and water (20 mL) was added sodium bicarbonate (1.92 g, 22.90 mmol, 5 eq), sodium periodate (3.92 g, 18.32 mmol, 4 eq) and ruthenium trichloride (28.50 mg, 137.38 µmol, 0.03 eq). The mixture was stirred at 25°C for 16 h. TLC (petroleum ether/ethyl acetate = 0/1, R_{f} = 0.5) showed the starting material was consumed and a new major spot was generated. The reaction mixture was quenched with water (50 mL) at 0°C and extracted with dichloromethane 150 mL (50 mL × 3). The aqueous layer was acidified to pH = 3 with acetic acid (1 M, 40 mL) and then extracted with dichloromethane 60 mL (3 x 20 mL). The combined organic layers were washed with aqueous sodium chloride (60 mL), dried over sodium sulfate, filtered and concentrated under reduced pressure to give **1D** (*trans*-3-[[tert-butyl(dimethyl)silyl]oxymethyl]oxirane-2-carboxylic acid) (0.5 g, yield 46.99%) as a yellow oil. **¹H NMR:** (400 MHz, CHLOROFORM-*d*) δ ppm 3.97 (dd, *J =* 12.0, 2.25 Hz, 1 H) 3.82 (dd, *J =* 12.0, 3.2 Hz, 1 H) 3.54 (d, *J* = 1.60 Hz, 1 H) 3.34 - 3.40 (m, 1 H) 0.90 (s, 9 H) 0.08 (d, *J* = 3.20 Hz, 6 H).

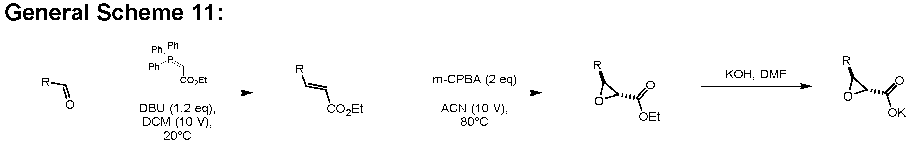

### Step 1: Procedure for preparation of 2B

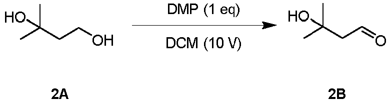

To a solution of **2A** (2 g, 19.2 mmol, 1.0 eq) in dichloromethane (20 mL) was added Dess-Martin periodane (8.15 g, 19.2 mmol, 1.0 eq) at 0°C under nitrogen. The mixture was stirred at 25°C for 2 h. The reaction mixture was then poured into water (100 mL) at 0°C, stirred for 30 min and extracted with ethyl acetate 90 mL (3 × 30 mL). The combined organic layers were washed with brine 20 mL, dried over Na₂SO₄, filtered and concentrated under reduced pressure to give a residue. The residue was purified by column chromatography (petroleum ether/ethyl acetate=3/1) to give **2B** (3-hydroxy-3-methyl-butanal) (0.26 g, 13% yield) as a colourless oil. **¹H NMR** (400 MHz, DMSO-*d*₆) δ ppm 9.64 - 9.81 (m, 1 H), 4.64 - 4.89 (m, 1 H), 2.40 (d, *J* = 3.2 Hz, 2 H), 1.21 (s, 6 H).

### Step 2: Procedure for preparation of 2C

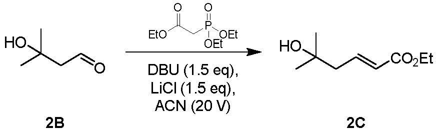

To a solution of ethyl 2-(diethoxyphosphoryl)acetate (856 mg, 3.82 mmol , 1.5 eq) in acetonitrile (2 mL) was added 2,3,4,6,7,8,9,10-octahydropyrimido[1,2-a]azepine (581 mg, 3.82 mmol, 1.5 *eq)* and lithium chloride (162 mg, 3.82 mmol, 1.5 *eq).* After stirring for 15 min, **2B** (0.26 g, 2.55 mmol, 1.0 eq) in acetonitrile (0.6 mL) was added at 0°C. The mixture was stirred at 25°C for 1 h. The reaction mixture was then poured into water (5 mL) at 25°C and extracted with ethyl acetate 9 mL (3 mL × 3). The combined organic layers were washed with brine (2 mL), dried over Na₂SO₄, filtered and concentrated under reduced pressure to give a residue. The residue was purified by column chromatography (petroleum ether/ethyl acetate = 3/1) to give **2C** ethyl (E)-5-hydroxy-5-methyl-hex-2-enoate (260 mg, 59% yield) as a colourless oil. **¹H NMR** (400 MHz, CDCl₃) δ ppm 7.01 (dt, *J* = 15.6, 7.8 Hz, 1 H), 5.89 (d, *J* = 15.6 Hz, 1 H), 4.20 (q, *J* = 7.2 Hz, 2 H), 2.39 (d, *J* = 7.8 Hz, 2 H), 1.26 - 1.32 (m, 9 H).

### Step 3: Procedure for preparation of 2D

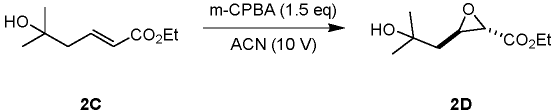

To a solution of **2C** (0.26 g, 1.51 mmol, 1.0 eq) in acetonitrile (2.6 mL) was added 3-chlorobenzoperoxoic acid (460 mg, 2.26 mmol, 85 wt%, 1.5 eq) under nitrogen. The mixture was stirred at 75°C for 12 h. The reaction mixture was then poured into a saturated solution of sodium sulfite (5 mL) at 0°C, and extracted with ethyl acetate (5 mL × 3). The combined organic layers were washed with brine (2 mL), dried over Na₂SO₄, filtered and concentrated under reduced pressure. The residue was purified by column chromatography (petroleum ether/ethyl acetate=87/13) to give **2D** (*trans*-ethyl 3-(2-hydroxy-2-methyl-propyl)oxirane-2-carboxylate) (170 mg, 60% yield) as a colourless oil. **¹H NMR** (400 MHz, CDCl₃) δ ppm 4.19 - 4.31 (m, 2 H), 3.37 (ddd, *J* = 7.6, 4.0, 2.0 Hz, 1 H), 3.23 (d, *J* = 2.0 Hz, 1 H), 1.94 (dd, *J* = 14.4, 4.0 Hz, 1 H), 1.62 (dd, *J =* 14.4, 7.6 Hz, 1 H), 1.35 (s, 6 H), 1.31 (t, *J* = 7.2 Hz, 3 H).

### Step 4: Procedure for preparation of 2E

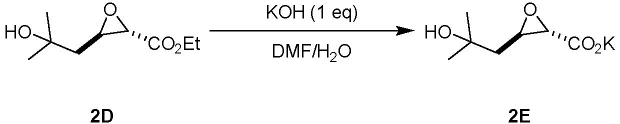

To a solution of **2D** (170 mg, 903 µmol, 1.0 eq) in *N*,*N*-dimethylformamide (0.85 mL) was added a solution of potassium hydroxide (50.7 mg, 903 µmol, 1.0 *eq*) in water (0.085 mL). The mixture was stirred at 25°C for 1 h. TLC showed the reactant was consumed and one new spot was observed. The reaction was added to acetonitrile (60 mL), filtered and the solid was lyophilized to give **2E** (*trans*-[3-(2-hydroxy-2-methyl-propyl)oxirane-2-carbonyl]oxypotassium) (105 mg, 59% yield) as a yellow solid. **¹H NMR** (400 MHz, DMSO-*d*₆) δ ppm 4.35 (br s, 1 H), 2.80 (br t, *J = 5.6* Hz, 1 H), 2.59 (s, 1 H), 1.50 - 1.55 (m, 1 H), 1.39 - 1.47 (m, 1 H), 1.13 (d, *J* = 8.4 Hz, 6 H).

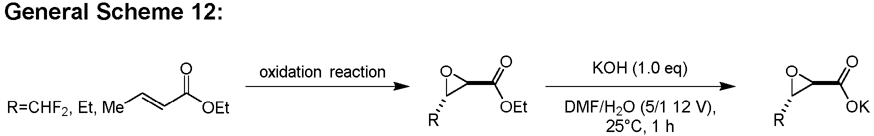

### Step 1: Procedure for preparation of 3B

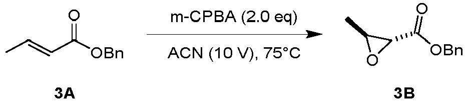

To a solution of **3A** (10 g, 56.7 mmol, 1 eq) in acetonitrile (100 mL) was added 3-chloroperoxybenzoic acid (23 g, 114 mmol, 85% purity, 2.0 eq) in portions and the mixture was stirred at 75°C for 12 hrs under a nitrogen atmosphere. The reaction mixture was then quenched by the addition of saturated sodium sulfite solution (500 mL) at 25°C, and extracted with ethyl acetate (1 L × 2). The combined organic layers were washed with saturated sodium bicarbonate aqueous solution (200 mL × 2), dried over Na₂SO₄, filtered and concentrated under reduced pressure to give a residue. The residue was purified by column chromatography (petroleum ether/ethyl acetate = 5/1) to give **3B** (*trans*-benzyl 3-methyloxirane-2-carboxylate) (13 g, yield 59.6 %) as a colourless oil. **¹H NMR** (400 MHz, DMSO-*d*₆) δ ppm 7.27 - 7.51 (m, 5 H), 5.08 - 5.25 (m, 2 H), 3.41 (d, *J* = 1.6 Hz, 1 H), 3.20 (qd, *J* = 5.2, 2.0 Hz, 1 H), 1.30 (d, *J* = 5.2 Hz, 3 H).

### Step 2: Procedure for preparation of 3C

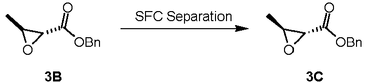

**3B** (13 g) was separated by SFC (column: DAICEL CHIRALCEL OJ (250mm*30mm,10um);mobile phase: [column: (s,s) WHELK-O1 (250mm*30mm,10um);mobile phase: [CO₂-IPA(0.1% NH₃H₂O)]; B%:10%, isocratic elution mode) to give **3C** (5 g, yield 38.4%) as a colourless oil. **¹H NMR** (400 MHz, DMSO-*d*₆) δ ppm 7.03 - 7.67 (m, 5 H), 4.98 - 5.29 (m, 2 H), 3.41 (d, *J = 1.6* Hz, 1 H), 3.20 (qd, *J* = 5.2, 2.0 Hz, 1 H), 1.30 (d, *J = 5.2* Hz, 3 H). LCMS (ESI+): m/z 234.1 (M+1)⁺

### Step 3: Procedure for preparation of 3D.

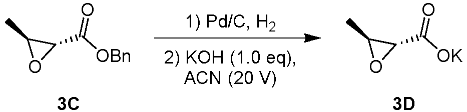

To a mixture of palladium on carbon (2.77 g, 2.60 mmol, 10% purity, 0.1 eq) in methanol (50 mL) was added **3C** (5 g, 26.0 mmol, 1.0 eq) at 25°C under argon. The mixture was stirred at 25°C for 2 h under hydrogen (15 psi). TLC showed the starting material was consumed and a new spot was detected. The reaction mixture was filtered and the filtrate was concentrated under reduced pressure to give (2*R*,3*S*)-3-methyloxirane-2-carboxylic acid (2.5 g, yield 94%) at 25°C as a colourless oil. To a solution of (2R,3S)-3-methyloxirane-2-carboxylic acid (2.5 g, 24.5 mmol, 1.0 eq) in acetonitrile (30 mL) was added a solution of potassium hydroxide (1.37 g, 24.49 mmol, 1.0 *eq*) in water (1.8 mL). The mixture was stirred at 25°C for 1 h. Acetonitrile was then added to precipitate the product and the mixture was filtered and concentrated under reduced pressure to give **3D** [(2*R*,3*S*)-3-methyloxirane-2-carbonyl]oxypotassium (3 g, yield 83%) as a white solid. **¹H NMR** (400 MHz, DMSO-*d*₆) δ ppm 2.76 (m, 1 H), 2.63 (d, *J =* 1.6 Hz, 1 H), 1.17 (d, *J = 5.2* Hz, 3 H).

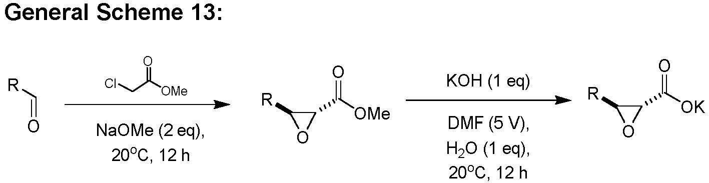

### Step 1: Procedure for preparation of 4B.

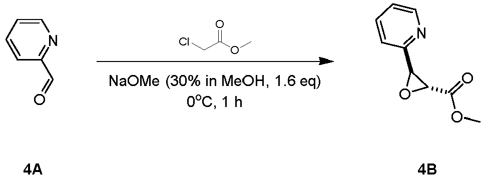

**4A** (1 g, 9.34 mmol, 1 eq) was added into a solution of sodium methoxide (2.69 g, 14.9 mmol, 30% purity, 1.6 eq) at 0°C under nitrogen. Then methyl 2-chloroacetate (1.62 g, 14.9 mmol, 1.6 eq) was added dropwise at 0°C. The mixture was stirred at 0°C for 1 h. LCMS showed 84% of the desired mass was detected. Acetic acid (0.52 mL, 0.6 eq) was added to the reaction mixture at 0°C and then water (30 mL) was added. The aqueous solution was extracted with ethyl acetate (30 mL x 3). The combined organic layers were washed with brine (20 mL), dried over anhydrous Na₂SO₄, filtered and the filtrate was concentrated under reduced pressure. The residue was purified by silica gel chromatography (PE/EA = 7/1 to 5/1) to get **4B** (methyl 3-(2-pyridyl)oxirane-2-carboxylate) (1.1 g, 63.1% yield) as a brown solid. **¹H NMR:** (400 MHz-CDCl₃) δ ppm 8.60 (d, *J = 4.8* Hz, 1 H), 7.72 (td, *J* = 7.6, 1.6 Hz, 1 H), 7.27 - 7.33 (m, 2 H), 4.25 (d, *J* = 1.6 Hz, 1 H), 3.84 (s, 3 H), 3.75 (d, *J = 1.6* Hz, 1 H). **LCMS (ESI+):** m/z 180.1 (M+H)⁺

### Step 2: Procedure for preparation of 4C

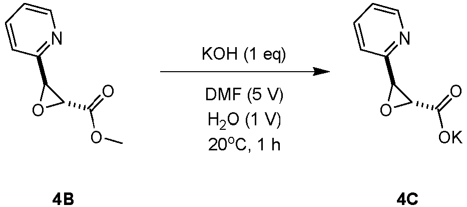

To a solution of **4B** (500 mg, 2.79 mmol, 1 eq) in *N*,*N*-dimethylformamide (2.5 mL) was added a solution of potassium hydroxide (156 mg, 2.79 mmol, 1 eq) in water (0.5 mL). The mixture was stirred at 20°C for 1 h. The reaction mixture was then added dropwise to acetonitrile (60 mL), filtered and the filter cake was dried *in vacuo* to give **4C** 3-(2-pyridyl)oxirane-2-carboxylic acid (260 mg, 56.4% yield) as a white solid. **¹H NMR:** (400 MHz, DMSO-*d*₆) δ ppm 8.42 - 8.54 (m, 1 H), 7.76 (td, *J* = 7.6, 2.0 Hz, 1 H), 7.30 (ddd, *J* = 7.6, 4.8, 1.2 Hz, 1 H), 7.22 (d, *J* = 8.0 Hz, 1 H), 3.78 (d, *J* = 2.0 Hz, 1 H), 3.17 (d, *J =* 2.0 Hz, 1 H).

### LCMS Method A:

| | | | | |
|---|---|---|---|---|
| WUXICD05 | | | | |
| Instrument | | Agilent 1260 LC & Agilent G6125C | | |
| Software | | Agilent Open Lab CDS 2.6 | | |
| | Column | Waters XBridge^{®} C18 5 µm, 2.1*50mm | | |
| | Mobile Phase | A: 0.05% NH₃·H₂O in water (v/v) | | |
| | | B: 100% Acetonitrile | | |
| | Gradient | Time(min) | B(%) | Flow(mL/min) |
| | | 0.00 | 5 | 0.8 |
| | | 0.40 | 5 | 0.8 |
| | | 3.40 | 90 | 0.8 |
| HPLC | | 3.90 | 100 | 0.8 |
| | | 4.00 | 5 | 0.8 |
| | | 4.00 | 5 | 1.0 |
| | | 4.50 | 5 | 1.0 |
| | Post time(min) | Off | | |
| | Column Temp | 45°C | | |
| | Detector | DAD | | |
| MS | Ionization | ESI | | |
| | Drying Gas | N2 | | |
| | Drying Gas Flow | 10(L/min) | | |
| | Nebulizer Pressure | 40(psi) | | |
| | Drying Gas Temperature | 350 °C | | |
| | Capillary Voltage | 3500(V) | | |
| | MS Polarity | Positive | | |
| | MS Mode | Scan | | |
| | Mass Range | 100-1500 | | |

### LCMS Method B:

| | | | | |
|---|---|---|---|---|
| Method name | | WUXIAB05 | | |
| Instrument | | Agilent 1260 LC & Agilent 6125C MSD | | |
| Software | | Agilent Open Lab CDS 2.6 | | |
| | Column | Agilent poroshell 120 EC-C18 3.0*50mm,2.7µm | | |
| | Mobile Phase | A: 0.0375% TFA in water (v/v) | | |
| | | B: 0.01875% TFA in Acetonitrile (v/v) | | |
| | Gradient | Time(min) | B(%) | Flow(mL/min) |
| | | 0.00 | 05 | 1.0 |
| | | 0.40 | 05 | 1.0 |
| HPLC | | 3.00 | 99 | 1.0 |
| | | 4.00 | 99 | 1.0 |
| | | 4.10 | 5 | 1.0 |
| | | 4.50 | 5 | 1.0 |
| | Post time(min) | Off | | |
| | Column Temp | 45°C | | |
| | Detector | DAD | | |
| MS | Ionization source | ESI | | |
| | Drying Gas | N₂ | | |
| | Drying Gas Flow | 10(L/min) | | |
| | Nebulizer Pressure | 40(psi) | | |
| | Drying Gas Temperature | 350 °C | | |
| | Capillary Voltage | 3500(V) Positive | | |
| | MS Polarity | Positive | | |
| | MS Mode | Scan | | |
| | Mass Range | 100-2000 | | |

### LCMS Method C:

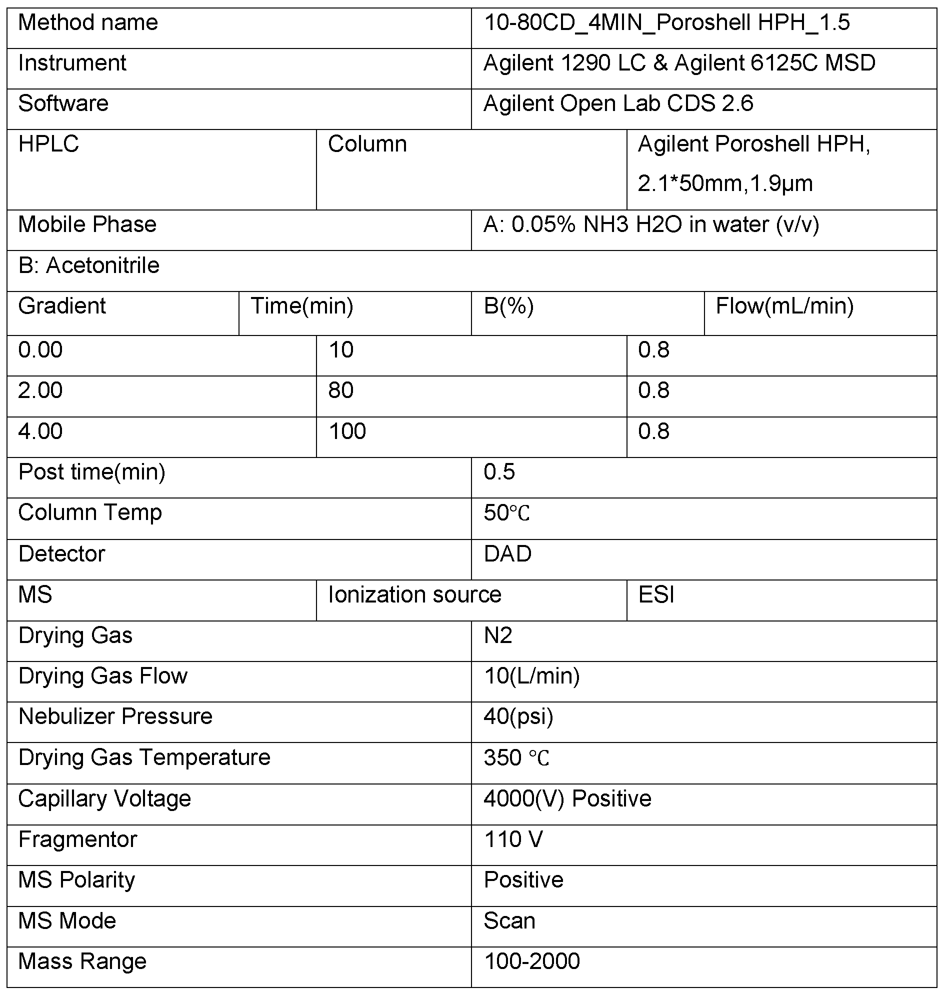

### LCMS Method D:

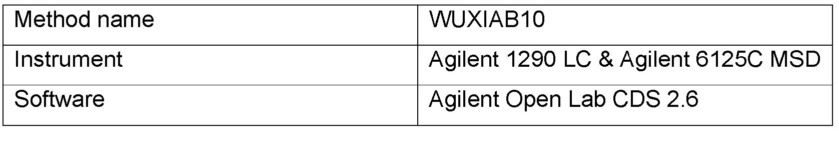

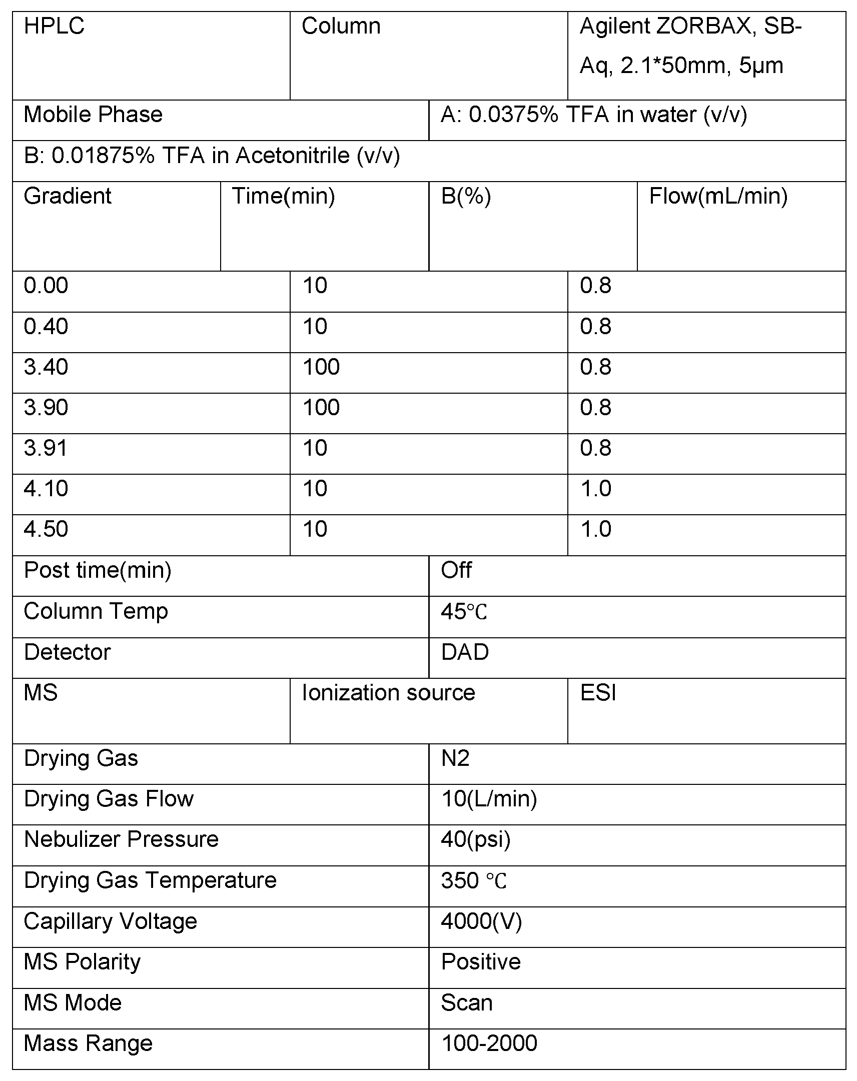

### LCMS Method E:

5_95AB_6min-220-254: LC/MS( The gradient was 5%B in 0.40min and 5-95% B in 2.60 min , hold on 95% B in 1.00min, and then 95-5%B in 0.01min, the flow rate was 1.0 ml/min. Mobile phase A was 0.04% Trifluoroacetic Acid in water, mobile phase B was 0.02% Trifluoroacetic Acid in acetonitrile. The column used for chromatography was a Halo C18, 3.0*30mm column (5um particles). Detection methods are diode array (DAD) detection .MS mode was positive electrospray ionization. MS range was 100-1000.

### LCMS Method F:

NEG5_95CD_6min-220-254-ELSD : LC/MS **(**The gradient was 5%B in 0.40min and 5-95% B at 0.40-3.40 min ,hold on 95% B for 0.45 min, and then 95-5%B in 0.01min, the flow rate was 0.8 ml/min. Mobile phase A was H₂O+10 mM NH₄HCO₃, mobile phase B was Acetonitrile. The column used for chromatography was a Xbridge Shield RP18 2.1*50 mm column (5um particles). Detection methods are diode array (DAD) and evaporative light scattering (ELSD) detection as well as positive electrospray ionization (MS).) MS range was 100-1000.

### LCMS Method G:

| | | | | |
|---|---|---|---|---|
| Method name | | WUXIN05 | | |
| Instrument | | Agilent 1260 LC & Agilent 6125C MSD | | |
| Software | | Agilent Open Lab CDS 2.6 | | |
| | Column | Agilent poroshell 120 EC-C18 3.0*50mm,2.7µm | | |
| | Mobile Phase | A: water | | |
| | | B: ACN | | |
| | Gradient | Time(min) | B(%) | Flow(mL/min) |
| | | 0.00 | 5 | 1.0 |
| | | 0.40 | 5 | 1.0 |
| HPLC | | 3.00 | 99 | 1.0 |
| | | 4.00 | 99 | 1.0 |
| | | 4.10 | 5 | 1.0 |
| | | 4.50 | 5 | 1.0 |
| | Post time(min) | Off | | |
| | Column Temp | 45°C | | |
| | Detector | DAD | | |
| MS | Ionization source | ESI | | |
| | Drying Gas | N₂ | | |
| | Drying Gas Flow | 10(L/min) | | |
| | Nebulizer Pressure | 40(psi) | | |
| | Drying Gas Temperature | 350 °C | | |
| | Capillary Voltage | 3500(V) Positive | | |
| | MS Polarity | Positive | | |
| | MS Mode | Scan | | |
| | Mass Range | 100-2000 | | |

### LCMS Method H:

5_95CD_6min-220-254-ELSD : LC/MS( The gradient was 5%B in 0.40min and 5-95% B at 0.40-3.40 min ,hold on 95% B for 0.45min, and then 95-5%B in 0.01min, the flow rate was 0.8 ml/min. Mobile phase A was H2O+10mM NH4HCO3, mobile phase B was Acetonitrile. The column used for chromatography was a Xbridge C18 2.1*50mm column (5um particles). Detection methods are diode array (DAD) and evaporative light scattering (ELSD) detection. MS mode was positive electrospray ionization. MS range was 100-1000.

### LCMS Method I:

| | | | | |
|---|---|---|---|---|
| Method name | | WUXIAB00 | | |
| Instrument | | Agilent 1290 LC & Agilent 6125C MSD | | |
| Software | | Agilent Open Lab CDS 2.6 | | |
| | Column | Agilent ZORBAX 5µm SB-Aq, 2.1*50mm | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| | | 0.00 | 0.0 | 0.6 |
| | | 0.40 | 0.0 | 0.6 |
| HPLC | | 3.40 | 100 | 0.6 |
| | | 3.90 | 100 | 0.6 |
| | | 3.91 | 0.0 | 0.6 |
| | | 4.10 | 0.0 | 1.0 |
| | | 4.50 | 0.0 | 1.0 |
| | Post time(min) | Off | | |
| | Column Temp | 45°C | | |
| | Detector | DAD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 4000(V) Positive | | |
| | MS Polarity | Positive | | |
| | MS Mode | Scan | | |
| | Mass Range | 100-2000 | | |

### LCMS Method J:

| | | | | |
|---|---|---|---|---|
| Method name | | 5-95(3-4.5)N_1_40_Xbridge C18_5cm | | |
| Instrument | | Agilent 1290 LC & Agilent 6125C MSD | | |
| Software | | Agilent Open Lab CDS 2.6 | | |
| | Column | Agilent ZORBAX 5µm SB-Aq, 2.1*50mm | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| | | 0.00 | 5 | 1 |
| HPLC | | 3.00 | 95 | 1 |
| | | 4.50 | 95 | 1 |
| | Post time(min) | 1.00 | | |
| | Column Temp | 40°C | | |
| | Detector | DAD&ELSD | | |
| MS | Ionization source | ESI | | |
| | Drying Gas | N₂ | | |
| | Drying Gas Flow | 10(L/min) | | |
| | Nebulizer Pressure | 40(psi) | | |
| | Drying Gas Temperature | 350 °C | | |
| | Capillary Voltage | 3500(V) Positive | | |
| | MS Polarity | Positive | | |
| | MS Mode | Scan | | |
| | Mass Range | 100-2000 | | |

### LCMS Method K:

| | | | | |
|---|---|---|---|---|
| Method name | | WUXIN10_NH4AC | | |
| Instrument | | Agilent 1290 LC & Agilent 6125C MSD | | |
| Software | | Agilent Open Lab CDS 2.6 | | |
| | Column | Agilent ZORBAX, SB-Aq, 2.1*50mm, 5µm | | |
| | Mobile Phase | A: 10mM NH4AC In Water | | |
| | | B: Acetonitrile | | |
| | Gradient | Time(min) | B(%) | Flow(mL/min) |
| | | 0.00 | 10 | 0.8 |
| | | 0.40 | 10 | 0.8 |
| HPLC | | 3.40 | 100 | 0.8 |
| | | 3.90 | 100 | 0.8 |
| | | 3.91 | 10 | 0.8 |
| | | 4.10 | 10 | 1.0 |
| | | 4.50 | 10 | 1.0 |
| | Post time(min) | Off | | |
| | Column Temp | 45°C | | |
| | Detector | DAD&ELSD | | |
| MS | Ionization source | ESI | | |
| | Drying Gas | N₂ | | |
| | Drying Gas Flow | 10(L/min) | | |
| | Nebulizer Pressure | 40(psi) | | |
| | Drying Gas Temperature | 350 °C | | |
| | Capillary Voltage | 3500(V) Positive | | |
| | MS Polarity | Positive | | |
| | MS Mode | Scan | | |
| | Mass Range | 100-2000 | | |

### LCMS Method L:

| | | | | |
|---|---|---|---|---|
| Method name | | WUXIN10_Aq | | |
| Instrument | | Agilent 1260 LC & Agilent 6125C MSD | | |
| Software | | Agilent Open Lab CDS 2.6 | | |
| | Column | Agilent ZORBAX, SB-Aq, 2.1*50mm, 5µm | | |
| | Mobile Phase | A: Water | | |
| | | B: Acetonitrile | | |
| | Gradient | Time(min) | B(%) | Flow(mL/min) |
| | | 0.00 | 10 | 0.8 |
| | | 0.40 | 10 | 0.8 |
| HPLC | | 3.40 | 100 | 0.8 |
| | | 3.90 | 100 | 0.8 |
| | | 3.91 | 10 | 0.8 |
| | | 4.10 | 10 | 1.0 |
| | | 4.50 | 10 | 1.0 |
| | Post time(min) | Off | | |
| | Column Temp | 45°C | | |
| | Detector | DAD | | |
| | Ionization source | ESI | | |
| | Drying Gas | N₂ | | |
| | Drying Gas Flow | 7(L/min) | | |
| | Nebulizer Pressure | 15(psi) | | |
| MS | Drying Gas Temperature | 300 °C | | |
| | Capillary Voltage | 4000(V) Positive | | |
| | MS Polarity | Positive | | |
| | MS Mode | Scan | | |
| | Mass Range | 100-1000 | | |

### LCMS Method M:

| | | | | |
|---|---|---|---|---|
| Method name | | WUXIN25_1 | | |
| Instrument | | Agilent 1290 LC & Agilent 6125C MSD | | |
| Software | | Agilent Open Lab CDS 2.6 | | |
| | Column | Agilent ZORBAX, SB-Aq, 2.1*50mm, 5µm | | |
| | Mobile Phase | A: Water | | |
| | | B: Acetonitrile | | |
| | Gradient | Time(min) | B(%) | Flow(mL/min) |
| | | 0.00 | 25 | 0.8 |
| | | 0.40 | 25 | 0.8 |
| HPLC | | 3.40 | 100 | 0.8 |
| | | 3.90 | 100 | 0.8 |
| | | 3.91 | 25 | 0.8 |
| | | 4.10 | 25 | 1.0 |
| | | 4.50 | 25 | 1.0 |
| | Post time(min) | Off | | |
| | Column Temp | 45°C | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) Positive | | |
| | MS Polarity | Positive | | |
| | MS Mode | Scan | | |
| | Mass Range | 100-2000 | | |

### LCMS Method N:

| | | | | |
|---|---|---|---|---|
| Method name | | WUXICD25 | | |
| Instrument | | Agilent 1290 LC & Agilent 6125C MSD | | |
| Software | | Agilent Open Lab CDS 2.6 | | |
| | Column | Waters Xbridge C18, 4.6*50mm,3.5um | | |
| | Mobile Phase | A: 0.05% NH₃·H₂O in water (v/v) | | |
| | | B: Acetonitrile | | |
| | Gradient | Time(min) | B(%) | Flow(mL/min) |
| | | 0.00 | 25 | 0.8 |
| | | 0.40 | 25 | 0.8 |
| HPLC | | 3.40 | 100 | 0.8 |
| | | 3.90 | 100 | 0.8 |
| | | 3.91 | 25 | 0.8 |
| | | 4.10 | 25 | 1 |
| | | 4.50 | 25 | 1 |
| | Post time(min) | Off | | |
| | Column Temp | 45°C | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method O:

| | | | | |
|---|---|---|---|---|
| Method name | | 5-95(3-4.5)AB_1_40_Xbridge C18_5cm | | |
| Instrument | | Agilent 1290 LC & Agilent 6125C MSD | | |
| Software | | Agilent Open Lab CDS 2.6 | | |
| HPLC | Column | Waters Xbridge C18, 4.6*50mm,3.5um | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| | | 0.00 | 5 | 1 |
| | | 3.00 | 95 | 1 |
| | | 4.50 | 95 | 1 |
| | Post time(min) | 1.00 | | |
| | Column Temp | 40.0 | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method P:

| | | | | |
|---|---|---|---|---|
| Method name | | 5-95(4.5)CD_1_40_Xbridge C18_5cm | | |
| Instrument | | Agilent 1290 LC & Agilent 6125C MSD | | |
| Software | | Agilent Open Lab CDS 2.6 | | |
| | Column | Waters Xbridge C18, 4.6*50mm,3.5um | | |
| | Mobile Phase | A: 0.05%NH3•H2O In Water(V/V) | | |
| | | B: Acetonitrile (v/v) | | |
| | Gradient | Time(min) | B(%) | Flow(mL/min) |
| HPLC | | 0.00 | 5 | 1 |
| | | 4.50 | 95 | 1 |
| | Post time(min) | 1.50 | | |
| | Column Temp | 40.0 | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method Q:

| | | | | |
|---|---|---|---|---|
| Method name | | 5-95(4.5)AB_1_40_Xbridge C18_5cm | | |
| Instrument | | Agilent 1290 LC & Agilent 6125C MSD | | |
| Software | | Agilent Open Lab CDS 2.6 | | |
| | Column | Waters Xbridge C18, 4.6*50mm,3.5um | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| HPLC | | 0.00 | 5 | 1 |
| | | 4.50 | 95 | 1 |
| | Post time(min) | 1.00 | | |
| | Column Temp | 40.0 | | |
| | Detector | DAD&ELSD | | |
| MS | Ionization source | ESI | | |
| | Drying Gas | N₂ | | |
| | Drying Gas Flow | 10(L/min) | | |
| | Nebulizer Pressure | 40(psi) | | |
| | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method R:

| | | | | |
|---|---|---|---|---|
| Method name | | 5-95(3-4.5)CD_1_40_Xbridge C18_5cm | | |
| Instrument | | Agilent 1290 LC & Agilent 6125C MSD | | |
| Software | | Agilent Open Lab CDS 2.6 | | |
| | Column | Waters Xbridge C18, 4.6*50mm,3.5um | | |
| | Mobile Phase | A: 0.05% NH3•H2O in water | | |
| | | B: Acetonitrile (v/v) | | |
| | Gradient | Time(min) | B(%) | Flow(mL/min) |
| HPLC | | 0.00 | 5 | 1 |
| | | 3.00 | 95 | 1 |
| | | 4.50 | 95 | 1 |
| | Post time(min) | 1.00 | | |
| | Column Temp | 40.0 | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method S:

| | | | | |
|---|---|---|---|---|
| Method name | | 30-95(3-4.5)AB_1_40_Xbridge C18_5cm | | |
| Instrument | | Agilent 1290 LC & Agilent 6125C MSD | | |
| Software | | Agilent Open Lab CDS 2.6 | | |
| | Column | Waters Xbridge C18, 4.6*50mm,3.5um | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| HPLC | | 0.00 | 30 | 1 |
| | | 3.00 | 95 | 1 |
| | | 4.50 | 95 | 1 |
| | Post time(min) | 1.00 | | |
| | Column Temp | 40.0 | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method T:

| | | | | |
|---|---|---|---|---|
| Method name | | WUXIN10 | | |
| Instrument | | Agilent 1290 LC & Agilent 6125C MSD | | |
| Software | | Agilent Open Lab CDS 2.6 | | |
| HPLC | Column | Agilent ZORBAX, SB-Aq, 2.1*50mm, 5µm | | |
| | Mobile Phase | A: water (v/v) | | |
| | | B: Acetonitrile | | |
| | Gradient | Time(min) | B(%) | Flow(mL/min) |
| | | 0.00 | 10 | 0.8 |
| | | 0.40 | 10 | 0.8 |
| | | 3.40 | 100 | 0.8 |
| | | 3.90 | 100 | 0.8 |
| | | 3.91 | 10 | 0.8 |
| | | 4.10 | 10 | 1.0 |
| | | 4.50 | 0 | 1.0 |
| | Post time(min) | Off | | |
| | Column Temp | 45°C | | |
| | Detector | DAD&ELSD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |

### LCMS Method U:

| | | | | |
|---|---|---|---|---|
| Method name | | 10-100(4-6)AB_1_40_GEMINI_C18-5cm | | |
| Instrument | | Agilent 1290 LC & Agilent 6125C MSD | | |
| Software | | Agilent Open Lab CDS 2.6 | | |
| HPLC | Column | Phenomenex Gemini C18, 4.6*50mm,3.5µm | | |
| | Mobile Phase | A: 0.04% TFA in water (v/v) | | |
| | | B: 0.02% TFA in Acetonitrile (v/v) | | |
| | Gradient | Time(min) | B(%) | Flow(mL/min) |
| | | 0.00 | 10 | 1 |
| | | 4.00 | 100 | 1 |
| | | 6.00 | 100 | 1 |
| | Post time(min) | 1.5min | | |
| | Column Temp | 40°C | | |
| | Detector | DAD | | |
| | Ionization source | ESI | | |
| | Drying Gas | N₂ | | |
| | Drying Gas Flow | 10(L/min) | | |
| | Nebulizer Pressure | 40(psi) | | |
| MS | Drying Gas Temperature | 350 °C | | |
| | Capillary Voltage | 3500(V) | | |
| | MS Polarity | Positive | | |
| | MS Mode | Scan | | |
| | Mass Range | 100-2000 | | |
| ELSD | Evaporator Temperature | 40°C | | |
| | Nebulizer Temperature | 40°C | | |
| | Gas Flow | 1.60 | | |
| | PMT Gain | 1.0 | | |

### BIOLOGICAL EXAMPLES

### Biological Example A - MutSβ Assay

| Title | **MutSβ ADP Glo Assay Protocol** |
|---|---|
| Plate Type | 384 well plate, non-binding, white, low volume (Greiner: 784904) |

### Reagents

MutSβ (supplied by AMRI) stored at -70°C
ADP Glo Kinase Assay Kit (Promega: V9102) stored at -20°C
ATP (supplied within the ADP Glo Kit) stored at -20°C
1M KCI (Sigma Aldrich: P9333) stored at 2-8°C
1 M HEPES pH 7.5 (Gibco: M15630-080) stored at 2-8°C
1M MgCl₂ (Sigma Aldrich: M1028-100mL) stored at 2-8°C
Tween-20 (Sigma Aldrich: P1379) (10% solution) stored at 2-8°C
500mM DTT (Sigma Aldrich: 10197777001) stored at -20°C
CAG loop DNA for MutSβ (made in house) stored at -20°C
MilliQ water stored at room temperature

### Buffer preparation (final conditions):

| **Component** | **Final Concentration** |
|---|---|
| Test compound | 10mM |
| DMSO | 1% |
| HEPES pH 7.5 | 25 mM |
| KCI | 150 mM |
| Tween-20 | 0.01% (v/v) |
| MgCl₂ | 5mM |
| DTT | 1 mM |
| Milli Q water | 81.7% of total volume |

### Reaction Mix for MutSβ

| **Component** | **Stock Concentration** | **Working Concentration** | **Final Concentration** |
|---|---|---|---|
| MutSβ | 11.2µM | 33.34nM | 20nM |
| CAG DNA | 50µM | 50.01nM | 30nM |
| ATP | 10mM | 62.5 µM | 25 µM |

### Standard Operating Procedure for MutSβ assay

**1.** Prepare the buffer fresh each day
**2.** Take the enzyme from the freezer, thaw in room temperature water and centrifuge at 13,000 rpm for 10 minutes at 2-8C, then make up enzyme and ATP stocks
**3.** Quality Control the multidrop head to a %CV of <2%
**4.** Add 3µL of 33.34nM MutSβ and 50.01nM CAG looped DNA solution (final concentrations) to each well in columns 1 and 3-23 of the plate
**5.** Pre-incubate the MutSβ and buffer mix for 10 minutes with the compounds
**6.** Wash the multidrop head through with MilliQ water (for at least 30 seconds)
**7.** Add 3µL of MutSβ Assay buffer (without enzyme) to columns 2 and 24_for the vehicle controls
**8.** Wash the multidrop head through with MilliQ water (for at least 30 seconds)
**9.** Add 2µL of 62.5µM ATP to each well of the whole plate, then wash with MilliQ water (for a minimum of 30 seconds)
**10.** Centrifuge plate for 1 minute at 1000 rpm
**11.** Incubate for 2 hours at room temperature
**12.** Add 5µL of ADP Glo reagent to each well of the whole plate and wash the multidrop head with MilliQ water (for a minimum of 30 seconds)
**13.** Incubate for 40 minutes at room temperature (minimum requirement)
**14.** Add 10µL of Kinase Detection Reagent to each well of the whole plate
**15.** Wash the multidrop head with MilliQ water more than 1 minute and then dry
**16.** Remove the head and put the cover back on for overnight storage
**17.** Incubate for 30 minutes at room temperature (minimum requirement)
**18.** Read on a microplate reader

### CAG Looped DNA sequence

5' TCATCGATCGCAGGCTTCAGATAGG 3' SEQ ID 1
3' GGTAGCTAGC CGAAGTCTATCTCC 5' SEQ ID 2

### Results

| **Example** | **MutSβ IC₅₀ (ADP-Glo, µM)** |
|---|---|
| 1 | ** |
| 2 | ** |
| 3 | **** |
| 4 | ** |
| 5 | ** |
| 6 | ** |
| 7 | ** |
| 8 | ** |
| 9 | * |
| 10 | * |
| 11 | * |
| 12 | * |
| 13 | * |
| 14 | * |
| 15 | ** |
| 16 | ** |
| 17 | ** |
| 18 | ** |
| 19 | ** |
| 20 | ** |
| 21 | ** |
| 22 | * |
| 23 | * |
| 24 | ** |
| 25 | ** |
| 26 | ** |
| 27 | ** |
| 28 | ** |
| 29 | *** |
| 30 | *** |
| 31 | *** |
| 32 | *** |
| 33 | *** |
| 34 | *** |
| 35 | *** |
| 36 | *** |
| 37 | **** |
| 38 | **** |
| 39 | **** |
| 40 | **** |
| 41 | **** |
| 42 | **** |
| 43 | **** |
| 44 | **** |
| 45 | **** |
| 46 | *** |
| 47 | *** |
| 48 | **** |
| 49 | * |
| 50 | * |
| 51 | * |
| 52 | ** |
| 53 | ** |
| 54 | ** |
| 55 | ** |
| 56 | ** |
| 57 | *** |
| 58 | *** |
| 59 | *** |
| 60 | *** |
| 61 | *** |
| 62 | **** |
| 63 | **** |
| 64 | **** |
| 65 | **** |
| 66 | **** |
| 67 | **** |
| 68 | **** |
| 69 | **** |
| 70 | **** |
| 71 | **** |
| 72 | **** |
| 73 | **** |
| 74 | **** |
| 75 | *** |
| 76 | ** |
| 77 | ** |
| 78 | ** |
| 79 | * |
| 80 | ** |
| 81 | ** |
| 82 | ** |
| 83 | ** |
| 84 | ** |
| 85 | ** |
| 86 | ** |
| 87 | *** |
| 88 | *** |
| 89 | *** |
| 90 | *** |
| 91 | *** |
| 92 | *** |
| 93 | *** |
| 94 | *** |
| 95 | *** |
| 96 | *** |
| 97 | *** |
| 98 | *** |
| 99 | **** |
| 100 | **** |
| 101 | **** |
| 102 | **** |
| 103 | **** |
| 104 | **** |
| 105 | **** |
| 106 | **** |
| 107 | **** |
| 108 | **** |
| 109 | **** |
| 110 | **** |
| 111 | **** |
| 112 | **** |
| 113 | **** |
| 114 | **** |
| 115 | **** |
| 116 | **** |
| 117 | **** |
| 118 | **** |
| 119 | **** |
| 120 | **** |
| 121 | **** |
| 122 | **** |
| 123 | **** |
| 124 | **** |
| 125 | **** |
| 126 | **** |
| 127 | **** |
| 128 | **** |
| 129 | **** |
| 130 | **** |
| 131 | **** |
| 132 | **** |
| 133 | **** |
| 134 | **** |
| 135 | **** |
| 136 | **** |
| 137 | **** |
| 138 | **** |
| 139 | **** |
| 140 | * |
| 141 | ** |
| 142 | *** |
| 143 | ** |
| 144 | *** |
| 145 | *** |
| 146 | **** |
| 147 | **** |
| 148 | **** |
| 149 | **** |
| 150 | **** |
| 151 | **** |
| 152 | **** |
| 153 | **** |
| 154 | **** |
| 155 | **** |
| 156 | **** |
| 157 | **** |
| 158 | **** |
| 159 | ** |
| 160 | ** |
| 161 | ** |
| 162 | ** |
| 163 | ** |
| 164 | ** |
| 165 | **** |
| 166 | ** |
| 167 | * |
| 168 | *** |
| 169 | **** |
| 170 | ** |
| 171 | ** |
| 172 | **** |
| 173 | ** |
| 174 | *** |
| 175 | * |
| 176 | * |
| 177 | *** |
| 178 | * |
| 179 | **** |
| 180 | **** |
| 181 | **** |
| 182 | **** |

### Key:

| | |
|---|---|
| **** | Denotes an IC₅₀ of 0.05 µM or less |
| *** | Denotes an IC₅₀ of 0.05 µM to 0.1 µM |
| ** | Denotes an IC₅₀ of 0.1 µM to 1 µM |
| * | Denotes an IC₅₀ of greater than 1 µM |

### Biological Example B - LoQ Assay Data

### Materials and Methods

The cell line that was used in the assay below was generated as follows:

### Construct generation

The pcDNA^{™}4/TO vector contains two tetracycline operator 2 (TetO2) sites within the human cytomegalovirus immediate-early (CMV) promoter for tetracycline regulated expression of your gene of interest (Yao et al., 1998). The TetO2 sequences serve as binding sites for four Tet repressor molecules (comprising two Tet repressor homodimers) and confer tetracycline-responsiveness to the gene of interest. In the absence of tetracycline, expression of your gene of interest is repressed by the binding of Tet repressor homodimers to the TetO2 sequences. Addition of tetracycline to the cells de-represses the hybrid CMV/TetO2 promoter in pcDNA^{™}4/TO and allows expression of your gene of interest. The pcDNA^{™}6/TR regulatory plasmid is designed for use with the T-REx^{™} System for tetracycline-regulated expression of the gene of interest in mammalian cells. (www.lifetechnologies.com).

The expression sequence comprises exon 1 huntingtin with ~100 pure CAG repeats, a P2A self-cleaving peptide and a nanoluc sequence followed by a polyA signal. Full plasmid sequence is indicated (Seq ID NO:3). The plasmid contains sequences for ampicillin (bacterial propagation) and zeocin (mammalian cell lines) resistance.

### Cell culture

The U-2 OS cell line (purchased from LGC standards, ATCC) is derived from a moderately differentiated sarcoma from the tibia of an adolescent female. Karyotyping has shown that although there are a lot of chromosomal abnormalities the tumour suppressor genes p53 and pRb are still fully functional in the U2OS cell line.

Routine culture of the adherent cells in non-PDL plastic ware in a humidified 37^{O}C incubator with 5% CO2. Cells were maintained in McCoy's 5A (modified) medium with GlutaMAX supplement (Life Technologies 3660-021) and tetracycline-free FBS (Clontech 631106). Maintenance medium for the clonal cell lines expressing Tet repressor and exon 1 HTT/nanoluc constructs were additionally supplemented with zeocin to 100 ug/ml and blasticidin to 1.25 ug/ml. Cells were passaged at 70-80% confluence once or twice a week, either to a split ratio or defined seeding density using DPBS and TrypLE express enzyme (1x, no phenol red, Life technologies 12604-039) was used to dissociate the cells. Cells were frozen with Cell Banker (AMSBIO 11891) or with medium plus 10% DMSO and stored in liquid nitrogen vapour phase.

### Compound treatment protocol

Stable, clonal cell lines (e.g. 1C6) are leveraged either identify potential druggable targets, and to assess the effectiveness of therapeutic compounds against a Huntington's disease, by way of quantifying CAG repeats expansion rates.

### Materials

### Cell culture

| | |
|---|---|
| • Trypan Blue | Gibco^{™} (Cat# 15250061) |
| • Nexcelom counting slides | Nexcelom (Cat# SD100) |
| • Penicillin-Streptomycin (P/S) (10,000 U/mL) | Gibco^{™} (Cat# 15140122) |
| • McCoys 5A (Modified) medium | Gibco^{™} (Cat# 16600082) |
| • Tet-System Approved FBS | Gibco^{™} (Cat#A47362-01) |
| • Zeocin^{®} 100 mg/mL | Invivogen (Cat# ant-zn-05) |
| • Blasticidin 10 mg/mL | Invivogen (Cat# ant-bl-05) |
| • Puromycin 10 mg/mL | Invivogen (Cat# ant-pr-1) |
| • 0.25% Trypsin EDTA | Gibco^{™} (Cat# 15050065) |
| • PBS -MgCl₂ -CaCl₂ | Gibco^{™} (Cat# 10010023) |
| • Freezing Medium Cryo-SFM | Sigma Aldrich (Cat# C-29912) |

### Compound Treatment

| | |
|---|---|
| • Tetracycline | Gibco^{™} (Cat# A39246) |
| • Test compounds | |
| • DMSO | Fisher (Cat# BP231-100) |

### 2.3 DNA extraction

| | |
|---|---|
| • Quick-DNA^{™} 96 Kit^{™} 10 x 96 preparations | Zymo (Cat# D3012) |
| • Quick-DNA Microprep Plus Kit | Zymo (Cat# D4074) |
| • B-mercaptoethanol | MP (Cat# 190242) |

### 2.4 Consumables

| |
|---|
| • Centrifuge tubes |
| • 50 mL falcon tubes |
| • 96-well flat bottom microplates (sterile) |
| • 96-well white opaque microplates |
| • T75 culture flasks |
| • Sterile 100 mL single use reservoirs |
| • Cryovials |
| • Foil Plate seals |

### 2.5 Instrumentation

| | |
|---|---|
| • T4 Automated cell counter | Nexcelom |
| • GloMAX Explorer | Promega^{®} |

### Method

All work should be conducted under aseptic sterile technique, in a laminar flow hood.

### Cell Thawing, Passaging, and Freezing

### 3.1.1 Thawing:

- Place a vial of the cell line U20S-IC6 (1-2x10⁶ cells/mL), produced as described above, in a water bath at 37°C for 30 s. Once thawed, quickly transfer the cells into a 15 mL falcon tube containing 9 mL of McCoys 5A media, supplemented with 10% Tet-free FBS.
- Pellet the cells by centrifugation at 300 x g for 5 m and discard the supernatant.
- Resuspend the cell pellet in 10 mL fresh media and transfer to a sterile T75 culture flask.
- Inspect the culture after 24 h and maintain the cells below 80% confluency, typically passaging every 3-4 days.

### 3.1.2 Passaging:

- To passage cells, aspirate the media and wash once with 2 mL PBS.
- Aspirate the PBS and add 2 mL 0.25% Trypsin EDTA for 2 m at 37°C or until the cells detach.
- Resuspend the detached cells with 8 mL McCoys 5A, 10% Tet-Free FBS (total volume 10 mL), and transfer to a 50 mL Falcon tube.
- Centrifuge the cell suspension at 300 x g for 5 m to pellet the cells.
- Aspirate the supernatant and resuspend the cell pellet in 10 mL McCoys 5A, 10% Tet-Free FBS.
- Perform a ratio split (typical 2:10) by transferring 2 mL of the cell suspension to a fresh T75 flask and add 8 mL McCoys 5A, 10% Tet-Free FBS.
- Return the passaged culture to the incubator at 37°C, 5% CO₂.

### 3.1.3 Freezing:

- Aspirate the media from a T75 flask of U20S-IC6 and wash once with 2 mL PBS.
- Aspirate the PBS and add 2 mL 0.25% Trypsin EDTA for 2 m at 37°C or until the cells detach.
- Resuspend the detached cells with 8 mL McCoys 5A, 10% Tet-Free FBS (total volume 10 mL), and transfer to a 50 mL Falcon tube.
- Take a 60 µL sample of the cell suspension into a fresh 1.5 mL Eppendorf tube and add 20 µL Trypan Blue. Mix and transfer 20 µL of the solution to a clean cell counting slide to determine the cell count per mL and the overall viability.
- Centrifuge the cell suspension at 300 x g for 5 m to pellet the cells.
- Aspirate the supernatant and resuspend the cell pellet in sufficient CryoSFM media to achieve a cell count of 1-2 x 10⁶ cells/mL. Alternatively, complete media supplemented with 10% DMSO can be used for cryopreservation.
- Aliquot the cell suspension in 1 mL volumes into labelled cryovials and transfer to a -80°C freezer in a Mr Frosty container.

### Lo-Q platform Assay for Candidate Compounds

The Lo-Q platform has been optimised to run as a 2-week assay, following the standard processes detailed in the schematic in Figure 1A.

### Assay initiation - Cell seeding:

- Cell seeding for Lo-Q platform assays should be initiated on Mondays (am) to ensure Tetracycline pulsing can be maintained every other day.
- The Friday prior to initiating any Lo-Q platform assay ensure there are sufficient cells in culture to seed the required number of assay plates. If required, passage the cells, otherwise perform a media change to McCoys 5A, 10% Tet-free FBS, 1.25 µg/mL Blasticidin^{®}, and 100 µg/mL Zeocin^{®}.
- On the Monday morning, aspirate the media from the required number of culture flasks containing U20S-IC6 cells and wash with 2 mL PBS.
- Aspirate the PBS and add 2 mL 0.25% Trypsin EDTA. Incubate the plate at 37°C for ~2 m, or until >90% of the cells have detached.
- Resuspend the detached cells with 8 mL McCoys 5A, 10% Tet-Free FBS (total volume 10 mL), and transfer to a 50 mL Falcon tube.
- Take a 60 µL sample of the cell suspension into a fresh 1.5 mL Eppendorf tube and add 20 µL Trypan Blue. Mix and transfer 20 µL of the solution to a clean cell counting slide. Determine the cell count per mL and the overall viability.
- Dilute a sufficient volume of the cell suspension in McCoys 5A, 10% Tet-Free FBS, to achieve a cell density of 5,000 cells/well at a volume of 200 µL per well of a 96-well plate (25,000 cells/mL).
- Using sterile clear flat bottom 96-well plates, fill the top row (A1-12) with 200 µL PBS. These wells must be kept empty for subsequent downstream analysis processing purposes.
- Next, transfer the diluted cell suspension to a sterile reservoir and using a 1250 µL multichannel integra, pipette 200 µL per well of the clear flat bottom 96-well plates (rows B-H).
- Place the seeded 96-well plates in an incubator at 37°C, 5% CO2, for ~24 h.
- At least 3 'day 0' samples should be taken at this point. Using the leftover cells from the trypsinised T75 flasks, prepare 3 x 1.5 mL Eppendorf tubes containing a minimum of 3 x 10⁵ cells.
- Centrifuge the tubes to pellet the cells and remove the supernatant.
- Store the cell pellets at -80°C until ready to extract the DNA using a Quick-DNA Microprep Plus Kit from Zymo, according to the manufacturer's instructions.

### Initial Compound Pre-treatment:

The test compounds are added to the cells approximately 24 h prior to the first Tetracycline pulse. This ensures that potential effects of slower acting compounds are not obscured by the quick de-repression of the CAG expansion by the additional of the Tetracycline. For each instance of Tetracycline pulsing, the test compounds are refreshed.
- Using a fresh sterile 96-well plate, prepare an intermediate compound dilution of each of the 6 DMSO stocks created in section 3.2.2.
- Create the intermediate stocks by diluting 2 µL of each DMSO stock in 18 µL McCoys 5A, 10% Tet-Free FBS. See Figure 1B.
- Next, using the repeat dispense function of a 12.5 µL integra digital pipette, dispense 2 µL of each intermediate compound dilution or DMSO control into triplicate wells of the 96-well plates containing the U20S-IC6 cells seeded 24 h prior. See example plate layout in Figure 1C.
- Return the treated 96-well plates to the incubator for ~24 h at 37°C, 5% CO2.

### Initial Tetracycline Pulse:

- Approximately 24 h post initial compound pre-treatment, prepare the intermediate compound dilution plate again, in the same manner as for 3.2.3, and set the plate aside.
- In the meantime, perform a media change on the 96-well plates pre-treated with test compound, replacing all media, except for the 'no Tet' control, with 198 µL McCoys 5A, 10% Tet-Free FBS, supplemented with 2 x Tetracycline (2 µg/mL). For the 'no Tet' control wells, replace the media with 198 µL McCoys 5A, 10% Tet-Free FBS.
- Immediately after media change, re-add the intermediate test compound dilutions as previously described in 3.2.3 and return the plate to the incubator for ~48 h.

### Tetracycline Pulse & stable clone maintenance:

To ensure that the U20S-IC6 clonal cell line retains expression of the 2 stably transfected Lo-Q platform plasmid constructs, the cells must be exposed to the relevant section antibiotics for a 72 h period every 7 days of the assay.
- Approximately 48 h (Friday) after the initial Tetracycline pulse, perform another media change in the same manner as 3.2.4, but ensure all media is additionally supplemented with 1.25 µg/mL Blasticidin^{®}, and 100 µg/mL Zeocin^{®}.
- After the media change, prepare and add the intermediate compound dilutions as before and return the plate to the incubator for ~72 h.

### Standard Lo-Q Platform Assay Process:

- After the first week of the assay, the standard process should be followed is outlined in Figure 1D. Passaging Lo-Q Platform plates:
- Cells should be passaged every Monday at a 1/8 dilution unless visual observation by light microscope reveals impeded proliferation, or cytotoxicity.
- Prior to performing any cell passaging, prepare the intermediate compound dilution plate as per 3.2.4. Set the plate aside until required after passaging is complete.
- Next, using a multi-channel 300 µL integra pipette, aspirate the media from each triplicate, using a custom multi aspirate and dispense function. Do not use the same pipette tips for different treatment condition triplicates.
- Wash the cells once with 200 µL PBS.
- Detach the cells by adding 25 µL of 0.25% Trypsin EDTA to each well and incubating for 2-3 m at 37°C. Ensure the >95% of the cells have detached before quenching with media. If necessary, tap the 4 sides of the plate gentle to detach persistent cells.
- During trypsinisation prepare fresh 96-well plates for seeding. Add 175 µL of appropriate media to each well. Adjust this volume if the passage ratio has been altered due to the confluency of the condition in question. The 'No Tet' control and all other conditions should receive McCoys 5A, 10% Tet-Free FBS and McCoys 5A, 10% Tet-Free FBS, 2 x Tetracycline (2 µg/mL) respectively. Ensure the top row is filled with 200 µL PBS.
- Quench the trypsinisation by adding 175 µL of appropriate media to the wells.
- To perform the 1/8 ratio passage, using a multi-channel P300 pipette, gently mix the quenched cells and transfer 25 µL to the fresh 96-well plates prepared earlier.
- Immediately add the intermediate dilutions of the test compounds to the newly passage plates as per 3.2.4 and return the plates to the incubator for 48 h at 37°C, 5% CO₂.

### NanoGlo^{®}:

- In the first instance of passaging the cells, a NanoGlo^{®} assay can be performed with the leftover unused cells to determine successful Tetracycline activation and infer compound cytotoxicity.
- Centrifuge the 96-well plates containing the unused cells that were quenched after trysinisation at 300 x g for 5 m.
- Aspirate the media and add 50 µL fresh McCoys 5A, 10% Tet-free FBS.
- To each well, add 50 µL NanoGlo^{®} reagent and incubate in the dark for up to 10 m shaking.
- Transfer the contents of each well to a solid white opaque 96-well plate and measure luminescence on a compatible plate reader (GloMax Explorer).

### Cell processing for DNA extraction:

At the end of the assay period, the cells should be retained for DNA extraction.
- Centrifuge the 96-well plates of cells at 300 x g for 5 m.
- Aspirate the media and wash the cells once with PBS.
- Seal the plate with a foil plate seal and store at -80°C until ready to extract the DNA using the Quick-DNA^{™} 96 Kit^{™} from Zymo, according to the manufacturer's instructions.

### Candidate Compound treatment plan variation:

In some instances, the assay described above can be modified to achieve specific compound exposure times during treatment regimes. Compound pulsing for 2, 4, 8, and 24 h can be implemented under the existing assay format, with the following adjustments:
- Each morning, test compounds are added in the presence of Tetracycline containing media for the desired time.
- After which the media is removed, the wells washed 1 x with PBS, and 200 µL fresh McCoys 5A, 10% Tet-free FBS + Tetracycline is added.
- The cells remain in this media until the following morning, when the wells are aspirated, and 200 µL fresh McCoys 5A, 10% Tet-free FBS + Tetracycline is added along with the test compounds.
- For the 24 h compound pulse, cells will remain in constant contact with test compound and Tetracycline throughout the 2-week assay, with replenishment every morning.
- Test compound pulse assays require treatments every day for the 2-week period, including the weekend. Therefore, during Friday, Saturday, and Sunday treatments, all media should be additionally supplemented with 1.25 µg/mL Blasticidin^{®}, and 100 µg/mL Zeocin^{®}.

### Results

| **Example** | **IC₅₀ (µM)** |
|---|---|
| 6 | **** |
| 8 | *** |
| 148 | **** |
| 169 | *** |
| 174 | *** |
| 180 | *** |
| 181 | **** |

### Key:

| | |
|---|---|
| **** | Denotes an IC₅₀ of 0.1 µM or less |
| *** | Denotes an IC₅₀ of 0.1 µM to 0.5 µM |
| ** | Denotes an IC₅₀ of 0.5 µM to 1 µM |
| * | Denotes an IC₅₀ of 1 µM to 5 µM |

SEQUENCES

SEQ ID NO:1

5' TCATCGATCGCAGGCTTCAGATAGG 3'

SEQ ID NO: 2

3' GGTAGCTAGC CGAAGTCTATCTCC 5'

SEQ ID NO: 3

pcDNA4/TO-100CAG (7160bp) Vector

Key: CMV promoter (bold), TetO operator (underline), Exon 1 HTT with 100 CAG repeats (italics), P2A self-cleaving peptide (bold and underline), Nanoluc reporter protein (italics and underline), HSV TK PolyA (bold and italics)

5'
GATGTACGGGCCAGATATACGCGTTGACATTGATTATTGACTAGTTATTAATAGTAAT
CAATTACGGGGTCATTAGTTCATAGCCCATATATGGAGTTCCGCGTTACATAACTTA
CGGTAAATGGCCCGCCTGGCTGACCGCCCAACGACCCCCGCCCATTGACGTCAAT
AATGACGTATGTTCCCATAGTAACGCCAATAGGGACTTTCCATTGACGTCAATGGG
TGGAGTATTTACGGTAAACTGCCCACTTGGCAGTACATCAAGTGTATCATATGCCA
AGTACGCCCCCTATTGACGTCAATGACGGTAAATGGCCCGCCTGGCATTATGCCC
AGTACATGACCTTATGGGACTTTCCTACTTGGCAGTACATCTACGTATTAGTCATCG
CTATTACCATGGTGATGCGGTTTTGGCAGTACATCAATGGGCGTGGATAGCGGTTT
GACTCACGGGGATTTCCAAGTCTCCACCCCATTGACGTCAATGGGAGTTTGTTTTG
GAACCAAAATCAACGGGACTTTCCAAAATGTCGTAACAACTCCGCCCCATTGACG
CAAATGGGCGGTAGGCGTGTACGGTGGGAGGTCTATATAAGCAGAGCTCTCCCTA
TCAGTGATAGAGATCTCCCTATCAGTGATAGAGATCGTCGACGAGCTCGTTTAGTGA
ACCGTCAGATCGCCTGGAGACGCCATCCACGCTGTTTTGACCTCCATAGAAGACAC
CGGGACCGATCCAGCCTCCGGACTCTAGCGTTTAAACTTAAGCTTACCATGGCGAC
CCTGGAAAAGCTGATGAAGGCCTTCGAGTCCCTCAAGTCCTTCCAGCAGCAGCAGC
AGCAGCAGCAGCAGCAGCAGCAGCAGCAGCAGCAGCAGCAGCAGCAGCAGCAGCA
GCAGCAGCAGCAGCAGCAGCAGCAGCAGCAGCAGCAGCAGCAGCAGCAGCAGCA

GCAGCAGCAGCAGCAGCAGCAGCAGCAGCAGCAGCAGCAGCAGCAGCA
GCAGCAGCAGCAGCAGCAGCAGCAGCAGCAGCAGCAGCAGCAGCAGCA
GCAGCAGCAGCAGCAGCAGCAGCAGCAGCAGCAGCAGCAGCAGCAGCA
GCAGCAGCAGCAGCAGCAACAGCCGCCACCGCCGCCGCCGCCGCCGCCTCC
TCAGCTTCCTCAGCCGCCGCCGCAGGCACAGCCGCTGCTGCCTCAGCCGCAGCCG
CCCCCGCCGCCGCCCCCGCCGCCACCCGGCCCGGCTGTGGCTGAGGAGCCGCTG
CACCGACCAGGTACCGGCAGCGGCGCCACAAACTTCTCTCTGCTAAAGCAAGCAG
GTGATGTTGAAGAAAACCCCGGGCCTGGATCCATGGTCTTCACACTCGAAGATTTC
GTTGGGGACTGGCGACAGACAGCCGGCTACAACCTGGACCAAGTCCTTGAACAGG
GAGGTGTGTCCAGTTTGTTTCAGAATCTCGGGGTGTCCGTAACTCCGATCCAAAGGA
TTGTCCTGAGCGGTGAAAATGGGCTGAAGATCGACATCCATGTCATCATCCCGTATG
AAGGTCTGAGCGGCGACCAAATGGGCCAGATCGAAAAAATTTTTAAGGTGGTGTAC
CCTGTGGATGATCATCACTTTAAGGTGATCCTGCACTATGGCACACTGGTAATCGAC
GGGGTTACGCCGAACATGATCGACTATTTCGGACGGCCGTATGAAGGCATCGCCGT
GTTCGACGGCAAAAAGATCACTGTAACAGGGACCCTGTGGAACGGCAACAAAATTAT
CGACGAGCGCCTGATCAACCCCGACGGCTCCCTGCTGTTCCGAGTAACCATCAACG
GAGTGACCGGCTGGCGGCTGTGCGAACGCATTCTGGCGTAAGAATTCCGGCAATAA
AAAGACAGAATAAAACGCACGGTGTTGGGTCGTTTGTTCCGTTACATAACTTACGGT
AAATGGCCCGCCTGGCTGACCGCCCAACGACCCCCGCCCATTGACGTCAATAATGA
CGTATGTTCCCATAGTAACGCCAATAGGGACTTTCCATTGACGTCAATGGGTGGAGT
ATTTACGGTAAACTGCCCACTTGGCAGTACATCAAGTGTATCATATGCCAAGTACGC
CCCCTATTGACGTCAATGACGGTAAATGGCCCGCCTGGCATTATGCCCAGTACATGA
CCTTATGGGACTTTCCTACTTGGCAGTACATCTACGTATTAGTCATCGCTATTACCAT
GGTGATGCGGTTTTGGCAGTACATCAATGGGCGTGGATAGCGGTTTGACTCACGGG
GATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGGTA
GGCGTGTACGGTGGGAGGTCTATATAAGCAGAGCTATCGATGCCACCATGGTGAGC
AAGGGCGAGGAGCTGTTCACCGGGGTGGTGCCCATCCTGGTCGAGCTGGACGGCG
ACGTAAACGGCCACAAGTTCAGCGTGTCCGGCGAGGGCGAGGGCGATGCCACCTA
CGGCAAGCTGACCCTGAAGTTCATCTGCACCACCGGCAAGCTGCCCGTGCCCTGGC
CCACCCTCGTGACCACCCTGACCTACGGCGTGCAGTGCTTCAGCCGCTACCCCGAC
CACATGAAGCAGCACGACTTCTTCAAGTCCGCCATGCCCGAAGGCTACGTCCAGGA
GCGCACCATCTTCTTCAAGGACGACGGCAACTACAAGACCCGCGCCGAGGTGAAGT
TCGAGGGCGACACCCTGGTGAACCGCATCGAGCTGAAGGGCATCGACTTCAAGGA
GGACGGCAACATCCTGGGGCACAAGCTGGAGTACAACTACAACAGCCACAACGTCT
ATATCATGGCCGACAAGCAGAAGAACGGCATCAAGGTGAACTTCAAGATCCGCCAC
AACATCGAGGACGGCAGCGTGCAGCTCGCCGACCACTACCAGCAGAACACCCCCAT

CGGCGACGGCCCCGTGCTGCTGCCCGACAACCACTACCTGAGCACCCAGTCCGCC
CTGAGCAAAGACCCCAACGAGAAGCGCGATCACATGGTCCTGCTGGAGTTCGTGAC
CGCCGCCGGGATCACTCTCGGCATGGACGAGCTGTACAAGGCTCGAGTCTAGAGG
GCCCGTTTAAACCCGCTGATCAGCCTCGACTGTGCCTTCTAGTTGCCAGCCATCTGT
TGTTTGCCCCTCCCCCGTGCCTTCCTTGACCCTGGAAGGTGCCACTCCCACTGTCCT
TTCCTAATAAAATGAGGAAATTGCATCGCATTGTCTGAGTAGGTGTCATTCTATTCTG
GGGGGTGGGGTGGGGCAGGACAGCAAGGGGGAGGATTGGGAAGACAATAGCAGG
CATGCTGGGGATGCGGTGGGCTCTATGGCTTCTGAGGCGGAAAGAACCAGCTGGG
GCTCTAGGGGGTATCCCCACGCGCCCTGTAGCGGCGCATTAAGCGCGGCGGGTGT
GGTGGTTACGCGCAGCGTGACCGCTACACTTGCCAGCGCCCTAGCGCCCGCTCCTT
TCGCTTTCTTCCCTTCCTTTCTCGCCACGTTCGCCGGCTTTCCCCGTCAAGCTCTAA
ATCGGGGGCTCCCTTTAGGGTTCCGATTTAGTGCTTTACGGCACCTCGACCCCAAAA
AACTTGATTAGGGTGATGGTTCACGTAGTGGGCCATCGCCCTGATAGACGGTTTTTC
GCCCTTTGACGTTGGAGTCCACGTTCTTTAATAGTGGACTCTTGTTCCAAACTGGAA
CAACACTCAACCCTATCTCGGTCTATTCTTTTGATTTATAAGGGATTTTGCCGATTTC
GGCCTATTGGTTAAAAAATGAGCTGATTTAACAAAAATTTAACGCGAATTAATTCTGT
GGAATGTGTGTCAGTTAGGGTGTGGAAAGTCCCCAGGCTCCCCAGCAGGCAGAAGT
ATGCAAAGCATGCATCTCAATTAGTCAGCAACCAGGTGTGGAAAGTCCCCAGGCTCC
CCAGCAGGCAGAAGTATGCAAAGCATGCATCTCAATTAGTCAGCAACCATAGTCCCG
CCCCTAACTCCGCCCATCCCGCCCCTAACTCCGCCCAGTTCCGCCCATTCTCCGCC
CCATGGCTGACTAATTTTTTTTATTTATGCAGAGGCCGAGGCCGCCTCTGCCTCTGA
GCTATTCCAGAAGTAGTGAGGAGGCTTTTTTGGAGGCCTAGGCTTTTGCAAAAAGCT
CCCGGGAGCTTGTATATCCATTTTCGGATCTGATCAGCACGTGTTGACAATTAATCAT
CGGCATAGTATATCGGCATAGTATAATACGACAAGGTGAGGAACTAAACCATGGCCA
AGTTGACCAGTGCCGTTCCGGTGCTCACCGCGCGCGACGTCGCCGGAGCGGTCGA
GTTCTGGACCGACCGGCTCGGGTTCTCCCGGGACTTCGTGGAGGACGACTTCGCC
GGTGTGGTCCGGGACGACGTGACCCTGTTCATCAGCGCGGTCCAGGACCAGGTGG
TGCCGGACAACACCCTGGCCTGGGTGTGGGTGCGCGGCCTGGACGAGCTGTACGC
CGAGTGGTCGGAGGTCGTGTCCACGAACTTCCGGGACGCCTCCGGGCCGGCCATG
ACCGAGATCGGCGAGCAGCCGTGGGGGCGGGAGTTCGCCCTGCGCGACCCGGCC
GGCAACTGCGTGCACTTCGTGGCCGAGGAGCAGGACTGACACGTGCTACGAGATTT
CGATTCCACCGCCGCCTTCTATGAAAGGTTGGGCTTCGGAATCGTTTTCCGGGACG
CCGGCTGGATGATCCTCCAGCGCGGGGATCTCATGCTGGAGTTCTTCGCCCACCCC
AACTTGTTTATTGCAGCTTATAATGGTTACAAATAAAGCAATAGCATCACAAATTTCAC
AAATAAAGCATTTTTTTCACTGCATTCTAGTTGTGGTTTGTCCAAACTCATCAATGTAT
CTTATCATGTCTGTATACCGTCGACCTCTAGCTAGAGCTTGGCGTAATCATGGTCAT
AGCTGTTTCCTGTGTGAAATTGTTATCCGCTCACAATTCCACACAACATACGAGCCG

GAAGCATAAAGTGTAAAGCCTGGGGTGCCTAATGAGTGAGCTAACTCACATTAATTG
CGTTGCGCTCACTGCCCGCTTTCCAGTCGGGAAACCTGTCGTGCCAGCTGCATTAA
TGAATCGGCCAACGCGCGGGGAGAGGCGGTTTGCGTATTGGGCGCTCTTCCGCTT
CCTCGCTCACTGACTCGCTGCGCTCGGTCGTTCGGCTGCGGCGAGCGGTATCAGCT
CACTCAAAGGCGGTAATACGGTTATCCACAGAATCAGGGGATAACGCAGGAAAGAA
CATGTGAGCAAAAGGCCAGCAAAAGGCCAGGAACCGTAAAAAGGCCGCGTTGCTGG
CGTTTTTCCATAGGCTCCGCCCCCCTGACGAGCATCACAAAAATCGACGCTCAAGTC
AGAGGTGGCGAAACCCGACAGGACTATAAAGATACCAGGCGTTTCCCCCTGGAAGC
TCCCTCGTGCGCTCTCCTGTTCCGACCCTGCCGCTTACCGGATACCTGTCCGCCTTT
CTCCCTTCGGGAAGCGTGGCGCTTTCTCATAGCTCACGCTGTAGGTATCTCAGTTCG
GTGTAGGTCGTTCGCTCCAAGCTGGGCTGTGTGCACGAACCCCCCGTTCAGCCCGA
CCGCTGCGCCTTATCCGGTAACTATCGTCTTGAGTCCAACCCGGTAAGACACGACTT
ATCGCCACTGGCAGCAGCCACTGGTAACAGGATTAGCAGAGCGAGGTATGTAGGCG
GTGCTACAGAGTTCTTGAAGTGGTGGCCTAACTACGGCTACACTAGAAGAACAGTAT
TTGGTATCTGCGCTCTGCTGAAGCCAGTTACCTTCGGAAAAAGAGTTGGTAGCTCTT
GATCCGGCAAACAAACCACCGCTGGTAGCGGTTTTTTTGTTTGCAAGCAGCAGATTA
CGCGCAGAAAAAAAGGATCTCAAGAAGATCCTTTGATCTTTTCTACGGGGTCTGACG
CTCAGTGGAACGAAAACTCACGTTAAGGGATTTTGGTCATGAGATTATCAAAAAGGA
TCTTCACCTAGATCCTTTTAAATTAAAAATGAAGTTTTAAATCAATCTAAAGTATATAT
GAGTAAACTTGGTCTGACAGTTACCAATGCTTAATCAGTGAGGCACCTATCTCAGCG
ATCTGTCTATTTCGTTCATCCATAGTTGCCTGACTCCCCGTCGTGTAGATAACTACGA
TACGGGAGGGCTTACCATCTGGCCCCAGTGCTGCAATGATACCGCGAGACCCACGC
TCACCGGCTCCAGATTTATCAGCAATAAACCAGCCAGCCGGAAGGGCCGAGCGCAG
AAGTGGTCCTGCAACTTTATCCGCCTCCATCCAGTCTATTAATTGTTGCCGGGAAGC
TAGAGTAAGTAGTTCGCCAGTTAATAGTTTGCGCAACGTTGTTGCCATTGCTACAGG
CATCGTGGTGTCACGCTCGTCGTTTGGTATGGCTTCATTCAGCTCCGGTTCCCAACG
ATCAAGGCGAGTTACATGATCCCCCATGTTGTGCAAAAAAGCGGTTAGCTCCTTCGG
TCCTCCGATCGTTGTCAGAAGTAAGTTGGCCGCAGTGTTATCACTCATGGTTATGGC
AGCACTGCATAATTCTCTTACTGTCATGCCATCCGTAAGATGCTTTTCTGTGACTGGT
GAGTACTCAACCAAGTCATTCTGAGAATAGTGTATGCGGCGACCGAGTTGCTCTTGC
CCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCC
AGTTCGATGTAACCCACTCGTGCACCCAACTGATCTTCAGCATCTTTTACTTTCACCA
GCGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTTA
TCAGGGTTATTGTCTCATGAGCGGATACATATTTGAATGTATTTAGAAAAATAAACAA
ATAGGGGTTCCGCGCACATTTCCCCGAAAAGTGCCACCTGACGTCGACGGATCGGG

AGATCTCCCGATCCCCTATGGTGCACTCTCAGTACAATCTGCTCTGATGCCGCATAG
TTAAGCCAGTATCTGCTCCCTGCTTGTGTGTTGGAGGTCGCTGAGTAGTGCGCGAG
CAAAATTTAAGCTACAACAAGGCAAGGCTTGACCGACAATTGCATGAAGAATCTGCT
TAGGGTTAGGCGTTTTGCGCTGCTTCGC 3'

## Claims

1. A compound, or a pharmaceutically acceptable salt thereof, having the structural formula (I) below: 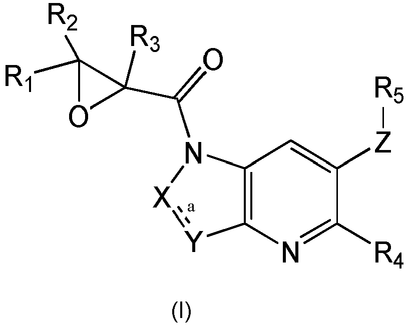 wherein:
R₁ is selected from hydrogen or a group:
-L^{B}-X^{B}-Q^{B}
wherein:
L^{B} is absent or -[CR_{L1}R_{L2}]ₘ-, wherein:
m is an integer selected from 1, 2, 3 or 4;
each occurrence of R_{L1} and R_{L2} is independently selected from hydrogen or (1-2C)alkyl), or R_{L1} and R_{L2} taken together may form an oxo group;
X^{B} is absent or is selected from the group consisting of -O-, -S-, -S(O)-, - S(O)₂-, -C(O)-, -C(O)O-, -OC(O)-, -C(O)N(R_{XB1})-, -N(R_{XB1})C(O)-, -N(R_{XB1})-, -N(R_{XB1})C(O)O-, -OC(O)N(R_{XB1})-, -N(R_{XB1})C(O)N(R_{XB2})-, -S(O)₂N(R_{XB1})-, or - N(R_{XB1})SO₂-, wherein R_{XB1} and R_{XB2} are each independently selected from hydrogen and (1-2C)alkyl;
Q^{B} is selected from a group consisting of hydrogen, (1-6C)alkyl, -[CR_{Q1}R_{Q2}]ₙ-, -[CR_{Q1}R_{Q2}]ₙ-(3-10C)cycloalkyl, -[CR_{Q1}R_{Q2}]ₙ-(3 to 10-membered heterocyclyl), - [CR_{Q1}R_{Q2}]ₙ-aryl and -[CR_{Q1}R_{Q2}]ₙ₋(5 to 10-membered heteroaryl);
wherein:
integer n is 0, 1 or 2; and
R_{Q1} and R_{Q2} are each independently selected from hydrogen and (1-2C)alkyl; and
any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy, NR_{Q3}R_{Q4}, C(O)R_{Q3}, C(O)OR_{Q3}, OC(O)R_{Q3}, C(O)N(R_{Q3})R_{Q4}, N(R_{Q3})C(O)R_{Q4}, S(O)_{y}R_{Q3} (where y is 0, 1 or 2), SO₂N(R_{Q3})R_{Q4} or N(R_{Q3})SO₂R_{Q4}; wherein R_{Q3} and
R_{Q4} are each independently selected from H or (1-2C)alkyl;
R₂ is selected from hydrogen and (1-3C)alkyl;
R₃ is selected from hydrogen and (1-3C)alkyl;
bond a is either a single bond or a double bond, wherein:
when bond a is a single bond:
X is selected from -C(R_{X1})(R_{X2})-, -C(=O)-, -C(=NH)-, -C(=S)-; and
Y is selected from -C(R_{Y1})(R_{Y2})-, -C(=O)-, -C(=NH)-, -C(=S)-; or
when bond a is a double bond:
X is -C(R_{X1})- and
Y is -C(R_{Y1})-;
wherein:
R_{X1} and R_{X2} are each independently selected from hydrogen, halo, hydroxy, (1-3C)alkyl or (1-3C)alkoxy; and any alkyl is optionally further substituted with one or more substituents selected from: halo, hydroxy or (1-3C)alkoxy; or
R_{Y1} and R_{Y2} are each independently selected from hydrogen, halo, hydroxy, (1-3C)alkyl or (1-3C)alkoxy; and any alkyl is optionally further substituted with one or more substituents selected from: halo, hydroxy or (1-3C)alkoxy; or
R_{X1} and R_{X2} are linked such that, together with the carbon atom to which they are attached, form a (3-6C)cycloalkyl ring; or
or R_{Y1} and R_{Y2} are linked such that, together with the carbon atom to which they are attached, form a (3-6C)cycloalkyl ring, optionally substituted with halo, hydroxy, (1-3C)alkyl or (1-3C)alkoxy; and any alkyl is optionally further substituted with one or more substituents selected from: halo, hydroxy or (1-3C)alkoxy;
R₄ is selected from hydrogen, halo, cyano, hydroxy, NR₄ₐR_{4b}, (1-6C)alkyl, (1-6C)alkoxy, (1-6C)haloalkyl, (1-6C)haloalkoxy, -[CH₂]ᵣ-(3-12C)cycloalkyl, -[CH₂]ᵣ-(3- to 12-membered)heterocyclyl, -[CH₂]ᵣ-aryl or -[CH₂]ᵣ-heteroaryl wherein each of R₄ₐ and
R_{4b} is independently selected from hydrogen or (1-3C)alkyl and integer r is 0, 1 or 2;
wherein:
any alkyl, cycloalkyl, heterocyclyl, aryl or heteroaryl in an R₄ group is optionally substituted by one or more R₁₆ substituents independently selected from halo, hydroxy, cyano, (1-3C)alkyl, (1-3C)alkoxy, (1-3C)haloalkyl, (1-3C)haloalkoxy or NR₄ₐR_{4b}, wherein R₄ₐ and R_{4b} are each independently selected from H or (1-2C)alkyl;
Z is selected from -C(R_{Z2})(R_{Z3})-, -O-, or -NR_{Z1}, wherein:
R_{Z1} is selected from hydrogen or (1-2C)alkyl;
R_{Z2} and R_{Z3} are each independently selected from hydrogen or (1-3C)alkyl; or
R_{Z2} and R_{Z3} are linked such that, together with the carbon atom to which they are attached, form a (3-5C)cycloalkyl or heterocyclyl ring;
R₅ is selected from phenyl, naphthyl or heteroaryl, wherein R₅ is optionally substituted by one or more substituents R_{A}, wherein each R_{A} is independently selected from: halo, hydroxy, cyano, (1-6C)alkyl, (1-6C)alkoxy, (1-6C)haloalkyl, (1-6C)haloalkoxy, cycloalkyl, (3-6-membered)heterocyclyl and -[CH₂]ₛ-NR₅ₐR_{5b}; wherein R₅ₐ and R_{5b} are each independently selected from hydrogen or (1-2C)alkyl and integer s is 0, 1 or 2.

2. A compound according to claim 1, or a pharmaceutically acceptable salt thereof, wherein R₁ is selected from hydrogen or a group:
-L^{B}-X^{B}-Q^{B}
wherein:
L^{B} is absent or -[CR_{L1}R_{L2}]ₘ-, wherein:
m is an integer selected from 1, 2 or 3;
each occurrence of R_{L1} and R_{L2} is independently selected from hydrogen or (1-2C)alkyl);
X^{B} is absent or is selected from the group consisting of -O-, -S-, -S(O)-, - S(O)₂-, -C(O)-, -C(O)O-, -OC(O)-, -C(O)N(R_{XB1})-, -N(R_{XB1})C(O)- or -N(R_{XB1})-, wherein R_{XB1} is selected from hydrogen and (1-2C)alkyl;
Q^{B} is selected from a group consisting of hydrogen, (1-6C)alkyl, -[CR_{Q1}R_{Q2}]ₙ-, -[CR_{Q1}R_{Q2}]ₙ-(3-10C)cycloalkyl, -[CR_{Q1}R_{Q2}]ₙ-(3 to 10-membered heterocyclyl), - [CR_{Q1}R_{Q2}]ₙ-aryl or -[CR_{Q1}R_{Q2}]ₙ₋(5 to 10-membered heteroaryl);
wherein:
integer n is 0, 1 or 2; and
R_{Q1} and R_{Q2} are each independently selected from hydrogen and (1-2C)alkyl; and
any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy, N(R_{Q3})R_{Q4}, C(O)R_{Q3}, C(O)OR_{Q3}, OC(O)R_{Q3}, C(O)N(R_{Q3})R_{Q4} or N(R_{Q3})C(O)R_{Q4}; wherein R_{Q3} and R_{Q4} are each independently selected from H or (1-2C)alkyl.

3. A compound according to claim 1 or claim 2, or a pharmaceutically acceptable salt thereof, wherein R₁ is selected from hydrogen or a group:
-L^{B}-X^{B}-Q^{B}
wherein:
L^{B} is absent or -[CR_{L1}R_{L2}]ₘ-, wherein:
m is an integer selected from 1 or 2;
each occurrence of R_{L1} and R_{L2} is independently selected from hydrogen or (1-2C)alkyl);
X^{B} is absent or is selected from the group consisting of -O-, -S-, -C(O)-, - C(O)N(R_{XB1})-, -N(R_{XB1})C(O)- or -N(R_{XB1})-, wherein R_{XB1} is selected from hydrogen and (1-2C)alkyl;
Q^{B} is selected from a group consisting of hydrogen, (1-6C)alkyl, -[CR_{Q1}R_{Q2}]ₙ-, -[CR_{Q1}R_{Q2}]ₙ-(3-6C)cycloalkyl, -[CR_{Q1}R_{Q2}]ₙ-(3 to 6-membered heterocyclyl), - [CR_{Q1}R_{Q2}]ₙ-phenyl or -[CR_{Q1}R_{Q2}]ₙ₋(6 or 6-membered heteroaryl);
wherein:
integer n is selected from 0, 1 or 2; and
R_{Q1} and R_{Q2} are each independently selected from hydrogen and (1-2C)alkyl; and
any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy or NR_{Q3}R_{Q4}; wherein R_{Q3} and R_{Q4} are each independently selected from H or (1-2C)alkyl;
optionally wherein R₁ is selected from hydrogen or a group:
-L^{B}-X^{B}-Q^{B}
wherein:
L^{B} is absent or -[CR_{L1}R_{L2}]ₘ-, wherein:
m is an integer selected from 1 or 2;
each occurrence of R_{L1} and R_{L2} is independently selected from hydrogen or (1-2C)alkyl);
X^{B} is absent or is selected from the group consisting of -O-, -S-, -C(O)-, - C(O)N(R_{XB1})-, -N(R_{XB1})C(O)-, -N(R_{XB1})-, wherein R_{XB1} is selected from hydrogen and (1-2C)alkyl;
Q^{B} is selected from a group consisting of hydrogen, (1-4C)alkyl, -[CR_{Q1}R_{Q2}]ₙ-, -[CR_{Q1}R_{Q2}]ₙ-(3-6C)cycloalkyl, -[CR_{Q1}R_{Q2}]ₙ-(3 to 6-membered heterocyclyl), - [CR_{Q1}R_{Q2}]ₙ-phenyl or -[CR_{Q1}R_{Q2}]ₙ₋(5 or 6-membered heteroaryl);
wherein:
integer n is selected from 0, 1 or 2; an R_{Q1} and R_{Q2} are each independently selected from hydrogen or (1-2C)alkyl; and
any Q^{B} is optionally further substituted with one or more substituents independently selected from: halo, oxo, hydroxy, cyano, (1-2C)alkyl, (1-2C)alkoxy, (1-2C)haloalkyl, (1-2C)haloalkoxy or NR_{Q3}R_{Q4}; wherein R_{Q3} and R_{Q4} are each independently selected from hydrogen or methyl.

4. A compound according to any one of the preceding claims, or a pharmaceutically acceptable salt thereof, wherein R₁ is selected from hydrogen or a group:
-L^{B}-X^{B}-Q^{B}
wherein:
L^{B} is absent or -[CR_{L1}R_{L2}]-, wherein:
each occurrence of R_{L1} and R_{L2} is independently selected from hydrogen or methyl;
X^{B} is absent or is selected from the group consisting of -O-, -C(O)N(R_{XB1})-, wherein R_{XB1} is selected from hydrogen and methyl;
Q^{B} is selected from a group consisting of hydrogen, (1-4C)alkyl, -[CR_{Q1}R_{Q2}]ₙ-(3-6C)cycloalkyl, -[CR_{Q1}R_{Q2}]ₙ-(4 to 6-membered heterocyclyl), -[CR_{Q1}R_{Q2}]ₙ-phenyl or -[CR_{Q1}R_{Q2}]ₙ₋(5 or 6-membered heteroaryl);
wherein:
integer n is selected from 0 or 1; and
R_{Q1} and R_{Q2} are each independently selected from hydrogen and methyl; and
any Q^{B} is optionally further substituted with one or more substituents independently selected from: halo, oxo, hydroxy, cyano, (1-3C)alkyl, (1-3C)alkoxy, (1-3C)haloalkyl, (1-3C)haloalkoxy or NR_{Q3}R_{Q4}; wherein R_{Q3} and R_{Q4} are each independently selected from H or (1-2C)alkyl;
optionally wherein R₁ is a group of the formula:
-L^{B}-X^{B}-Q^{B}
wherein:
L^{B} is absent or -[CR_{L1}R_{L2}]-, wherein each occurrence of R_{L1} and R_{L2} is independently selected from hydrogen or (1-2C)alkyl);
X^{B} is absent or is selected from the group consisting of -O-, -S-, -C(O)-, - C(O)N(R_{XB1})-, -N(R_{XB1})C(O)-, -N(R_{XB1})-, wherein R_{XB1} is selected from hydrogen and (1-2C)alkyl;
Q^{B} is selected from a group consisting of hydrogen, (1-4C)alkyl, (3-6C)cycloalkyl, 3 to 6-membered heterocyclyl (e.g. oxetane, azetidine, 1,3-dioxolane or morpholine), phenyl or 5 or 6-membered heteroaryl;
wherein:
any Q^{B} is optionally further substituted with one or more substituents independently selected from: halo, oxo, hydroxy, cyano, (1-2C)alkyl, (1-2C)alkoxy.

5. A compound according to any one of the preceding claims, or a pharmaceutically acceptable salt thereof, wherein R₁ is a group with a formula selected from:

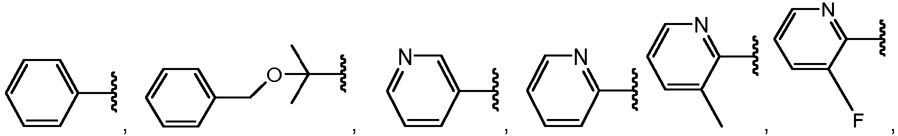

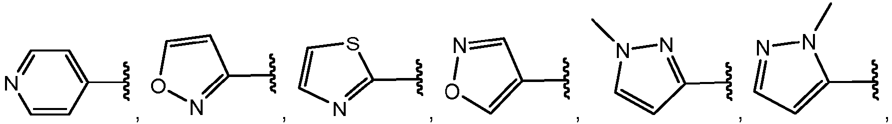

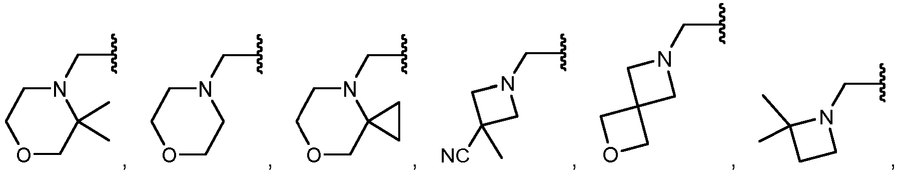

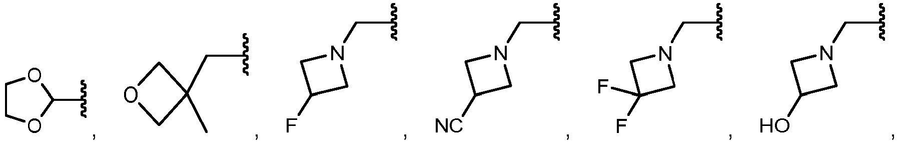

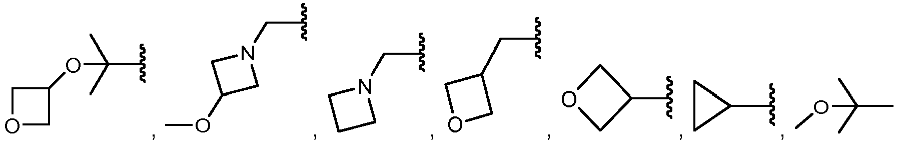

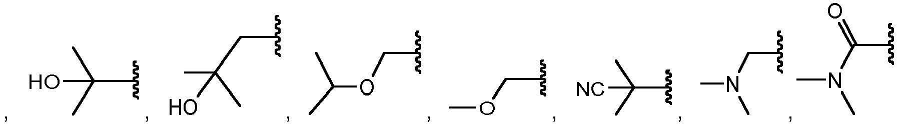

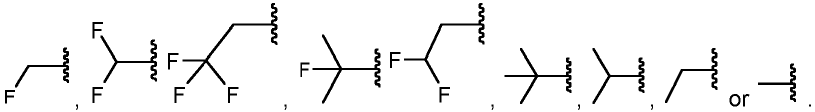

6. A compound according to any one of the preceding claims, or a pharmaceutically acceptable salt thereof, wherein R₂ is selected from hydrogen or methyl; optionally wherein R₂ is hydrogen.

7. A compound according to any one of the preceding claims, or a pharmaceutically acceptable salt thereof, wherein R₃ is selected from hydrogen or methyl; optionally wherein R₃ is hydrogen.

8. A compound according to any one of the preceding claims, or a pharmaceutically acceptable salt thereof, wherein bond a is either a single bond or a double bond, and wherein:
when bond a is a single bond:
X is -CH₂-, and
Y is selected from -C(R_{Y1})(R_{Y2})- or -C(=O)-; or
when bond a is a double bond:
X is -CH- and
Y is -C(R_{Y1})-;
wherein:
R_{Y1} and R_{Y2} are each independently selected from hydrogen or (1-2C)alkyl; or
or R_{Y1} and R_{Y2} are linked such that, together with the carbon atom to which they are attached, form a (3-4C)cycloalkyl ring.

9. A compound according to any one of the preceding claims, or a pharmaceutically acceptable salt thereof, wherein bond a is a single bond and:
X is -CH₂-, and
Y is -C(R_{Y1})(R_{Y2})-;
wherein:
R_{Y1} and R_{Y2} are each independently selected from hydrogen or methyl; or
R_{Y1} and R_{Y2} are linked such that, together with the carbon atom to which they are attached, form a cyclopropyl ring.

10. A compound according to any one of the preceding claims, or a pharmaceutically acceptable salt thereof, wherein R₄ is selected from hydrogen, halo (e.g. F or Cl), NR₄ₐR_{4b}, (1-4C)alkyl (e.g. CH₃ or CD₃), (1-4C)alkoxy, (1-4C)haloalkyl, (1-4C)haloalkoxy, -[CH₂]ᵣ-(3-6C)cycloalkyl, -[CH₂]ᵣ-(3- to 7-membered)heterocyclyl, -[CH₂]ᵣ-phenyl or -[CH₂]ᵣ-(5- or 6-membered heteroaryl;
wherein:
each of R₄ₐ and R_{4b} is independently selected from hydrogen or (1-2C)alkyl and integer r is 0 or 1; and
any alkyl, cycloalkyl, heterocyclyl, phenyl or heteroaryl in an R₄ group is optionally substituted by one or more substituents independently selected from halo, hydroxy, cyano or amino
optioally wherein R₄ is selected from hydrogen, halo (e.g. For Cl), NR₄ₐR_{4b}, (1-3C)alkyl (e.g. CH₃ or CD₃), (1-2C)alkoxy, (1-3C)haloalkyl, (3-6C)cycloalkyl, (4- to 6-membered)heterocyclyl, phenyl or (5- or 6-membered heteroaryl;
wherein:
each of R₄ₐ and R_{4b} is independently selected from hydrogen or (1-2C)alkyl and integer r is 0 or 1; and
any alkyl, cycloalkyl, heterocyclyl, phenyl or heteroaryl in an R₄ group is optionally substituted by one or more substituents independently selected from halo, hydroxy, cyano or amino.

11. A compound according to any one of the preceding claims, or a pharmaceutically acceptable salt thereof, wherein R₄ is selected from hydrogen, halo (e.g. For Cl), NR₄ₐR_{4b}, (1-3C)alkyl (e.g. CH₃ or CD₃), (1-2C)alkoxy, (1-3C)haloalkyl, (3-6C)cycloalkyl, (4- to 6-membered)heterocyclyl, phenyl or (5- or 6-membered heteroaryl;
wherein:
each of R₄ₐ and R_{4b} is independently selected from hydrogen or methyl;
any alkyl, cycloalkyl, heterocyclyl, phenyl or heteroaryl in an R₄ group is optionally substituted by one or more substituents independently selected from halo, hydroxy, cyano or amino.

12. A compound according to any one of the preceding claims, or a pharmaceutically acceptable salt thereof, wherein R₄ is selected from hydrogen, F or Cl, NR₄ₐR_{4b}, (1-2C)alkyl (e.g. CH₃ or CD₃), methoxy, (1-2C)haloalkyl, (3-4C)cycloalkyl, (4- to 5-membered)heterocyclyl or (5- or 6-membered heteroaryl;
wherein:
each of R₄ₐ and R_{4b} is independently selected from hydrogen or methyl;
any alkyl, cycloalkyl, heterocyclyl or heteroaryl in an R₄ group is optionally substituted by one or more substituents independently selected from halo, hydroxy, cyano or amino;
optionally wherein R₄ is methyl.

13. A compound according to any one of the preceding claims, or a pharmaceutically acceptable salt thereof, wherein Z is selected from -C(R_{Z2})(R_{Z3})-, -O-, - or -NR_{Z1}; wherein:
R_{Z1} is selected from hydrogen or methyl; and
R_{Z2} and R_{Z3} are independently selected from hydrogen or methyl;
optionally wherein Z is selected from -CH₂-, -O- or -NR_{Z1}; wherein:
R_{Z1} is selected from hydrogen or methyl;
further optionally wherein Z is -O-.

14. A compound according to any one of the preceding claims, or a pharmaceutically acceptable salt thereof, wherein R₅ is selected from phenyl, 5- or 6-membered heteroaryl, or a bicyclic heteroaryl (e.g. a bicyclic heteroaryl comprising a phenyl ring fused to a 4- to 6 membered heterocyclic ring);
wherein R₅ is optionally substituted by one or more substituents R_{A}, wherein each R_{A} is independently selected from: halo, hydroxy, cyano, (1-4C)alkyl, (1-4C)alkoxy, (1-4C)haloalkyl, (1-4C)haloalkoxy, (3-6C)cycloalkyl, (3-6-membered)heterocyclyl and NR₅ₐR_{5b}; wherein R₅ₐ and R_{5b} are each independently selected from hydrogen or methyl;
optionally wherein R₅ is selected from phenyl, pyridyl, or a phenyl ring fused to a 4- to 6 membered heterocyclic ring;
wherein R₅ is optionally substituted by one or more substituents R_{A}, wherein each R_{A} is independently selected from: halo, hydroxy, cyano, (1-2C)alkyl, (1-2C)alkoxy, (1-2C)haloalkyl, (1-2C)haloalkoxy, (3-6C)cycloalkyl or (3-6-membered) heterocyclyl;

15. A compound according to any one of the preceding claims, or a pharmaceutically acceptable salt thereof, wherein R₅ is a group of the formula: 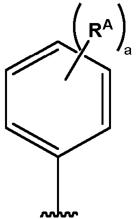 wherein a is an integer selected from 0, 1, 2, 3 or 4;
each occurrence of R_{A} is independently as defined in any one of the preceding claims;
optionally wherein each occurrence of R_{A} is independently selected from: halo, hydroxy, cyano, (1-2C)alkyl, (1-2C)alkoxy, (1-2C)haloalkyl, (1-2C)haloalkoxy, (3-6C)cycloalkyl or (3-6-membered)heterocyclyl;
optionally wherein each occurrence of R_{A} is independently selected from fluoro, chloro, hydroxy, cyano, or methoxy;
further optionally wherein each occurrence of R_{A} is independently selected from fluoro or chloro.

16. A compound according to any one of the preceding claims, or a pharmaceutically acceptable salt thereof, wherein R₅ is a group of the formula: 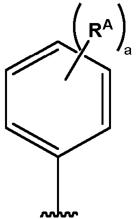 wherein a is an integer selected from 0, 1, 2 or 3;
each occurrence of R_{A} is independently selected from fluoro, chloro, hydroxy, cyano, methyl, methoxy, fluoromethyl, fluoromethoxy, (3-6C)cycloalkyl or (3-6-membered) heterocyclyl;
optionally wherein each occurrence of R_{A} is independently selected from fluoro, chloro, hydroxy, cyano, or methoxy;
further optionally wherein each occurrence of R_{A} is independently selected from fluoro or chloro;

17. A compound according to any one of the preceding claims, or a pharmaceutically acceptable salt thereof, wherein R₅ is a group of the formula: 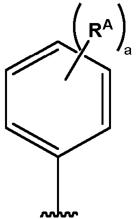 wherein:
a is an integer selected from 0, 1, 2;
each occurrence of R_{A} is independently as defined in any one of the preceding claims; and
at least one occurrence of R_{A} is in the meta position.

18. A compound according to any one of the preceding claims, or a pharmaceutically acceptable salt thereof, wherein R₅ is a group with a formula selected from: 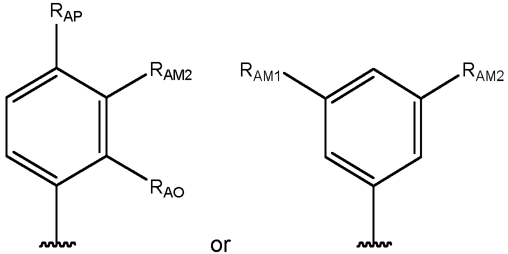
each of R_{AO} and R_{AP} are independently selected from hydrogen, fluoro and chloro; R_{AM1} is selected from fluoro and chloro; and
R_{AM2} is selected from fluoro and chloro.
optionally wherein R₅ is a group a formula: 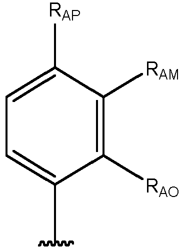
each of R_{AO} and R_{AP} are independently selected from hydrogen, fluoro and chloro; R_{AM} is selected from fluoro and chloro;
wherein at least one of R_{AO} and R_{AP} is hydrogen, and the other is selected from fluoro and chloro.

19. A compound according to any one of the preceding claims, or a pharmaceutically acceptable salt thereof, wherein the compound has a structural formula (II) shown below: 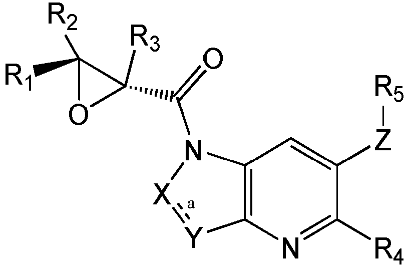 wherein R₁, R₂, R₃, R₄, R₅, X, Y, Z and bond a are as defined in any one of the preceding claims.

20. A compound or a pharmaceutically acceptable salt thereof, selected from any one of the following:
trans-(6-(3-chloro-4-fluorobenzyl)-5-methyl-2,3-dihydro-1H-pyrrolo[3,2-b]pyridin-1-yl)(3-((dimethylamino)methyl)oxiran-2-yl)methanone;
trans-(6-(3-chloro-4-fluorobenzyl)-5-methyl-2,3-dihydro-1H-pyrrolo[3,2-b]pyridin-1-yl)(3-(methoxymethyl)oxiran-2-yl)methanone;
trans-(6-(3-chloro-4-fluorobenzyl)-5-methyl-2,3-dihydro-1H-pyrrolo[3,2-b]pyridin-1-yl)(3-(fluoromethyl)oxiran-2-yl)methanone;
trans-(6-(3-chloro-4-fluorobenzyl)-5-methyl-2,3-dihydro-1H-pyrrolo[3,2-b]pyridin-1-yl)(3-ethyloxiran-2-yl)methanone;
trans-(6-(3-chloro-4-fluorobenzyl)-5-methyl-2,3-dihydro-1H-pyrrolo[3,2-b]pyridin-1-yl)(3-methyloxiran-2-yl)methanone;
(6-(3-chloro-4-fluorobenzyl)-5-methyl-2,3-dihydro-1H-pyrrolo[3,2-b]pyridin-1-yl)((2R,3S)-3-methyloxiran-2-yl)methanone;
(6-(3-chloro-4-fluorobenzyl)-5-methyl-2,3-dihydro-1H-pyrrolo[3,2-b]pyridin-1-yl)(oxiran-2-yl)methanone;
(R)-(6-(3-chlorobenzyl)-5-methyl-2,3-dihydro-1H-pyrrolo[3,2-b]pyridin-1-yl)(oxiran-2-yl)methanone;
trans-(6-(3-chloro-4-fluorobenzyl)-5-(oxetan-3-yl)-2,3-dihydro-1H-pyrrolo[3,2-b]pyridin-1-yl)(3-methyloxiran-2-yl)methanone;
trans-(6-(3-chloro-4-fluorobenzyl)-2,3-dihydro-1H-pyrrolo[3,2-b]pyridin-1-yl)(3-methyloxiran-2-yl)methanone;
trans-(6-(3-chloro-4-fluorobenzyl)-5-(dimethylamino)-2,3-dihydro-1H-pyrrolo[3,2-b]pyridin-1-yl)(3-methyloxiran-2-yl)methanone;
trans-(6-(3-chloro-4-fluorobenzyl)-5-(isoxazol-5-yl)-2,3-dihydro-1H-pyrrolo[3,2-b]pyridin-1-yl)(3-methyloxiran-2-yl)methanone;
trans-(6-(3-chloro-4-fluorobenzyl)-5-cyclopropyl-2,3-dihydro-1H-pyrrolo[3,2-b]pyridin-1-yl)(3-methyloxiran-2-yl)methanone;
trans-(6-(3-chloro-4-fluorobenzyl)-5-(isoxazol-4-yl)-2,3-dihydro-1H-pyrrolo[3,2-b]pyridin-1-yl)(3-methyloxiran-2-yl)methanone;
trans-(6-(3-chloro-4-fluorobenzyl)-5-(hydroxymethyl)-2,3-dihydro-1H-pyrrolo[3,2-b]pyridin-1-yl)(3-methyloxiran-2-yl)methanone;
trans-(6-(3-chloro-4-fluorobenzyl)-5-ethyl-2,3-dihydro-1H-pyrrolo[3,2-b]pyridin-1-yl)(3-methyloxiran-2-yl)methanone;
(6-(3-chloro-4-fluorobenzyl)-5-methoxy-2,3-dihydro-1H-pyrrolo[3,2-b]pyridin-1-yl)((2R,3S)-3-methyloxiran-2-yl)methanone;
trans-(6-(3-chloro-4-fluorobenzyl)-5-(methyl-d3)-2,3-dihydro-1H-pyrrolo[3,2-b]pyridin-1-yl)(3-methyloxiran-2-yl)methanone;
trans-(5-chloro-6-(3-chloro-4-fluorobenzyl)-2,3-dihydro-1H-pyrrolo[3,2-b]pyridin-1-yl)(3-methyloxiran-2-yl)methanone;
(6-(3-chloro-4-fluorobenzyl)-5-fluoro-2,3-dihydro-1H-pyrrolo[3,2-b]pyridin-1-yl)((2R,3S)-3-methyloxiran-2-yl)methanone;
(5-chloro-6-(3-chloro-4-fluorobenzyl)-2,3-dihydro-1H-pyrrolo[3,2-b]pyridin-1-yl)((2R,3S)-3-methyloxiran-2-yl)methanone;
trans-(6-(4-fluorophenoxy)-5-methyl-2,3-dihydro-1H-pyrrolo[3,2-b]pyridin-1-yl)(3-(2-methoxypropan-2-yl)oxiran-2-yl)methanone;
trans-(6-(3,4-difluorophenoxy)-5-methyl-2,3-dihydro-1H-pyrrolo[3,2-b]pyridin-1-yl)(3-(2-methoxypropan-2-yl)oxiran-2-yl)methanone;
trans-(6-(2,3-difluorophenoxy)-5-methyl-2,3-dihydro-1H-pyrrolo[3,2-b]pyridin-1-yl)(3-(2-methoxypropan-2-yl)oxiran-2-yl)methanone;
trans- (6-(3-fluorophenoxy)-5-methyl-2,3-dihydro-1H-pyrrolo[3,2-b]pyridin-1-yl)(3-(2-(oxetan-3-yloxy)propan-2-yl)oxiran-2-yl)methanone;
trans-(6-(4-fluorophenoxy)-5-methyl-2,3-dihydro-1H-pyrrolo[3,2-b]pyridin-1-yl)(3-(2-fluoropropan-2-yl)oxiran-2-yl)methanone;
trans-(6-(3,4-difluorophenoxy)-5-methyl-2,3-dihydro-1H-pyrrolo[3,2-b]pyridin-1-yl)(3-(oxetan-3-ylmethyl)oxiran-2-yl)methanone;
trans-(6-(2,3-difluorophenoxy)-5-methyl-2,3-dihydro-1H-pyrrolo[3,2-b]pyridin-1-yl)(3-((3-methyloxetan-3-yl)methyl)oxiran-2-yl)methanone;
trans-(6-(3,4-difluorophenoxy)-5-methyl-2,3-dihydro-1H-pyrrolo[3,2-b]pyridin-1-yl)(3-(2-hydroxy-2-methylpropyl)oxiran-2-yl)methanone;
trans-(6-(2,3-difluorophenoxy)-5-methyl-2,3-dihydro-1H-pyrrolo[3,2-b]pyridin-1-yl)(3-(2-hydroxy-2-methylpropyl)oxiran-2-yl)methanone;
trans-(3-(2,2-difluoroethyl)oxiran-2-yl)(6-(4-fluorophenoxy)-5-methyl-2,3-dihydro-1H-pyrrolo[3,2-b]pyridin-1-yl)methanone;
(6-(2,3-difluorophenoxy)-5-methyl-2,3-dihydro-1H-pyrrolo[3,2-b]pyridin-1-yl)((2R,3S)-3-((3-methyloxetan-3-yl)methyl)oxiran-2-yl)methanone;
trans-(6-(3-fluorophenoxy)-5-methyl-2,3-dihydro-1H-pyrrolo[3,2-b]pyridin-1-yl)(3-(2-fluoropropan-2-yl)oxiran-2-yl)methanone;
trans-(6-(3-fluorophenoxy)-5-methyl-2,3-dihydro-1H-pyrrolo[3,2-b]pyridin-1-yl)(3-(2,2,2-trifluoroethyl)oxiran-2-yl)methanone;
trans-(3-(2,2-difluoroethyl)oxiran-2-yl)(6-(3-fluorophenoxy)-5-methyl-2,3-dihydro-1H-pyrrolo[3,2-b]pyridin-1-yl)methanone;
(6-(2,3-difluorophenoxy)-5-methyl-2,3-dihydro-1H-pyrrolo[3,2-b]pyridin-1-yl)((2R,3S)-3-(2-hydroxy-2-methylpropyl)oxiran-2-yl)methanone;
trans-2-(3-(6-(3-fluorophenoxy)-5-methyl-2,3-dihydro-1H-pyrrolo[3,2-b]pyridine-1-carbonyl)oxiran-2-yl)-2-methylpropanenitrile;
trans-2-(3-(6-(2,3-difluorophenoxy)-5-methyl-2,3-dihydro-1H-pyrrolo[3,2-b]pyridine-1-carbonyl)oxiran-2-yl)-2-methylpropanenitrile;
(6-(3,4-difluorophenoxy)-5-methyl-2,3-dihydro-1H-pyrrolo[3,2-b]pyridin-1-yl)((2S,3R)-3-(2-hydroxy-2-methylpropyl)oxiran-2-yl)methanone;
(6-(3-fluorophenoxy)-5-methyl-2,3-dihydro-1H-pyrrolo[3,2-b]pyridin-1-yl)((2R,3S)-3-(oxetan-3-ylmethyl)oxiran-2-yl)methanone;
(6-(3,4-difluorophenoxy)-5-methyl-2,3-dihydro-1H-pyrrolo[3,2-b]pyridin-1-yl)((2R,3S)-3-(oxetan-3-ylmethyl)oxiran-2-yl)methanone;
2-((2S,3R)-3-(6-(3-fluorophenoxy)-5-methyl-2,3-dihydro-1H-pyrrolo[3,2-b]pyridine-1-carbonyl)oxiran-2-yl)-2-methylpropanenitrile;
trans-(3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone;
2-((2S,3R)-3-(6-(2,3-difluorophenoxy)-5-methyl-2,3-dihydro-1H-pyrrolo[3,2-b]pyridine-1-carbonyl)oxiran-2-yl)-2-methylpropanenitrile;
((2R,3R)-3-(1,3-dioxolan-2-yl)oxiran-2-yl)(6-(2,3-difluorophenoxy)-5-methyl-2,3-dihydro-1H-pyrrolo[3,2-b]pyridin-1-yl)methanone;
trans-2-(3-(6-(4-fluorophenoxy)-5-methyl-2,3-dihydro-1H-pyrrolo[3,2-b]pyridine-1-carbonyl)oxiran-2-yl)-2-methylpropanenitrile;
trans-(6-(3-fluorophenoxy)-5-methyl-2,3-dihydro-1H-pyrrolo[3,2-b]pyridin-1-yl)(3-(oxetan-3-ylmethyl)oxiran-2-yl)methanone;
trans-(6-(2,3-difluorophenoxy)-5-methyl-2,3-dihydro-1H-pyrrolo[3,2-b]pyridin-1-yl)(3-(isoxazol-4-yl)oxiran-2-yl)methanone;
trans-(3-(tert-butyl)oxiran-2-yl)(6-(4-fluorophenoxy)-5-methyl-2,3-dihydro-1H-pyrrolo[3,2-b]pyridin-1-yl)methanone;
Trans-(6-(3-fluoro-4-morpholinophenoxy)-5-methyl-2,3-dihydro-1H-pyrrolo[3,2-b]pyridin-1-yl)(3-(oxetan-3-yl)oxiran-2-yl)methanone;
trans-(3-(2-(benzyloxy)propan-2-yl)oxiran-2-yl)(6-(3-fluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-(2-hydroxypropan-2-yl)oxiran-2-yl)methanone;
trans-(6-(3-fluorophenoxy)-5-methyl-2,3-dihydro-1H-pyrrolo[3,2-b]pyridin-1-yl)(3-(2-hydroxypropan-2-yl)oxiran-2-yl)methanone;
trans-(6-(3,4-difluorophenoxy)-5-methyl-2,3-dihydro-1H-pyrrolo[3,2-b]pyridin-1-yl)(3-phenyloxiran-2-yl)methanone;
trans-(6-(3-chloro-4-fluorophenoxy)-5-methyl-2,3-dihydro-1H-pyrrolo[3,2-b]pyridin-1-yl)(3-phenyloxiran-2-yl)methanone;
(6-(2,3-difluorophenoxy)-5-methyl-2,3-dihydro-1H-pyrrolo[3,2-b]pyridin-1-yl)((2R,3R)-3-(2-hydroxypropan-2-yl)oxiran-2-yl)methanone;
trans-(6-(3-chloro-4-fluorophenoxy)-5-methyl-2,3-dihydro-1H-pyrrolo[3,2-b]pyridin-1-yl)(3-cyclopropyloxiran-2-yl)methanone;
trans-(6-(4-fluorophenoxy)-5-methyl-2,3-dihydro-1H-pyrrolo[3,2-b]pyridin-1-yl)(3-(1-methyl-1H-pyrazol-3-yl)oxiran-2-yl)methanone;
trans-(6-(2,3-difluorophenoxy)-5-methyl-2,3-dihydro-1H-pyrrolo[3,2-b]pyridin-1-yl)(3-(pyridin-3-yl)oxiran-2-yl)methanone;
trans-(6-(2,3-difluorophenoxy)-5-methyl-2,3-dihydro-1H-pyrrolo[3,2-b]pyridin-1-yl)(3-(pyridin-2-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-3-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-4-yl)oxiran-2-yl)methanone;
trans-(6-(3,4-difluorophenoxy)-5-methyl-2,3-dihydro-1H-pyrrolo[3,2-b]pyridin-1-yl)(3-(pyridin-2-yl)oxiran-2-yl)methanone;
(6-(3-chloro-4-fluorophenoxy)-5-methyl-2,3-dihydro-1H-pyrrolo[3,2-b]pyridin-1-yl)((2S,3R)-3-phenyloxiran-2-yl)methanone;
trans-(6-(3,4-difluorophenoxy)-5-methyl-2,3-dihydro-1H-pyrrolo[3,2-b]pyridin-1-yl)(3-(oxetan-3-yl)oxiran-2-yl)methanone;
trans-(6-(4-fluorophenoxy)-5-methyl-2,3-dihydro-1H-pyrrolo[3,2-b]pyridin-1-yl)(3-(1-methyl-1H-pyrazol-5-yl)oxiran-2-yl)methanone;
trans-(6-(2,3-difluorophenoxy)-5-methyl-2,3-dihydro-1H-pyrrolo[3,2-b]pyridin-1-yl)(3-(oxetan-3-yl)oxiran-2-yl)methanone;
(6-(3,4-difluorophenoxy)-5-methyl-2,3-dihydro-1H-pyrrolo[3,2-b]pyridin-1-yl)((2R,3S)-3-(pyridin-2-yl)oxiran-2-yl)methanone;
trans-(6-(3,4-difluorophenoxy)-5-methyl-2,3-dihydro-1H-pyrrolo[3,2-b]pyridin-1-yl)(3-(isoxazol-5-yl)oxiran-2-yl)methanone;
(6-(2,3-difluorophenoxy)-5-methyl-2,3-dihydro-1H-pyrrolo[3,2-b]pyridin-1-yl)((2R,3S)-3-(oxetan-3-yl)oxiran-2-yl)methanone;
(6-(2,3-difluorophenoxy)-5-methyl-2,3-dihydro-1H-pyrrolo[3,2-b]pyridin-1-yl)((2R,3S)-3-(pyridin-3-yl)oxiran-2-yl)methanone;
(6-(3-chloro-4-fluorophenoxy)-5-methyl-2,3-dihydro-1H-pyrrolo[3,2-b]pyridin-1-yl)((2R,3S)-3-cyclopropyloxiran-2-yl)methanone;
(6-(3,4-difluorophenoxy)-5-methyl-2,3-dihydro-1H-pyrrolo[3,2-b]pyridin-1-yl)(3-(isoxazol-3-yl)oxiran-2-yl)methanone;
trans-(6-(3,4-difluorophenoxy)-5-methyl-2,3-dihydro-1H-pyrrolo[3,2-b]pyridin-1-yl)(3-(isoxazol-5-yl)oxiran-2-yl)methanone;
trans-(6-(2,3-difluorophenoxy)-5-methyl-2,3-dihydro-1H-pyrrolo[3,2-b]pyridin-1-yl)(3-(3-fluoropyridin-2-yl)oxiran-2-yl)methanone;
trans-(6-(2,3-difluorophenoxy)-5-methyl-2,3-dihydro-1H-pyrrolo[3,2-b]pyridin-1-yl)(3-(3-methylpyridin-2-yl)oxiran-2-yl)methanone;
trans-(6-(3-chloro-4-fluorophenoxy)-5-methyl-2,3-dihydro-1H-pyrrolo[3,2-b]pyridin-1-yl)(3-isopropyloxiran-2-yl)methanone;
trans-(3-cyclopropyloxiran-2-yl)(6-(3,4-difluorophenoxy)-5-methyl-2,3-dihydro-1H-pyrrolo[3,2-b]pyridin-1-yl)methanone;
trans-(6-(3-fluoro-2-hydroxyphenoxy)-5-methyl-2,3-dihydro-1H-pyrrolo[3,2-b]pyridin-1-yl)(3-(morpholinomethyl)oxiran-2-yl)methanone;
trans-(6-(3-fluoro-2-methoxyphenoxy)-5-methyl-2,3-dihydro-1H-pyrrolo[3,2-b]pyridin-1-yl)(3-(morpholinomethyl)oxiran-2-yl)methanone;
trans-(3-(azetidin-1-ylmethyl)oxiran-2-yl)(6-(3-fluoro-2-methoxyphenoxy)-5-methyl-2,3-dihydro-1H-pyrrolo[3,2-b]pyridin-1-yl)methanone;
trans-(3-(azetidin-1-ylmethyl)oxiran-2-yl)(6-(3-fluorophenoxy)-5-methyl-2,3-dihydro-1H-pyrrolo[3,2-b]pyridin-1-yl)methanone;
trans-(6-(2,5-difluorophenoxy)-5-methyl-2,3-dihydro-1H-pyrrolo[3,2-b]pyridin-1-yl)(3-(morpholinomethyl)oxiran-2-yl)methanone;
trans-(6-(2-fluorophenoxy)-5-methyl-2,3-dihydro-1H-pyrrolo[3,2-b]pyridin-1-yl)(3-(morpholinomethyl)oxiran-2-yl)methanone;
trans-(6-(2,4-difluorophenoxy)-5-methyl-2,3-dihydro-1H-pyrrolo[3,2-b]pyridin-1-yl)(3-((dimethylamino)methyl)oxiran-2-yl)methanone;
(6-(2-fluorophenoxy)-5-methyl-2,3-dihydro-1H-pyrrolo[3,2-b]pyridin-1-yl)((2R,3S)-3-(morpholinomethyl)oxiran-2-yl)methanone;
trans-(6-(3-fluorophenoxy)-5-methyl-2,3-dihydro-1H-pyrrolo[3,2-b]pyridin-1-yl)(3-((3-methoxyazetidin-1-yl)methyl)oxiran-2-yl)methanone;
trans-(3-((3-fluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(3-((3-fluoroazetidin-1-yl)methyl)oxiran-2-yl)(6-(4-fluorophenoxy)-5-methyl-2,3-dihydro-1H-pyrrolo[3,2-b]pyridin-1-yl)methanone;
trans-(6-(3,4-difluorophenoxy)-5-methyl-2,3-dihydro-1H-pyrrolo[3,2-b]pyridin-1-yl)(3-(morpholinomethyl)oxiran-2-yl)methanone;
trans-(6-(3-fluorophenoxy)-5-methyl-2,3-dihydro-1H-pyrrolo[3,2-b]pyridin-1-yl)(3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-(morpholinomethyl)oxiran-2-yl)methanone;
trans-(3-((dimethylamino)methyl)oxiran-2-yl)(6-(3-fluorophenoxy)-5-methyl-2,3-dihydro-1H-pyrrolo[3,2-b]pyridin-1-yl)methanone;
trans-(3-(azetidin-1-ylmethyl)oxiran-2-yl)(6-(4-fluorophenoxy)-5-methyl-2,3-dihydro-1H-pyrrolo[3,2-b]pyridin-1-yl)methanone;
trans-(3-((2-oxa-6-azaspiro[3.3]heptan-6-yl)methyl)oxiran-2-yl)(6-(3-fluorophenoxy)-5-methyl-2,3-dihydro-1H-pyrrolo[3,2-b]pyridin-1-yl)methanone;
trans-(6-(4-fluorophenoxy)-5-methyl-2,3-dihydro-1H-pyrrolo[3,2-b]pyridin-1-yl)(3-(isopropoxymethyl)oxiran-2-yl)methanone;
(6-(2,3-difluorophenoxy)-5-methyl-2,3-dihydro-1H-pyrrolo[3,2-b]pyridin-1-yl)((2R,3S)-3-(morpholinomethyl)oxiran-2-yl)methanone;
trans-1-((3-(6-(2,3-difluorophenoxy)-5-methyl-2,3-dihydro-1H-pyrrolo[3,2-b]pyridine-1-carbonyl)oxiran-2-yl)methyl)azetidine-3-carbonitrile;
(6-(2,3-difluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone;
trans-(6-(3,4-difluorophenoxy)-5-methyl-2,3-dihydro-1H-pyrrolo[3,2-b]pyridin-1-yl)(3-((dimethylamino)methyl)oxiran-2-yl)methanone;
trans-(3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((2,2-dimethylazetidin-1-yl)methyl)oxiran-2-yl)methanone;
trans-(6-(2,3-difluorophenoxy)-5-methyl-2,3-dihydro-1H-pyrrolo[3,2-b]pyridin-1-yl)(3-((3-fluoroazetidin-1-yl)methyl)oxiran-2-yl)methanone;
trans-(3-((2-oxa-6-azaspiro[3.3]heptan-6-yl)methyl)oxiran-2-yl)(6-(3,4-difluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((3-hydroxyazetidin-1-yl)methyl)oxiran-2-yl)methanone;
trans-(3-((2-oxa-6-azaspiro[3.3]heptan-6-yl)methyl)oxiran-2-yl)(6-(2,3-difluorophenoxy)-5-methyl-2,3-dihydro-1H-pyrrolo[3,2-b]pyridin-1-yl)methanone;
trans-(6-(2,3-difluorophenoxy)-5-methyl-2,3-dihydro-1H-pyrrolo[3,2-b]pyridin-1-yl)(3-((dimethylamino)methyl)oxiran-2-yl)methanone;
(6-(3,4-difluorophenoxy)-5-methyl-2,3-dihydro-1H-pyrrolo[3,2-b]pyridin-1-yl)((2R,3S)-3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-((3-methoxyazetidin-1-yl)methyl)oxiran-2-yl)methanone;
(6-(3-fluorophenoxy)-5-methyl-2,3-dihydro-1H-pyrrolo[3,2-b]pyridin-1-yl)((2R,3S)-3-((3-methoxyazetidin-1-yl)methyl)oxiran-2-yl)methanone;
((2R,3S)-3-((3,3-difluoroazetidin-1-yl)methyl)oxiran-2-yl)(6-(3-fluorophenoxy)-5-methyl-2,3-dihydro-1H-pyrrolo[3,2-b]pyridin-1-yl)methanone;
trans-(6-(3-chlorophenoxy)-5-methyl-2,3-dihydro-1H-pyrrolo[3,2-b]pyridin-1-yl)(3-(morpholinomethyl)oxiran-2-yl)methanone;
(6-(2,3-difluorophenoxy)-5-methyl-2,3-dihydro-1H-pyrrolo[3,2-b]pyridin-1-yl)((2R,3S)-3-((3-fluoroazetidin-1-yl)methyl)oxiran-2-yl)methanone;
trans-(6-(3-chloro-4-fluorophenoxy)-5-methyl-2,3-dihydro-1H-pyrrolo[3,2-b]pyridin-1-yl)(3-(morpholinomethyl)oxiran-2-yl)methanone;
(6-(2,3-difluorophenoxy)-5-methyl-2,3-dihydro-1H-pyrrolo[3,2-b]pyridin-1-yl)((2R,3S)-3-((3-hydroxyazetidin-1-yl)methyl)oxiran-2-yl)methanone;
trans-(3-(azetidin-1-ylmethyl)oxiran-2-yl)(6-(2,3-difluorophenoxy)-5-methyl-2,3-dihydro-1H-pyrrolo[3,2-b]pyridin-1-yl)methanone;
trans-(3-(azetidin-1-ylmethyl)oxiran-2-yl)(6-(3,4-difluorophenoxy)-5-methyl-2,3-dihydro-1H-pyrrolo[3,2-b]pyridin-1-yl)methanone;
trans-(6-(3,4-difluorophenoxy)-5-methyl-2,3-dihydro-1H-pyrrolo[3,2-b]pyridin-1-yl)(3-(methoxymethyl)oxiran-2-yl)methanone;
trans-(6-(3-fluorophenoxy)-5-methyl-2,3-dihydro-1H-pyrrolo[3,2-b]pyridin-1-yl)(3-(isopropoxymethyl)oxiran-2-yl)methanone;
((2R,3S)-3-(azetidin-1-ylmethyl)oxiran-2-yl)(6-(2,3-difluorophenoxy)-5-methyl-2,3-dihydro-1H-pyrrolo[3,2-b]pyridin-1-yl)methanone;
((2R,3S)-3-((2-oxa-6-azaspiro[3.3]heptan-6-yl)methyl)oxiran-2-yl)(6-(2,3-difluorophenoxy)-5-methyl-2,3-dihydro-1H-pyrrolo[3,2-b]pyridin-1-yl)methanone;
trans-(6-(3-chlorophenoxy)-5-methyl-2,3-dihydro-1H-pyrrolo[3,2-b]pyridin-1-yl)(3-((dimethylamino)methyl)oxiran-2-yl)methanone;
(6-(2,3-difluorophenoxy)-5-methyl-2,3-dihydro-1H-pyrrolo[3,2-b]pyridin-1-yl)((2R,3S)-3-((2,2-dimethylazetidin-1-yl)methyl)oxiran-2-yl)methanone;
(6-(2,3-difluorophenoxy)-5-methyl-2,3-dihydro-1H-pyrrolo[3,2-b]pyridin-1-yl)((2R,3S)-3-((dimethylamino)methyl)oxiran-2-yl)methanone;
trans-(6-(3-chloro-4-fluorophenoxy)-3,3,5-trimethyl-2,3-dihydro-1H-pyrrolo[3,2-b]pyridin-1-yl)(3-((dimethylamino)methyl)oxiran-2-yl)methanone;
((2R,3S)-3-((dimethylamino)methyl)oxiran-2-yl)(6-(3-fluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-((dimethylamino)methyl)oxiran-2-yl)methanone;
(6-(3,4-difluorophenoxy)-5-methyl-2,3-dihydro-1H-pyrrolo[3,2-b]pyridin-1-yl)((2R,3S)-3-((dimethylamino)methyl)oxiran-2-yl)methanone;
((2R,3S)-3-(azetidin-1-ylmethyl)oxiran-2-yl)(6-(3,4-difluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-(methoxymethyl)oxiran-2-yl)methanone;
(6-(3-chloro-4-fluorophenoxy)-3,3,5-trimethyl-2,3-dihydro-1H-pyrrolo[3,2-b]pyridin-1-yl)((2R,3S)-3-((dimethylamino)methyl)oxiran-2-yl)methanone;
(6-(3-chlorophenoxy)-5-methyl-2,3-dihydro-1H-pyrrolo[3,2-b]pyridin-1-yl)((2R,3S)-3-(morpholinomethyl)oxiran-2-yl)methanone;
(6-(3-chloro-4-fluorophenoxy)-5-methyl-2,3-dihydro-1H-pyrrolo[3,2-b]pyridin-1-yl)((2R,3S)-3-(morpholinomethyl)oxiran-2-yl)methanone;
trans-(3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone;
((2R,3S)-3-(azetidin-1-ylmethyl)oxiran-2-yl)(6-(3-chloro-4-fluorophenoxy)-5-methyl-2,3-dihydro-1H-pyrrolo[3,2-b]pyridin-1-yl)methanone;
trans-(6-(3-chloro-4-fluorophenoxy)-5-methyl-2,3-dihydro-1H-pyrrolo[3,2-b]pyridin-1-yl)(3-(fluoromethyl)oxiran-2-yl)methanone;
(6-(3-chloro-4-fluorophenoxy)-5-methyl-2,3-dihydro-1H-pyrrolo[3,2-b]pyridin-1-yl)((2S,3R)-3-((dimethylamino)methyl)oxiran-2-yl)methanone;
(6-(3-chloro-4-fluorophenoxy)-5-methyl-2,3-dihydro-1H-pyrrolo[3,2-b]pyridin-1-yl)((2R,3R)-3-(fluoromethyl)oxiran-2-yl)methanone;
trans-1-((3-(6-(2,3-difluorophenoxy)-5-methyl-2,3-dihydro-1H-pyrrolo[3,2-b]pyridine-1-carbonyl)oxiran-2-yl)methyl)-3-methylazetidine-3-carbonitrile;
trans-(6-(2,6-difluorophenoxy)-5-methyl-2,3-dihydro-1H-pyrrolo[3,2-b]pyridin-1-yl)(3-(morpholinomethyl)oxiran-2-yl)methanone;
trans-(6-(2,3-difluorophenoxy)-5-methyl-2,3-dihydro-1H-pyrrolo[3,2-b]pyridin-1-yl)(3-((3,3-dimethylmorpholino)methyl)oxiran-2-yl)methanone;
trans-(3-((7-oxa-4-azaspiro[2.5]octan-4-yl)methyl)oxiran-2-yl)(6-(2,3-difluorophenoxy)-5-methyl-2,3-dihydro-1H-pyrrolo[3,2-b]pyridin-1-yl)methanone;
3-((5-methyl-1-((2R,3S)-3-methyloxirane-2-carbonyl)-2,3-dihydro-1H-pyrrolo[3,2-b]pyridin-6-yl)oxy)benzonitrile;
(6-(benzo[d][1,3]dioxol-5-yloxy)-5-methyl-2,3-dihydro-1H-pyrrolo[3,2-b]pyridin-1-yl)((2R,3S)-3-methyloxiran-2-yl)methanone;
(6-(4-fluoro-3-hydroxyphenoxy)-5-methyl-2,3-dihydro-1H-pyrrolo[3,2-b]pyridin-1-yl)((2R,3S)-3-methyloxiran-2-yl)methanone;
(6-(3-fluoro-4-hydroxyphenoxy)-5-methyl-2,3-dihydro-1H-pyrrolo[3,2-b]pyridin-1-yl)((2R,3S)-3-methyloxiran-2-yl)methanone;
trans-(6-(3-chloro-4-fluorophenoxy)-5-methyl-2,3-dihydro-1H-pyrrolo[3,2-b]pyridin-1-yl)(3-methyloxiran-2-yl)methanone;
(6-(3-chloro-4-fluorophenoxy)-5-methyl-2,3-dihydro-1H-pyrrolo[3,2-b]pyridin-1-yl)((2R,3S)-3-methyloxiran-2-yl)methanone;
(6-(4-fluorophenoxy)-5-methyl-2,3-dihydro-1H-pyrrolo[3,2-b]pyridin-1-yl)((2R,3S)-3-methyloxiran-2-yl)methanone;
(6-(3,4-difluorophenoxy)-5-methyl-2,3-dihydro-1H-pyrrolo[3,2-b]pyridin-1-yl)((2R,3S)-3-methyloxiran-2-yl)methanone;
trans-(6-(3-chloro-4-fluorophenoxy)-5-methyl-2,3-dihydro-1H-pyrrolo[3,2-b]pyridin-1-yl)(3-ethyloxiran-2-yl)methanone;
(6-(3-chloro-4-fluorophenoxy)-5-methyl-2,3-dihydro-1H-pyrrolo[3,2-b]pyridin-1-yl)((2R,3S)-3-ethyloxiran-2-yl)methanone;
trans-3-((1-(3-(difluoromethyl)oxirane-2-carbonyl)-5-methyl-2,3-dihydro-1H-pyrrolo[3,2-b]pyridin-6-yl)oxy)benzonitrile;
trans-(3-(difluoromethyl)oxiran-2-yl)(6-(3-fluorophenoxy)-5-methyl-2,3-dihydro-1H-pyrrolo[3,2-b]pyridin-1-yl)methanone;
((2R,3R)-3-(difluoromethyl)oxiran-2-yl)(6-(3-fluorophenoxy)-5-methyl-2,3-dihydro-1H-pyrrolo[3,2-b]pyridin-1-yl)methanone;
trans-(3-(difluoromethyl)oxiran-2-yl)(6-(3,4-difluorophenoxy)-5-methyl-2,3-dihydro-1H-pyrrolo[3,2-b]pyridin-1-yl)methanone;
trans-(3-(difluoromethyl)oxiran-2-yl)(6-(4-fluorophenoxy)-5-methyl-2,3-dihydro-1H-pyrrolo[3,2-b]pyridin-1-yl)methanone;
((2R,3R)-3-(difluoromethyl)oxiran-2-yl)(6-(3,4-difluorophenoxy)-5-methyl-2,3-dihydro-1H-pyrrolo[3,2-b]pyridin-1-yl)methanone;
trans-3-(6-(2,3-difluorophenoxy)-5-methyl-2,3-dihydro-1H-pyrrolo[3,2-b]pyridine-1-carbonyl)-N,N-dimethyloxirane-2-carboxamide;
trans-3-(6-(3,4-difluorophenoxy)-5-methyl-2,3-dihydro-1H-pyrrolo[3,2-b]pyridine-1-carbonyl)-N,N-dimethyloxirane-2-carboxamide;
(2R,3R)-3-(6-(3,4-difluorophenoxy)-5-methyl-2,3-dihydro-1H-pyrrolo[3,2-b]pyridine-1-carbonyl)-N,N-dimethyloxirane-2-carboxamide;
trans-3-(6-(3-fluorophenoxy)-5-methyl-2,3-dihydro-1H-pyrrolo[3,2-b]pyridine-1-carbonyl)-N,N-dimethyloxirane-2-carboxamide;
(2S,3S)-3-(6-(3-fluorophenoxy)-5-methyl-2,3-dihydro-1H-pyrrolo[3,2-b]pyridine-1-carbonyl)-N,N-dimethyloxirane-2-carboxamide;
trans-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide;
(2R,3R)-3-(6-(3-chloro-4-fluorophenoxy)-5-methyl-2,3-dihydro-1H-pyrrolo[3,2-b]pyridine-1-carbonyl)-N,N-dimethyloxirane-2-carboxamide;
trans-(6-((3,4-difluorophenyl)amino)-5-methyl-2,3-dihydro-1H-pyrrolo[3,2-b]pyridin-1-yl)(3-(oxetan-3-ylmethyl)oxiran-2-yl)methanone;
trans-(6-((3-chloro-4-fluorophenyl)(methyl)amino)-5-methyl-2, 3-dihydro-1H-pyrrolo[3,2-b]pyridin-1-yl)(3-methyloxiran-2-yl)methanone;
trans-(6-((3-chloro-4-fluorophenyl)amino)-5-methyl-2,3-dihydro-1H-pyrrolo[3,2-b]pyridin-1-yl)(3-methyloxiran-2-yl)methanone;
(6-((3-chloro-4-fluorophenyl)amino)-5-methyl-2,3-dihydro-1H-pyrrolo[3,2-b]pyridin-1-yl)((2R,3S)-3-methyloxiran-2-yl)methanone;
(6-(3-chloro-4-fluorophenoxy)-5-methoxy-2,3-dihydro-1H-pyrrolo[3,2-b]pyridin-1-yl)((2R,3S)-3-methyloxiran-2-yl)methanone;
(6-(3-chloro-4-fluorophenoxy)-5-fluoro-2,3-dihydro-1H-pyrrolo[3,2-b]pyridin-1-yl)((2R,3S)-3-methyloxiran-2-yl)methanone;
(5-chloro-6-(3-chloro-4-fluorophenoxy)-2,3-dihydro-1H-pyrrolo[3,2-b]pyridin-1-yl)((2R,3S)-3-methyloxiran-2-yl)methanone;
trans-(6-(3-chloro-4-fluorobenzyl)-3,3,5-trimethyl-2,3-dihydro-1H-pyrrolo[3,2-b]pyridin-1-yl)( 3-methyloxiran-2-yl)methanone;
(6-(3-chloro-4-fluorobenzyl)-3,3,5-trimethyl-2,3-dihydro-1H-pyrrolo[3,2-b]pyridin-1-yl)((2R,3S)-3-methyloxiran-2-yl)methanone;
trans-(6-(3-chloro-4-fluorobenzyl)-3,3-dimethyl-2,3-dihydro-1H-pyrrolo[3,2-b]pyridin-1-yl)( 3-methyloxiran-2-yl)methanone;
(6-(3-chloro-4-fluorobenzyl)-3,3-dimethyl-2,3-dihydro-1H-pyrrolo[3,2-b]pyridin-1-yl)(oxiran-2-yl)methanone;
trans-(6'-(3-chloro-4-fluorobenzyl)-5'-methylspiro[cyclopropane-1,3'-pyrrolo[3,2-b]pyridin]-1'(2'H)-yl)(3-methyloxiran-2-yl)methanone;
trans-(6-(3-fluorophenoxy)-2,2,5-trimethyl-2,3-dihydro-1H-pyrrolo[3,2-b]pyridin-1-yl)(3-methyloxiran-2-yl)methanone;
trans-(6-(3-chloro-4-fluorophenoxy)-5-methyl-1H-pyrrolo[3,2-b]pyridin-1-yl)(3-methyloxiran-2-yl)methanone;
(6-(3-chloro-4-fluorophenoxy)-5-methyl-1H-pyrrolo[3,2-b]pyridin-1-yl)((2R,3S)-3-methyloxiran-2-yl)methanone;
(6-(3-chloro-4-fluorophenoxy)-3,3,5-trimethyl-2,3-dihydro-1H-pyrrolo[3,2-b]pyridin-1-yl)((2R,3S)-3-methyloxiran-2-yl)methanone; or
(6'-(4-fluorophenoxy)-5'-methylspiro[cyclopropane-1,3'-pyrrolo[3,2-b]pyridin]-1'(2'H)-yl)((2R,3S)-3-methyloxiran-2-yl)methanone.

21. A pharmaceutical composition comprising a compound according to any one of claims 1 to 20, or a pharmaceutically acceptable salt thereof, and one or more pharmaceutically acceptable excipients.

22. A compound according to any one of claims 1 to 20 or a pharmaceutically acceptable salt thereof, or a pharmaceutical composition according to claim 21:
a. for use in therapy;
b. for use in the inhibition of MutSβ activity, e.g. in cellular systems;
c. for use in the treatment of a disease or disorder in which MutSβ activity is implicated;
d. for use in the treatment of a repeat expansion disease or disorder; optionally wherein the repeat expansion disease or disorder is selected from benign adult familial myoclonic epilepsy (BAFME); brachydactyly and cleidocranial dysplasia (BCCD); blepharophimosis, ptosis and epicanthus inversus (BPES); Baratela-Scott syndrome (BSS); cerebellar ataxia, neuropathy and vestibular areflexia syndrome (CANVAS); congenital central hypoventilation syndrome (CCHS); dentatorubropallidoluysian atrophy (DRPLA); early infantile epileptic encephalopathy type 1 (EIEE1); familial adult myoclonic epilepsy (FAME); Fuch's endothelial corneal dystrophy type 3 (FECD3); Dentatorubral pallidoluysian atrophy; fragile XE syndrome (FRAXE); Friedreich ataxia (FRDA) frontotemporal dementia/amyotrophic lateral sclerosis (FTD/ALS); fragile X-associated premature ovarian infertility (FXPOI); fragile X syndrome (FXS); fragile X-associated tremor ataxia syndrome (FXTAS); global development delay, progressive ataxia and elevated glutamine (GDPAG); Huntington' s disease (HD); Huntington disease-like 2 (HDL2); hand-foot-genital syndrome (HFGS); holoprosencephaly type 5 (HPE5); mental retardation with isolated growth hormone deficiency (MRGH); myotonic dystrophy type 1 (DM1); myotonic dystrophy type 2 (DM2); progressive myoclonus epilepsy type 1 or Unverricht-Lundborg diseae (EPM1); neuronal intranuclear inclusion disease (NIID); oculopharyngodistal myopathy type 1 (OPDM1); oculopharyngodistal myopathy type 2 (OPDM2); oculopharyngeal myopathy with leukoencephalopathy type 1 (OPML1); spinobulbar muscular atrophy (SBMA); spinocerebellar ataxias type 1, 2, 3, 6, 7, 8, 10, 12, 17, 27B, 31, 36 or 37 (SCA1, SCA2, SCA3, SCA6, SCA7, SCA8, SCA10, SCA12, SCA17, SCA27B, SCA31, SCA36, SCA37); synpolydactyly (SPD); X-linked dystonia parkinsonism (XDP); X-linked mental retardation and abnormal genitalia (XLAG); or X-linked mental retardation with or without growth hormone deficiency (XLMR, XLMRGHD);
e. for use in the treatment of a triplet repeat disease or disorder, e.g. dentatorubropallidoluysian atrophy (DRPLA); Fuch's endothelial corneal dystrophy type 3 (FECD3); fragile XE syndrome (FRAXE); Friedreich ataxia (FRDA); fragile X-associated premature ovarian infertility (FXPOI); fragile X syndrome (FXS); fragile X-associated tremor ataxia syndrome (FXTAS); Huntington' s disease (HD); Huntington disease-like 2 (HDL2); myotonic dystrophy type 1 (DM1); neuronal intranuclear inclusion disease (NIID); spinobulbar muscular atrophy (SBMA); spinocerebellar ataxias type 1, 2, 3, 6, 7, 8, 17 or 27B (SCA1, SCA2, SCA3, SCA6, SCA7, SCA8, SCA17 or SCA27B);
f. for use in the treatment of Huntington's disease (HD);
g. for use in inhibiting somatic expansion of expanded simple nucleotide repeats in a subject, e.g. in the treatment of a repeat expansion disease or disorder;
h. for use in the inhibition of somatic expansion of expanded simple nucleotide repeats in a subject carrying a disease allele for a repeat expansion disease or disorder;
i. for use in the treatment of Huntington's disease, wherein the compound or pharmaceutical composition is administered in combination with one or more disease modification therapies, e.g. RNA interference approaches (such as tominersen, WVE-03, AMT-130, BV-101, VO-659 or SPK-miHTT), mis-splicing (such as PTC-518) and antibody therapeutics (such as INT-41, NI-302, C6-17 or Vtx-003);
j. for use in the treatment of Huntington's disease, wherein the compound or pharmaceutical composition is administered in combination with one or more Huntingtin lowering therapies (such as RNAi, miRNA (such as AMT-130), antisense oligonucleotides (such as tominersen), and small-molecule splicing modulators (such as PTC-518); and novel methods to clear the mHTT protein, such as proteolysis-targeting chimeras);
k. for use in gene editing *in vitro, ex vivo or in vivo*; optionally wherein the gene editing method is selected from CRISPR-Cas9, CRISPR-Cas12, Fanzor, ZFN (zinc finger nucleases), TALENs, base editors and prime editors;
l. for use as an agent to enhance the efficiency of prime editors;
m. for use as an agent to reduce off-target editing (errors) of prime editors in cellular systems;
n. for use as an agent to both enhance efficiency and reduce error of prime editors in cellular systems;
o. for use as an agent to enhance the efficiency of prime editor therapeutics *ex vivo* (e.g. allogeneic editing of hematopoietic cells); or
p. for use as an agent to enhance the efficiency of prime editor therapeutics *in vivo.*

23. The use of compound according to any one of claims 1 to 20 or a pharmaceutically acceptable salt thereof, or a pharmaceutical composition according to claim 21:
a. in inhibiting somatic expansion of expanded simple nucleotide repeats in a cellular assay system;
b. as an agent to enhance the efficiency of prime editors in a cellular system;
c. as an agent to reduce off-target editing (errors) of prime editors in cellular systems;
d. as an agent to both enhance efficiency and reduce error of prime editors in cellular systems;
e. as an agent to enhance the efficiency of prime editor therapeutics *ex vivo* (e.g. allogeneic editing of hematopoietic cells);
f. as an agent to inhibit mismatch repair *in vitro, ex vivo* or *in vivo*;
g. in testing an agent for the ability to inhibit ATP hydrolysis by MutSβ in the presence or absence of a suitable heteroduplex DNA substrate; or
h. in testing an agent for the ability to inhibit ATP-dependent recruitment of MutLα or MutLβ or MutLγ by MutSβ.

24. A method of increasing the efficiency of a gene editing technique or reducing the error rate of a gene editing technique, the method comprising conducting the gene editing technique in the presence of a compound according to any one of claims 1 to 20, or a pharmaceutically acceptable salt thereof; optionally wherein the gene editing technique is a gene editing technique in which host cell mis-match repair (MMR) factors are known to decrease the efficiency of the gene editing, e.g.:
homology-directed repair (HDR),
non-homologous end joining (NHEJ),
ZFN (zinc finger nucleases),
TALENs (Transcription activator like effector nucleases),
CRISPR-Cas9 nucleases,
CRISPR-Cas12 nucleases,
base editing (fusion of catalytically dead dCas9 enzymes to DNA deaminases which enable single nucleotide substitutions),
prime editing (fusion of a Cas9 nickase to a reverse transcriptase),
PASTE (programmable addition via site-specific targeting elements which leverages prime editing to insert AttB sites and then facilitate serine integrase proteins to insert larger sequences), or
any editing method using a single-stranded oligonucleotide (ssODN) as a template including nuclease-free editing methods.

25. A method of prime editing, the method comprising contacting a cell with: (1) a Cas9 nickase to nick a non-target strand; (2) a prime editing single guide RNA (pegRNA) to bind the target strand; (3) a reverse transcriptase to reverse transcribe from the 3' end of the non-target strand according to the information provided by the pegRNA; and (4) a compound according to any one of claims 1 to 20, or a pharmaceutically acceptable salt thereof.
